# EUROPEAN PATENT SPECIFICATION

(11) **EP 3 166 934 B1**
(45) Date of publication and mention of the grant of the patent: **23.05.2018**
(21) Application number: 15738268.0
(22) Date of filing: 07.07.2015
(51) Int. Cl.: C07D 313/00, C07H 17/08, A61P 31/04, A61K 31/365

(54) **NOVEL MACROLIDE ANTIBIOTICS**
NEUARTIGE MAKROLIDANTIBIOTIKA
NOUVEAUX ANTIBIOTIQUES MACROLIDES

(30) Priority: 07.07.2014 EP 14002319
(43) Date of publication of application: 17.05.2017
(73) Proprietor: Helmholtz-Zentrum für Infektionsforschung GmbH, 38124 Braunschweig (DE); Leibniz-Institut für Naturstoff-Forschung und Infektionsbiologie e.V. -Hans-Knöll-Institut- (HKI), 07745 Jena (DE)
(72) Inventor: SURUP, Frank, 34346 Hannoversch Münden (DE); STEINMETZ, Heinrich, 31139 Hildesheim (DE); MOHR, Kathrin, 38102 Braunschweig (DE); VIEHRIG, Konrad, 66111 Saarbrücken (DE); MÜLLER, Rolf, 66440 Blieskastel (DE); NETT, Markus, 07745 Jena (DE); SCHIEFERDECKER, Sebastian, 07747 Jena (DE); DAHSE, Hans-Martin, 07749 Jena (DE); WOLLING, Michael, 30625 Hannover (DE); KIRSCHNING, Andreas, 29221 Celle (DE)
(74) Representative: Forstmeyer, Dietmar
(86) International application number: PCT/EP2015/001384
(87) International publication number: WO 2016/005049

(56) References cited:
- WO-A2-01/83800
- PAITAN Y ET AL: "THE FIRST IN THE BIOSYNTHESIS OF THE POLYKETIDE ANTIBIOTIC TA OF MYXOCOCCUS XANTHUS CODES FOR A UNIQUE PKS MODULE COUPLED TO A PEPTIDE SYNTHETASE", JOURNAL OF MOLECULAR BIOLOGY, ACADEMIC PRESS, UNITED KINGDOM, vol. 286, no. 1, 1 January 1999 (1999-01-01), pages 465-474, XP000920956, ISSN: 0022-2836, DOI: 10.1006/JMBI.1998.2478
- PFEIFER B A ET AL: "BIOSYNTHESIS OF POLYKETIDES IN HETEROLOGOUS HOSTS", MICROBIOLOGY AND MOLECULAR BIOLOGY REVIEWS, AMERICAN SOCIETY FOR MICROBIOLOGY, US, vol. 65, no. 1, 1 March 2001 (2001-03-01), pages 106-118, XP000997626, ISSN: 1092-2172, DOI: 10.1128/MMBR.65.1.106-118.2001

## Description

### Field of the invention

The present invention relates to a compound according to general formula (I), which exhibits bioactivity; methods for the production of the compound; to pharmaceutical compositions comprising one or more of the compound(s); and to the use of the compound(s) as a medicament, e.g. an antibiotic.

### Background of the invention

Myxobacteria are a prolific source of novel secondary metabolites, e. g. the anticancer drug epothilone, the antibacterial sorangicin and the antifungal soraphen. In recent years, full genome sequencing of various strains led to the identification of novel secondary metabolites with antibacterial, antifungal or anticancer activity.

In view of the rapid decline in the effectiveness of antibiotics due to the emergence of resistance, there is a need for a constant supply of new antibiotics for effective treatment of bacterial infections. For instance, the steadily rising occurrence of methicillin-resistant *Staphylococcus aureus* (MRSA) and vancomycin-resistant *Staphylococcus aureus* (VRSA) strains poses a serious public health threat, since *Staphylococcus aureus* (*S. aureus) inter alia* causes severe infections of the skin, respiratory disease, and food poisoning.

Therefore, the problem underlying the present invention is to provide novel compounds having antibacterial and/or antiproliferative activity, especially compounds having antimicrobial activity against resistant or multiresistant Gram-positive bacteria.

### Summary and description of the invention

The present invention relates to a compound of the general formula (I): 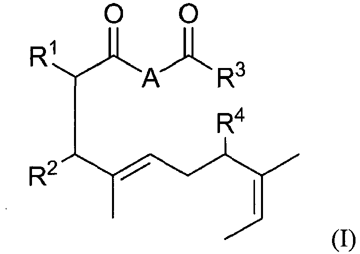 or a pharmacologically acceptable salt thereof, wherein

A represents a group of the formula: 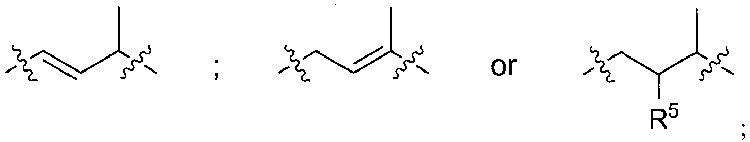
R¹, R², R³ and, if present, R⁵ each independently represents a hydrogen atom; a halogen atom; a hydroxyl group; an amino group; a mercapto group; a C₁-C₁₂ alkyl group; a C₂-C₁₂ alkenyl group; a C₂-C₁₂ alkynyl group; a heteroalkyl group containing 1 to 11 carbon atoms; a C₃-C₁₀ cycloalkyl group, a heterocycloalkyl group containing 3 to 10 ring atoms, a (C₁-C6)alkyl-(C₃-C₇)cycloalkyl group, a (C₁-C6)heteroalkyl-(C₃-C₇)cycloalkyl group, a C₆-C₁₄ aryl group, a heteroaryl group containing 5 to 14 ring atoms, an ar-(C₁-C₆)alkyl group, or a heteroar-(C₁-C₆)alkyl group containing 5 to 10 ring atoms;
R⁴ represents a hydrogen atom; a halogen atom; a hydroxyl group; an amino group; a C₁-C₁₂ alkyl group; or a heteroalkyl group containing 1 to 11 carbon atoms; or
R⁴ and R³ are taken together to form an oxygen or sulphur atom, or a group -NH-.

Compounds are usually described herein using standard nomenclature or the definitions presented below. For compounds having asymmetric centers, it should be understood that (unless otherwise specified) all of the optical isomers and mixtures thereof are encompassed. Compounds with two or more asymmetric elements can also be present as mixtures of diastereomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms. Recited compounds are further intended to encompass compounds in which one or more atoms are replaced with an isotope (i.e., an atom having the same atomic number but a different mass number). By way of general example, and without limitation, isotopes of hydrogen include tritium and deuterium and isotopes of carbon include ¹¹C, ¹³C, and ¹⁴C.

Compounds according to the formulas provided herein, which have one or more stereogenic centers, have an enantiomeric excess of at least 50%. For example, such compounds may have an enantiomeric excess of at least 60%, 70%, 80%, 85%, 90%, 95%, or 98%. Some embodiments of the compounds have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example a chiral HPLC column.

Certain compounds are described herein using a general formula that includes variables, *e.g.* R¹, R², R³, and R⁴. Unless otherwise specified, each variable within such a formula is defined independently of any other variable, and any variable that occurs more than one time in a formula is defined independently at each occurrence. Thus, for example, if a group is shown to be substituted with 0-2 R*, the group may be unsubstituted or substituted with up to two R* groups and R* at each occurrence is selected independently from the definition of R*. Also, combinations of substituents and/or variables are permissible only if such combinations result in stable compounds, i.e., compounds that can be isolated, characterized and tested for biological activity.

As used herein a wording defining the limits of a range of length such as, e. g., "from 1 to 5" means any integer from 1 to 5, i. e. 1, 2, 3, 4 and 5. In other words, any range defined by two integers explicitly mentioned is meant to comprise and disclose any integer defining said limits and any integer comprised in said range. For example, the term "C₁-C₆" refers to 1 to 6, *i.e.* 1, 2, 3, 4, 5 or 6, carbon atoms.

A "pharmacologically acceptable salt" of a compound disclosed herein is an acid or base salt that is generally considered in the art to be suitable for use in contact with the tissues of human beings or animals without excessive toxicity or carcinogenicity, and preferably without irritation, allergic response, or other problem or complication. Such pharmaceutical salts include mineral and organic acid salts of basic residues such as amines, as well as alkali or organic salts of acidic residues such as carboxylic acids.

Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to 4 (*i.e.,* 0, 1, 2, 3, or 4) and the like. Similarly, pharmaceutically acceptable cations include, but are not limited to sodium, potassium, calcium, aluminum, lithium and ammonium. Those of ordinary skill in the art will recognize further pharmacologically acceptable salts for the compounds provided herein. In general, a pharmacologically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of nonaqueous media, such as ether, ethyl acetate, ethanol, isopropanol or acetonitrile, is preferred.

It will be apparent that each compound of formula (I) may, but need not, be present as a hydrate, solvate or non-covalent complex. In addition, the various crystal forms and polymorphs are within the scope of the present invention, as are prodrugs of the compounds of formula (I) provided herein.

A "prodrug" is a compound that may not fully satisfy the structural requirements of the compounds provided herein, but is modified *in vivo,* following administration to a subject or patient, to produce a compound of formula I provided herein. For example, a prodrug may be an acylated derivative of a compound as provided herein. Prodrugs include compounds wherein hydroxy, carboxy, amine or sulfhydryl groups are bonded to any group that, when administered to a mammalian subject, cleaves to form a free hydroxy, carboxy, amino, or sulfhydryl group, respectively. Examples of prodrugs include, but are not limited to, acetate, formate, phosphate and benzoate derivatives of alcohol and amine functional groups within the compounds provided herein. Prodrugs of the compounds provided herein may be prepared by modifying functional groups present in the compounds in such a way that the modifications are cleaved *in vivo* to generate the parent compounds.

A "substituent," as used herein, refers to a molecular moiety that is covalently bonded to an atom within a molecule of interest. For example, a "ring substituent" may be a moiety such as a halogen, alkyl group, haloalkyl group, hydroxy, cyano, amino, nitro, mercapto, or other substituent described herein that is covalently bonded to an atom (preferably a carbon or nitrogen atom) that is a ring member. The term "substituted," as used herein, means that any one or more hydrogens on the designated atom is replaced with a selection from the indicated substituents, provided that the designated atom's normal valence is not exceeded, and that the substitution results in a stable compound, *i.e*. a compound that can be isolated, characterized and tested for biological activity. When a substituent is oxo (*i.e*., =O), then 2 hydrogens on the atom are replaced. An oxo group that is a substituent of an aromatic carbon atom results in a conversion of -CH- to -C(=O)- and a loss of aromaticity. For example a pyridyl group substituted by oxo is a pyridone.

As used herein, "comprising", "including", "containing", "characterized by", and grammatical equivalents thereof are inclusive or open-ended terms that do not exclude additional, unrecited elements or method steps. "Comprising", etc. is to be interpreted as including the more restrictive term "consisting of".

As used herein, "consisting of' excludes any element, step, or ingredient not specified in the claim.

When trade names are used herein, it is intended to independently include the trade name product formulation, the generic drug, and the active pharmaceutical ingredient(s) of the trade name product.

In general, unless defined otherwise, technical and scientific terms used herein have the same meaning as commonly understood by one of ordinary skill in the art to which this invention belongs, and are consistent with general textbooks and dictionaries.

The expression "optionally substituted" refers to groups in which one or more hydrogen atoms, e.g. 1 to 5 hydrogen atoms, and more preferably 1 to 3 hydrogen atoms, have been replaced each independently of the others by fluorine, chlorine, bromine or iodine atoms or by OH, =O, SH, =S, NH₂, =NH, CN or NO₂ groups. The expression "optionally substituted" refers furthermore to groups in which one or more hydrogen atoms, e.g. 1 to 5 hydrogen atoms, and more preferably 1 to 3 hydrogen atoms, have been replaced each independently of the others by unsubstituted C₁-C₆alkyl, (C₁-C₆) haloalkyl (e.g. a fluoromethyl, trifluoromethyl, chloromethyl, (1- or 2-)haloethyl (e.g. (1- or 2-) chloroethyl), or (2- or 3-) halopropyl (e.g. (2- or 3-) fluoropropyl) group), (C₁-C₆) hydroxyalkyl (e.g. a hydroxymethyl, (1- or 2-)hydroxyethyl, or (2- or 3-) hydroxypropyl group), unsubstituted C₂-C₆alkenyl, unsubstituted C₂-C₆alkynyl, unsubstituted C₁-C₆heteroalkyl, unsubstituted C₃-C₇cycloalkyl, unsubstituted C₂-C₇heterocycloalkyl, unsubstituted C₆-C₁₀aryl, unsubstituted C₁-C₈heteroaryl, unsubstituted C₇-C₁₂aralkyl or unsubstituted C₂-C₁₁heteroaralkyl groups.

The expression alkyl or alkyl group denotes a saturated, straight-chain or branched hydrocarbon group that contains from 1 to 20 carbon atoms, preferably from 1 to 12 carbon atoms, more preferably from 1 to 6 carbon atoms, wherein said alkyl group may be optionally substituted. Examples of an unsubstituted alkyl group include a methyl, ethyl, propyl, isopropyl, n-butyl, iso-butyl, *sec-*butyl, *tert*-butyl*,* n-pentyl, n-hexyl, 2,2-dimethylbutyl, and n-octyl group, and examples of an optionally substituted alkyl group include haloalkanes, e.g. a trifluoromethyl group, or a difluoromethyl group; a hydroxymethyl group, 2-hydroxyethyl group, and a methoxymethyl group.

The expression alkenyl or alkenyl group refers to an at least partially unsaturated, straight-chain or branched hydrocarbon group that contains one or more double bond(s) and from 2 to 20 carbon atoms, preferably from 2 to 12 carbon atoms, more preferably from 2 to 6 carbon atoms, wherein said alkenyl group may be optionally substituted. Examples of an unsubstituted alkenyl group include an ethenyl (vinyl), propenyl (allyl), iso-propenyl, butenyl, isoprenyl and hex-2-enyl group. Preferably, an alkenyl group has one or two, especially one, double bond(s).

The expression alkynyl or alkynyl group refers to an at least partially unsaturated, straight-chain or branched hydrocarbon group that contains one or more triple bond(s) and from 2 to 20 carbon atoms, preferably from 2 to 12 carbon atoms, more preferably from 2 to 6, *e.g.* 2, 3 or 4, carbon atoms, wherein said alkynyl group may be optionally substituted. Examples of an unsubstituted alkynyl group include an ethynyl (acetylenyl), propynyl, butynyl or propargyl group. Preferably, an alkynyl group has one or two, especially one, triple bond(s).

As used herein, the expression "heteroalkyl" or heteroalkyl group also includes heteroalkenyl (group) and heteroalkynyl (group), and accordingly refers to an alkyl, alkenyl or alkynyl (straigt chain or branched) group as defined above, in which one or more, preferably 1, 2, 3 or 4, carbon atoms have been replaced each independently of the others by an oxygen, nitrogen, phosphorus, boron, selenium, silicon or sulphur atom, preferably by an oxygen, sulphur or nitrogen atom, or by an SO or SO₂ group, wherein said heteroalkyl group may be optionally substituted. As a result, the expression heteroalkyl encompasses groups containing 1 to 19 carbon atoms, preferably from 1 to 11 carbon atoms, more preferably from 1 to 5, *e.g*. 1, 2, 3 or 4, carbon atoms, and accordingly may also be referred to as C₁-C₁₉, C₁-C₁₁, and C₁-C₅ heteroalkyl, respectively. Further, the expression heteroalkyl also encompasses groups derived from a carboxylic acid, such as, for example, acyl, acylalkyl, alkoxycarbonyl, acyloxy, acyloxyalkyl, carboxyalkylamide or alkoxycarbonyloxy. Examples of heteroalkyl groups are alkylamino, dialkylamino, alkylaminoalkyl, dialkylaminoalkyl, acylalkyl, alkoxycarbonyl, alkylcarbamoyl, alkylamido, alkylcarbamoylalkyl, alkylamidoalkyl, alkylcarbamoyloxyalkyl, alkylureidoalkyl, alkoxycarbonyloxy, alkoxy, or alkoxyalkyl. The expression alkoxy or alkoxy group refers to an alkyl group, in which one or more non-adjecent CH₂ group(s) are replaced by oxygen, wherein the alkyl moiety of the alkoxy group may be optionally substituted. The expression alkylthio or alkylthio group refers to an alkyl group, in which one or more non-adjecent CH₂ group(s) are replaced by sulfur, wherein the alkyl moiety of the alkylthio group may be optionally substituted.

Further examples of heteroalkyl groups are groups of formulae: R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a direct bond, a C₁₋C₆ alkylene, a C₂-C₆ alkenylene or a C₂-C₆ alkynylene group. Specific examples of heteroalkyl groups include acyl, methoxy, trifluoromethoxy, ethoxy, n-propyloxy, isopropyloxy, *tert*-butyloxy, methoxymethyl, ethoxymethyl, methoxyethyl, methylamino, ethylamino, dimethylamino, diethylamino, isopropylethylamino, methylaminomethyl, ethylaminomethyl, diisopropylaminoethyl, dimethylaminomethyl, dimethylaminoethyl, acetyl, propionyl, butyryloxy, acetyloxy, methoxycarbonyl, ethoxycarbonyl, isobutyrylamino-methyl, N-ethyl-N-methylcarbamoyl, N-methylcarbamoyl, cyano, nitrile, isonitrile, thiocyanate, isocyanate, isothiocyanate and alkylnitrile.

The expression cycloalkyl or cycloalkyl group refers to a saturated or partially unsaturated cyclic group that contains one or more rings (preferably 1 or 2), containing from 3 to 14 ring carbon atoms, preferably from 3 to 10 (more preferably 3, 4, 5, 6 or 7) ring carbon atoms, wherein the cycloalkyl group may be optionally substituted. In an embodiment a partially unsaturated cyclic group has one, two or more double bonds, such as a cycloalkenyl group. Specific examples of an unsubstituted cycloalkyl group are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, cyclopentylcyclohexyl, and a cyclohex-2-enyl group.

The expression heterocycloalkyl or heterocycloalkyl group refers to a cycloalkyl group as defined above in which one or more, preferably 1, 2 or 3, ring carbon atoms have been replaced each independently of the others by an oxygen, nitrogen, or sulphur atom (preferably oxygen or nitrogen), or by a SO or SO2 group. A heterocycloalkyl group has preferably 1 or 2 ring(s) containing from 3 to 10 (more preferably 3, 4, 5, 6 or 7, and most preferably 5, 6 or 7) ring atoms. Examples are an aziridinyl, oxiranyl, thiiranyl, oxaziridinyl, dioxiranyl, azetidinyl, oxetanyl, thietanyl, diazetidinyl, dioxetanyl, dithietanyl, pyrrolidinyl, tetrahydrofuranyl, thiolanyl, phospholanyl, silolanyl, azolyl, thiazolyl, isothiazolyl, imidazolidinyl, pyrazolidinyl, oxazolidinyl, isoxazolidinyl, thiazolidinyl, isothiazolidinyl, dioxolanyl, dithiolanyl, piperazinyl, morpholinyl, thiomorpholinyl, trioxanyl, azepanyl, oxepanyl, thiepanyl, homopiperazinyl, or urotropinyl group. Further examples are a 2-pyrazolinyl group, and also a lactam, a lactone and a cyclic imide. The heterocycloalkyl group can be optionally substituted, and may be saturated or mono-, di- or tri-unsaturated. As a result, the expression heterocycloalkyl group also encompasses a group derived from a carbohydrate or saccharide, such as furanoses or pentoses, e.g. arabinose, ribose, xylose, lyxose or desoxyribose, or pyranoses/ hexoses or derivatives thereof, e.g. allose, altrose, glucose, mannose, gulose, idose, galactose, talose, 6-carboxy-D-glucose, 6-carboxy-D-galactose, *N*-acetylchitosamine, glucosamine, *N*-acetylchondrosamin, fucose, rhamnose, or chinovose.

The expression alkylcycloalkyl or alkylcycloalkyl group refers to a group containing both cycloalkyl and also an alkyl, alkenyl or alkynyl group in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two ring systems having from 3 to 10 (preferably 3, 4, 5, 6 or 7) carbon atoms, and one or two alkyl, alkenyl or alkynyl groups having 1 or 2 to 6 carbon atoms, the cyclic groups being optionally substituted.

The expression heteroalkylcycloalkyl or heteroalkylcycloalkyl group refers to alkylcycloalkyl groups as defined above in which one or more (preferably 1, 2 or 3) carbon atoms have been replaced each independently of the others by an oxygen, nitrogen, silicon, selenium, phosphorus or sulphur atom (preferably oxygen, sulphur or nitrogen). A heteroalkylcycloalkyl group preferably contains 1 or 2 ring systems having from 3 to 10 (preferably 3, 4, 5, 6 or 7) ring atoms, and one or two alkyl, alkenyl, alkynyl or heteroalkyl groups having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being optionally substituted and saturated or mono-, di- or tri-unsaturated.

The expressions aryl, Ar or aryl group refer to an aromatic group that contains one or more rings containing from 6 to 14 ring carbon atoms (C₆-C₁₄), preferably from 6 to 10 (C₆-C₁₀), more preferably 6 ring carbon atoms, wherein the aryl group may be optionally substituted. Examples of an unsubstituted aryl group include a phenyl, naphthyl, biphenyl, or indanyl group.

The expression heteroaryl refers to an aromatic group that contains one or more rings containing from 5 to 14 ring atoms, preferably from 5 to 10 (more preferably 5 or 6) ring atoms, and contains one or more (preferably 1, 2, 3 or 4) oxygen, nitrogen, phosphorus or sulphur ring atoms (preferably O, S or N), wherein the heteroaryl group may be optionally substituted. Examples of an unsubstituted. Examples of an unsubstituted heteroaryl group include 2-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl.

The expression aralkyl or aralkyl group refers to a group containing both aryl and also alkyl, alkenyl, alkynyl and/or cycloalkyl groups in accordance with the above definitions, wherein the aralkyl group may be optionally substituted. As a result, the expression aralkyl group encompasses groups such as, for example, arylalkyl, arylalkenyl, arylalkynyl, arylcycloalkyl, arylcycloalkenyl, alkylarylcycloalkyl and alkylarylcycloalkenyl groups, wherein all of said groups may be optionally substituted. Specific examples of an unsubstituted aralkyl group include toluene, xylene, mesitylene, styrene, benzyl chloride, o-fluorotoluene, 1H-indene, tetralin, dihydronaphthalene, indanone, phenylcyclopentyl, cumene, cyclohexylphenyl, fluorene and indan. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl or heteroaralkyl group refers to an aralkyl group as defined above in which one or more (preferably 1, 2, 3 or 4) carbon atoms have been replaced each independently of the others by an oxygen, nitrogen, silicon, selenium, phosphorus, boron or sulphur atom (preferably oxygen, sulphur or nitrogen), that is to say to groups containing both aryl or heteroaryl and also alkyl, alkenyl, alkynyl and/or heteroalkyl and/or cycloalkyl and/or heterocycloalkyl groups in accordance with the above definitions, wherein the heteroaralkyl group may be optionally substituted. A heteroaralkyl group preferably contains one or two aromatic ring systems (1 or 2 rings) containing from 5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced each independently of the others by oxygen, sulphur or nitrogen atoms. Examples of heteroaralkyl groups are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl, heteroalkylheteroarylalkyl and heteroarylheteroalkylheterocycloalkyl groups; all of which groups may be optionally substituted and the cyclic moieties of said groups being saturated or mono-, di- or tri-unsaturated. Specific examples of a heteroaralkyl group include a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

The expression "halogen" or "halogen atom" as preferably used herein means fluorine, chlorine, bromine, or iodine.

The activity and more specifically the bioactivity of the compounds according to the present invention can be assessed using appropriate assays known to those skilled in the art, e.g. *in vitro* or *in vivo* assays. For instance, the antimicrobial activity against Gram-positive bacteria and the cytotoxic activity against cell lines may be determined via a growth inhibition assay, such as the assays provided in Examples 2 and 3 below, which are thus embodiments of standard *in vitro* assays.

Preferred is a compound of formula (I), or a pharmacologically acceptable salt thereof, wherein
R¹, R² and, if present, R⁵ each independently represents a hydrogen atom; a halogen atom; a hydroxyl group; an amino group; a mercapto group; a C₁-C₁₂ alkyl group; a C₂-C₁₂ alkenyl group; a C₂-C₁₂ alkynyl group; a heteroalkyl group containing 1 to 11 carbon atoms; a C₃-C₁₀ cycloalkyl group, a heterocycloalkyl group containing 3 to 10 ring atoms, a (C₁-C6)alkyl-(C₃-C₇)cycloalkyl group, a (C₁-C6)heteroalkyl-(C₃-C₇)cycloalkyl group, a C₆-C₁₄ aryl group, a heteroaryl group containing 5 to 14 ring atoms, an ar-(C₁-C₆)alkyl group, or a heteroar-(C₁-C₆)alkyl group containing 5 to 10 ring atoms; and
R³ and R⁴ are taken together to form an oxygen atom.

Further preferred is a compound of formula (I), or a pharmacologically acceptable salt thereof, wherein
R¹ and R² each independently represents a hydrogen atom; a halogen atom; a hydroxyl group; a heteroalkyl group containing 1 to 11 carbon atoms; or a heterocycloalkyl group containing 3 to 10 ring atoms. R³, R⁴ and, if present R⁵, are as defined above.

In the compound of formula (I), R¹ can represent a hydrogen atom; a hydroxyl group or a heteroalkyl group containing 1 to 11 carbon atoms; and R² can represent a hydroxyl group; or a heterocycloalkyl group containing 3 to 10 ring atoms.

In the compound of formula (I), R³ and R⁴ are preferably taken together to form an oxygen atom. Preferably, R¹ can represent a heteroalkyl group containing 1 to 11 carbon atoms. R² can preferably represent a heterocycloalkyl group containing 3 to 10 ring atoms.

In the compound of formula (I), R⁵, if present, can preferably represent a hydrogen atom; a halogen atom; a hydroxyl group; an amino group; a C₁-C₁₂ alkyl group; a C₂-C₁₂ alkenyl group; a C₂-C₁₂ alkynyl group; or a heteroalkyl group containing 1 to 11 carbon atoms.

The compound of formula (I) may be selected from the group consisting of:

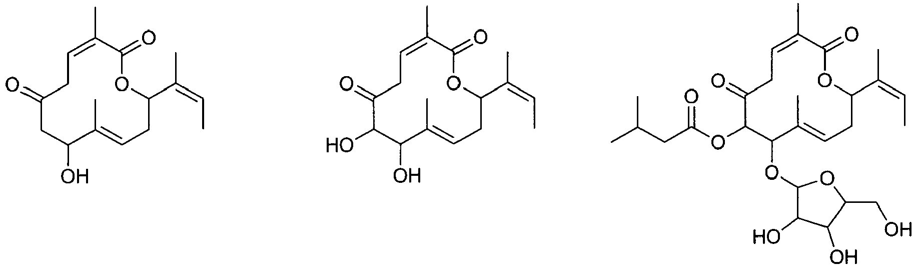

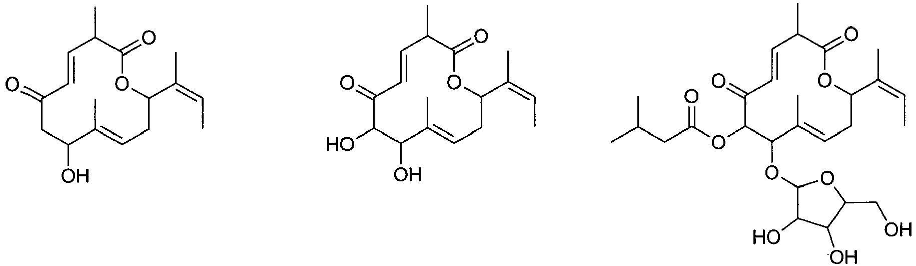

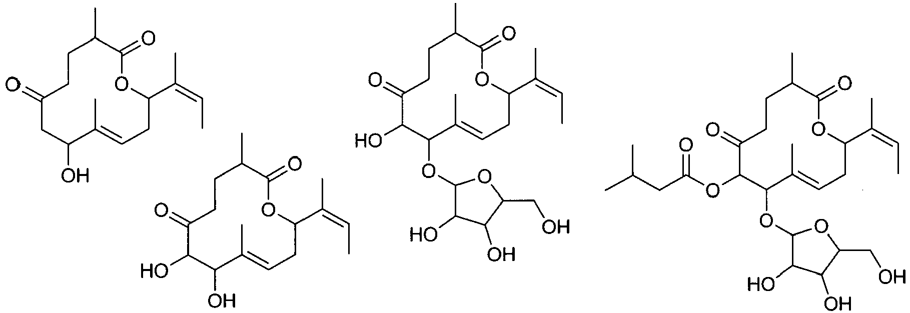

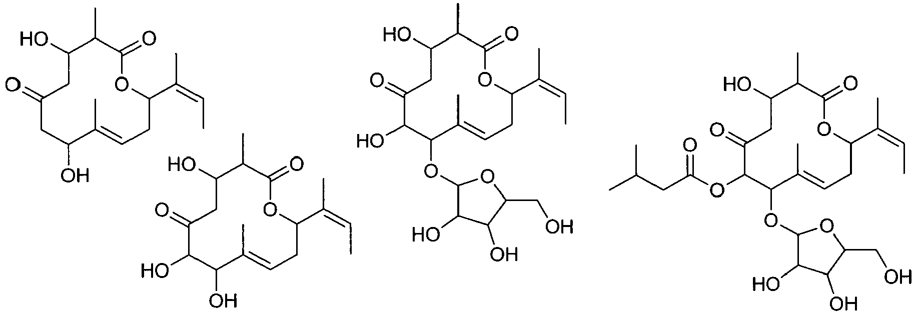

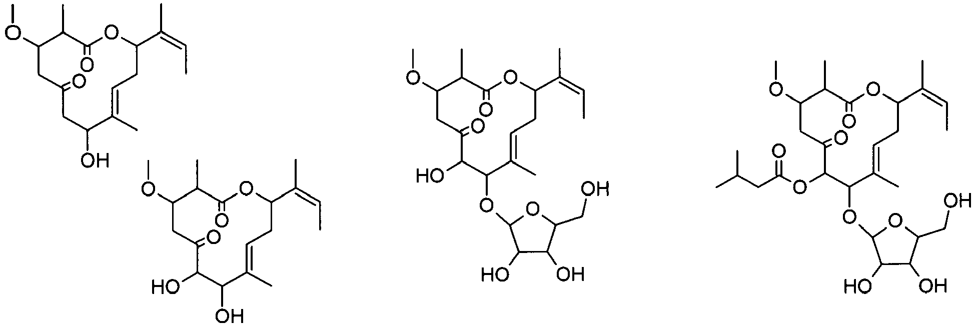

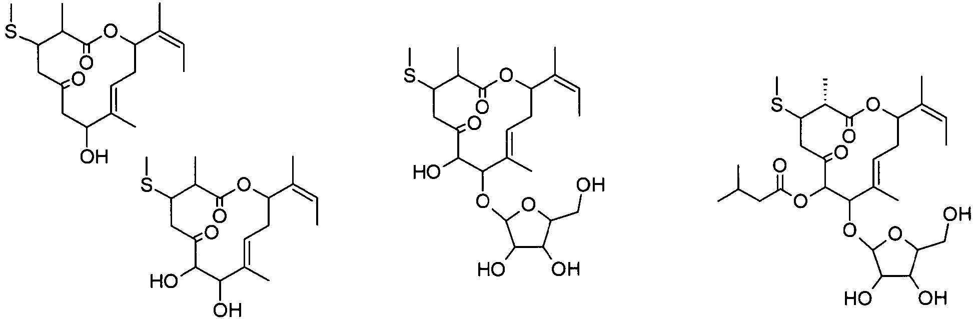

In a preferred embodiment, the compound of formula (I) is compound (**1**) - disciformycin A; compound (**2**) - disciformycin B; compound (**3**) - gulmirecin A; compound (**4**) - gulmirecin B; compound (**5**) - disciformycin C, or compound (**6**) - disciformycin D having the structure as depicted below:

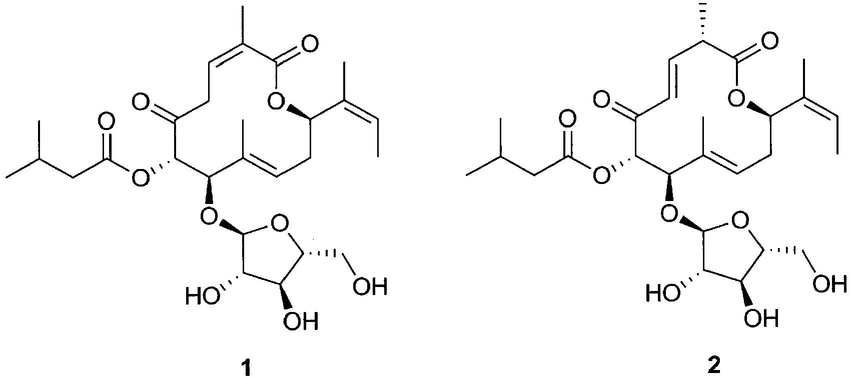

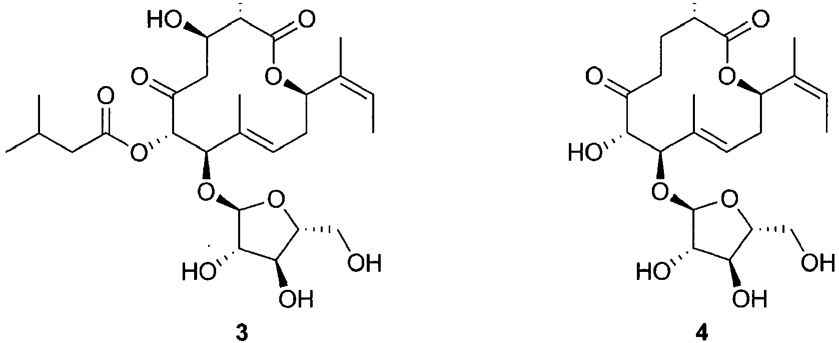

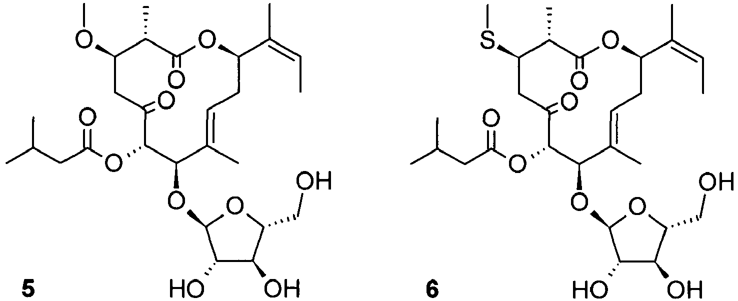

The compound of formula (I), or a pharmacologically acceptable salt, solvate or hydrate thereof, and also a formulation and/or pharmaceutical composition containing the same for therapeutic use are within the scope of the present invention. The present invention also relates to the use of a compound of formula (I) as active ingredient in the preparation or manufacture of a medicament.

A pharmaceutical composition according to the present invention comprises at least one compound of formula (I) and, optionally, one or more carrier substance(s), excipient(s) and/or adjuvant(s). Pharmaceutical compositions may additionally comprise, for example, one or more of water, buffers (*e.g*., neutral buffered saline or phosphate buffered saline), ethanol, mineral oil, vegetable oil, dimethylsulfoxide, carbohydrates (*e.g*., glucose, mannose, sucrose or dextrans), mannitol, proteins, adjuvants, polypeptides or amino acids such as glycine, antioxidants, chelating agents such as EDTA or glutathione and/or preservatives. Furthermore, one or more other active ingredients may (but need not) be included in the pharmaceutical compositions provided herein. For instance, the compounds of the invention may advantageously be employed in combination with another antibiotic or antifungal agent, an anti-viral agent, an anti histamine, a non-steroidal anti-inflammatory drug, a disease modifying anti-rheumatic drug, another cytostatic drug, a drug with smooth muscle activity modulatory activity or mixtures of the aforementioned.

A pharmaceutical composition may be formulated for any appropriate route of administration, including, for example, parenteral administration. The term parenteral as used herein includes subcutaneous, intradermal, intravascular such as, *e.g*., intravenous, intramuscular, spinal, intracranial, intrathecal, intraocular, periocular, intraorbital, intrasynovial and intraperitoneal injection, as well as any similar injection or infusion technique.

Carrier substances are, for example, cyclodextrins such as hydroxypropyl β-cyclodextrin, micelles or liposomes, excipients and/or adjuvants. Customary excipients include, for example, inert diluents such as, *e.g*., calcium carbonate, sodium carbonate, lactose, calcium phosphate or sodium phosphate, granulating and disintegrating agents such as, *e.g*., corn starch or alginic acid, binding agents such as, *e.g.,* starch, gelatin or acacia, and lubricating agents such as, *e.g.,* magnesium stearate or stearic acid. Examples of adjuvants are aluminum hydroxide, aluminum phosphate, calcium phosphate hydroxide, paraffin oil, squalene, thimerosal, detergents, Freund's complete adjuvant, or Freund's incomplete adjuvant.

For the prevention and/or treatment of bacterial infections, especially infections with Gram-positive bacteria, or other conditions, e.g. a prolifertive disease, the dose of the biologically active compound according to the invention may vary within wide limits and may be adjusted to individual requirements. Active compounds according to the present invention are generally administered in a therapeutically effective amount. The expression "therapeutically effective amount" denotes a quantity of the compound(s) that produces a result that in and of itself helps to ameliorate, heal, or cure the respective condition or disease. Preferred doses range from about 0.1 mg to about 140 mg per kilogram of body weight per day (about 0.5 mg to about 7 g per patient per day). The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, and rate of excretion, drug combination (*i.e*. other drugs being used to treat the patient) and the severity of the particular disease undergoing therapy.

The invention further relates to a combination preparation containing at least one compound according to the invention and at least one further active pharmaceutical ingredient. The combination preparation of the invention can be used as a medicament, in particular in the treatment or prophylaxis of bacterial infections with Gram-positive bacteria, such as an infection with *Bacillus subtilis, Mycobacterium aurum, Mycobacterium smegmatis, Mycobacterium vaccae, Paenibacillus polymyxa,* or staphylococci, e.g. *Staphylococcus aureus, Staphylococcus aureus* Newman, *Staphylococcus aureus* (MRSA), *Staphylococcus aureus* N315 (MRSA), *Staphylococcus aureus* Mu50 (MRSA/VRSA), *Staphylococcus auricularis,* and *Staphylococcus carnosus.*

Preferably, in the combination preparation of the invention the further active pharmaceutical ingredient is another antibiotic. The other antibiotic can be selected from the group consisting of β-lactam antibiotics, including penams, carbapenams, oxapenams, penems, carbapenems, monobactams, cephems, carbacephems, oxacephems, and monobactams; aminoglycoside antibiotics, including amikacin, arbekacin, astromicin, bekanamycin, dibekacin, framycetin, gentamicin, hygromycin B, isepamicin, kanamycin, neomycin, netilmicin, paromomycin, paromomycin sulfate, ribostamycin, sisomicin, spectinomycin, streptomycin, tobramycin, and verdamicin; quinolone antibiotics, including ciprofloxacin, enoxacin, gatifloxacin, grepafloxacin, levofloxacin, lomefloxacin, moxifloxacin, nalidixic acid, norfloxacin, ofloxacin, sparfloxacin, temafloxacin, and trovafloxacin; or glycopeptide antibiotics, e.g. vancomycin, telavancin, bleomycin, ramoplanin, and decaplanin; linezolid; or daptomycin.

Preferred compounds of the invention will have certain pharmacological properties. Such properties include, but are not limited to, bioavailability (especially with regard to oral administration), metabolic stability and sufficient solubility, such that the dosage forms can provide therapeutically effective levels of the compound *in vivo.*

The compound according to the invention as well as the pharmaceutical composition or combination preparation according to the invention can be used as a medicament, which can be administered to a patient (*e.g*. parenterally to a human or an other mammal), with dosages as described herein, and will be present within at least one body fluid or tissue of the patient. As used herein, the term "treatment" encompasses both disease-modifying treatment and symptomatic treatment, either of which may be prophylactic, *i.e.,* before the onset of symptoms, in order to prevent, delay or reduce the severity of symptoms, or therapeutic, *i.e.,* after the onset of symptoms, in order to reduce the severity and/or duration of symptoms. In particular, the conditions or diseases that can be ameliorated, prevented or treated using a compound of formula (I), a pharmaceutical composition or a combination preparation according to the invention include bacterial infections and antiproliferative diseases, e.g. cancer. Thus, the present invention also provides methods for treating patients suffering from said diseases. A method for the treatment of a subject which is in need of such treatment, comprises the administration of a compound, a pharmaceutical composition, or a combination preparation according to the invention. The term "subject" refers to patients, including, but not limited to primates (especially humans), domesticated companion animals (such as dogs, cats, horses) and livestock (such as cattle, pigs, sheep).

A recombinant polyketide synthase (PKS) capable of synthesizing a compound according to general formula (I), wherein the PKS comprises at least one polypeptide, or a functional variant thereof, according to any one of SEQ ID NOs. 11 to 19 or SEQ ID NOs. 34 to 46 .

The term "functional variant" as used herein denotes a polypeptide having a sequence that is at least 85%, 90%, 95% or 99% identical to a polypeptide sequence described herein. A "functional variant" of a polypeptide may retain amino acids residues recognized as conserved for the polypeptide in nature, and/or may have non-conserved amino acid residues. Amino acids can be, relative to the native polypeptide, substituted (different), inserted, or deleted, but the variant has generally similar (enzymatic) activity or function as compared to a polypeptide described herein. A "functional variant" may be found in nature or be an engineered mutant (recombinant) thereof.

The term "identity" refers to a property of sequences that measures their similarity or relationship. Identity is measured by dividing the number of identical residues by the total number of residues and multiplying the product by 100.

The terms "protein", "polypeptide", "peptide" as used herein define an organic compound made of two or more amino acid residues arranged in a linear chain, wherein the individual amino acids in the organic compound are linked by peptide bonds, i.e. an amide bond formed between adjacent amino acid residues. By convention, the primary structure of a protein is reported starting from the amino-terminal (N) end to the carboxyl-terminal (C) end.

Preferably, the PKS enzyme of the invention is a not naturally occurring PKS. The PKS of the invention may also be a hybrid PKS comprising modules, domains, and/or portions thereof, or functional variants thereof, from two or more PKSs or from one or more nonribosomal peptide-synthetase(s) (NRPSs). The PKS of the invention preferably includes one or more of the open reading frame(s) (ORF(s)) indicated in Table A or Table B below. Preferably, the PKS of the invention includes 1, 2, 3, 4, 5 or 6 ORF(s) selected from the group consisting of *difB, difC, difD, difE, difF,* and *difG*; or 1, 2, 3, 4, 5, or 6 ORF(s) selected from the group consisting of *gulA, gulB, gulC, gulD, gulE,* and *gulF.*

**Table A. Annotated ORFs in the dif gene cluster, closest homologues found by BLAST-search, and SEQ ID NOs. of the nucleotide and corresponding amino acid sequence.**

| ORF | closest homologue | source organism | SEQ ID NO |
|---|---|---|---|
| *difA* | cytochrome 450 | *P. mucilaginosus* 3016 | 2, 11 |
| *difB* | polyketide synthase | *S. aurantiaca* | 3, 12 |
| *difC* | polyketide synthase | *M. xanthus* Dk 1622 | 4, 13 |
| *difD* | polyketide synthase | *S. aurantiaca* | 5, 14 |
| *difE* | polyketide synthase | *N. punctiforme* PCC 73102 | 6, 15 |
| *difF* | polyketide synthase | *S. cellulosum* So ce56 | 7, 16 |
| *difG* | polyketide synthase | *S. aurantiaca* | 8, 17 |
| *difH* | hypothetical protein | *S. cellulosum* So ce56 | 9, 18 |
| *difI* | CRP-like protein | *M. fulvus* HW-1 | 10, 19 |

**Table B. Annotated ORFs in the gul gene cluster, closest homologues found by BLAST-search, and SEQ ID NOs. of the nucleotide and corresponding amino acid sequence.**

| ORF | closest homologue | source organism | SEQ ID NO |
|---|---|---|---|
| *gulG* | cytochrome P450 | *P. mucilaginosus* 3016 | 21, 34 |
| *gulA* | polyketide synthase | *S. aurantiaca* Sg a15 | 22, 35 |
| *gulB* | polyketide synthase | *S. aurantiaca* Sg a15 | 23, 36 |
| *gulC* | polyketide synthase | *M. stipitatus* DSM 14675 | 24, 37 |
| *gulD* | polyketide synthase | *S. aurantiaca* Sg a15 | 25, 38 |
| *gulE* | polyketide synthase | *S. aurantiaca* DW4/3-1 | 26, 39 |
| *gulF* | polyketide synthase | *S*. *cellulosum* So ce38 | 27, 40 |
| *gulH* | hypothetical protein | *S. cellulosum* So ce56 | 28, 41 |
| *gulI* | cAMP-binding protein | *Myxococcus* sp. (contaminant ex DSM 436) | 29, 42 |
| *gulJ* | hypothetical protein | *S. cellulosum* So0157-2 | 30, 43 |
| *gulR1* | LysR family transcriptional regulator | *C. fuscus* DSM 2262 | 31, 44 |
| *gulK* | sui1 family protein | *S. aurantiaca* DW4/3-1 | 32, 45 |
| *gulR2* | TetR family transcriptional regulator | *K. racemifer* DSM 44963 | 33, 46 |

The present invention also provides isolated, synthetic or recombinant nucleic acids that encode PKSs of the invention. Said nucleic acids include nucleic acids that include a portion or all of a PKS of the invention, nucleic acids that further include regulatory sequences, such as promoter and translation initiation and termination sequences, and can further include sequences that facilitate stable maintenance in a host cell, i.e., sequences that provide the function of an origin of replication or facilitate integration into host cell chromosomal or other DNA by homologous recombination.

Preferably, the invention relates to an isolated, synthetic or recombinant nucleic acid comprising:
(i) a sequence encoding a PKS of the invention, wherein the sequence has a sequence identity to the full-length sequence of SEQ ID NO: 1 or SEQ ID NO: 20 from at least 85%, 90%, 95%, 96%, 97%, 98%, 98.5%, 99%, or 99.5% to 100%;
(ii) a sequence encoding a portion of a PKS of the invention, wherein the sequence has a sequence identity to the full-length sequence of any of SEQ ID NOs. 2 to 10 or SEQ ID NOs. 21 to 33 from at least 85%, 90%, 95%, 96%, 97%, 98%, 98.5%, 99%, or 99.5% to 100%;
(iii) a sequence completely complementary to any nucleic acid sequence of (i) or (ii); or
(iv) a sequence encoding a polypeptide according to any of SEQ ID NOs. 11 to 19 or SEQ ID NOs. 34 to 46 .

The phrases "nucleic acid" or "nucleic acid sequence" as used herein refer to an oligonucleotide, nucleotide, polynucleotide, or to a fragment of any of these, to DNA of genomic or synthetic origin which may be single-stranded or double-stranded and may represent a sense or antisense strand, natural or synthetic in origin. "Oligonucleotide" includes either a single stranded polydeoxynucleotide or two complementary polydeoxynucleotide strands that may be chemically synthesized. Such synthetic oligonucleotides have no 5' phosphate and thus will not ligate to another oligonucleotide without adding a phosphate with an ATP in the presence of a kinase. A synthetic oligonucleotide can ligate to a fragment that has not been dephosphorylated. A "coding sequence" of or a "nucleotide sequence encoding" a particular polypeptide or protein, is a nucleic acid sequence which is transcribed and translated into a polypeptide or protein when placed under the control of appropriate regulatory sequences. The nucleic acids used to practice this invention may be isolated from a variety of sources, genetically engineered, amplified, and/or expressed/ generated recombinantly. Techniques for the manipulation of nucleic acids, such as, e.g., subcloning, labeling probes (e.g., random-primer labeling using Klenow polymerase, nick translation, amplification), sequencing, hybridization and the like are well described in the scientific and patent literature, see, e.g., Sambrook, et al, Molecular Cloning: A Laboratory Manual, 2nd Ed., Cold Spring Harbor, N. Y., VoIs. 1-3, (1989) ; Current Protocolsin Molecular Biology, Ausubel, ed. John Wiley & Sons, Inc., New York (1997); Laboratory Techniques In Biochemistry And Molecular Biology: Hybridization With Nucleic Acid Probes, Part I. Theory and Nucleic Acid Preparation, Tijssen, ed. Elsevier, N.Y. (1993). A nucleic acid encoding a polypeptide of the invention is assembled in appropriate phase with a leader sequence capable of directing secretion of the translated polypeptide or fragment thereof.

The term "isolated" as used herein means that the material, e.g., a nucleic acid, a polypeptide, a vector, a cell, is removed from its original environment, e.g., the natural environment if it is naturally occurring. For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition and still be isolated in that such vector or composition is not part of its natural environment.

The term "synthetic" as used herein means that the material, e.g. a nucleic acid, has been synthesized in vitro by well-known chemical synthesis techniques, as described in, e.g., Adams (1983) J. Am. Chem. Soc. 105:661; Belousov (1997) Nucleic Acids Res. 25:3440-3444; Frenkel (1995) Free Radic. Biol. Med. 19:373-380; Blommers (1994) Biochemistry 33:7886-7896; Narang (1979) Meth. Enzymol. 68:90; Brown (1979) Meth. Enzymol. 68:109; Beaucage (1981) Tetra. Lett. 22: 1859.

The term "recombinant" means that the nucleic acid is adjacent to a "backbone" nucleic acid to which it is not adjacent in its natural environment. Backbone molecules according to the invention include nucleic acids such as cloning and expression vectors, self-replicating nucleic acids, viruses, integrating nucleic acids and other vectors or nucleic acids used to maintain or manipulate a nucleic acid insert of interest. Recombinant polypeptides of the invention, generated from these nucleic acids can be individually isolated or cloned and tested for a desired activity. Any recombinant expression system can be used, including bacterial, mammalian, yeast, insect or plant cell expression systems.

Also provided is a vector comprising at least one nucleic acid according to the invention. The vector may be a cloning vector, an expression vector or an artificial chromosome.

As used herein, the term "vector" refers to a nucleic acid molecule capable of transporting another nucleic acid to which it has been linked. Vectors, including cloning and expression vectors, comprise a nucleic acid of the invention or a functional equivalent thereof. Nucleic acids of the invention can be incorporated into a recombinant replicable vector, for example a cloning or expression vector. The vector may be used to replicate the nucleic acid in a compatible host cell. Thus, the invention also provides a method of making polynucleotides of the invention by introducing a polynucleotide of the invention into a replicable vector, introducing the vector into a compatible host cell, and growing the host cell under conditions which bring about replication of the vector. The vector may be recovered from the host cell. Suitable host cells are described below. The vector into which the expression cassette or nucleic acid of the invention is inserted may be any vector which may conveniently be subjected to recombinant DNA procedures, and the choice of the vector will often depend on the host cell into which it is to be introduced. A variety of cloning and expression vectors for use with prokaryotic and eukaryotic hosts are described by Sambrook, et al, Molecular Cloning: A Laboratory Manual, 2nd Ed., Cold Spring Harbor, N. Y., (1989).

A vector according to the invention may be an autonomously replicating vector, i.e. a vector which exists as an extra-chromosomal entity, the replication of which is independent of chromosomal replication, e. g. a plasmid. Alternatively, the vector may be one which, when introduced into a host cell, is integrated into the host cell genome and replicated together with the chromosome(s) into which it has been integrated.

One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication, and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. The terms "plasmid" and "vector" can be used interchangeably herein as the plasmid is the most commonly used form of vector. However, the invention is intended to include such other forms of expression vectors, such as cosmid, viral vectors (e.g., replication defective retroviruses, adenoviruses and adeno-associated viruses) and phage vectors which serve equivalent functions.

Vectors according to the invention may be used in vitro, for example for the production of RNA or used to transfect or transform a host cell.

A vector of the invention may comprise two or more, for example three, four or five, nucleic acids of the invention, for example for overexpression.

The recombinant expression vectors of the invention comprise a nucleic acid of the invention in a form suitable for expression of the nucleic acid in a host cell, which means that the recombinant expression vector includes one or more regulatory sequences, selected on the basis of the host cells to be used for expression, which is operationally linked to the nucleic acid sequence to be expressed.

Within a vector, such as an expression vector, "operationally linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell), i.e. the term "operationally linked" refers to a juxtaposition wherein the components described are in a relationship permitting them to function in their intended manner. A regulatory sequence such as a promoter, enhancer or other expression regulation signal "operationally linked" to a coding sequence is positioned in such a way that expression of the coding sequence is achieved under condition compatible with the control sequences or the sequences are arranged so that they function in concert for their intended purpose, for example transcription initiates at a promoter and proceeds through the DNA sequence encoding the polypeptide.

The term "regulatory sequence" or "control sequence" is intended to include promoters, operators, enhancers, attenuators and other expression control elements (e.g., polyadenylation signal). Such regulatory sequences are described, for example, in Goeddel; Gene Expression Technology: Methods in Enzymology 185, Academic Press, San Diego, CA (1990).

The term regulatory or control sequences includes those sequences which direct constitutive expression of a nucleotide sequence in many types of host cells and those which direct expression of the nucleotide sequence only in a certain host cell (e.g. tissue-specific regulatory sequences).

A vector or expression construct for a given host cell may thus comprise the following elements operationally linked to each other in a consecutive order from the 5'-end to 3'-end relative to the coding strand of the sequence encoding the polypeptide of the invention: (i) a promoter sequence capable of directing transcription of the nucleotide sequence encoding the polypeptide in the given host cell; (ii) optionally, a signal sequence capable of directing secretion of the polypeptide from the given host cell into a culture medium; (iii) optionally, a sequence encoding for a C-terminal, N-terminal or internal epitope tag sequence or a combination of the aforementioned allowing purification, detection or labeling of the polypeptide; (iv) a nucleic acid sequence of the invention encoding a polypeptide of the invention; and preferably also (v) a transcription termination region (terminator) capable of terminating transcription downstream of the nucleotide sequence encoding the polypeptide. Particular named bacterial promoters include lad, lacZ, T3, T7, SP6, K1F, tac, tet, gpt, lambda P_{R}, P_{L} and trp. Eukaryotic promoters include CMV immediate early, HSV thymidine kinase, early and late SV40, LTRs from retrovirus and mouse metallothionein-I. Selection of the appropriate vector and promoter is well within the level of ordinary skill in the art. Downstream of the nucleotide sequence according to the invention there may be a 3' untranslated region containing one or more transcription termination sites (e. g. a terminator). The origin of the terminator is less critical. The terminator can, for example, be native to the DNA sequence encoding the polypeptide. Preferably, the terminator is endogenous to the host cell (in which the nucleotide sequence encoding the polypeptide is to be expressed). In the transcribed region, a ribosome binding site for translation may be present. The coding portion of the mature transcripts expressed by the constructs will include a translation initiating AUG (or TUG or GUG in prokaryotes) at the beginning and a termination codon appropriately positioned at the end of the polypeptide to be translated.

Enhanced expression of a polynucleotide of the invention may also be achieved by the selection of heterologous regulatory regions, e.g. promoter, secretion leader and/or terminator regions, which may serve to increase expression and, if desired, secretion levels of the protein of interest from the expression host and/or to provide for the inducible control of the expression of a polypeptide of the invention. It will be appreciated by those skilled in the art that the design of the expression vector can depend on such factors as the choice of the host cell to be transformed, the level of expression of protein desired, etc. The vectors, such as expression vectors, of the invention can be introduced into host cells to thereby produce proteins or peptides, encoded by nucleic acids as described herein.

The vectors, such as recombinant expression vectors, of the invention can be designed for expression of a portion or all of a PKS of the invention in prokaryotic or eukaryotic cells. For example, a portion or all of a PKS of the invention can be expressed in bacterial cells such as E. coli, Bacillus strains, insect cells (using baculovirus expression vectors), filamentous fungi, yeast cells or mammalian cells. Suitable host cells are discussed further in Goeddel, Gene Expression Technology: Methods in Enzymology 185, Academic Press, San Diego, CA (1990). Representative examples of appropriate hosts are described hereafter. Appropriate culture media and conditions for the above-described host cells are known in the art.

As set out above, the term "control sequences" or "regulatory sequences" is defined herein to include at least any component which may be necessary and/or advantageous for the expression of a polypeptide. Any control sequence may be native or foreign to the nucleic acid sequence of the invention encoding a polypeptide. Such control sequences may include, but are not limited to, a promoter, a leader, optimal translation initiation sequences (as described in Kozak, 1991 , J. Biol. Chem. 266:19867- 19870), a secretion signal sequence, a pro-peptide sequence, a polyadenylation sequence, a transcription terminator. At a minimum, the control sequences typically include a promoter, and transcriptional and translational stop signals. A stably transformed microorganism is one that has had one or more DNA fragments introduced such that the introduced molecules are maintained, replicated and segregated in a growing culture. Stable transformation may be due to multiple or single chromosomal integration(s) or by (an) extrachromosomal element(s) such as (a) plasmid vector(s). A plasmid vector is capable of directing the expression of polypeptides encoded by particular DNA fragments. Expression may be constitutive or regulated by inducible (or repressible) promoters that enable high levels of transcription of functionally associated DNA fragments encoding specific polypeptides.

Expression vectors of the invention may also include a selectable marker gene to allow for the selection of bacterial strains that have been transformed, e.g., genes which render the bacteria resistant to drugs such as chloramphenicol, erythromycin, kanamycin, neomycin, tetracycline, as well as ampicillin and other penicillin derivatives like carbenicillin. Selectable markers can also include biosynthetic genes, such as those in the histidine, tryptophan and leucine biosynthetic pathways.

The appropriate polynucleotide sequence may be inserted into the vector by a variety of procedures. In general, the polynucleotide sequence is ligated to the desired position in the vector following digestion of the insert and the vector with appropriate restriction endonucleases. Alternatively, blunt ends in both the insert and the vector may be ligated. A variety of cloning techniques are disclosed in Ausubel et al. Current Protocols in Molecular Biology, John Wiley 503 Sons, Inc. 1997 and Sambrook et al, Molecular Cloning: A Laboratory Manual 2nd Ed., Cold Spring Harbor Laboratory Press (1989). The polynucleotide sequence may also be cloned using homologous recombination techniques including in vitro as well as in vivo recombination. Such procedures and others are deemed to be within the scope of those skilled in the art. The vector may be, for example, in the form of a plasmid, a viral particle, or a phage. Other vectors include chromosomal, nonchromosomal and synthetic polynucleotide sequences, derivatives of SV40; bacterial plasmids, phage DNA, baculovirus, yeast plasmids, vectors derived from combinations of plasmids and bacteriophage DNA, viral DNA such as vaccinia, adenovirus, fowl pox virus and pseudorabies.

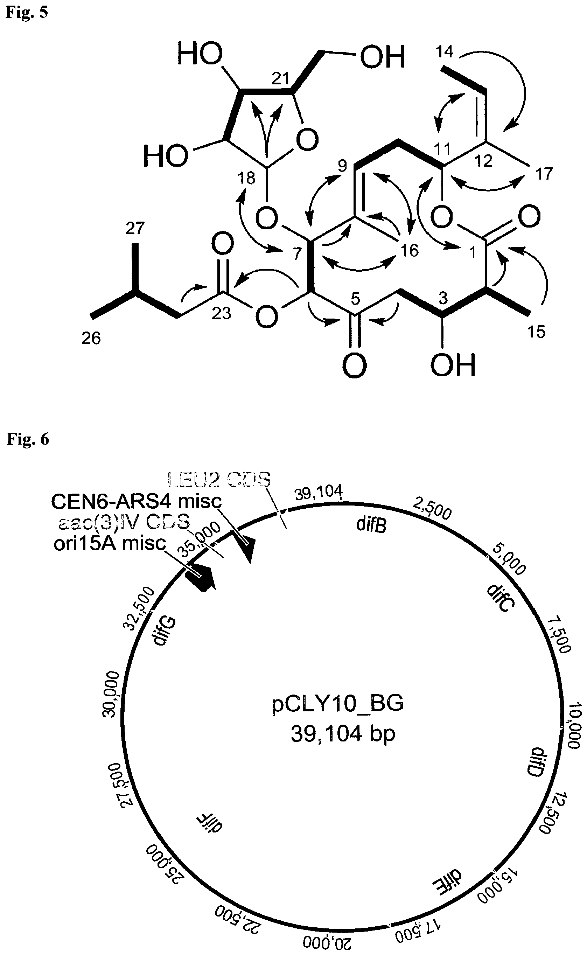

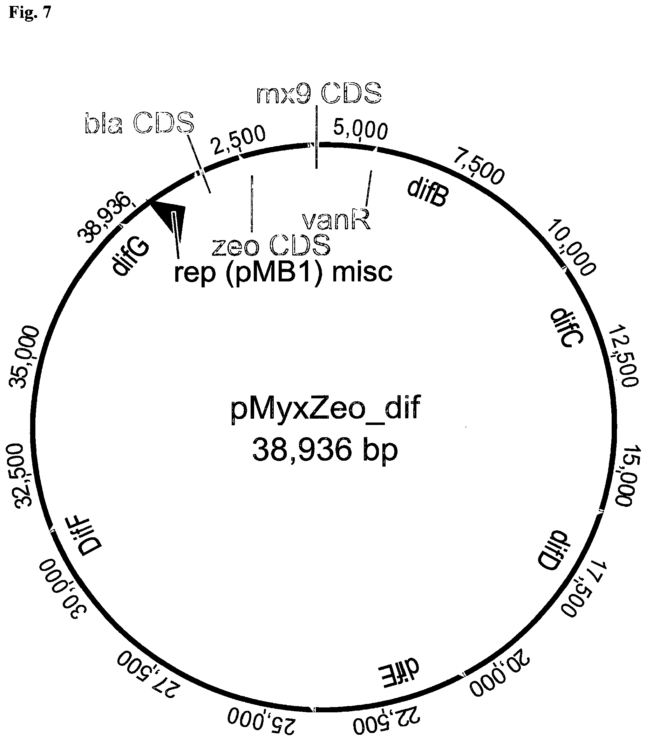

Preferred vectors of the invention include, but are not limited to, the plasmid pCLY10_BG (cf. Fig. 6) and the plasmid pMyxZeo_dif (deposited at DSMZ). The plasmid pCLY10_BG contains the six PKS genes *difB, difC, difD, difE, difF,* and *difG* and selection markers for propargation in *S. cerevisiae* and *E. coli.* The map of the plasmid pCLY10_BG is shown in Fig. 6. The plasmid pMyxZeo_dif contains the six PKS genes *difB, difC, difD, difE, difF,* and *difG,* a zeocin resistance cassette, the mx9 integrase gene, and the vanillate promotor/repressor system. The plasmid pMyxZeo_dif allows integration into the genome and controllable expression in myxobacterium *Myxococcus xanthus* DK1622. The map of the plasmid pMyxZeo_dif is shown in Fig. 7.

The invention also provides host cells, i.e. transformed cells comprising a nucleic acid sequence of the invention, e.g. a sequence encoding one or more polypeptides or all of a NRPS of the invention, or a vector of the invention. The host cell may be any of the host cells familiar to those skilled in the art, including prokaryotic cells, eukaryotic cells, such as bacterial cells, fungal cells, yeast cells, mammalian cells, insect cells, or plant cells.

Preferred mammalian cells include e.g. Chinese hamster ovary (CHO) cells, COS cells, 293 cells, PerC6 cells, hybridomas, Bowes melanoma or any mouse or any human cell line. Exemplary insect cells include any species of *Spodoptera* or *Drosophila,* including *Drosophila* S2 and *Spodoptera* Sf-9. Exemplary fungal cells include any species of *Aspergillus.* Preferred yeast cell include, e. g. a cell from a *Candida, Hansenula, Kluyveromyces, Pichia, Saccharomyces, Schizosaccharomyces,* or *Yarrowia* strain, more preferably from *Kluyveromyces lactis, S. cerevisiae, Hansenula polymorpha, Yarrowia lipolytica,* or *Pichia pastoris.* According to the invention, the host cell may be a prokaryotic cell. Preferably, the prokaryotic host cell is a bacterial cell. The term "bacterial cell" includes both Gram-negative and Gram-positive as well as archaeal microorganisms. Suitable bacteria may be selected from e.g. *Myxobacterium, Escherichia, Anabaena, Caulobacter, Gluconobacter, Rhodobacter, Pseudomonas, Paracoccus, Ralstonia, Bacillus, Brevibacterium, Corynebacterium, Rhizobium (Sinorhizobium), Flavobacterium, Klebsiella, Enterobacter, Lactobacillus, Lactococcus, Methylobacterium, Staphylococcus* or *Streptomyces.* Preferably, the bacterial cell is selected from the group consisting of *Angiococcus disciformis, Pyxidicoccus fallax, B. subtilis, B. amyloliquefaciens, B. licheniformis, B. puntis, B. megaterium, B. halodurans, B. pumilus, G. oxydans, Caulobacter crescentus* CB 15, *Methylobacterium extorquens, Rhodobacter sphaeroides, Pseudomonas putida, Paracoccus zeaxanthinifaciens, Paracoccus denitrificans, Ralstonia eutropha, E. coli, C. glutamicum, Staphylococcus carnosus, Streptomyces lividans, Sinorhizobium melioti* and *Rhizobium radiobacter.* The selection of an appropriate host is within the abilities of those skilled in the art.

The vector can be introduced into the host cells using any of a variety of techniques, including transformation, transfection, transduction, viral infection, gene guns, or Ti-mediated gene transfer. Particular methods include calcium phosphate transfection, DEAE-Dextran mediated transfection, lipofection, or electroporation (Davis, L., Dibner, M., Battey, I., Basic Methods in Molecular Biology, (1986)). The nucleic acids or vectors of the invention may be introduced into the cells for screening, thus, the nucleic acids enter the cells in a manner suitable for subsequent expression of the nucleic acid. The method of introduction is largely dictated by the targeted cell type. Exemplary methods include CaPO₄ precipitation, liposome fusion, lipofection (e.g., LIPOFECTIN™), electroporation, viral infection, etc. The candidate nucleic acids may stably integrate into the genome of the host cell (for example, with retroviral introduction) or may exist either transiently or stably in the cytoplasm (i.e. through the use of traditional plasmids, utilizing standard regulatory sequences, selection markers, etc.). As many pharmaceutically important screens require human or model mammalian cell targets, retroviral vectors capable of transfecting such targets can be used.

Where appropriate, the engineered host cells can be cultured in conventional nutrient media modified as appropriate for activating promoters, selecting transformants or amplifying the nucleic acids of the invention. Following transformation of a suitable host strain and growth of the host strain to an appropriate cell density, the selected promoter may be induced by appropriate means (e.g., temperature shift or chemical induction) and the cells may be cultured for an additional period to allow them to produce the desired polypeptide or fragment thereof. Cells can be harvested by centrifugation, disrupted by physical or chemical means, and the resulting crude extract is retained for further purification. Microbial cells employed for expression of proteins can be disrupted by any convenient method, including freeze-thaw cycling, sonication, mechanical disruption, or use of cell lysing agents. Such methods are well known to those skilled in the art. The expressed polypeptide or fragment thereof can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the polypeptide. If desired, high performance liquid chromatography (HPLC) can be employed for final purification steps. The constructs in host cells can be used in a conventional manner to produce the gene product encoded by the recombinant sequence. Depending upon the host employed in a recombinant production procedure, the polypeptides produced by host cells containing the vector may be glycosylated or may be non-glycosylated. Polypeptides of the invention may or may not also include an initial methionine amino acid residue. Cell-free translation systems can also be employed to produce a polypeptide of the invention. Cell-free translation systems can use mRNAs transcribed from a DNA construct comprising a promoter operationally linked to a nucleic acid encoding the polypeptide or fragment thereof. In some aspects, the DNA construct may be linearized prior to conducting an in vitro transcription reaction. The transcribed mRNA is then incubated with an appropriate cell-free translation extract, such as a rabbit reticulocyte extract, to produce the desired polypeptide or fragment thereof.

Host cells containing the polynucleotides of interest, e.g., nucleic acids of the invention, can be cultured in conventional nutrient media modified as appropriate for activating promoters, selecting transformants or amplifying genes. The culture conditions such as temperature, pH and the like, are those previously used with the host cell selected for expression and will be apparent to the ordinarily skilled artisan. The clones which are identified as having the specified enzyme activity may then be sequenced to identify the polynucleotide sequence encoding a portion or all of a PKS of the invention.

Recombinant DNA can be introduced into the host cell by any means, including, but not limited to, plasmids, cosmids, phages, yeast artificial chromosomes or other vectors that mediate transfer of genetic elements into a host cell. These vectors can include an origin of replication, along with *cis*-acting control elements that control replication of the vector and the genetic elements carried by the vector. Selectable markers can be present on the vector to aid in the identification of host cells into which genetic elements have been introduced. Means for introducing genetic elements into a host cell (e.g. cloning) are well known to the skilled artisan. Other cloning methods include, but are not limited to, direct integration of the genetic material into the chromosome. This can occur by a variety of means, including cloning the genetic elements described herein on non-replicating plasmids flanked by homologous DNA sequences of the host chromosome; upon transforming said recombinant plasmid into a host the genetic elements can be introduced into the chromosome by DNA recombination. Such recombinant strains can be recovered if the integrating DNA fragments contain a selectable marker, such as antibiotic resistance. Alternatively, the genetic elements can be directly introduced into the chromosome of a host cell without use of a non-replicating plasmid. This can be done by synthetically producing DNA fragments of the genetic elements in accordance to the present invention that also contains homologous DNA sequences of the host chromosome. Again if these synthetic DNA fragments also contain a selectable marker, the genetic elements can be inserted into the host chromosome.

A portion or all of a PKS of the invention may be favorably expressed in any of the above host cells. Thus, the present invention provides a wide variety of host cells comprising one or more of the isolated, synthetic or recombinant nucleic acids and/or PKSs of the present invention. The host cell, when cultured under suitable conditions, can be capable of producing a disciformycin according to formula (I) that it otherwise does not produce, or produces at a lower level, in the absence of a nucleic acid of the invention.

A preferred host cell of the invention is a bacterial cell from *Myxococcus xanthus* DK1622. Particularly preferred as a host cell is DK1622 attB::dif20, a result of a transformation of *M. xanthus* DK1622 with plasmid pMyxZeo_dif and subsequent integration of the heterologous plasmid DNA into the genome of *M. xanthus* DK1622.

The invention also relates to an isolated, synthetic or recombinant polypeptide having an amino acid sequence according to any of SEQ ID NOs. 11 to 19, SEQ ID NOs. 34 to 46, or an amino acid sequence encoded by a nucleic acid of the invention.

Also the following methods for producing a compound of formula (I) lie within the scope of the present invention: chemical synthesis, semisynthesis, and biosynthesis including recombinant techniques. Since the structural complexity of the compounds precludes facile total synthetic access, semisynthetic as well as biotechnological approaches are commonly pursued in pharmaceutical research and development. It is understood that the production of compounds of formula (I) is not limited to the use of the particular organism described herein, which is given for illustrative purpose only. The invention also includes the use of any mutants which are capable of producing a compound of formula (I) including natural mutants as well as artificial mutants, e.g. genetically manipulated mutants and the expression of the gene cluster responsible for biosynthesis in a producer strain or by heterologous expression in host strains.

For instance, a compound of formula (I) can be produced by a method comprising the steps: (a) culturing a host cell comprising at least one nucleic acid or a vector according to the invention; and (b) separating and retaining the compound from the culture broth.

The culturing step can be performed in liquid culture, by growing the respective host cell in media containing one or several different carbon sources, and one or different nitrogen sources. Also salts are essential for growth and production. Suitable carbon sources are different mono-, di-, and polysaccharides like maltose, glucose or carbon from amino acids like peptones. Nitrogen sources are ammonium, nitrate, urea, chitin or nitrogen from amino acids. The following inorganic ions support the growth or are essential in synthetic media: Mg-ions, Ca-ions, Fe-ions, Mn-ions, Zn-ions, K-ions, sulfate-ions, Cl-ions, phosphate-ions. The host cell may be a microorganism, e.g. *Angiococcus disciformis* strain AngJ1 (DSM 27408) or *Pyxidicoccus fallax* HKI 727 (DSM 28991). Temperatures for growth and production are between 15°C to 40 °C, preferred temperatures are between 25 °C and 35 °C, especially at 30 °C. The pH of the culture solution is from 5 to 8, preferably a pH of 7.3 to 7.5.

A compound of formula (I) can also be obtained by chemical synthesis in a number of ways well known to one skilled in the art of organic synthesis using usual chemical reaction and synthesis methods, e.g. by the synthesis provided in Example 5 below.

### Brief Description of the Figures

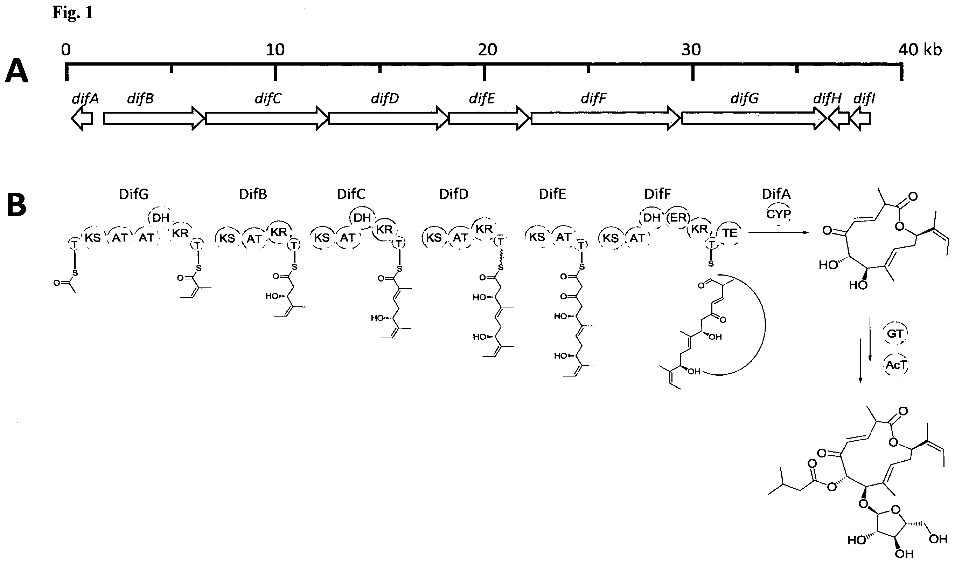

***Figure 1****.* (**A**) Biosynthesis gene cluster. Putative polyketide synthase (PKS) encoding genes are shown in dark grey arrows, whereas genes encoding non-PKS genes are shown as light grey arrows. **(B)** PKS domain organization is shown together with a biosynthesis proposal. Disciformycin assembly is expected to start at DifG, incorporating a malonate and a methylmalonate unit. Polyketide extension is then expected to continue at modules DifB-DifF. Product release takes place by macrocyclization at the TE-domain of DifF. The hydroxy-group at carbon atom C6, to which the valerate is attached in the final compound, does not result from reduction of a keto group. Instead, this hydroxy-group is expected to result from oxidation by a tailoring enzyme during or after assembly. The putative cytochrome P450 gene difA, located adjacent to the PKS genes is considered the most likely candidate for this tailoring step. Suitable genes for further post-PKS modifications, i.e. acylation and glucosylation, could not be found in the vicinity of the PKS gene cluster with structure of disciformycin. The abbreviations have the following meaning: AcT = acyl transferase; AT = acyl transferase domain; CYP = cytochrome P450; DH = dehydrogenase domain; ER = enoyl reductase domain; GT = glycosyl transferase; KS = ketosynthetase domain; KR = ketoreductase domain; T = thiolation domain; TE = thioesterase domain.

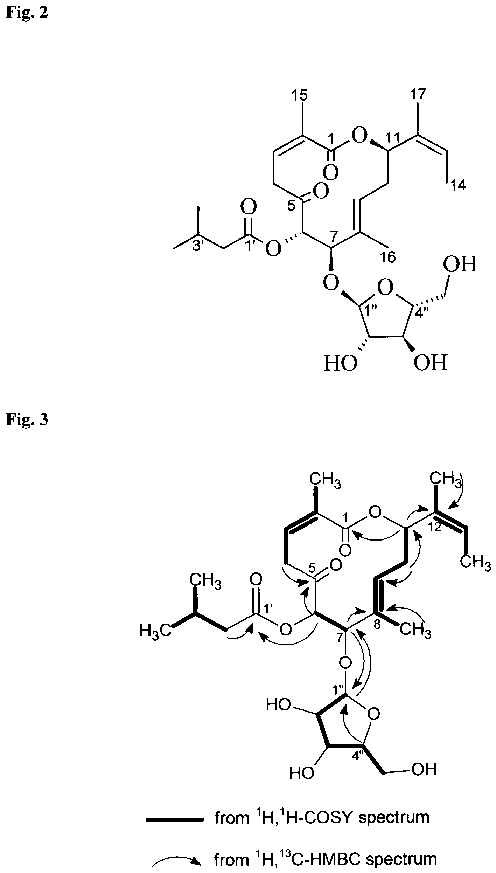

***Figure 2******.*** Structure of disciformycin A (**1**) with carbon atoms numbered.
***Figure 3******.*** Structure of disciformycin A (**1**) with selected COSY and HMBC correlations. ¹H,¹H-COSY and ¹H,¹H-TOCSY correlations led to six ¹H-spins systems. These partial structures were subsequently linked by series of ¹H, ¹³C-HMBC correlations: correlation of methyl group C-15 (δ_{H} 1.96) to C-1 (δ_{C} 169.1), of methylene C-4 (δ_{H} 4.01, 3.58) and methine C-6 (δ_{H} 5.05) to C-5 (δ_{C} 203.9), of methine C-7 (δ_{H} 4.26) and methyl C-16 (δ_{H} 1.91) to C-8 (δ_{C} 134.9), as well as 11-H (δ_{H} 5.61) and 17-H (δ_{H} 1.65) to C-12 (δ_{C} 134.9) established the carbon skeleton of the aglycon. The deep field shift of 11-H (δ_{H} 5.61) indicated an ester linkage at this position which was verified by a HMBC correlation between 11-H and C-1, establishing the lactone ring closure of the aglycon. The configuration of the methyl-substituted double bonds was derived from ROESY correlations. A strong ROESY correlation between 15-H and 3-H demonstrated a Z configuration for the Δ^{2,3} double bond, while the absence of a ROESY correlation between 16-H (δ_{H} 1.91) and 9-H (δ_{H} 5.32) showed an E configuration for the Δ^{8,9} bond. Eventually, a ROESY correlation between 13-H (δ_{H} 5.41) and 17-H (δ_{H} 1.65) indicated a Δ^{12,13} Z configuration in the side chain of the aglycon. A ¹H, ¹³C- HMBC correlation from 6-H to C-1' (δ_{C} 173.2) proved the ester linkage of 3-methylbutyric acid to C-6, simultaneously explaining the deep field shift of 6-H (δ_{H} 5.01).
   Since carbon nuclei in furanose sugars are generally less shielded than in related pyranoses and the anomeric configuration can be differentiated by their chemical shifts, the ¹³C NMR data of **1** are characteristic for an α-arabinofuranose configuration (observed δ_{C} 110.6; 83.8; 79.1; 86.3; 63.0, methyl furanoside δ_{C} 109.3; 81.9; 77.5; 84.9; 62.4).^{[1]} The absolute configuration of the arabinose moiety was determined to be D-(-)-arabinose by GC-MS comparison of the (-)-2-butyl glycoside derivative to authentic standards.^{[2]}
   The relative configuration of the aglycon could be derived from vicinal coupling constants and ROESY spectra (Figure 2). The large coupling constant of 9.5 Hz between 6-H and 7-H and the absence of a ROESY correlation indicated their trans configuration. Strong NOEs were observed for 6-H and 10-Hb with methyl 16-H₃, but not with 11-H. On the other side 7-H shows a strong NOE with 9-H, which itself correlates to 11-H and 10-Ha, indicating a cisoidal relation between 7-H and 11-H.
   Having the relative configuration of the aglycon assigned the absolute configuration remained to be established. In this case the usual chemical derivatization was dispensable because the inherent information of the absolute configuration of the d-(-)-arabinosyl residue could be utilized: As a strong ROESY correlation between 1"-H and 7-H confirmed the typical solution conformation of the glycoside. Consequently, a weak but unambiguous correlation between 4"-H and methyl 16-H₃ permitted assigning the configuration of the chiral center C-7 (Figure 2). Thus the absolute configuration of the disciformycin A aglycon (**1**) was assigned as (6S,7R,11R).
   1D and 2D NMR data of disciformycin B (**2**) showed that the only difference to **1** was the shifting of the Δ^{2,3} double bond to position Δ^{3,4} with E configuration as indicated by the large coupling constant (*J*_{3,4} = 15.3 Hz). Coupling constants and ROESY correlations of the C-6 to C-11 part remained largely unchanged compared to **1.** Therefore, a (6*S*,7*R*,11*R*) configuration can be concluded for **2** as well. To determine the configuration of C-2 a structure model of compound **2** was calculated by MM+ with HyperChem. Due to the rigid structure parts, i.e. the double bond, the keto and the α,β-unsaturated ester, the core part of **2** is locked in a twisted-like configuration, with protons 4-H, 6-H and methyl group 16-H₃ pointing above the main plain. Strong ROESY correlations between 4-H and 2-H on the one and from 3-H to methyl group 15-H₃ on the other hand indicate a 2S configuration.

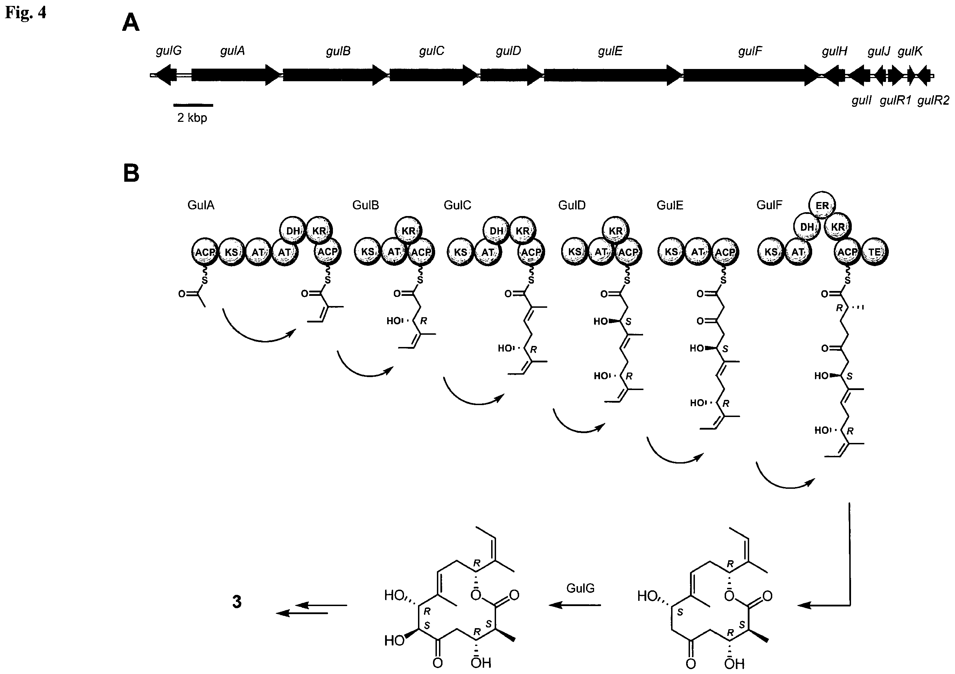

***Figure 4******.*** (**A**) Organization of the gul biosynthetic gene cluster. (**B**) Molecular assembly line deduced from gulA - gulF and proposed biosynthesis of **3.** Domain notation: ACP, acyl carrier protein; KS, β-ketoacyl synthase; AT, acyl transferase; DH, dehydrogenase; KR, ketoreductase; ER, enoyl reductase; TE, thioesterase. Note that the DH and ER domain of GulF are skipped for the formation of **3,** while they are used for the assembly of **4.**
***Figure 5******.*** Structure of gulmirecin A (**3**) with carbon atoms numbered including COSY (bold lines) and selected HMBC (arrows) correlations.
   The identified carbonyl signals in the ¹³C NMR spectrum could be assigned to a ketone (C-5) as well as to two ester functions (C-1, C-23). Aside from the carbonyl groups, four additional carbon atoms (C-8, C-9, C-12, C-13) are sp²-hybridized according to their chemical shifts (cf. Table 5 in Example 6 below). Thus, **3** must feature two carbon-carbon double bonds and two ring structures in order to comply with the required degrees of unsaturation. The signals in the ¹H NMR spectrum were attributed to their directly attached carbon atoms by heteronuclear single-quantum coherence (HSQC). Proton-proton correlation spectroscopy (COSY) revealed six discrete spin systems, all of which could be connected through heteronuclear multiple bond correlation (HMBC) spectroscopy (Fig. 5). The elucidation of the 12-membered lactone ring started from the spin system including CH₃-15 and CH-2 to CH₂-4. The ¹H and ¹³C chemical shifts of CH-3 indicated a secondary alcohol function. Heteronuclear long-range correlations of H₃-15 established the carboxylate group (C-1) at CH-2. Likewise, the placement of the ketone function next to CH₂-4 could be inferred from HMBC interactions between C-5 and the protons at C-3 and C-4. A correlation between H-11 and C-1 in the HMBC spectrum, together with the chemical shift of C-11, unveiled the ester linkage. The resulting partial structure could be expanded with another COSY-derived fragment, which covered CH-9 and CH₂-10 in addition to CH-11. As evidenced by homonuclear and heteronuclear correlations, the olefinic CH-9 must be located adjacent to the quaternary C-8, which possesses a resonance at 133.1 ppm. The two remaining neighboring groups at C-8 were identified as CH₃-16 and CH-7 on the basis of HMBC data. The proton of the methine in position 7 exhibits a ³*J* coupling to H-6. Except for H-3 and H-4a, H-6 is the only proton correlating with C-5 in the HMBC spectrum, thereby allowing a closure of the macrolide ring. The three substituents at C-6, C-7 and C-11 were identified as an isovaleryl, a furanose, and a 2-but-2-en-yl residue. These moieties were connected to the macrolide on the basis of ¹H,¹³C long-range interactions from H-6 to C-23, from H-7 to C-18, and from H₂-10 to C-12 to give the full planar structure of **3.**
   The configuration at C-3 was determined as R by preparation of the diastereomeric α-methoxy-α-trifluoromethylphenylacetic acid (MTPA) ester derivatives, removal of the furanose with In(OTf)₃, and calculation of the Δδ*^{RS}* values. Subsequently, the stereochemistry of the chiral centers C-2, C-6, C-7, and C-11 was concluded from a 2D NOESY experiment. Since no NOE correlation was detected between H-2 and H-3, the two protons must be anti-oriented, *i.e.* they are placed on opposite sides of the macrolide ring. Together with the preceding Mosher analysis, a 2*S* configuration could hence be deduced. In the NOESY spectrum, H-3 exhibits crosspeaks with both hydrogen atoms at C-4, suggesting proton-proton torsion angles of about 60° and a staggered conformation, which is also in good agreement with the respective ³*J*_{HH} values (Table 5). It is evident that the spatial orientations of H-4a and H-4b cannot be derived from their NOE interactions with H-3. However, H-4b also shows an exclusive NOE correlation with H-7, which is only possible when H-4b occupies an axial position and H-7 is located on the same side of the macrolide ring as H-3. The lack of a NOE correlation between H-6 and H-7 hence established the 6*S*, 7*R* configuration. Diagnostic NOEs were observed from H-9 to H-7, H-10b and H-11. The latter proton correlates with H-10b, but not with H-10a, which itself shows crosspeaks with its geminal partner and H₃-16. Taken together, H-10b must have an axial orientation and H-11 should be found on the same side of the molecule as H-3 and H-7, indicating an 11*R* configuration. The NOE-derived 2*S*, 3*R,* 6*S*, 7*R,* 11*R* stereochemistry of the macrolide core was confirmed using a biosynthetic approach that has proven reliable for the configurational assignment of oxygen-bearing stereogenic centers in macrolides and other polyketide natural products.
   To resolve the stereochemistry of the furanose moiety in **3,** the sugar was cleaved from the macrolide core by treatment with In(OTf)₃, followed by a derivatization with 2,4-dihydro-5-methyl-2-phenyl-3*H*-pyrazol-3-one (PMP). Co-chromatography against the PMP derivatives of commercially available pentoses indicated **3** to contain either arabinose or xylose. Since a chromatographic separation of the corresponding PMP derivatives could not be achieved, the released sugar was independently subjected to reductive amination with anthranilic acid. The product was compared to accordingly prepared standards and, in this way, the sugar residue of **3** was identified as arabinose. A subsequent conversion of the unmodified sugar into a thiacarbamoyl-thiazolidine derivative and chromatographic analysis revealed D-(-)-arabinose as the enantiomer present in **3.** The α configuration of the arabinofuranoside was concluded from its ¹³C chemical shifts, and established the *S* configuration of the anomeric carbon C-18. In conclusion, the absolute stereochemistry of **3** was established as depicted above.
   An inspection of the NMR spectra of gulmirecin B (**4**) revealed the structural relatedness to **3.** The missing double bond equivalent (in comparison to **3**) could be readily attributed to the absence of the isovalerate moiety at C-6 and the corresponding carbonyl function. Furthermore, the secondary alcohol at C-3 was replaced by a fully reduced methylene function (*δ*_{H}, 1.66 ppm; *δ*_{C} 28.0 ppm) in **4.** This suggests the dehydratase (DH) and enoyl reductase (ER) domain of GulF to be operational (Fig. 4), and lends additional support to the involvement of the annotated gene cluster in gulmirecin biosynthesis. The proposed stereochemistry of **4** was established on the basis of NOE correlations and biosynthetic reasoning. Upon irradiation at the resonance frequency of H-4b, NOE's were observed with H₂-3, H-4a, H-7 and H₃-15. The latter correlation is only possible, when the methyl group at C-2 resides on the same side of the macrolide ring as H-7.
***Figure 6******.*** Plasmid map of the plasmid vector pCLY10_BG containing the core PKS genes of the disciformycin PKS gene cluster, i.e. *difB, difC, difD, difE, difF,* and *difG,* and homology arms and selection markers for propagation in *S. cerevisiae* and *E. coli.*
***Figure 7*****.** Plasmid map of pMyxZeo_dif containing the core PKS genes of the disciformycin PKS gene cluster, i.e. *difB, difC, difD, difE, difF,* and *difG,* driven by the vanillate-inducible Pvan promotor. This recombinant plasmid can integrate into the genome of M. xanthus DK1622. Induction of *difBCDEFG* expression leads to production of compounds P1 and P2.
***Figure 8******.*** Assembly of the *dif*-PKS gene cluster by TAR. The six PKS genes *difB-difG* were amplified as 4 over-lapping PCR products. In parallel, a part of the pCLY10 plasmid was amplified by PCR, carrying homologous sequences for assembly at each end.
***Figure 9******.*** Total ion chromatograms of four cultivations of *M. xanthus* DK1622 mutants containing inducible heterologous gene cluster. A: mutant with *dif*-cluster, not induced. B: Mutant control with different cluster, induced. C and D: mutants with *dif*-cluster, induced with KVan. Mass peaks P1 and P2 are only observed in extracts of the induced mutants with the *dif-*cluster. Their mass and chemical structure is shown below the chromatograms. The chemical structures of P1 and P2 were elucidated using NMR.

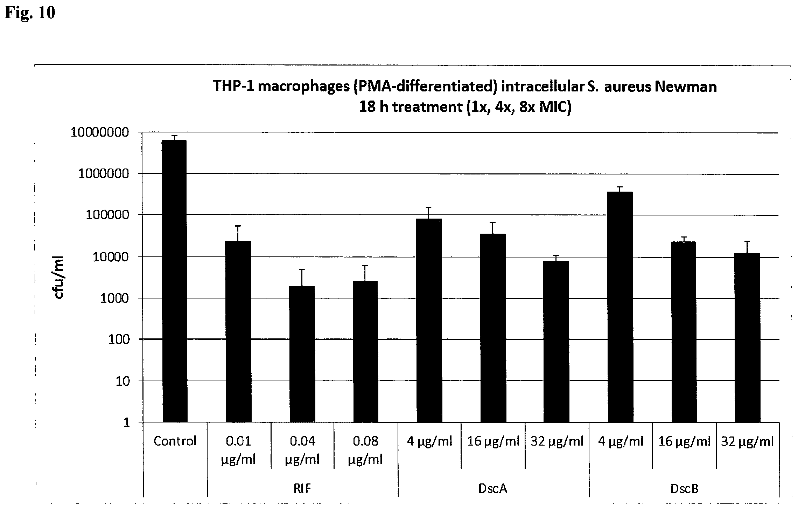

***Figure 10******.*** Intracellular activity of disciformycins A (DscA) and B (DscB) at various concentrations compared to rifampicin (RIF).

The present invention is now further illustrated by the following examples from which further features, embodiments and advantages of the present invention may be taken.

### EXAMPLES

The compounds of the present invention can be prepared in a number of ways well known to one skilled in the art of organic synthesis. The compounds of the present invention can be synthesized using methods known in the art of synthetic organic chemistry or by biotechnological approaches such as fermentation as appreciated by those skilled in the art.

**General Experimental Procedures:** Optical rotations were determined with a Perkin-Elmer 241 or a JASCO P-1020 polarimeter. UV spectra were recorded with a Shimadzu UV-Vis spectrophotometer UV-2450 or a Varian UV/Visible Cary spectrophotometer. IR spectra were recorded on a Bruker FT-IR (IFS 55) spectrometer. NMR spectra were recorded with Bruker AM 300 (¹H 300 MHz, ¹³C 75 MHz), Avance III 500 (¹H 500 MHz, ¹³C 125 MHz), ARX 600 (¹H 600 MHz, ¹³C 150 MHz) and Bruker Ascend 700 (with a 5 mm TXI cryoprobe (¹H 700 MHz, ¹³C 175 MHz)) spectrometers. HRESIMS mass spectra were obtained with an Exactive Mass Spectrometer (Thermo-Scientific) or an Agilent 1200 series HPLC-UV system combined with an ESI-TOF-MS (Maxis, Bruker) [column 2.1×50 mm, 1.7 µm, C₁₈ Acquity UPLC BEH (Waters), solvent A: H₂O + 0.1 % formic acid; solvent B: AcCN + 0.1 % formic acid, gradient: 5 % B for 0.5 min increasing to 100 % B in 19.5 min, maintaining 100 % B for 5 min, F_{R} = 0.6 mLmin⁻¹, UV detection 200-600 nm].

### Example 1 Biosynthesis and biophysical analysis of disciformycin A and B

**Cultivation A (10 L scale):** *Angiococcus disciformis* AngJ1 was cultivated in 10 L of medium E (per liter: skimmed milk 4 g; soy meal 4 g; yeast extract 2 g; starch 10 g; MgSO₄ x 7 H₂O 1 g; Fe-EDTA 8 mg, glycerol 5 g; behenyl alcohol 35 mg) in a biofermenter b10 (Aktiengesellschaft für Biotechnologische Verfahrenstechnik, Schweiz) at 30 °C for 216h. The pH was regulated with potassium hydroxide (2.5 %) and sulfuric acid between 7.3 and 7.5. The stirrer speed was 100 - 400 rpm, aerated with 0.05 vvm compressed air. The dissolved oxygen content within the fermentation broth was regulated by the stirrer speed to pO₂ 20 %. Fermentation was carried out with addition of 1 % adsorber resin XAD-16.

**Purification A:** For isolation of the active metabolites, XAD-16 was harvested by centrifugation; cells were separated from XAD-16 by flotation and discarded. The XAD was eluted with 750 mL acetone and 750 mL methanol, separately. Bioassays against *S. aureus* showed that the antibacterial activity was concentrated in the acetone extract. The organic solvent of the acetone fraction was evaporated *in vacuo* until mostly water remained. This was extracted twice with ethylacetate. The ethylacetate was removed *in vacuo*; the material was dissolved in 85 % aqueous methanol and extracted twice with heptane; subsequently the lower methanol phase was adjusted to 70 % methanol and extracted twice with dichloromethane. Bioassays revealing the water fraction contained the main antibacterial activity, antibiotic metabolite **1** was isolated in a bioassay guided fractionation strategy. The extract was fractionated by RP MPLC (column 480 x 30mm, ODS/AQ C18 (Kronlab), gradient 37 % to 100 % methanol in 60 min, flow 30 mL/min, UV peak detection at 210 nm). Active fractions were combined and further fractionated by preparative RP HPLC column 250 x 21 mm, VP Nucleodur C18 Gravity 5 µm, gradient 37 % to 100 % methanol in 25 min, 50 mM sodium acetate, flow 20 mL/min). A final step of preparative RP HPLC (column 250 x 21 mm, VP Nucleodur C18 Gravity 5 µm, gradient 28 % to 55 % methanol in 25 min, 0.5 % acetic acid, flow 20 mL/min) provided 1.0 mg of disciformycin A (**1**).

**Cultivation B (70 L scale):** *A. disciformis* AngJ1 was cultivated in 70 L of medium P (peptone [Marcor] 2 g/L, starch 8 g/L, probion single cell protein 4 g/L, yeast extract 2 g/L, CaCl₂ 1 g/L, MgSO₄ 1 g/L, Fe-EDTA 8 mg/L, pH 7.5) in a biofermenter P150 (Bioengineering AG, Wald CH) at 30 °C for 148h. The fermentation parameters were as described for the 10L fermentation.

**Purification B:** For isolation of disciformycin A (**1**) and B (**2**), XAD-16 and wet cell mass were harvested by centrifugation. Combined cells and XAD were washed with 30 % methanol (1.5 L), and subsequently extracted with methanol (4 L) and acetone (1 L). Methanol and acetone extracts were combined, evaporated and subjected to a solvent partition. The extract was dissolved in 70 % aqueous methanol and extracted twice with dichloromethane. The obtained material (10.0 g) was fractionated on silica gel 100 (0.063-0.200 mm, approximately 1 kg) by a gradient from dichloromethane/methanol 98:2 to 8:2 (98:2, 95:5, 92.5:7.5 90:10, 80:20 500-750 mL each). The bioactive fraction "90:10" (219.2 mg) was subjected to preparative RP HPLC (column 250 x 21 mm, VP Nucleodur C18 Gravity 5 µm, gradient 30 % to 55 % acetonitrile in 25 min, flow 20 mL/min) and afforded 74.3 mg of material containing **1** and **2.** A final step of silica gel HPLC (column 250 x 21 mm, Nucleosil 100 7 µm, isocratic *tert*-butylmethylether/heptane 1:3 with methanol 2 %, flow 25 mL/min) provided 25.4 mg of **1** and 7.6mg of **2**.

**Disciformycin A (1):** colorless, amorphous powder, [α]²⁰_{D} -51 (c 0.1, MeOH); UV (MeOH) λₘₐₓ 229 (sh); IR (KBr) 3431, 2961, 2929, 2874, 1731, 1647, 1455, 1383, 1254, 1193, 1124, 1076, 1006, 873 cm⁻¹; ¹H, ¹³C, COSY, HMBC and ROESY NMR data see Table 1; HRESIMS *m*/*z* 547.2510 [M+Na]⁺ (calcd for C₂₇H₄₀O₁₀Na, 547.2514).

**Table 1. NMR spectroscopic data of disciformycin A (1) in [D₄]methanol (¹H at 600 MHz, ¹³C at 150 MHz).**

| # | ¹³C, mult. | ¹H, mult. | COSY | HMBC | ROESY |
|---|---|---|---|---|---|
| 1 | 169.1, C | | | | |
| 2 | 134.5, C | | | | |
| 3 | 130.0, CH | 5.88, ddq (8.1, 7.3, 1.5) | 4a, 4b | 4, 15 | 15>4b |
| 4 | 44.0, CH₂ | 4.01, dd (18.3, 8.1) | 3, 4b, 15 | 2, 3, 5 | 4b, 16 |
| | | 3.58, dd (18.3, 7.3) | 3, 4a | | 4a |
| 5 | 203.9, C | | | | |
| 6 | 80.8, CH | 5.05, d (9.5) | 7 | 5, 7, 8, 1' | 16, 4a >4b |
| 7 | 84.2, CH | 4.26, d (9.5) | 6 | 5, 6, 8, 9, 1" | 9, 1" > 16 |
| 8 | 134.9, C | | | | |
| 9 | 128.8, CH | 5.32, brd (11.0) | 10a, 10b, 16 | 7, 16 | 7, 10b, 11 |
| 10 | 31.8, CH₂ | 2.82, ddd (14.7, 11.7, 11.0) | 9, 10b, 11 | 8,9, 11 | 10b, 16, 17 |
| | | | 9, 10a, 11, 16 | | 9, 10a, 11 |
| | | 2.13, m | | | |
| 11 | 75.4, CH | 5.58, dd (11.7, 3.3) | 10a, 10b | 1, 9, 10, 12, 13,17 | 10b, 14>17 |
| 12 | 134.9, C | | | | |
| 13 | 123.6, CH | 5.41, qq (6.6, 1.5) | 14, 17 | 11, 14, 17 | 17 |
| 14 | 13.1, CH₃ | 1.74, dq (6.6, 1.5) | 13, 17 | 12, 13 | 11 |
| 15 | 21.2, CH₃ | 1.96, brs | 3, 4a | 1, 2, 3 | 3 |
| 16 | 12.3, CH₃ | 1.91, dd (1.5, 1.5) | 9, 10b | 7, 8, 9 | 6, 10a |
| 17 | 18.3, CH₃ | 1.65, qd (1.5, 1.5) | 13, 14 | 11, 12, 13 | 13 |
| 1' | 174.5, C | | | | |
| 2' | 43.4, CH₂ | 2.30, d (7.3) | 3' | 1', 3', 4'/5' | 4'/5' |
| 3' | 27.0, CH | 2.11, m | 2', 4'/5' | 1', 2', 4'/5' | |
| 4'/5' | 22.7, CH₃ | 1.00/1.01, d (6.6) | 3' | 2', 3', 4'/5' | 2' |
| 1" | 110.6, CH | 5.13, brs | 2" | 7, 3", 4" | 7 |
| 2" | 83.8, CH | 4.06, m | 1", 3" | 1", 3", 4" | |
| 3" | 79.1, CH | 3.88, dd. (5.9, 2.9) | 2", 4" | 1", 2", 5" | |
| 4" | 86.3, CH | 3.94, td (5.7, 3.3) | 3", 5a", 5b" | 2", 3" | |
| 5" | 63.0, CH₂ | 3.73, dd (12.0, 3.3) | 4", 5b" | 3", 4" | |
| | | 3.64, dd (12.0, 5.7) | 4", 5a" | 3", 4" | |

**Disciformycin B (2):** colorless, amorphous powder, [α]_{D} +64 (*c* 0.4, MeOH); UV (MeOH) λₘₐₓ 221 (sh), 240 (3.67); IR (KBr) 3429, 2961, 2936, 2875, 1738, 1704, 1627, 1455, 1379, 1299, 1178, 1074, 1031, 856 cm⁻¹; ¹H, ¹³C, COSY, HMBC and ROESY NMR data see Table 2; HRESIMS *m*/*z* 547.2516 [M+Na]⁺ (calcd for C₂₇H₄₀O₁₀Na, 547.2514).

**Table 2. NMR spectroscopic data of disciformycin B (2) in CHCl₃-d (¹H at 700 MHz, ¹³C at 175 MHz).**

| # | ¹³C, mult. | ¹H, mult. | COSY | HMBC | ROESY |
|---|---|---|---|---|---|
| 1 | 171.5, C | | | | |
| 2 | 43.0, CH | 3.35, dqd (9.3, 6.6, 1.1) | 3, 15 | 3, 4, 15 | 4,15 |
| 3 | 145.9, CH | 6.60, dd (15.3, 9.3) | 2, 4 | 2, 4, 5, 15 | 15>>2 |
| 4 | 129.9, CH | 6.37, dd (15.3, 1.1) | 3 | 2, 3, 5 | 2, 6, 16 |
| 5 | 192.1, C | | | | |
| 6 | 78.3, CH | 5.32, d (10.3) | 7 | 5, 7, 8, 1' | 4, 16 |
| 7 | 81.1, CH | 4.09, d (10.3) | 6 | 5, 6, 8, 9, 16, 1" | 9, 1" |
| 8 | 133.3, C | | | | |
| 9 | 129.4, CH | 5.42, m | 10a, 10b | 8, 11 | 7, 10b |
| 10 | 32.1, CH₂ | 2.87, ddd (14.6,11.7,11.0) | 9, 10b | 8, 9, 11 | 10b, 16, 17 |
| | | 2.04, m | 9, 10a | 8, 9 | 9, 10a |
| 11 | 72.8, CH | 5.33, dd (11.7, 2.8) | 10a, 10b | 1, 9, 10, 12, 13, 17 | 10b, 14 |
| 12 | 133.0, C | | | | |
| 13 | 123.4, CH | 5.40, m | 14 | 11, 12, 14, 17 | 14, 17 |
| 14 | 13.0, CH₃ | 1.71, dq (6.9, 1.3) | 13 | 12, 13 | 11 |
| 15 | 14.1, CH₃ | 1.27, d (6.6) | 2 | 1, 2, 3 | 2, 3 |
| 16 | 12.5, CH₃ | 1.91, brs | 9 | 7, 8, 9 | 6, 10a |
| 17 | 18.0, CH₃ | 1.69, dq (1.5, 1.3) | | 11, 12, 13 | 10a, 13 |
| 1' | 172.5, C | | | | |
| 2' | 42.8, CH₂ | 2.36, dd (14.8, 7.3) | 3' | 1', 3', 4'/5' | 4'/5' |
| | | 2.31, dd (14.8, 7.3) | | | |
| 3' | 25.8, CH | 2.16, tsept. (7.3, 6.8) | 2', 4'/5' | 1', 2', 4'/5' | 4'/5' |
| 4'/5' | 22.4, CH₃ | 1.02, d (6.8) | 3' | 2', 3', 4'/5' | 2', 3' |
| 1" | 108.2, CH | 5.19, m | 2" | 7, 3", 4" | 7 |
| 2" | 78.2, CH | 4.03, m | 1", 3" | 1", 3" | |
| 3" | 78.3, CH | 4.03, m | 2", 4" | 2", 5" | |
| 4" | 88.1, CH | 4.13, m | 3", 5"a, 5"b | 1", 3" | 16, 5" |
| 5" | 62.1, CH₂ | 3.89, dd (11.6, 2.6) | 4", 5"b | 3" | |
| | | 3.83, dd (11.6, 1.7) | 4", 5"a | 3", 4" | |

### Example 1A Biosynthesis and biophysical analysis of disciformycin C and D

Cultivation was performed according to cultivation B (70 L scale) in Example 1 above.

For isolation of the active metabolites, purification B as described in Example 1 above was conducted, except that the final step of silica gel HPLC was replaced by a further RP HPLC step (column 250 x 21 mm, VP Nucleodur C18 Gravity 5 µm, gradient 30 % to 55 % acetonitrile in 25 min, flow 20 mL/min) where the fraction containing **1** and **2** was further fractionized to thereby obtain 1.0 mg of disciformycin C (**5**) and 7.4 mg disciformycin D (**6**) in addition to **1** and **2**.

### Structure elucidation of disciformycins C (5) and D (6)

A peak at *m*/*z* 579.2776 in the HRESIMS spectrum of **5** provided the molecular formula C₂₈H₄₄O₁₁, which implies a formal addition of CH₄O in comparison to disciformycins A (**1**) and B (**2**). The NMR spectra (Table 1A) were highly similar to those of **1** and **2**. However, the key difference was the shortfall of signals for the C2/C3 respectively C3/C4 double bond. Instead, additional signals of two methines (δ_{H}, δ_{C} 2.51, 47.5; 3.68, 76.7) and one methoxy moiety (δ_{H}, δ_{C} 3.25, 57.5) were observed. ¹H,¹³C HMBC correlations from H₃-15 to C-1/C-2/C-3 and the methoxy signal to C-3 established constitution of **5**. The stereochemistry of the new stereo center C-3 was assigned by the strong ¹H,¹H ROESY correlation from H-3 to H₃-15 together with the absence of a ROESY correlation from H-3 to H-2.

The molecular formula of disciformycin D (**6**) was deduced as C₂₈H₄₄O₁₀S from its [M+Na]⁺ ion peak cluster at *m*/*z* 595.2549 in the HRESIMS spectrum. The NMR spectra of **6** were highly similar to those of **5.** The key difference was the high field shift of the signals for C-3 (δ_{C} 41.8, δ_{H} 3.22) and 3SMe (δ_{C} 14.4, δ_{H} 2.07). Therefore, disciformycin D (**6**) was deduced as the 3-thioether derivative of **5.**

**Table 1A: NMR data (¹H 700 MHz, ¹³C 175 MHz) of disciformycin C (5) and D (6) in CH₃OH-d₄ for 5 and CHCl₃-d for 6.**

| | **5** | | **6** | |
|---|---|---|---|---|
| # | ¹³C. mult. | ¹H | ¹³C | ¹H |
| 1 | 175.1, C | | 174.4, C | |
| 2 | 47.5, CH | 2.51, br s | 44.6, CH | 2.52, m |
| 3 | 76.7, CH | 3.68, m | 41.8, CH | 3.22, m |
| 4 | 47.5, CH₂ | 3.02, m | 41.4, CH₂ | 3.03, m |
| 5 | 205.0, C | | 201.3, C | |
| 6 | 80.6, CH | 5.01, m | 78.4, CH | 4.96, d (9.8) |
| 7 | 84.1, CH | 4.15, d (9.5) | 82.3, CH | 4.17, d (9.8) |
| 8 | 135.8, C | | 132.6, C | |
| 9 | 129.0, CH | 5.30, m | 129.8, CH | 5.45, dd (11.3,1.8) |
| 10 | 32.9, CH₂ | 2.82, dt(14.3,11.8) | 32.1, CH₂ | 2.73, ddd (14.7,11.8,11.3) |
| | | 1.97, m | | 2.00, m |
| 11 | 73.1, CH | 5.81, dd (11.8,1.9) | 71.0, CH | 5.91, dd (11.8,2.3) |
| 12 | 124.5, CH | | 123.6, CH | |
| 13 | 135.0, C | 5.38, m | 133.2, C | 5.36, br q (7.0) |
| 14 | 13.3, CH₃ | 1.67, m | 13.0, CH₃ | 1.66, d6 (7.0,1.5) |
| 15 | 15.1, CH₃ | 1.14, d (6.9) | 17.3, CH₃ | 1.29, d (6.7) |
| 16 | 12.3, CH₃ | 1.85, s | 11.7, CH₃ | 1.79, s |
| 17 | 18.5, CH₃ | 1.71, br s | 18.4, CH₃ | 1.71, t (1.5) |
| 1' | 174.5, C | | 172.7, C | |
| 2' | 43.6, CH₂ | 2.28, d (7.3) | 42.6, CH₂ | 2.27, t (7.3) |
| 3' | 27.2, CH | 2.09, m | 25.8, CH | 2.11, m |
| 4'/5' | 22.8, CH₃ | 0.97, t (6.5) | 22.3, CH₃ | 0.97, d (6.7) |
| 1" | 110.7, CH | 5.09, s | 108.3, CH | 5.15 br s |
| 2" | 83.9, CH | 4.01, dd (3.0,1.3) | 84.0, CH | 4.03, m |
| 3" | 79.3, CH | 3.82, m | 78.1, CH | 4.03, brs |
| 4" | 86.3, CH | 3.86, td (5.4,3.2) | 88.0, CH | 4.14, m |
| 5" | 63.1, CH₂ | 3.68, dd (11.8,3.2) | 62.0, CH₂ | 3.88, dd (11.8,2.8) |
| | | 3.59, dd (11.8,5.4) | | 3.81, (11.8, 2.0) |
| 3OMe | 57.5, CH₃ | 3.25, s | | |
| 3SMe | | | 14.4, CH₃ | 2.07, s |

### Example IB Heterologous expression system and precursor compounds

To corroborate the biosynthesis proposal as depicted in Fig. 1 and to enable generation of novel disciformycin derivatives by, e.g., semisynthetic approaches, a heterologous expression system for the production of (a) disciformycin precursor(s), expressing of the PKS genes *difB-difG* was developed. As producer, the related myxobacterium *Myxococcus xanthus* DK1622 was chosen, because of its favourable growth characterisitcs and established genetic tools.

### Cloning of the dif cluster by transformation-associated recombination (TAR)

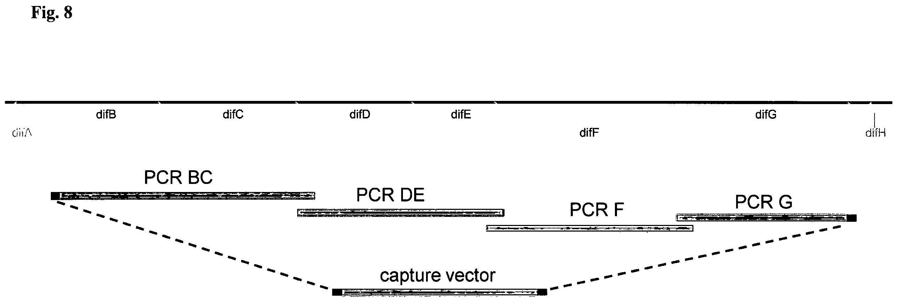

Transformation-associated recombination (TAR) is a cloning strategy that allows linear-to-linear homologous recombination of two or more DNA molecules with suitable homologous sequence overlaps to be assembled as an extrachromosomal plasmid in yeast cells. To select for sucessfully assembled clones, a genetically modified baker's yeast strain with a selectable mutation is used, *S. cerevisiae* ATCC 4004247. The strategy for cluster assembly of the PKS genes *difB-difG* is shown in Fig. 8.

The PKS genes *difB-difG* were amplified as PCR products that overlap with the neighboring fragment over 30-150 base pairs according to the assembly strategy depicted in Fig. 8. The respective PCR products - PCR BC, PCR DE, PCR F, and PCR G - were generated using genomic DNA from *A. disciformis* AngJ1 (DSM 27408) as template. The oligonucleotide primers for conducting the PCR reactions for generating the products PCR BC, PCR DE, PCR F, and PCR G were generated using the primer3 software tool [36]. A linear plasmid DNA fragment with homology arms of 40 base pairs was generated using plasmid pCLY10 DNA as template. The homologous overhangs for *difB* and *difG* were introduced via the oligonucleotide primers (cf. Fig. 8).

An equimolar mixture of all purified single DNA fragment PCR products was then used for transformation of LEU2-deficient *S*. *cerevisiae* ATCC4004247. Clones were selected on leucine-free selection medium. The map of the resulting plasmid pCLY10_BG is shown in Fig. 6.

### Modification of the plasmid for expression in M. xanthus

The obtained *S. cerevisiae* clones were screened with colony PCR for correct assembly of the plasmid. Positive clones were then cultivated, their plasmids isolated and transformed into *E. coli* for plasmid propagation and subsequent restriction analysis. Plasmid DNA from one clone that showed the correct restriction fragments was then transformed into *E. coli* GB-dir for exchange of the plasmid backbone, which is necessary to allow integration and controllable expression in myxobacterium *Myxococcus xanthus* DK1622. As a linear capture vector, a PCR product amplified from pMyxZeoPvan plasmid (generated previously by overlap PCR from two plasmids: pMyxoZeo [37], which contains the zeocin resistance cassette and the mx9 integrase gene and pMR3679 [38], which contains the vannilate promotor/repressor system. was generated with primers containing a sequence overhang that is homologous to *difB* and *difG* sequences on pCLY10_BG. The plasmid map of the resulting integrative expression plasmid pMyxoZeo_dif is shown in Fig. 7.

Exchange of the plasmid backbone was performed with recombination strain *E. coli* GB-dir according to standard recombineering protocols [39]. GB-dir clones with recombined plasmid pMyxoZeo_dif were selected by zeocin and ampicillin resistance and verified with colony PCR and restriction analysis. Correct pMyxoZeo_dif plasmids pMyxZeoDif16 and pMyxZeoDif20 were obtained. The sequence of plasmid pMyxZeoDif16 was verified by Illumina sequencing to exclude acquired mutations in the biosynthesis genes.

### Heterologous expression of precursor compounds P1 and P2 in M. xanthus

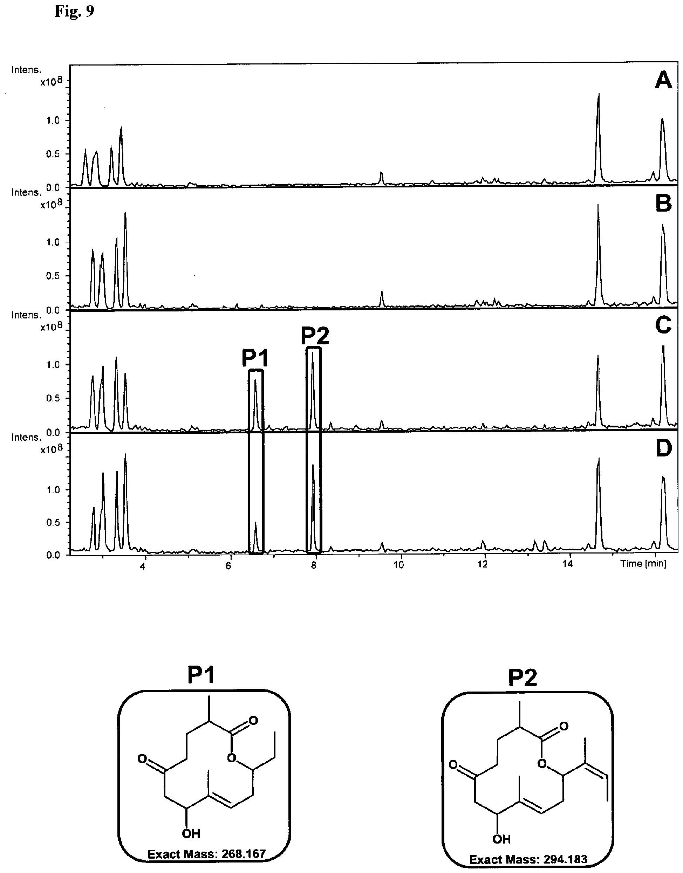

Plasmids pMyxZeoDif16 and pMyxZeoDif20 were transformed into *M. xanthus* DK1622 following standard protocols. DK1622 clones were selected on CTT Agar with zeocin and genetically verified by PCR. One genetically verified clone of DK1622 attB::dif20 was then grown in triplicates in a 20 ml scale in liquid culture and induced with either 1 mM or 2 mM potassium vanillate (Kvan) when the culture showed visible planctonic growth. For comparison, a non-induced culture and a zeocin-resistant control mutant DK1622 attB::CPN13, carrying another heterologous gene cluster integrated in its genome were cultivated in parallel. Cultivation was continued until the colour of the cultures was turning brown, indicating the dying phase. Cells and growth medium were then extracted twice with ethyl acetate, the extracts dried, redissolved in methanol and subjected to MS-analysis, as shown in Fig. 9. The chromatograms of extracts from *M. xanthus* DK1622 attB::dif20 cultures (panel A, C and D) and one control clone attB::CPN13 (panel B) were compared. New masses were detected in extracts of induced DK1622 attB2::dif20 cultures (panel C and D), which are absent in the extracts of the non-induced culture and in the induced control culture of attB::CPN13 (panel A and B). The new mass peaks P1 and P2 are marked in boxes in the chromatograms in Fig. 9 and are expected to result from expression of the PKS encoded by the *dif-genes.* Mass peak P1 has a m/z of [M + H]⁺ = 269.2 and P2 has a m/z of [M + H]⁺ = 295.2.

### Structure elucidation of products P1 and P2

To obtain further insight into the compound identity, cultivation was scaled up to 400 ml CYE-medium and 800 ml CTT-medium, induced and extracted as before and the putative precursors P1 and P2 were isolated by preparative LC-MS on a Waters Autopurifier (Eschborn, Germany) high pressure gradient system. From the culture in CYE medium, 0.7 mg of each compound P1 and P2 were obtained, whereas from the culture in CTT, 0.3 mg of P1 and 1.0 mg of P2 were obtained. Subsequently, 0.7 mg of each purified compounds P1 and P2 were dissolved in DMSO-d₆ for NMR measurements on a Bruker Ascend 700 spectrometer (data not shown). By comparison of the obtained spectra for P1 and P2 with the previously recorded spectra of disciformycins A and B, the structures of P1 and P2 were elucidated to consist of the disciformycin macrolacton core, lacking the post-PKS modifications and posessing fully saturated C2-C3 and C3-C4-bonds, where the disciformycins A and B have a double bond, respectively. The structure of P1 is identical to P2 except for its side chain, which lacking two carbon atoms C13 and C14. The structures of P1 and P2 are shown in Fig. 9.

### Example 2 Assessment of antimicrobial activity of disciformycin A and B

**Bacterial strains.** Bacterial wildtype strains used in susceptibility assays were either part of HZI's strain collection or purchased from the German Collection of Microorgansims and Cell Cultures (*Deutsche Sammlung von Mikroorganismen und Zellkulturen,* DSMZ).

**Susceptibility testing.** Minimum inhibitory concentration (MIC) was determined by microbroth dilution. In brief, overnight cultures of bacteria (EBS medium) were diluted to OD₆₀₀ 0.01. Disciformycin A (**1**) or B (**2**) dissolved in DMSO was added directly to the cultures in 96-well plates (Sarstedt, flat bottom) in duplicate and the compound was tested in serial dilution. Methanol was tested as negative control and showed no activity against the test organisms. ^{[a]}Oxytetracyclin hydrochloride, ^{[b]}Gentamycin, and ^{[c]}Vancomycin were tested as positive control. After 16 h incubation at 900 rpm and 30 °C on a microplate shaker (Titramax, Heidolph) absorbance at 600 nm was measured using a microplate reader (POLARstar Omega, BMG Labtech). MIC values were determined by sigmoidal curve fitting. The results are shown in Table 3.

**Table 3. Antimicrobial activity of disciformycin A (1) and B (2)**

| | Bacterium | DSM/ATCC | **1** | **2** | **Ref.** [^{a,b,c}] |
|---|---|---|---|---|---|
| Gram + | | | | | |
| | *Bacillus subtilis* | 10 | 4.2 | 0.83 | 4.2 ^{[a]} |
| | *Paenibacillus polymyxa* | 36 | 16.6 | 16.6 | 1.7 ^{[a]} |
| | *Staphylococcus aureus* | 346 | 16.6 | 3.3 | 0.21 ^{[a]} |
| | *Staphylococcus aureus* Newman | / | 8.0 | 1.2 | / |
| | *Staphylococcus aureus* (MRSA) | 11822 | 4.0 | 0.6 | 0.83 ^{[c]} |
| | *Staphylococcus aureus* N315 (MRSA) | / | 8.0 | 1.2 | / |
| | *Staphylococcus aureus* Mu50 (MRSA/VRSA) | 700699 | 2.0 | 0.6 | / |
| | *Staphylococcus carnosus* | 20501 | 7.8 | 2.4 | / |
| | *Mycobacterium sp.* | 43270 | n.i. | n.i. | 0.52 ^{[a]} |
| | *Mycobacterium diernhoferi* | 43524 | 33.3 | 33.3 | ≤ 0.052 ^{[a]} |
| | *Micrococcus luteus* | 20030 | 67 | n.i. | 0.42 ^{[a]} |
| | *Nocardioides simplex* | 20130 | 33.3 | 16.6 | 3.3 ^{[a]} |

| Gram - | | | | | |
|---|---|---|---|---|---|
| | *Pseudomonas aeruginosa* | 50071 | n.i. | n.i. | 42.0 ^{[b]} |
| | *Chromobacterium violaceum* | 30191 | n.i. | n.i. | 0.83 ^{[a]} |
| | *Escherichia coli* | 1116 | n.i. | n.i. | 0.83 ^{[a]} |

| | | | | | |
|---|---|---|---|---|---|
| **n.i.** = no inhibition up to 67 µg/ml | | | | | |

As demonstrated above, disciformycin A (**1**) and B (**2**) have an excellent antimicrobial activity against Gram-positive bacteria, and particularly against staphylococci, such as *S. carnosus* DSM-20501 (7.8/2.4; MIC in µg/mL for **1** and **2**, respectively) and *S. aureus* Newman^{[5]} (8.0/1.2). In addition, both tested strains of methicillin-resistant *S. aureus* (MRSA) were inhibited: *S*. *aureus* DSM-11822 (4.0/0.6) and *S. aureus* N315^{[6]} (8.0/1.2). Moreover, these MRSA strains show reduced susceptibility to other antibiotic classes, such as macrolides and quinolones, indicating a putative novel target to be addressed by compounds according to the invention, e.g. **1** and **2**, respectively. Notably, the MICs are in the range of reserve antibiotic vancomycin (cf. Table 3). Most importantly, no cross-resistance was observed to classes of antibiotics in use for human therapy, e.g. vancomycin, as demonstrated by the pronounced activity of **1** and **2** against the methicillin- and vancomycin-resistant *S. aureus* (MRSA/VRSA) Mu50 (ATCC 700699) (2.0/0.6).

### Example 3 Assessment of antiproliferative activity of disciformycin A and B

**Cytotoxicity assay.** Half maximal inhibitory concentrations (IC₅₀) in µg/ml of disciformycin A (1) and disciformycin B (2) for inhibiting the proliferative activity of human HCT-116 colon carcinoma cells, mouse fibroblast cells L929, and Chinese hamster ovary CHO-K1 cells were determined. Cells were seeded at 6 x 10³ cells per well of 96-well plates (Coming CellBind®) in complete medium (180 µl) and directly treated with disciformycin dissolved in DMSO in serial dilution. The compound was tested in duplicate, as well as the internal solvent control. After 5 d incubation, 5 mg/ml MTT in PBS (20 µL) was added per well and it was further incubated for 2 h at 37 °C. The medium was then discarded and cells were washed with PBS (100 µl) before adding 2-propanol/10N HCl (250:1, v/v; 100 µl) in order to dissolve formazan granules. The absorbance at 570 nm was measured using a microplate reader (EL808, Bio-Tek Instruments Inc.). The amount of formazan, i.e. relative absorption, is thereby proportional to the number of actively proliferating cells. The results are shown in Table 4.

**Table 4. Antiproliferative activity of disciformycin A (1) and disciformycin B (2)**

| | IC₅₀ (µg/mL) | |
|---|---|---|
| | **1** | **2** |
| HCT-116 colon carcinoma cells (ACC-581) | 13.0 | > 10 |
| Mouse fibroblast cells L929 (ACC-2) | 29 | > 10 |
| Chinese hamster ovary CHO-K1 cells (ACC-110) | 16.6 | > 10 |

### Example 4 Comparison and prediction of the substrate specificity of the domains in the disciformycin PKS modules

Assembly of the polyketide is expected to start at C14 with incorporation of malonate (mal) and extension by methylmalonate (mmal), mal, mmal, mal, mal, mmal. The polyketide is released as a macrolide, by formation of an ester bond between the carboxyl group at C1 and the hydroxy group at C11. The order of the PKS modules is DifG, DifB, DifC, DifD, DifE, DifF.

**AT domains.** Prediction of the substrate specificity of the seven AT domains was done by multiple-alignments of their respective amino acid sequences and comparison of key residues to published data^{[3]}. Specifically, the specificity of the AT domains within the module sequence was predicted based on occupation of critical postion (AT fingerprint) according to Mohanty. The ATs of modules DifB, DifF, DifE and AT1 of module DifG have a motif characteristic for malonate specific ATs. The ATs of modules DifC, DifF and AT2 from DifG have a methylmalonate-specific motif. Accordingly, the predicted substrate of the AT-domains from modules DifB, DifD, DifE and DifGl is malonate, while AT-domains from modules DifC, DifF and DifG2 are predicted to be methylmalonate. This is consistent with the observations in the disciformycin molecule.

**KR domains.** The polyketide core of disciformycin contains three stereocenters and three double bonds, as can be taken from Fig. 2. The hydroxy group in S-configuration at C6 results from hydroxylation and not from keto-reduction, and is therefore excluded from the analysis. The configuration of hydroxy groups and double bonds that result from KR activity is determined by the respective KR domain that is responsible for reduction of the keto group during metabolite assembly. Stereoselectivity can be predicted from the primary sequence of the KR-domain, since most KRs group into two distinct types, A- and B-type, which accept their substrate in different ways, and thus produce a secondary alcohol of opposite configuration. Briefly, an A-type KR activity results in an L-configured alcohol, while B-type KRs yields D-configured alcohols. Further, a trans-configured double bond results from a dehydrogenase activity on a hydroxy group in a R-configuration, while a cis-double bond results from an S-configured hydroxy group. While B-type KR domains show a conserved "LDD"-motif in the loop region of the enzyme, A-type KRs lack this motif and instead contain a conserved Trp-residue in the catalytic centre.

The stereoselectivity of the KR-domains can be predicted from their primary sequence by multiple alignments and screening for sequence motifs^{[4]}. By multiple alignments of the five KR sequences, loop region and catalytic region were investigated for presence of key residues. The KR of DifB, which reduces the keto group at C11 lacks the typical B-type loop motif and is clearly A-type, because the LDD motif is absent and the critical Trp in the catalytic centre is present. KRs of DifC, DifD and DifF are all B-type, with the conserved LDD-motif being replaced by "LED" in DifD and "LQD" in DifF. The KR sequence of DifG shows neither the conserved LDD-motif nor the conserved Trp in the catalytic region. A prediction of its stereoselectivity is therefore not possible by this method. Observed and predicted stereochemistry agree for the KRs of DifC and DifD. For the Z Δ^{12,13} double bond and the E Δ^{8,9} double bond, the KR domain sequences correspond clearly to A-type KR for DifG and a B-type KR for DifC, respectively. The "*R*"-configured hydroxyl group at carbon atom C7, to which the arabinose is attached, results from keto-reduction by the KR of DifD. For the DifG KR, no clear predicition is possible, yet the observed cis-configuration of the double bond at this position suggests that the KR must be A-type, producing a hydroxy-group in S-configuration which is converted to a cis-double bond by the DifG DH domain. Reduction by DifB, which is clearly A-type, should lead to an S-configured OH-group at C11, but an R-configured OH-group is observed here. The cis-double bond at C3 (Z Δ^{2,3} double bond) is expected to result from DH activity on an S-OH group, but the corresponding DifF KR is B-type. In these two cases, prediction and observed stereochemistry disagree. A possible explanation might be an isomerization during macrocyclization. Notably, module DifF contains a domain of unknown function which appears to be an inactive ER domain. Sequence analysis by BLAST conserved domain search revealed a NADPH binding motif as well as a quinone reductase motif. This unusual domain may catalyze the isomerization which leads to the observed stereochemistry.

### Example 5 Synthesis of disciformycin

A compound of formula (I) according to the present invention can be synthesized using the starting materials and route of synthesis described below, together with synthetic methods known in the art of synthetic organic chemistry, or variations thereon as appreciated by those skilled in the art. Preferred methods include, but are not limited to, those methods described below or in the respective references cited. Unless otherwise specified all starting materials and reagents are of standard commercial grade, and are used without further purification, or are readily prepared from such materials by routine methods. Those skilled in the art of organic synthesis will recognize that starting materials and reaction conditions may be varied including additional steps employed to produce compounds encompassed by the present invention.

### (1) Preparation of (Z)-3-iodo-2-methylprop-2-en-1-ol^{[7]}

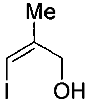

To a solution of CuI (12.8 mg, 67 µmol, 0.1 eq.) in THF (0.7 ml) at -20 °C was added propargyl alcohol (39.7 µl, 672 µmol, 1.0 eq.). A 3 M solution of methylmagnesium bromide in Et₂O (0.49 ml, 1.48 mmol, 2.2 eq.) was added slowly, the temperature was raised to -10 °C and the solution stirred for 30 min. A solution of I₂ (85.3 mg, 672 µmol, 1.0 eq.) in Et₂O/THF (0.3 ml, 1:1) was added and the mixture was allowed to warm to room temperature. The solution was poured into a mixture of NH₄Cl (0.7 ml) and brine (0.7 ml). The aqueous phase was extracted with Et₂O (3x0.4 ml), the combined organic phases were dried (MgSO₄) and concentrated. The residue was purified by column chromatography to furnish the product (100 mg, 504 µmol, 75 %) in good yield.

### (2) Preparation of (Z)-1-(((3-iodo-2-methylallyl)oxy)methyl)-4-methoxybenzene^{[8]}

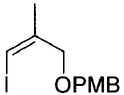

To a suspension of NaH (13.2 mg, 554 µmol 1.1 eq.) in DMF at 0 °C was slowly added a solution of (Z)-3-iodo-2-methylprop-2-en-1-ol (100 mg, 504 µmol, 1.0 eq.) in THF (0.3 ml). The resulting mixture was stirred for 30 min, prior to addition of PMBCl (68.4 µl, 504 µmol, 1.0 eq.) in THF (0.1 ml). After stirring for 2 h, Et₂O and H₂O were added. The phases were separated and the organic phase was washed with H₂O and brine. The dried (MgSO₄) organic layer was concentrated and the residue was purified by column chromatography to give the product (130 mg, 408 µmol, 81 %) in good yield.

### Preparation of (S)-4-benzyl-3-(2-(benzyloxy)acetyl)oxazolidin-2-one^{[9]}

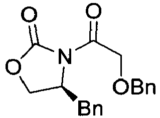

To a solution of (S)-4-benzyloxazolidin-2-one (40.0 mg, 224 µmol, 1.0 eq.) in THF (1.1 ml) at -78 °C was added 1.57 M nBuli in hexane (0.14 ml, 224 µmol, 1.0 eq.), followed by addition of benzyloxyactyl chloride (38.8 µl, 246 µmol, 1.1 eq.). After stirring for 1 h at -78 °C the solution was allowed to warm to room temperature over 30 min. The reaction was quenched by addition of NH₄Cl (0.2 ml). The mixture was concentrated to a slurry which was extracted with CH₂Cl₂ (2x0.4 ml). The combined organic phases were washed with 1 M NaOH (0.3 ml) and brine (0.3 ml), dried (Na₂SO₄) and concentrated. The residue was purified by column chromatography to give the product in very good yield (70.6 mg, 217 µmol, 97 %).

### (3) Preparation of (3S,4R,5R)-4-((acetyl-12-chloranyl)oxy)-5-(((acetyl-12-chloranyl)oxy)methyl)-2-bromotetrahydrofuran-3-yl 2-chloroacetate^{[10]}

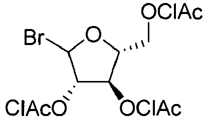

To a solution of D-Arabinose (32.7 mg, 218 µmol, 1.0 eq.) in methanol (0.8 ml) at 0 °C was dropwise added acetlychloride (12.7 µl, 178 µmol, 0.9 eq.). The reaction was stirred for 1 h at room temperature. After addition of pyridine (164 µl, 2.03 mmol, 9.3 eq.) the mixture was concentrated and the residue was coevaporated with CHCl₃.

The residue was dissolved in DMF (0.3 ml) and the solution cooled to 0 °C. Na₂CO₃ (104 mg, 981 µmol, 4.5 eq.) was added, followed by chloroacetyl chloride (78.0 µl, 981 µmol, 4.5 eq.) in DMF (0.1 ml). The reaction was stirred overnight at room temperature before being quenched by addition of H₂O. After stirring for 30 min, the solution was extracted with Et₂O (3x0.7 ml). The combined organic layers were dried (MgSO₄) and concentrated. The residue was purified by column chromatography to furnish the product (24.2 mg, 61.1 µmol, 28 %).

This compound (24.2 mg, 61.1 µmol, 1.0 eq.) was dissolved in AcOH (0.6 ml) and 45 % HBr in AcOH (0.8 ml) was added. The reaction was stirred for 1 h at room temperature. CH₂Cl₂ (0.8 ml) and ice-cold H₂O (0.7 ml) were added and the organic layer was washed with cold H₂O (0.7 ml) and cold saturated NaHCO₃ (2x0.3 ml). The dried organic phase was concentrated to give the product (19.3 mg, 43.4 µmol, 71 %), which was used in the following glycosylation without further purification.

### (4) Preparation of (Z)-2-methylbut-2-en-1-ol^{[11]}

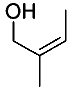

To a suspension of LiAlH₄ (96.0 mg, 2.53 mmol, 2.5 eq.) in Et₂O (1.2 ml) at 0 °C was slowly added a solution of angelica acid methyl ester (0.12 ml, 1.01 mmol, 1.0 eq.) in Et₂O (1.9 ml). The mixture was stirred at room temperature for 1 h before being treated with more LiAlH₄ (30.7 mg, 0.81 mmol, 0.8 eq.) in Et₂O (1.3 ml). The reaction was quenched after 30 min by addition of H₂O (0.3 ml), 15 % aqueous NaOH (0.3 ml) and more H₂O (1.0 ml). After filtration the solution was dried (MgSO₄) and concentrated. Kugelrohr distillation afforded the product (65.1 mg, 756 µmol, 75 %) as an oil.

### (5) Preparation of (Z)-2-methylbut-2-enal^{[12]}

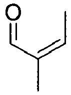

To a solution of (Z)-2-methylbut-2-en-1-ol (65.1 mg, 756 µmol, 1.0 eq.) in CH₂Cl₂ (1.1 ml) was added MnO₂ (1.08 g, 12.5 mmol, 16.5 eq.) and the reaction was stirred for 24 h at room temperature. The solid was filtered off and the solvent removed under reduced pressure. The product (50.9 mg, 605 µmol, 80 %) was obtained as a colourless liquid via short path distillation.

### (6) Preparation of (R,Z)-3-hydroxy-1-((S)-4-isopropyl-2-thioxothiazolidin-3-yl)-4-methylhex-4-en-1-one^{[13]}

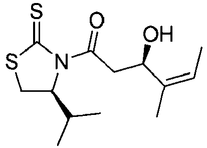

To a solution of N-Acetyl thiazolidinthione (209 mg, 1.03 mmol, 1.7 eq.) in CH₂Cl₂ (4.4 ml) at -40°C was added TiCl₄ (0.12 ml, 1.09 mmol, 1.8 eq.). After 5 min DIPEA (0.19 ml, 1.09 mmol, 1.8 eq.) was added and the deep red mixture was stirred for 2 h at -40°C before being cooled to -78 °C. (Z)-2-methylbut-2-enal (50.9 mg, 605 µmol, 1.0 eq.) in CH₂Cl₂ (0.6 ml) was added to the reaction. After 10 min the mixture was poured into pH7 phosphate buffer (22 ml) and the aqueous phase was washed with CH₂Cl₂ (3x). The combined organic phases were dried (Na₂SO₄) and concentrated. The product (134 mg, 466 µmol, 77%) was obtained via column chromatography of the residue.

### (7) Preparation of (R,Z)-3-((tert-butyldimethylsilyl)oxy)-1-((S)-4-isopropyl-2-thioxothiazolidin-3-yl)-4-methylhex-4-en-1-one^{[14]}

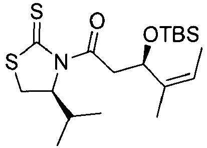

To a stirred solution of (R,Z)-3-hydroxy-1-((S)-4-isopropyl-2-thioxothiazolidin-3-yl)-4-methylhex-4-en-1-one (134 mg, 466 µmol, 1.0 eq.) in CH₂Cl₂ (1.0 ml) was added 2,6-lutidine (92.0 µl, 792 µmol, 1.7 eq.). The mixture was cooled to -78°C and TBSOTf (214 µl, 932 µmol, 2.0 eq.) was added dropwise. The reaction was stirred at -78°C for 1 h and at 0 °C for another 1 h. After addition of pH7 phosphate buffer (3.3 ml) and CH₂Cl₂ the phases were separated and the aqueous phase was extracted with CH₂Cl₂ (2x3.3 ml). The combined organic phases were washed with H₂O (3.3 ml), dried and concentrated. The product (185 mg, 461 µmol, 99 %) was obtained by column chromatography.

### (8) Preparation of (R,Z)-3-((tert-butyldimethylsilyl)oxy)-4-methylhex-4-enal^{[15]}

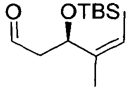

To a solution of (R,Z)-3-((tert-butyldimethylsilyl)oxy)-1-((S)-4-isopropyl-2-thioxothiazolidin-3-yl)-4-methylhex-4-en-1-one (185 mg, 461 µmol 1.0 eq.) in PhMe (1.1 ml) was added 1 M DIBALH (1.15 ml, 1.15 mmol, 2.5 eq.) at -78 °C. The reaction was quenched after 1 h by addition of EtOAc (0.9 ml), followed by addition of saturated Rochelle's salt (2.9 ml). After stirring at room temperature, the phases were separated and the aqueous phase was extracted with EtOAc. The combined organic phases were dried (Na₂SO₄) and concentrated. The residue was purified via column chromatography to furnish the product (105 mg, 433 µmol, 94 %) in very good yield.

### (9) Preparation of Ethyl (R,2E,6Z)-5-((tert-butyldimethylsilyl)oxy)-2,6-dimethylocta-2,6-dienoate^{[16]}

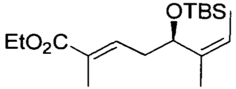

To a solution of (R,Z)-3-((tert-butyldimethylsilyl)oxy)-4-methylhex-4-enal (105 mg, 433 µmol, 1.0 eq.) in THF (6.4 ml) was added (carbethoxyethylidene)triphenylphosphorane (471 mg, 1.30 mmol, 3.0 eq.). The mixture was stirred for 24 h at reflux. The crude material was purified by column chromatography to give the product (141 mg, 433 µmol, 100 %).

### (10) Preparation of (R,2E,6Z)-5-((tert-butyldimethylsilyl)oxy)-2,6-dimethylocta-2,6-dien-1-ol^{[17]}

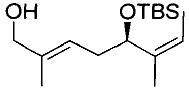

A solution of Ethyl (R,2E,6Z)-5-((tert-butyldimethylsilyl)oxy)-2,6-dimethylocta-2,6-dienoate (141 mg, 433 µmol, 1.0 eq) in THF (0.33 ml) was added to a suspension of LiAlH₄ (49.3 mg, 1.30 mmol, 3.0 eq.) in THF (0.08 ml) at 0°C. After stirring for 20 min at room temperature H₂O (0.05 ml) was added, followed by 2M NaOH (0.02 ml). The mixture was filtered through celite and was washed with brine, dried (MgSO₄) and concentrated to give the product (118 mg, 416 µmol, 96 %).

### (11) Preparation of (R,2E,6Z)-5-((tert-butyldimethylsilyl)oxy)-2,6-dimethylocta-2,6-dienal^{[18]}

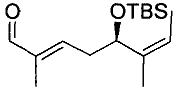

To a solution of (R,2E,6Z)-5-((tert-butyldimethylsilyl)oxy)-2,6-dimethylocta-2,6-dien-1-ol (118 mg, 416 µmol, 1.0 eq.) in Et₂O (2.0 ml) was added MnO₂ (542 mg, 6.24 mmol, 15.0 eq.) and the mixture was stirred for 2 h. The reaction was filtered over a silica gel column, eluting with EtOAc (16 ml). The resulting solution was concentrated to give the product (110mg, 391 µmol, 94 %).

### (12) Preparation of (S)-4-benzyl-3-((2S,3R,4E,7R,8Z)-2-(benzyloxy)-7-((tert-butyl-dimethylsilyl)oxy)-3-hydroxy-4,8-dimethyldeca-4,8-dienoyl)oxazolidin-2-one^{[19]}

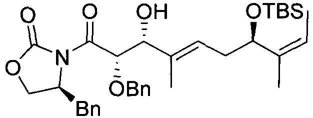

To a solution of (S)-4-benzyl-3-(2-(benzyloxy)acetyl)oxazolidin-2-one (70.6 mg, 217 µmol, 1.0 eq.) in PhMe (0.3 ml) was added Et₃N (39.1 µl, 282 µmol, 1.3 eq.). The mixture was cooled to -50 °C, followed by careful addition of Bu₂BOTf (77.4 µl, 239 µmol, 1.1 eq.). The reaction was stirred for 1.5 h at -50 °C. A solution of (R,2E,6Z)-5-((tert-butyldimethylsilyl)oxy)-2,6-dimethylocta-2,6-dienal (110 mg, 391 µmol, 1.8 eq.) in PhMe (0.3 ml) was added to the mixture via transfer cannula. The reaction was warmed to -30 °C and stirred for 1.5 h. The reaction was quenched by addition of MeOH (0.2 ml), pH7 buffer (0.2 ml), and H₂O₂ (0.2 ml) and was stirred for 1 h. The organic phase was separated and the aqueous phase was washed with Et₂O (3x15 ml). The combined organic phases were dried (MgSO₄) and concentrated. The product (102 mg, 167 µmοl, 77%) was purified using column chromatography.

### (13) Preparation of (S)-4-benzyl-3-((2S,3R,4E,7R,8Z)-2-(benzyloxy)-7-((tert-butyldimethylsilyl)oxy)-3-((tert-butyldiphenylsilyl)oxy)-4,8-dimethyldeca-4,8-dienoyl) oxazolidin-2-one^{[20]}

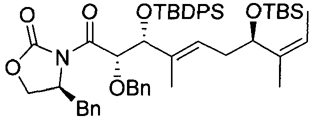

To a solution of (S)-4-benzyl-3-((2S,3R,4E,7R,8Z)-2-(benzyloxy)-7-((tertbutyldimethylsilyl)oxy)-3-hydroxy-4,8-dimethyldeca-4,8-dienoyl)oxazolidin-2-one (102 mg, 167 µmol, 1.0 eq.) in DMF (1.0 ml) at 0 °C was added imidazole (56.8 mg, 835 µmol, 5.0 eq.). A solution of TBDPSCl (0.13 ml, 501 µmol, 3.0 eq.) in DMF (0.2 ml) was added dropwise and the reaction was allowed to warm to room temperature overnight before being quenched by slow addition of saturated NH₄Cl. Et₂O was added and the phases were separated. The aqueous layer was extracted with Et₂O (4x). The combined organic phases were washed with saturated NH₄Cl (2x) and H₂O (2x), dried (MgSO₄) and concentrated. The product (139 mg, 164 µmol, 98 %) was obtained via column chromatography of the residue.

### (14) Preparation of (2R,3R,4E,7R,8Z)-2-(benzyloxy)-7-((tert-butyldimethylsilyl) oxy)-3-((tert-butyldiphenylsilyl)oxy)-4,8-dimethyldeca-4,8-dien-1-ol^{[21]}

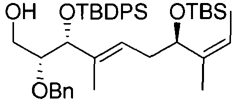

To a solution of (S)-4-benzyl-3-((2S,3R,4E,7R,8Z)-2-(benzyloxy)-7-((tertbutyldimethylsilyl)oxy)-3-((tert-butyldiphenylsilyl)oxy)-4,8-dimethyldeca-4,8-dienoyl)oxazolidin-2-one (139 mg, 164 µmοl, 1.0 eq.) in THF (0.8 ml) and H₂O (4.3 µl, 238 µmol, 1.5 eq.) at 0 °C was added a 0.65 M solution of lithium borohydride in THF (0.33 ml, 213 µmol, 1.3 eq.). After 1 h the reaction was quenched by addition of saturated NH₄Cl (0.8 ml). The aqueous phase was extracted with EtOAc (2x1.0 ml). The combined organic phases were washed with brine (0.6 ml), dried (Na₂SO₄) and concentrated. The residue was purified via column chromatography to give the pure product (92.9 mg, 138 µmol, 84 %).

### (15) (2S,3R,4E,7R,8Z)-2-(benzyloxy)-7-((tert-butyldimethylsilyl)oxy)-3-((tert-butyl diphenylsilyl)oxy)-4,8-dimethyldeca-4,8-dienal^{[22]}

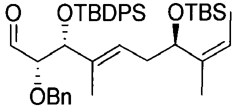

A solution of DMSO (21.6 µl, 304 µmol, 2.2 eq.) in CH₂Cl₂ (0.1 ml) was added to a solution of oxalyl chloride (13.0 µl, 152 µmol, 1.1 eq.) in CH₂Cl₂ (0.2 ml) at -60°C. After 5 min (2R,3R,4E,7R,8Z)-2-(benzyloxy)-7-((tert-butyldimethylsilyl)oxy)-3-((tert-butyldiphenylsilyl)oxy)-4,8-dimethyldeca-4,8-dien-1-ol (92.9 mg, 138 µmol, 1.0 eq.) in CH₂Cl₂ (0.2 ml) was added to the mixture and the solution was stirred at -60°C for 15 min. NEt₃ (95.9 µl 692 µmol, 5.0 eq.) was added and the mixture was stirred for 5 min at -60°C before being warmed to room temperature. The reaction was quenched by addition of H₂O (0.3 ml), the organic layer was washed with brine (0.3 ml), dried (MgSO₄) and concentrated to give the product (91.9 mg, 137 µmοl, 99 %).

### (16) Preparation of (5R,9R,E)-5-((1R)-(benzyloxy)(oxiran-2-yl)methyl)-9-((Z)-but-2-en-2-yl)-2,2,6,11,11,12,12-heptamethyl-3,3-diphenyl-4,10-dioxa-3,11-disilatridec-6-ene^{[23]}

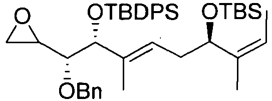

To a solution of Me₃S⁺I⁻ (42.0 mg, 206 µmol, 1.5 eq.) in THF (0.5 ml) was added KHMDS (35.7 mg, 179 µmol, 1.3 eq.). After 1 h, a solution of (2S,3R,4E,7R,8Z)-2-(benzyloxy)-7-((tertbutyldimethylsilyl)oxy)-3-((tert-butyldiphenylsilyl)oxy)-4,8-dimethyldeca-4,8-dienal (91.9 mg, 137 µmol, 1.0 eq.) in THF (0.2 ml) was added via transfer cannula. The reaction was quenched with H₂O (0.04 ml) after 30 min and the solution was concentrated. The obtained residue was redissolved in Et₂O (0.8 ml) and H₂O (0.5 ml) and the aqueous layer was washed with Et₂O (0.3 ml). The combined organic phases were washed with H₂O (0.3 ml) and brine (0.3 ml), dried (MgSO₄) and concentrated. The residue was purified by column chromatography, yielding the product (93.2 mg, 136 µmol, 99 %) in very good yield.

### (17) Preparation of (2Z,6R,7R,8E,11R,12Z)-6-(benzyloxy)-11-((tert-butyldimethyl silyl)oxy)-7-((tert-butyldiphenylsilyl)oxy)-1-((4-methoxybenzyl)oxy)-2,8,12-trimethyltetradeca-2,8,12-trien-5-ol^{[24]}

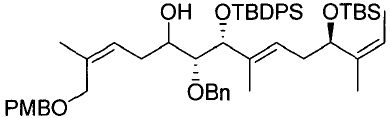

To a dry flask containing Mg (9.92 mg, 408 µmol, 3.0 eq.) in THF (0.1 ml), (Z)-1-(((3-iodo-2-methylallyl)oxy)methyl)-4-methoxybenzene (13.0 mg, 40.8 µmol, 0.3 eq.) was added. After 30 s THF (3 ml) was added and the reaction cooled to 0 °C. Additional (Z)-1-(((3-iodo-2-methylallyl)oxy)methyl)-4-methoxybenzene (117 mg, 367 µmol, 2.7 eq.) was added. The solution was stirred for further 2 h and then stirring of the reaction was stopped. After 3 h the mixture was added via transfer cannula to a 0.1 M solution of LiCuCl₄ (0.10 ml, 10.2 µmol, 0.1 eq.) in THF (0.6 ml) at -35°C. The reaction was stirred for 35 min, then (5R,9R,E)-5-((1R)-(benzyloxy)(oxiran-2-yl)methyl)-9-((Z)-but-2-en-2-yl)-2,2,6,11,11,12,12-heptamethyl-3,3-diphenyl-4,10-dioxa-3,11-disilatridec-6-ene (93.2 mg, 136 µmol, 1.0 eq.) in THF (0.6 ml) was added via transfer cannula. After 10 min the reaction was quenched by addition of saturated NH₄Cl (0.2 ml), together with addition of Et₂O (0.5 ml). The mixture was warmed to 10°C under vigorous stirring. Washing of the organic phase with brine multiple times was followed by extraction of the combined aqueous phases with Et₂O (1x). The combined organic phases were dried (MgSO₄) and concentrated. The residue was purified by column chromatography to furnish the product (115 mg, 132 µmol, 97 %) in very good yield.

### (18) Preparation of (6R,7R,11R,E)-6-(benzyloxy)-11-((Z)-but-2-en-2-yl)-7-((tert-butyldiphenylsilyl)oxy)-5-((Z)-4-((4-methoxybenzyl)oxy)-3-methylbut-2-en-1-yl)-8,13,13,14,14-pentamethyl-4,12-dioxa-2-thia-13-silapentadec-8-ene^{[25]}

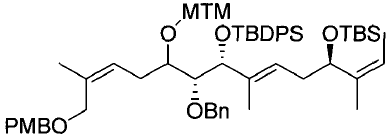

A stirred suspension of NaH (5.7 mg, 238 µmol, 1.8 eq.) in THF (0.2 ml) was treated with (2Z,6R,7R,8E,11R,12Z)-6-(benzyloxy)-11-((tert-butyldimethylsilyl)oxy)-7-((tert-butyldiphenylsilyl)oxy)-1-((4-methoxybenzyl)oxy)-2,8,12-trimethyltetradeca-2,8,12-trien-5-ol (115mg, 132 µmol, 1.0 eq.) for 30 min. Chloromethyl methyl sulfide (22.1 µl, 264 µmol, 2.0 eq.) was added and the mixture was stirred at 0°C for 3 h. After quench with saturated NH₄Cl, the aqueous layer was extracted multiple times with Et₂O. The combined organic phases were washed with brine, dried (Na₂SO₄) and concentrated. The residue was purified using column chromatography to give the product (121 mg, 129 µmol, 98 %).

### (19) Preparation of (2Z,6R,7R,8E,11R,12Z)-6-(benzyloxy)-11-((tert-butyldimethylsilyl)oxy)-7-((tert-butyldiphenylsilyl)oxy)-2,8,12-trimethyl-5-((methylthio) methoxy)tetradeca-2,8,12-trien-1-ol^{[16]}

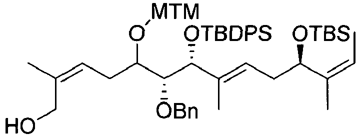

(6R,7R,11R,E)-6-(benzyloxy)-11-((Z)-but-2-en-2-yl)-7-((tert-butyldiphenylsilyl)oxy)-5-((Z)-4-((4-methoxybenzyl)oxy)-3-methylbut-2-en-1-yl)-8,13,13,14,14-pentamethyl-4,12-dioxa-2-thia-13-silapentadec-8-ene (121 mg, 129 µmol, 1.0 eq.) was dissolved in CH₂Cl₂ (5.7 ml) and H₂O (0.57 ml). DDQ (29.3 mg, 129 µmol, 1.0 eq.) was added at 0°C and the mixture was stirred for 1 h at 0 °C. The reaction was quenched by addition of saturated Na₂SO₃ solution (8.6 ml). After extraction of the aqueous phase with CH₂Cl₂ (3x30 ml) the combined organic phases were dried (MgSO₄) and concentrated. Purification by flash chromatography yielded the product (95.6 mg, 117 µmol, 91%).

### (20) Preparation of (2Z,6R,7R,8E,11R,12Z)-6-(benzyloxy)-11-((tert-butyldimethyl silyl)oxy)-7-((tert-butyldiphenylsilyl)oxy)-2,8,12-trimethyl-5-((methylthio) methoxy) tetradeca-2,8,12-trienoic acid^{[27]}

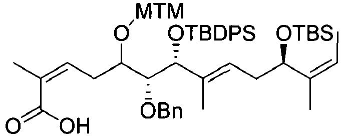

A) To a solution of (2Z,6R,7R,8E,11R,12Z)-6-(benzyloxy)-11-((tert-butyldimethylsilyl)oxy)-7-((tert-butyldiphenylsilyl)oxy)-2,8,12-trimethyl-5-((methylthio) methoxy)tetradeca-2,8,12-trien-1-ol (95.6 mg, 117 µmol, 1.0 eq.) in CH₂Cl₂ (1.2 ml) was added MnO₂ (101 mg 1.17 mmol, 10 eq.). The reaction was stirred for 2 h at room temperature and filtered through a Celite pad. The product (92.9 mg, 114 µmol, 98%) was obtained by concentration of the filtrate and purification via column chromatography.
B) The obtained aldehyde (92.9 mg, 114 µmol, 1.0 eq.) was dissolved in *tert*-butyl alcohol (1.5 ml). 2-methyl-2-butene (0.85 ml, 7.98 mmol, 70 eq.), NaClO₂ (227 mg, 2.51 mmol, 22 eq.) and sodium dihydrogenphosphate (227 mg, 1.89 mmol, 17 eq.) in H₂O (1.5 ml) were added. The reaction was stirred for 16 h at room temperature. 0.5 M KHSO₄ and EtOAc were added, the phases separated and the aqueous phase was washed with EtOAc. The combined organic phases were washed with 10 % NaHSO₃ and brine, dried (Na₂SO₄) and concentrated under reduced pressure. The product (91.0 mg, 110 µmol, 96%) was obtained by column chromatography.

### (21) Preparation of (2Z,6R,7R,8E,11R,12Z)-6-(benzyloxy)-7-((tert-butyldiphenylsilyl)oxy)-11-hydroxy-2,8,12-trimethyl-5-((methylthio)methoxy)tetradeca-2,8,12-trienoic acid^{[28]}

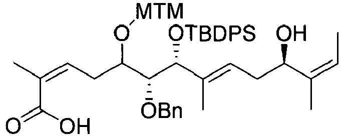

(2Z,6R,7R,8E,11R,12Z)-6-(benzyloxy)-11-((tert-butyldimethylsilyl)oxy)-7-((tert-butyldiphenylsilyl)oxy)-2,8,12-trimethyl-5-((methylthio)methoxy)tetradeca-2,8,12-trienoic acid (91.0 mg, 110 µmol, 1.0 eq.) was dissolved in ethanol (0.6 ml). The reaction was stirred at 55°C for 2 h after addition of PPTS (8.26 mg, 32.9 µmol, 0.3 eq.). After removal of the solvent under reduced pressure, the residue was dissolved in EtOAc, washed with brine, H₂O and dried (MgSO₄). The product (64.4 mg, 89.8 µmol, 82 %) was obtained by concentration of the organic phase, followed by column chromatography.

### (22) Preparation of (3Z,7R,8R,9E,12R)-7-(benzyloxy)-12-((Z)-but-2-en-2-yl)-8-((tert-butyldiphenylsilyl)oxy)-3,9-dimethyl-6-((methylthio)methoxy)oxacyclodo-deca-3,9-dien-2-one^{[29]}

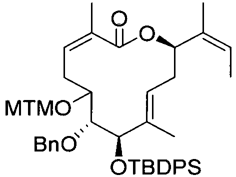

A mixture of (2Z,6R,7R,8E,11R,12Z)-6-(benzyloxy)-7-((tert-butyldiphenylsilyl)oxy)-11-hydroxy-2,8,12-trimethyl-5-((methylthio)methoxy)tetradeca-2,8,12-trienoic acid (64.4 mg, 89.8 µmol, 1.0 eq.), DIPEA (0.61 ml, 3.59 mmol, 40 eq.) and 2,4,6-trichlorbenzoylchloride (0.28 ml, 1.80 mmol, 20 eq.) in THF (4 ml) was stirred overnight at room temperature. The mixture was diluted with benzene (12 ml) and added to a solution of DMAP (548 mg, 4.49 mmol, 50 eq.) in benzene (55 ml) at 80°C over a period of 12 h. After stirring for another hour, the reaction was quenched by addition of saturated NaHCO₃. The aqueous phase was extracted with EtOAc (2x25 ml). The combined organic phases were dried (Na₂SO₄) and concentrated. The product (34.5 mg, 49.4 µmol, 55 %) was obtained by column chromatography.

### (23) Preparation of (3Z,7R,8R,9E,12R)-12-((Z)-but-2-en-2-yl)-8-((tert-butyldi-phenylsilyl)oxy)-7-hydroxy-3,9-dimethyl-6-((methylthio)methoxy)oxa-cyclododeca-3,9-dien-2-one^{[30]}

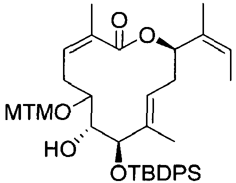

To a solution of di-tert-butylbiphenyl (263 mg, 988 µmol, 20 eq.) in THF (4.0 ml) was added activated lithium wire (24.0 mg, 3.46 mmol, 70 eq.) at 0 °C to give a green solution. The mixture was used to titrate a solution of (3Z,7R,8R,9E,12R)-7-(benzyloxy)-12-((Z)-but-2-en-2-yl)-8-((tert-butyldiphenylsilyl)oxy)-3,9-dimethyl-6-((methylthio)methoxy)oxacyclododeca-3,9-dien-2-one (34.5 mg, 49.4 µmol, 1.0 eq.) in THF (4.0 ml) at -78°C until the green color persisted (3 h). The reaction was quenched by addition of saturated NH₄Cl (9.0 ml). Et₂O (9.0 ml) was added and the phases were separated, followed by extraction of the aqueous phase with Et₂O (3x). The combined organic phases were dried (MgSO₄) and concentrated. The product (29.7 mg, 46.9 µmol, 95 %) was obtained by column chromatography of the residue.

### (24) Preparation of (3Z,7R,8R,9E,12R)-12-((Z)-but-2-en-2-yl)-8-((tert-butyldiphenylsilyl)oxy)-3,9-dimethyl-6-((methylthio)methoxy)-2-oxooxacyclo-dodeca-3,9dien-7-yl3-methylbutanoate^{[31]}

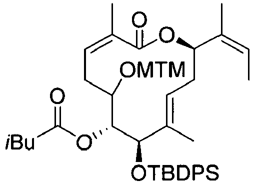

A mixture of (3Z,7R,8R,9E,12R)-12-((Z)-but-2-en-2-yl)-8-((tert-butyldiphenylsilyl)oxy)-7-hydroxy-3,9-dimethyl-6-((methylthio)methoxy)oxacyclododeca-3,9-dien-2-one (29.7 mg, 46.9 µmol, 1.0 eq.), pyridine (19.0 µl, 235 µmol, 5.0 eq.), DMAP (1.15 mg, 9.38 µmol, 0.2 eq.) and 3-methylbutanoyl chloride (28.9 µl, 235 µmol, 5.0 eq.) was stirred overnight at room temperature. The reaction mixture was diluted with CH₂Cl₂, washed with 1 M HCl, saturated NaHCO₃ and H₂O. The combined organic phases were dried (Na₂SO₄) and the solvent was removed under reduced pressure. The residue was purified using column chromatography to give the product (27.9 mg, 44.1 µmol, 94 %)

### (25) Preparation of (3Z,7R,8R,9E,12R)-12-((Z)-but-2-en-2-yl)-8-((tert-butyldiphenylsilyl)oxy)-6-hydroxy-3,9-dimethyl-2-oxooxacyclododeca-3,9-dien-7-yl 3-methylbutanoate^{[32]}

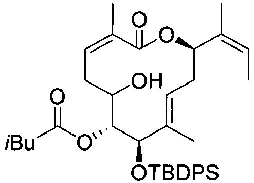

(3Z,7R,8R,9E, 12R)-12-((Z)-but-2-en-2-yl)-8-((tert-butyldiphenylsilyl)oxy)-3,9-dimethyl-6-((methylthio)methoxy)-2-oxooxacyclododeca-3,9-dien-7-yl 3-methylbutanoate (27.9 mg, 44.1 µmol, 1.0 eq.) was dissolved in a THF/H₂O mixture (, 4:1). After addition of 2,6-lutidine (15.4 µl, 132.3 µmol, 3.0 eq.) and AgNO₃ (37.5 mg, 220.5 µmol, 5.0 eq.), the mixture was stirred at room temperature for 45 min. After addition of Et₂O the solution was filtered through Celite. The combined organic phases were washed with saturated aqueous CuSO₄ solution (2x), H₂O (1x) and then dried (K₂CO₃). The residue obtained by removal of the organic solvent was purified using column chromatography, yielding clean product (24.6 mg, 38.8 µmol, 88 %).

### (26) Preparation of (3Z,7S,8R,9E,12R)-12-((Z)-but-2-en-2-yl)-8-((tert-butyldiphenylsilyl)oxy)-3,9-dimethyl-2,6-dioxooxacyclododeca-3,9-dien-7-yl-3-methylbutanoate^{[33]}

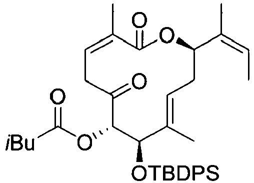

To a solution of oxalyl chloride (51 µl, 73.8 µmol, 2.0 eq.) at -78 °C in dichloromethane (4.4 µl) was added a solution of DMSO (10 µl, 147 µmol, 4.0 eq.) in dichloromethane (45 µl) over 5 min. The reaction mixture was stirred for another 10 min. A solution of (3Z,7R,8R,9E,12R)-12-((Z)-but-2-en-2-yl)-8-((tert-butyldiphenylsilyl)oxy)-6-hydroxy-3,9-dimethyl-2-oxooxacyclododeca-3,9-dien-7-yl 3-methylbutanoate (24.6 mg, 38.8 µmol, 1.0 eq.) in dichloromethane (47 µl) was added to the mixture over 5 min. After 1 hour at -78 °C triethylamine (32 µl, 233 µmol, 6.0 eq.) was added and the solution was allowed to warm up to room temperature. Addition of CH₂Cl₂ (233 µl) was followed by washing with H₂O. The organic phase was dried (MgSO₄) and concentrated. Column chromatography gave the product (23.3 mg, 36.9 µmol, 95 %) in good yield.

### (27) Preparation of (3Z,7S,8R,9E,12R)-12-((Z)-but-2-en-2-yl)-8-hydroxy-3,9-dimethyl-2,6-dioxooxacyclododeca-3,9-dien-7-yl 3-methylbutanoate^{[20]}

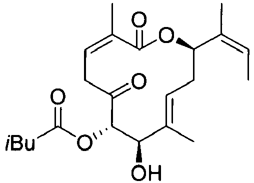

(3Z,7S,8R,9E,12R)-12-((Z)-but-2-en-2-yl)-8-((tert-butyldiphenylsilyl)oxy)-3,9-dimethyl-2,6-dioxooxacyclododeca-3,9-dien-7-yl 3-methylbutanoate (23.3 mg, 36.9 µmοl, 1.0 eq.) was dissolved in THF (1.0 ml) at 0 °C. A 1 M TBAF solution in THF (0.22 ml, 221 µmol, 6.0 eq.) was added dropwise. The reaction was allowed to warm to room temperature over 3 h and was stirred for another 4 h. The reaction was quenched by addition of a saturated NaHCO₃. The aqueous solution was extracted with EtOAc (4x). The combined organic phases were dried (MgSO₄) and concentrated. The product (14.2 mg, 36.2 µmol, 98 %) was obtained by column chromatography of the residue.

### (28) Preparation of (3Z,7S,8R,9E,12R)-8-(((2S,3S,4R,5R)-3,4-bis((acetyl-12-chloranyl)oxy)-5-(((acetyl-12-chloranyl)oxy)methyl)tetrahydrofuran-2-yl)oxy)-12-((Z)-but-2-en-2-yl)-3,9-dimethyl-2,6-dioxooxacyclododeca-3,9-dien-7-yl 3-methylbutanoate^{[34]}

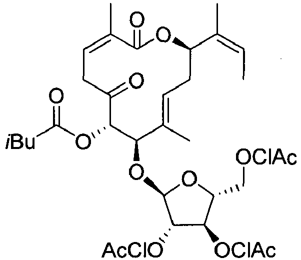

A mixture of (3Z,7S,8R,9E,12R)-12-((Z)-but-2-en-2-yl)-8-hydroxy-3,9-dimethyl-2,6-dioxooxacyclododeca-3,9-dien-7-yl 3-methylbutanoate (14.2 mg, 36.2 µmοl, 1.0 eq.) was dissolved in dichloromethane (0.30 ml), together with molecular sieves 4Å (30.0 mg), Hg(CN)₂ (17.4 mg, 68.8 µmol, 1.9 eq.) and HgBr₂ (6.52 mg, 18.1 µmol, 0.5 eq.). The mixture was stirred for 3 h at room temperature. A solution of (3S,4R,5R)-4-((acetyl-12-chloranyl)oxy)-5-(((acetyl-12-chloranyl)oxy)methyl)-2-bromotetrahydrofuran-3-yl 2-chloroacetate (19.3 mg, 43.4 µmol, 1.2 eq.) in dichloromethane (0.03 ml) was added and subsequently stirred for 4 d at room temperature. The mixture was filtered, washed with aqueous KI solution and H₂O, dried (NaSO₄) and the solvent was removed under reduced pressure. The residue was purified using column chromatography to give the product (17.8 mg, 23.5 µmol, 65 %).

### (29) Preparation of disciformycin^{[35]}

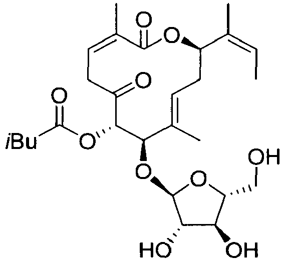

To a solution of (3Z,7S,8R,9E,12R)-8-(((2S,3S,4R,5R)-3,4-bis((acetyl-12-chloranyl)oxy)-5-(((acetyl-12-chloranyl)oxy)methyl)tetrahydrofuran-2-yl)oxy)-12-((Z)-but-2-en-2-yl)-3,9-dimethyl-2,6-dioxooxacyclododeca-3,9-dien-7-yl 3-methylbutanoate (17.8 mg, 23.5 µmol, 1.0 eq.) in THF/H₂O (1.3 ml, 2:1) was added LiOH·H₂O (8.89 mg, 211 µmol, 9.0 eq.) at 0 °C. After stirring at room temperature for 3 h, THF was removed under reduced pressure at ambient temperature. The residue was extracted with EtOAc multiple times, the combined organic phases were dried (MgSO₄) and concentrated. The product (10 mg, 19.1 µmol, 81 %) was obtained after column chromatography.

### Example 6 Biosynthesis and biophysical analysis of gulmirecin A and B

**Production and isolation:** For the production of gulmirecins, *P. fallax* strain HKI 727 (DSM 28991) was cultured at 30°C in Erlenmeyer flasks under oxic conditions with gentle shaking (130 rpm). The broth of a seven day old culture (50 1 total volume) grown in MD1 medium (casitone 0.3% (w/v), CaCl₂ x 2 H₂O 0.07% (w/v), MgSO₄ x 7 H₂O 0.2% (w/v), vitamin B₁₂ 0.00005% (w/v), and 1 ml trace elements solution SL-4 consisting of EDTA 0.05% (w/v), FeSO₄ x 7 H₂O 0.02% (w/v), ZnSO₄ x 7 H₂O 0.001% (w/v), MnCl₂ x 4 H₂O 0.0003% (w/v), H₃BO₃ 0.003% (w/v), CoCl₂ x 6 H₂O 0.020% (w/v), CuCl x 2 H₂O 0.0001% (w/v), NiCl₂ x 6 H₂O 0.0002% (w/v), and Na₂MoO₄ x 2 H₂O 0.0003% (w/v)) was filtered through a cellulose-based filter paper (retention capacity 12-25 µm) in order to remove the cell biomass. The filtrate was extracted three times with equivalent volumes of ethyl acetate. The organic layers were combined and residual water was removed following the addition of anhydrous sodium sulfate (30 g/l) by another filtration step. Subsequently, the organic extract was concentrated under reduced pressure. The residue was dissolved in a small amount of methanol and subjected to flash column chromatography using 45 g of Polygoprep 60-50 C₁₈ (Macherey-Nagel) as a stationary phase. To this end, the octadecyl phase had been suspended in 20% aqueous methanol and filled into a glass column (30 x 3 cm). Elution started with 150 ml of 20% methanol and was continued with the same volume of 40%, 60%, 80%, and 100% methanol. Antimicrobial activity screening against *S*. *aureus* indicated the 80% methanol fraction to contain the bioactive compounds. Furthermore, ¹H NMR analysis revealed the same fraction to feature unique signals in the olefinic range between 5.00 and 6.00 ppm.

Isolation of the gulmirecins was accomplished by two consecutive reverse-phase HPLC steps. The initial separation was conducted on a Nucleodur PFP column (250 x 10 mm, 5 µm; Macherey-Nagel) using a linear gradient of methanol in water + 0.1% trifluoroacetic acid and a flow rate of 2 ml/min: 10% methanol for 3 min, 10% → 100% methanol within 30 min, 100% methanol for 10 min. The gulmirecin-containing fraction was collected between 28 and 32 min post injection. Final purification was achieved on a Nucleodur C₁₈ HTec column (250 x 10 mm, 5 µm; Macherey-Nagel) using an isocratic flow (2 ml/min) of 70% methanol in water + 0.1% trifluoroacetic acid. Under these conditions, **3** had a retention time of 11.5 min and **4** possessed a retention time of 14.0 min. The elution of gulmirecins was detected by wavelength monitoring at 210 nm using a diode array detector. A total of 8.2 mg of gulmirecin A (**3**) and 2.1 mg of gulmirecin B (**4**) were isolated in this way.
**Gulmirecin A** (**3**): [α]²⁵D +98.4 (c 1.0, MeOH); UV (MeOH) λₘₐₓ (log *ε*) 203 (4.28); IR (film) νₘₐₓ 3344, 2938, 1733, 1456, 1374, 1251, 1167, 1074, 1022, 863, 792 cm⁻¹; HRESIMS *m*/*z* 541.2660 [M-H]⁻, calcd 541.2654 for C₂₇H₄₁O₁₁.

**Table 5. NMR spectroscopic data of gulmirecin A (3) in chloroform-dₗ (¹H at 500 MHz, ¹³C at 125 MHz).**

| Pos. | *δ*_{C} | *δ*_{H}, mult. (*J* in Hz) | HMBC | NOESY |
|---|---|---|---|---|
| 1 | 174.3 | | | |
| 2 | 45.1 | 2.76, dq (10.4,6.7) | 1, 3, 15 | 15 |
| 3 | 68.9 | 3.84, ddd (10.4, 4.0, 3.2) | 2, 5 | 4a, 4b, 15 |
| 4 | 44.3 | a: 3.03, dd (20.2, 3.2) | 2, 3, 5 | 3, 4b |
| | | b: 2.79, dd (20.2, 4.0) | 5 | 3, 4a, 7, 15 |
| 5 | 203.5 | | | |
| 6 | 80.4 | 5.01, d (9.2) | 5, 7, 23 | 16 |
| 7 | 83.5 | 4.18, d (9.2) | 6, 8, 9, 16, 18 | 4b, 9, 18 |
| 8 | 133.1 | | | |
| 9 | 129.9 | 5.46, ddd (11.5, 3.3, 1.5) | 7, 16 | 7, 10b, 11 |
| 10 | 31.9 | a: 2.67, dt (14.5, 11.5) | 8, 9, 11, 12 | 10b, 16 |
| | | b: 1.98, m | 8, 9, 12 | 9, 10a, 11 |
| 11 | 70.9 | 5.82, dd (11.5, 1.6) | 1,10,13, 17 | 9, 10b, 17 |
| 12 | 132.9 | | | |
| 13 | 123.6 | 5.35, dq (6.9, 1.6) | 14, 17 | 17 |
| 14 | 13.0 | 1.65, dd (6.9, 1.6) | 12, 13 | |
| 15 | 15.2 | 1.23, d (6.7) | 1, 2, 3 | 2, 3, 4b |
| 16 | 11.4 | 1.70, t (1.5) | 7, 8, 9 | 6, 10a |
| 17 | 18.3 | 1.67, t (1.6) | 11, 12, 13 | 11, 13 |
| 18 | 108.2 | 5.12, s | 7, 20, 21 | 7, 19 |
| 19 | 78.7 | 3.97, d (1.0) | | 18 |
| 20 | 78.0 | 3.98, dd (2.0, 1.0) | 22 | |
| 21 | 87.7 | 4.09, q (2.0) | 20, 22 | 22 |
| 22 | 61.8 | 3.78, dt (11.7, 2.0) | 20, 21 | 21 |
| 23 | 172.6 | | | |
| 24 | 42.8 | 2.26, d (7.1) | 23, 25, 26, 27 | 26, 27 |
| 25 | 25.7 | 2.09, m | 23, 24, 26, 27 | 26, 27 |
| 26 | 22.3 | 0.96, d (6.6) | 24, 25, 27 | 24, 25 |
| 27 | 22.3 | 0.96, d (6.6) | 24, 25, 26 | 24, 25 |

**Gulmirecin B** (**4**): [α]²⁵_{D} +113.5 (c 0.9, MeOH); UV (MeOH) λₘₐₓ (log *ε*) 202 (4.19); IR (film) νₘₐₓ 3306, 2929, 1717, 1652, 1558, 1539, 1506, 1456, 1376, 1175, 1020, 876 cm⁻¹; ¹H NMR (500 Mhz, methanol-*d*₄) δ_{H} [ppm] (*J* [Hz]) 1.08 (3 H, d, *J* 6.9, H-15), 1.63 (3H, d, *J* 1.4, H-16), 1.66 (2H, m, H-3), 1.68 (3H, m, H-17), 1.69 (3H, dd, *J* 7.0, 1.6, H-14), 1.94 (1H, m, H-10b), 2.46 (1H, ddd, *J* 20.5, 10.8, 4.2, H-4b), 2.66 (1H, dt, *J* 14.4, 11.8, H-10a), 2.73 (1H, m, H-2), 2.91 (1H, dt, *J* 20.5, 4.2, H-4a), 3.59 (1H, dd, *J* 11.8, 5.4, H-22b), 3.67 (1H, dd, *J* 11.8, 3.4, H-22a), 3.81 (1H, dd, *J* 5.8, 3.3, H-20), 3.90 (1H, ddd, *J* 5.8, 5.4, 3.4, H-21), 4.02 (1H, d, *J* 8.5, H-7),4.06 (1H, dd, *J* 3.3, 1.3, H-19), 4.07 (1H, d, *J* 8.5, H-6), 5.04 (1H, d, *J* 1.3, H-18), 5.37 (1H, dq*, J* 7.0*,* 1.6, H-13), 5.38 (1H, m, H-9), 5.87 (1H, dd, *J* 11.8, 2.8, H-11); ¹³C NMR (125 MHz, methanol-*d₄*) δ_{C} [ppm] 11.7 (C-16), 13.1 (C-14), 18.4 (C-17), 18.8 (C-15), 28.0 (C-3), 32.6 (C-10), 35.6 (C-4), 39.0 (C-2), 63.0 (C-22), 72.3 (C-11), 78.8 (C-20), 81.7 (C-6), 83.2 (C-19), 86.3 (C-21), 88.7 (C-7), 109.9 (C-18), 123.9 (C-13), 129.3 (C-9), 135.0 (C-12), 136.4 (C-8), 177.8 (C-1), 210.1 (C-5); HRESIMS *m*/*z* 465.2098 [M + Na]⁺, calcd 465.2095 for C₂₂H₃₄O₉Na.

### Example 7 Assessment of antimicrobial activity of gulmirecin A and B

**Agar diffusion assay.** Antimicrobial activities of the gulmirecins were determined in a primary screen against *Bacillus subtilis* ATCC 6633, *Staphylococcus aureus* SG511, *Staphylococcus auricularis* DSM 20609, *Mycobacterium vaccae* IMET 10670, *Pseudomonas aeruginosa* K799/61, *Escherichia coli* SG458, *Sporobolomyces salmonicolor* SBUG 549, *Candida albicans* ATCC14053 and *Penicillium notatum* JP 36. To this end, holes with 7 mm diameter were aseptically punched in the respective agar medium. Subsequently, the agar plates were inoculated with the test organisms. 0.5 mg of every test compound was dissolved in 1 mL of methanol, and 50 µL of this solution was transferred to a single hole. Ciprofloxacin and amphotericin B served as positive controls. After evaporation of the solvent, the agar plates were incubated depending on the growth conditions of the test organisms. A noticeable antimicrobial activity resulted in an inhibition zone of >10 mm. The test results are shown in Table 6 below, wherein the given values represent the diameters of the respective inhibition zone in the agar diffusion assay.

**Table 6. Antimicrobial activity of gulmirecin A (3) and B (4)**

| **Microorganism** | **3** | **4** | **Ciprofloxacin** | **Amphotericin B** |
|---|---|---|---|---|
| *Bacillus subtilis* | 35 | n.a. | 30 | n.a. |
| *Staphylococcus aureus* | 29 | 17 | 19 | n.a. |
| *Staphylococcus auricularis* | 31 | 19 | 20 | n.a. |
| *Mycobacterium vaccae* | 17 | n.a. | 24 | n.a. |
| *Pseudomonas aeruginosa* | n.a. | n.a. | 38 | n.a. |
| *Escherichia coli* | n.a. | n.a. | 34 | n.a. |
| *Sporobolomyces salmonicolor* | n.a. | n.a. | n.a. | 19 |
| *Candida albicans* | n.a. | n.a. | n.a. | 22 |
| *Penicillium notatum* | n.a. | n.a. | n.a. | 18 |
| n.a., no activity observed | | | | |

As demonstrated above, gulmirecin A (**3**) and B (**4**) have an excellent antimicrobial activity against Gram-positive bacteria, and particularly against staphylococci. However, they were inactive against Gram-negative bacteria, including *Escherichia coli* and *Pseudomonas aeruginosa.* Fungi, such as *Candida albicans, Penicillium notatum,* and *Sporobolomyces salmonicolor,* were also not affected by the gulmirecins.

**Bouillon dilution assay.** To determine the MIC₉₀ value of **3**, the test organism was cultured in 500 µl aliquots of trypric soy broth (peptone from casein 1.7% (w/v), peptone from soymeal 0.3 % (w/v), D-(+)-glucose 0.25 % (w/v), NaCl 0.5 % (w/v), K₂HPO₄ 0.25 % (w/v), pH 7.3) at 37 °C for 16 h. Before incubation, **3** was added in decreasing concentrations (100 µg/ml to 12.5 µg/ml). After incubation, the OD₆₀₀ was measured. The experiment was run in triplicate. Ciprofloxacin was used as a positive control.

The MIC₉₀ values of **3** against methicillin-resistant *S. aureus* (MRSA) and *S. auricularis* A were 23.05 µM and 22.48 µM, respectively. Ciprofloxacin, which served as a reference, exhibited an MIC₉₀ of 75.4 µM against the MRSA strain in the same test series.

### Example 8 Assessment of cytotoxic effects of gulmirecin A and B

The cytotoxic activity of gulmirecin A (**3**) and gulmirecin B (**4**) against human cells was evaluated in the well-established MTT assay using primary monocytes from human peripheral blood as well as transformed lung epithelial cell A-549 cell and leukemic Mono Mac 6 cells. Furthermore, **3** and **4** were tested against K-562, HUVEC, and HeLa cells.

**Isolation of primary monocytes, cell lines and growth conditions.** Human primary monocytes were isolated from leukocyte concentrates, obtained from the Institute of Transfusion Medicine at the University Hospital Jena, Germany. The concentrates were prepared from the blood of healthy adult human donors who had not taken any antiinflammatory medication for the 10 days prior to blood donation, as described. In brief, freshly withdrawn peripheral blood was pretreated with citrate-phosphate-dextrose solution as anticoagulant and processed with the 2C+ protocol of the Atreus Whole Blood Processing System (Terum BCT). Peripheral blood mononuclear cells (PBMC) were isolated by dextran sedimentation and centrifugation on LSM 1077 lymphocyte separation medium (PAA Laboratories). For isolation of monocytes, the PBMC were washed twice with ice-cold phosphate buffered saline (PBS) and plated (density 2 × 10⁷ cells/ml) in culture flasks (Greiner Bio-One) containing PBMC medium (RPMI 1640 medium supplemented with 100 U/ml penicillin, 100 µg/ml streptomycin and 2 mM L-glutamine) for 1.5 hours at 37 °C, 5% CO₂. Non-adherent cells were removed; adherent monocytes were scraped, washed with ice-cold PBS and resuspended in ice-cold PBS with a purity of >85%, defined by forward- and side-light scatter properties and detection of the CD14 surface molecule by flow cytometry (BD FACS Calibur). Monocytes were cultured in monocyte medium (RPMI 1640 supplemented with 2% heat-inactivated fetal calf serum (FCS, 10%, v/v), L-glutamine (2 mM), penicillin (100 U/ml), and streptomycin (100 µg/ml)).

The human monocytic cell line Mono Mac 6 was cultured in RPMI 1640 medium supplemented with FCS (10%, v/v), penicillin (100 U/ml), streptomycin (100 µg/ml), insulin (10 µg/ml), oxaloacetic acid (1 mM), sodium pyruvate (1 mM), and 1 x non-essential amino acids at 37 °C and 5% CO₂. A-549 cells (DSM ACC-107) were cultured in DMEM high glucose (4.5 g/l) medium supplemented with FCS (10% v/v), penicillin (100 U/ml) and streptomycin (100 µg/ml) at 37°C in a 5% CO₂ incubator. K-562 (DSM ACC 10) and HeLa cells (DSM ACC 57) were grown in RPMI 1640, whereas cells of HUVEC (ATCC CRL-1730) were cultured in DMEM medium. The respective media were supplemented with ultraglutamine 1 (10 ml/l), gentamicin sulfate (500 µl/l), and FCS (10% v/v).

**MTT assay.** Cells (3 × 10⁵ Mono Mac 6, 1 × 10⁵ A-549 cells, or 2 × 10⁶ monocytes per well) were seeded in a 96-well plate in the respective medium (100 µl/well). Monocytes were allowed to adhere for 1.5 hours (37°C, 5% CO₂) prior to treatment. Test compounds (0.3% DMSO as vehicle) were added to each well and samples were incubated for 48 hrs. Then, 20 µl of thiazolyl blue tetrazolium bromide (MTT, 5 mg/ml PBS) were added, and the incubation was continued at 37°C, 5% CO₂ until blue staining of the vehicle control. Formazan formation was stopped by adding 100 µl of lysis buffer (SDS, 10%, w/v in 20 mM HCl) and samples were shaken overnight. Absorbance of each well was measured at 570 nm in a Multiskan™ microplate spectrophotometer (Thermo Scientific).

Incubation of the cells with either **3** or **4** for 24 or 48 hours caused no reduction in cell viability at the concentrations required for antibacterial activity. In contrast, the reference compound staurosporine was highly cytotoxic for all three cell types.

**Evaluation of antiproliferative and cytotoxic effects.** The test substances were dissolved in methanol before being diluted in DMEM. The adherent cells were harvested at the logarithmic growth phase after soft trypsinization using 0.25% trypsin in PBS containing 0.02% EDTA. For each experiment, approximately 10,000 cells were seeded with 0.1 ml culture medium per well of the 96-well microplates. HeLa cells were pre-incubated for 48 h prior to the addition of the test compounds, which were carefully diluted on the subconfluent monolayers. Incubation was then conducted in a humidified atmosphere at 37°C and 5% CO₂. In case of K-562 cells, the number of viable cells in every well was determined using the CellTiter-Blue1 assay. The adherent HUVEC and HeLa cells were fixed by glutaraldehyde and stained with a 0.05% solution of methylene blue for 15 min. After gently washing, the stain was eluted with 0.2 ml of 0.33 N HCl in the wells. The optical densities were measured at 660 nm in a SUNRISE microplate reader (TECAN).

**3** and **4** did not inhibit the proliferation of K-562, HUVEC, or HeLa cells at a concentration of 100 µM.

### Example 9 Identification and analysis of the gul gene cluster

Retrobiosynthetic analysis of **3** suggests that its polyketide portion is assembled from an acetate starter unit and six polyketide extender molecules, including three malonyl-CoAs and three methylmalonyl-CoAs. Assuming a co-linear biosynthesis, the genome of *P. fallax* HKI 727 (DSM 28991) was screened for gene loci featuring seven PKS modules. The gulmirecin (*gul*) gene cluster was identified taking the substrate specificities of the gate-keeping acyl transferase (AT) domains in every PKS module and the reductive domains into consideration. The *gul* gene cluster includes six PKS genes (*gulA* - *gulF*) having the organization shown in Figure 4.

The oxygen-bearing stereogenic centers of **3** at C-3, C-7 and C-11 are introduced at different extension steps by NADPH-dependent reduction of the respective Claisen products. They are catalyzed by the ketoreductase (KR) domains of the PKS modules, which can be classified into two groups (A-type and B-type) on the basis of their distinct substrate orientation. Since the relative position of the substrate determines the stereochemical outcome of the reduction, the stereospecificity of KR domains can be inferred from their 3D architecture. By using a sequence-based model, a 3*R,* 7*S*, 11*R* configuration for the macrolide that is offloaded from the PKS assembly line was predicted. While the predictions for the chiral centers at C-3 and C-11 already matched the NOE-derived stereochemistry, the discrepancy at C-7 can be rationalized. The 7*S* configuration was predicted under the assumption that the AT domain of GulD selects malonyl-CoA as an extender unit. This means that the hydroxyl group at C-6 is not introduced during the GulD-catalyzed polyketide chain extension, but results from an independent reaction at a later biosynthetic stage. The cytochrome P450 GulG is a likely candidate for the expected hydroxylation due its sequence homology to macrolide carbon hydroxylases, such as EryK. Once the hydroxyl group is installed, the original PKS-derived 7*S* configuration will switch to 7*R*.

The structure of **3** suggests the skipping of two reductive domains in the PKS GulF during its assembly.

### Example 10 Assessment of resistance mutations in S. aureus

The reference strain *S. aureus* N315 (genome accession number NC_002745) has been sequentially exposed to increasing concentrations of disciformycin A (1). Resistant mutants developed at a frequency of ca. 10⁻⁸ as determined by colony-forming units (cfu) count of a defined inoculum treated with the 8x MIC of disciformycin A (1). Finally, ten independent resistant mutants were obtained that grew in the presence of 50 µg/mL disciformycin A (1). These mutants were analyzed by whole-genome sequencing and comparison to the wildtype reference genome. The results are shown in Table 7.

**Table 7. Mutations (amino acid changes) found in Disciformycin-resistant S. aureus N315 mutants.**

| **Mutant #** | **RpoB (β subunit)** | **RpoC (β' subunit)** |
|---|---|---|
| Mt50DscA.1 | Q575R, C593W, R594C | - |
| Mt50DscA.2 | Y507D, **D611N** | - |
| Mt50DscA.3 | - | H785R, T936A |
| Mt50DscA.4 | **D611N** | - |
| Mt50DscA.5 | C593W, R594C | D952N |
| Mt50DscA.6 | **D611N** | - |
| Mt50DscA.8 | Q575R, C593W, R594C | - |
| Mt50DscA.9 | R594C | I763M |
| Mt50DscA.10 | Q575R, C593W, R594C | - |
| Mt50DscA.11 | Y510H, C593W, R594C | - |

All of the mutations mapped to the β and β' subunit of *S*. *aureus* RNA polymerase. Importantly, no cross-resistance with rifampicin was observed. All of the Diciformycin A-resistant mutants were at least 8-fold resistant towards treatment with Disciformycins B-D and Gulmirecins A and B, which most probably exhibit the same mechanism of action. (Table 8).

**Table 8. Susceptibility of Disciformycin A-resistant S. aureus N315 mutants towards disciformycin and gulmirecin derivatives and rifampicin.**

| ***S. aureus* wildtype (WT) and mutants** | **MIC [µg/ml]** | | | | | | |
|---|---|---|---|---|---|---|---|
| | **DscA (1)** | **DscB (2)** | **DscC (5)** | **DscD (6)** | **GlmA (3)** | **GlmB (4)** | **RIF** |
| WT | 4 | 1 | 4 | 0.5 | 4 | 32 | 0.003 |
| Mt50DscA.1 | > 64 | > 64 | > 64 | 32 | > 64 | > 64 | 0.003 |
| Mt50DscA.2 | > 64 | > 64 | n.d. | n.d. | > 64 | > 64 | 0.005 |
| Mt50DscA.3 | > 64 | > 64 | > 64 | 64 | > 64 | > 64 | 0.003 |
| Mt50DscA.4 | > 64 | > 64 | n.d. | n.d. | > 64 | > 64 | 0.001 |
| Mt50DscA.5 | 64 | 16 | 32 | 4 | 64 | > 64 | 0.001 |
| Mt50DscA.6 | > 64 | > 64 | > 64 | 32 | > 64 | > 64 | 0.001 |
| Mt50DscA.8 | > 64 | > 64 | n.d. | n.d. | > 64 | > 64 | 0.001 |
| N315 Mt50DscA.9 | > 64 | > 64 | n.d. | n.d. | > 64 | > 64 | 0.001 |
| Mt50DscA.10 | > 64 | > 64 | n.d. | n.d. | > 64 | > 64 | 0.001 |
| Mt50DscA.11 | > 64 | 32 | n.d. | n.d. | > 64 | > 64 | 0.001 |

| | | | | | | | |
|---|---|---|---|---|---|---|---|
| (Dsc: Disciformycin; Glm: Gulmirecin; RIF: rifampicin; n.d. not determined) | | | | | | | |

### Example 11 Assessment of intra-macrophage activity

The PMA-differentiated human THP-1 cell line (macrophages) was used as cellular host for infection with *S. aureus* Newman at an MOI (multiplicity of infection) of 10. After 2 h, extracellular S. aureus cells were eliminated by lysostaphin treatment and compounds were added for 18 h at their respective 1x, 4x and 8x MIC. Intracellular bacteria were recovered and serial dilutions were streaked out on CASO-Agar for cfu counting. The reference drug rifampicin reduced the number of intracellular bacteria by ca. 4 logs (at 8x MIC). Likewise, disciformycin A and B reduced the number of intacellular bacteria by 3 logs without causing any apparent cytotoxicity (Figure 11).

### References

[1] a) P. K. Agrawal, Phytochemistry 1992, 31, 3307-3330; b) W. A. Bubb, Concepts Magn. Reson. A 2003, 19, 1-19.
[2] G. J. Gerwig, J. P. Kamerling, J.F.G. Vliegenthart Carbohydr. Res. 1978, 62, 349-357
[3] G. Yadav, R. S. Gokhale, D. Mohanty, Journal of molecular biology 2003, 328, 335-363.
[4] A. T. Keatinge-Clay, Chemistry & biology 2007, 14, 898-908.
[5] E. S. Duthie, J. Gen. Microbiol. 1952 7, 320-326.
[6] K. Okonogi, Y. Noji, M. Kondo, A. Imada, T. Yokota, J. Antimicrob. Chemother. 1989, 24, 637-645.
[7] a) D. J. Del Valle, M. J. Krische, J. Am. Chem. Soc. 2013, 135, 10986-10989;
   b) J. G. Duboudin, B. Jousseaume; A. Bonakdar, J. Organomet. Chem. 1979, 168, 227-232.
[8] J. Wennerberg, C. Olofsson, T. Frejd, J. Org. Chem. 1998, 63, 3595-3598.
[9] Y. Nakamura, M. Hirata, E. Kuwano, E. Taniguchi, Biosci. Biotechnol. Biochem. 1998, 62, 1550-1554.
[10] B. Fraser-Reid, P. Ganney, C. Ramamurty, A. M. Gómez, J. C. López, Chemical Communications 2013, 49, 3251-3253.
[11] P. Wipf, P. C. Fritch, J. Org. Chem. 1994, 59, 4875-4886.
[12] U. Vogeli, W. von Philipsborn, Org. Magn. Resonance 1975, 7, 617-627.
[13] T. Frenzel, M. Brünjes, M. Quitschalle, A. Kirschning, Org. Lett. 2006, 6, 135-138.
[14] L. A. Paquette, I. Efremov, J. Am. Chem. Soc. 2001, 123, 4492-4501.
[15] M. Bock, R. Dehn, A. Kirschning, Angew. Chem. Int. Ed. 2008, 47, 9134-9137.
[16] K. Kinoshita, P. G. Williard, C. Khosla, D. E. Cane, J. Am. Chem. Soc. 2001, 123, 2495-2502.
[17] Z. Cai, N. Yongpruksa, M. Harmata, Org. Lett. 2012, 14, 1661-1663.
[18] M. J. Martín, L. Coello, R. Fernández, F. Reyes, A. Rodriguez, C. Murcia, M. Garrazo, C. Mateo, F. Sánchez-Sancho, S. Bueno, C. de Eguilior, A. Francesch, S. Munt, C. Cuevas, J. Am. Chem. Soc. 2013, 135, 10164-10171.
[19] I. Paterson, E. A: Anderson, S. M. Dalby, J. H. Lim, P. Maltas, O. Loiseleur, J. Genovino, C. Moessner, Org. Biomol. Chem. 2012, 10, 5861-5872.
[20] J. D. Frein, R: E. Taylor, D. L. Sackett, Org. Lett. 2009, 11, 3186-3189.
[21] S. Nakamura, J. Inagaki, M. Kudo, T. Sugimoto, K. Obara, M. Nakajima, S. Hashimoto, Tetrahedron 2002, 58, 10353-10374.
[22] A. H. McNeill, E. J. Thomas, Tetrahedron 2011, 67, 257-266.
[23] S. J. Danishefsky, J. J. Masters, W. B. Young, J. T. Link, L. B. Snyder, T. V. Magee, D. K. Jung, R. C. A. Isaacs, W. G. Bornmann, C. A. Alaimo, C. A. Coburn, M. J. Di Grandi, J. Am. Chem. Soc. 1996, 118, 2843-2859.
[24] B. M. Trost, J. P. N. Papillon, T. Nussbaumer, J. Am. Chem. Soc. 2005, 127, 17921-17937.
[25] Y. Wang, M. Kurosu, Tetrahedron 2012, 68, 4797-4804.
[26] R. L. Beingessner, J. A. Farand, L. Barriault, J. Org. Chem. 2010, 75, 6337-6346.
[27] S. Shirokawa, M. Shinoyama, I. Ooi, S. Hosokawa, A. Nakazaki, S. Kobayashi, Org. Lett. 2007, 9, 849-852.
[28] C. Prakash, S. Saleh, I. A. Blair, Tetrahedron Letters 1989, 30, 19-22.
[29] B. Chatterjee, D. Mondal, S. Bera, Tetrahedron Asymetry 2012, 23, 1170-1185.
[30] S. J. Shimshock, R. E. Waltermire, P. DeShong, J. Am. Chem. Soc. 1991, 113, 8791-8796.
[31] J. Esteve, C. Jiménez, J. Nebot, J. Velasco, P. Romea, F. Urpí, Tetrahedron 2011, 67, 6045-6056.
[32] E. J. Corey, M. G. Bock, Tetrahedron Letters 1975, 38, 3269-3270.
[33] N. Yamazaki, C. Kibayashi, J. Am. Chem. Soc. 1989, 111, 1396-1408.
[34] M. Kiso, M. Fujita, M. Tanahashi, Y. Fujishima, Y. Ogawa, A. Hasegawa, Carbohydrate Research 1988, 177, 51-67.
[35] M. Yang, W. Ye, S. W. Schneller, Bioorg. Med. Chem. 2013, 21, 4374-4377.
[36] A. Untergasser, I. Cutcutache, T. Koressaar, J. Ye, B. C. Faircloth, M. Remm, S. G. Rozen, Nucleic acids research 2012, 40, e115.
[37] C. Osswald, G. Zipf, G. Schmidt, J. Maier, H. S. Bernauer, R. Muller, S. C. Wenzel, ACS synthetic biology 2014, 3, 759-772.
[38] A. A. Iniesta, F. Garcia-Heras, J. Abellon-Ruiz, A. Gallego-Garcia, M. Elias-Arnanz, Journal of bacteriology 2012, 194, 5875-5885.
[39] Y. Zhang, J. P. Muyrers, G. Testa, A. F. Stewart, Nature biotechnology 2000, 18, 1314-1317.

The features of the present invention disclosed in the specification, the claims and/or the drawings may both separately and in any combination thereof be material for realizing the invention.

## Claims

1. A compound of the general formula (I): 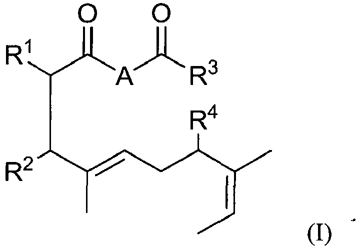 or a pharmacologically acceptable salt thereof, wherein
A represents a group of the formula: 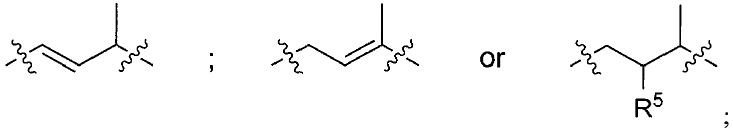
R¹, R², R³ and, if present, R⁵ each independently represents a hydrogen atom; a halogen atom; a hydroxyl group; an amino group; a mercapto group; a C₁-C₁₂ alkyl group; a C₂-C₁₂ alkenyl group; a C₂-C₁₂ alkynyl group; a heteroalkyl group containing 1 to 11 carbon atoms; a C₃-C₁₀ cycloalkyl group, a heterocycloalkyl group containing 3 to 10 ring atoms, a (C₁-C6)alkyl-(C₃-C₇)cycloalkyl group, a(C₁-C6)heteroalkyl-(C₃-C₇)cycloalkyl group, aC₆-C₁₄ aryl group, a heteroaryl group containing 5 to 14 ring atoms, an ar-(C₁-C₆)alkyl group, or a heteroar-(C₁-C₆)alkyl group containing 5 to 10 ring atoms;
R⁴ represents a hydrogen atom; a halogen atom; a hydroxyl group; an amino group; a C₁-C₁₂ alkyl group; or a heteroalkyl group containing 1 to 11 carbon atoms; or
R⁴ and R³ are taken together to form an oxygen or sulphur atom, or a group -NH-.

2. The compound according to claim 1, or a pharmacologically acceptable salt thereof, wherein
R¹, R² and, if present, R⁵ each independently represents a hydrogen atom; a halogen atom; a hydroxyl group; an amino group; a mercapto group; a C₁-C₁₂ alkyl group; a C₂-C₁₂ alkenyl group; a C₂-C₁₂ alkynyl group; a heteroalkyl group containing 1 to 11 carbon atoms; a C₃-C₁₀ cycloalkyl group, a heterocycloalkyl group containing 3 to 10 ring atoms, a (C₁-C6)alkyl-(C₃-C₇)cycloalkyl group, a (C₁-C6)heteroalkyl-(C₃-C₇)cycloalkyl group, a C₆-C₁₄ aryl group, a heteroaryl group containing 5 to 14 ring atoms, an ar-(C₁-C₆)alkyl group, or a heteroar-(C₁-C₆)alkyl group containing 5 to 10 ring atoms; and
R³ and R⁴ are taken together to form an oxygen atom.

3. The compound according to claim 1 or 2, or a pharmacologically acceptable salt thereof, wherein
R¹ and R² each independently represents a hydrogen atom; a halogen atom; a hydroxyl group; a heteroalkyl group containing 1 to 11 carbon atoms; or a heterocycloalkyl group containing 3 to 10 ring atoms.

4. The compound according to any one of claims 1 to 3, or a pharmacologically acceptable salt thereof, wherein
R¹ represents a hydrogen atom; a hydroxyl group or a heteroalkyl group containing 1 to 11 carbon atoms; and
R² represents a hydroxyl group; or a heterocycloalkyl group containing 3 to 10 ring atoms.

5. The compound according to any one of claims 1 to 4, or a pharmacologically acceptable salt thereof, wherein
R¹ represents a heteroalkyl group containing 1 to 11 carbon atoms; and
R² represents a heterocycloalkyl group containing 3 to 10 ring atoms.

6. The compound according to any one of claims 1 to 5, wherein R⁵ represents a hydrogen atom; a halogen atom; a hydroxyl group; an amino group; a C₁-C₁₂ alkyl group; a C₂-C₁₂ alkenyl group; a C₂-C₁₂ alkynyl group; or a heteroalkyl group containing 1 to 11 carbon atoms.

7. The compound according to any one of claims 1 to 6, wherein the compound is selected from the group consisting of:

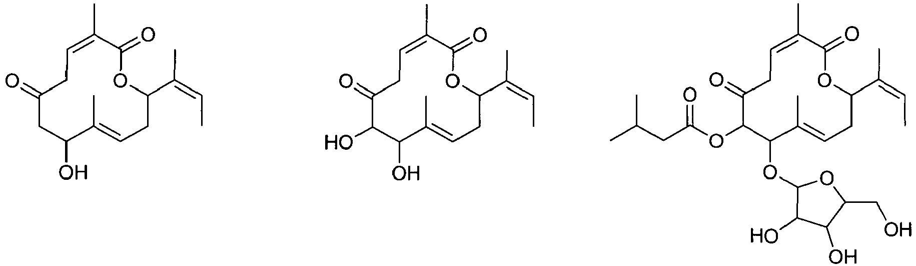

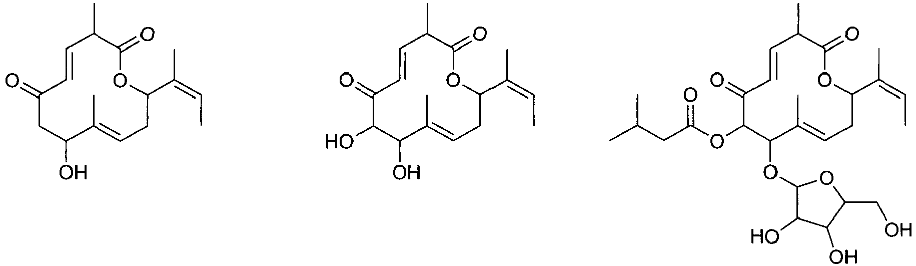

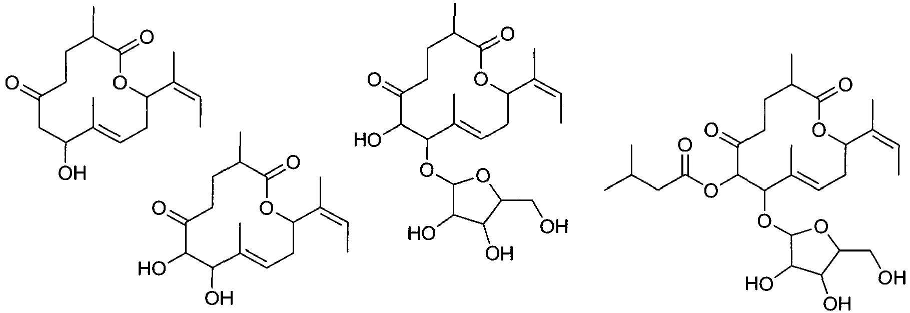

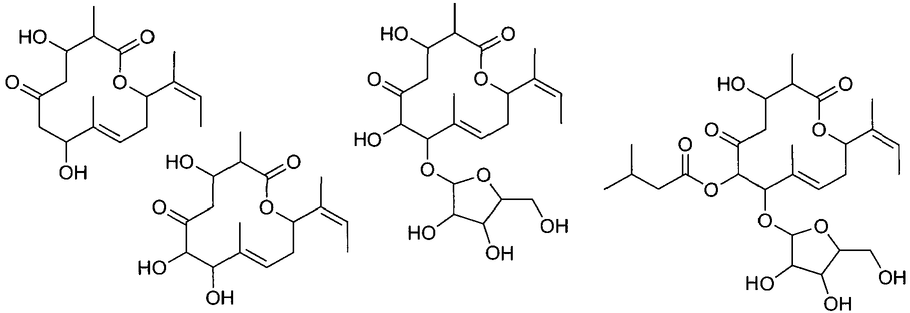

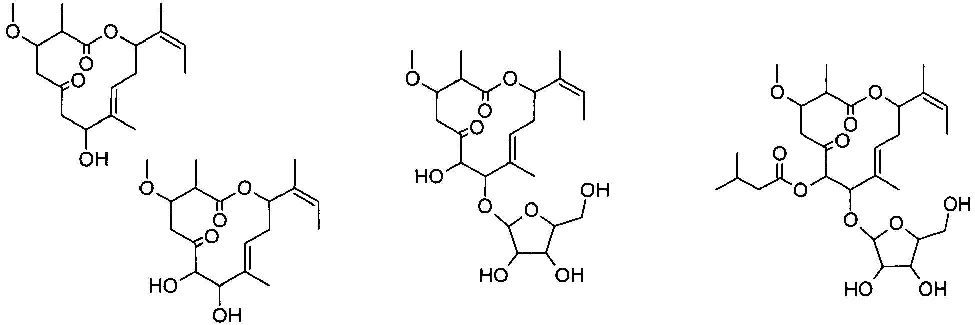

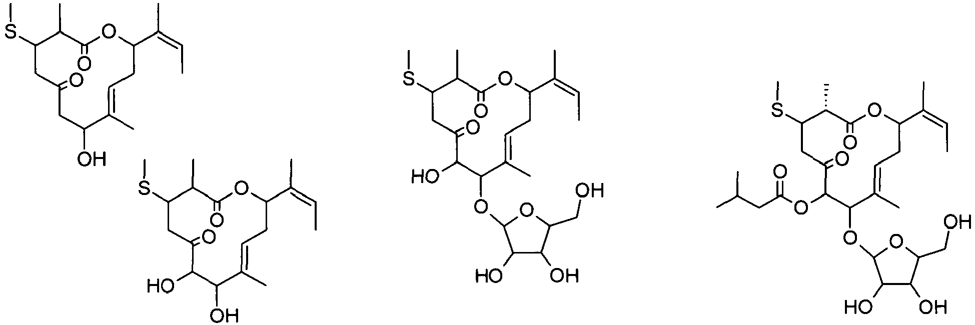

8. A pharmaceutical composition comprising at least one compound according to any one of claims 1 to 7 and, optionally, one or more carrier substance(s), excipient(s) and/or adjuvant(s).

9. A combination preparation containing at least one compound according to any one of claims 1 to 7 and at least one further active pharmaceutical ingredient.

10. The compound according to any one of claims 1 to 7, the pharmaceutical composition of claim 8, or the combination preparation of claim 9 for use as a medicament.

11. The compound according to any one of claims 1 to 7, the pharmaceutical composition of claim 8, or the combination preparation of claim 9 for use in the prevention and/or treatment of a bacterial infection.

12. A recombinant polyketide synthase (PKS) capable of synthesizing a compound according to general formula (I), wherein the PKS comprises at least one polypeptide, or a functional variant thereof, according to any one of SEQ ID NOs. 11 to 19 or SEQ ID NOs. 34 to 46.

13. An isolated, synthetic or recombinant nucleic acid comprising:
(i) a sequence encoding a PKS of the invention, wherein the sequence has a sequence identity to the full-length sequence of SEQ ID NO. 1 or SEQ ID NO. 20 from at least 85%, 90%, 95%, 96%, 97%, 98%, 98.5%, 99%, or 99.5% to 100%;
(ii) a sequence encoding a portion of a PKS of the invention, wherein the sequence has a sequence identity to the full-length sequence of any of SEQ ID NOs. 2 to 10 or SEQ ID NOs. 21 to 33 from at least 85%, 90%, 95%, 96%, 97%, 98%, 98.5%, 99%, or 99.5% to 100%;
(iii) a sequence completely complementary to any nucleic acid sequence of (i) or (ii); or
(iv) a sequence encoding a polypeptide according to any of SEQ ID NOs. 11 to 19 or SEQ ID NOs. 34 to 46.

14. A vector comprising at least one nucleic acid according to claim 13.

15. A host cell comprising at least one nucleic acid according to claim 13 or a vector according to claim 14.

16. A method for the preparation of a compound of formula (I), the method comprising the steps of:
(a) culturing a host cell of claim 15; and
(b) separating and retaining the compound from the culture broth.

## Patentansprüche

1. Verbindung der allgemeinen Formel (I): 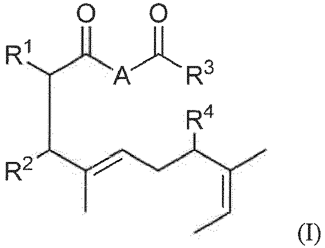 oder ein pharmazeutisch akzeptables Salz derselben, wobei
A eine Gruppe der Formel: 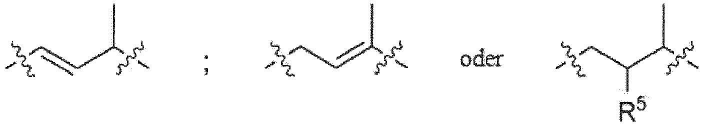 darstellt;
R¹, R², R³ und, sofern vorhanden, R⁵ jeweils unabhängig ein Wasserstoffatom; ein Halogenatom; eine Hydroxylgruppe; eine Aminogruppe; eine Mercaptogruppe; eine C₁-C₁₂ Alkylgruppe; eine C₂-C₁₂ Alkenylgruppe; eine C₂-C₁₂ Alkinylgruppe; eine Heteroalkylgruppe, die 1 bis 11 Kohlenstoffatome enthält; eine C₃-C₁₀ Cycloalkylgruppe, eine Heterocycloalkylgruppe, die 3 bis 10 Ringatome enthält; eine (C₁-C₆)- Alkyl- (C₃-C₇)-cycloalkylgruppe, eine (C₁-C₆)- Heteroalkyl- (C₃-C₇)- cycloalkylgruppe, eine C₆-C₁₄ Arylgruppe, eine Heteroarylgruppe, die 5 bis 14 Ringatome enthält, eine Ar- (C₁-C₆)-alkylgruppe oder eine Heteroar- (C₁-C₆)- alkylgruppe, die 5 bis 10 Ringatome enthält; darstellt;
R⁴ ein Wasserstoffatom; ein Halogenatom; eine Hydroxylgruppe; eine Aminogruppe; eine C₁-C₁₂ Alkylgruppe; oder eine Heteroalkylgruppe, die 1 bis 11 Kohlenstoffatome enthält; darstellt; oder
R⁴ und R³ zusammengefasst sind, um ein Sauerstoff- oder Schwefelatom oder eine Gruppe -NH- zu bilden.

2. Die Verbindung nach Anspruch 1, oder ein pharmazeutisch akzeptables Salz derselben, wobei R¹, R² und, sofern vorhanden, R⁵ jeweils unabhängig ein Wasserstoffatom; ein Halogenatom; eine Hydroxylgruppe; eine Aminogruppe; eine Mercaptogruppe; eine C₁-C₁₂ Alkylgruppe; eine C₂-C₁₂ Alkenylgruppe; eine C₂-C₁₂ Alkinylgruppe; eine Heteroalkylgruppe, die 1 bis 11 Kohlenstoffatome enthält; eine C₃-C₁₀ Cycloalkylgruppe, eine Heterocycloalkylgruppe, die 3 bis 10 Ringatome enthält; eine (C₁-C₆)- Alkyl- (C₃-C₇)-cycloalkylgruppe, eine (C₁-C₆)- Heteroalkyl- (C₃-C₇)- cycloalkylgruppe, eine C₆-C₁₄ Arylgruppe, eine Heteroarylgruppe, die 5 bis 14 Ringatome enthält, eine Ar- (C₁-C₆)-alkylgruppe oder eine Heteroar- (C₁-C₆)- alkylgruppe, die 5 bis 10 Ringatome enthält; darstellt; und
R³ und R⁴ zusammengefasst sind, um ein Sauerstoffatom zu bilden.

3. Die Verbindung nach Anspruch 1 oder 2, oder ein pharmazeutisch akzeptables Salz derselben, wobei R¹ und R² jeweils unabhängig ein Wasserstoffatom; ein Halogenatom; eine Hydroxylgruppe; eine Heteroalkylgruppe, die 1 bis 11 Kohlenstoffatome enthält; oder eine Heterocycloalkylgruppe, die 3 bis 10 Ringatome enthält; darstellt.

4. Die Verbindung nach einem der Ansprüche 1 bis 3, oder ein pharmazeutisch akzeptables Salz derselben, wobei
R¹ ein Wasserstoffatom; eine Hydroxylgruppe; oder eine Heteroalkylgruppe, die 1 bis 11 Kohlenstoffatome enthält; darstellt und
R² eine Heterocycloalkylgruppe, die 3 bis 10 Ringatome enthält; darstellt.

5. Die Verbindung nach einem der Ansprüche 1 bis 4, oder ein pharmazeutisch akzeptables Salz derselben, wobei
R¹ eine Heteroalkylgruppe, die 1 bis 11 Kohlenstoffatome enthält; darstellt und
R² eine Hydroxylgruppe; oder eine Heterocycloalkylgruppe, die 3 bis 10 Ringatome enthält; darstellt.

6. Die Verbindung nach einem der Ansprüche 1 bis 5, oder ein pharmazeutisch akzeptables Salz derselben, wobei R⁵ ein Wasserstoffatom; ein Halogenatom; eine Hydroxylgruppe; eine Aminogruppe; eine C₁-C₁₂ Alkylgruppe; eine C₂-C₁₂ Alkenylgruppe; eine C₂-C₁₂ Alkinylgruppe; oder eine Heteroalkylgruppe, die 1 bis 11 Kohlenstoffatome enthält; darstellt.

7. Die Verbindung nach einem der Ansprüche 1 bis 6, wobei die Verbindung aus der Gruppe ausgewählt ist, die aus: 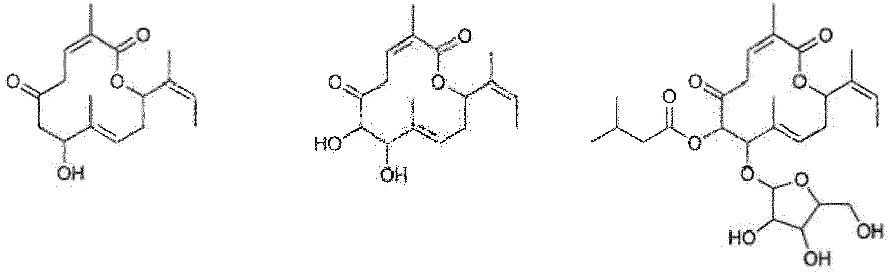 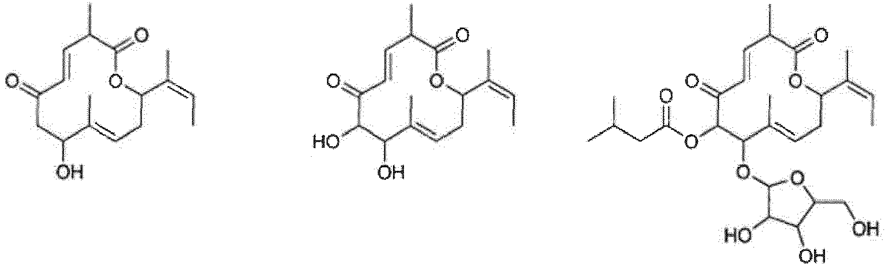 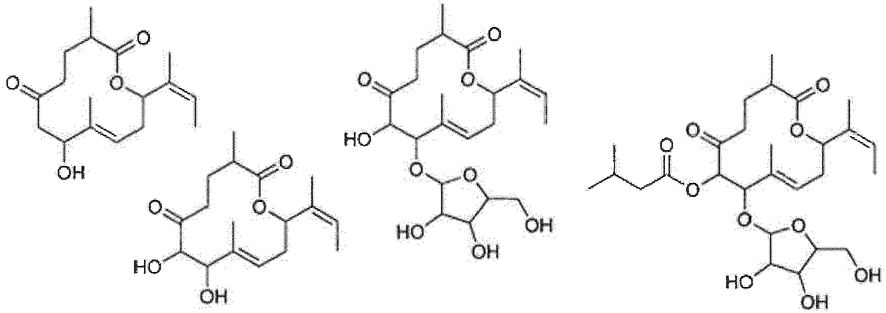 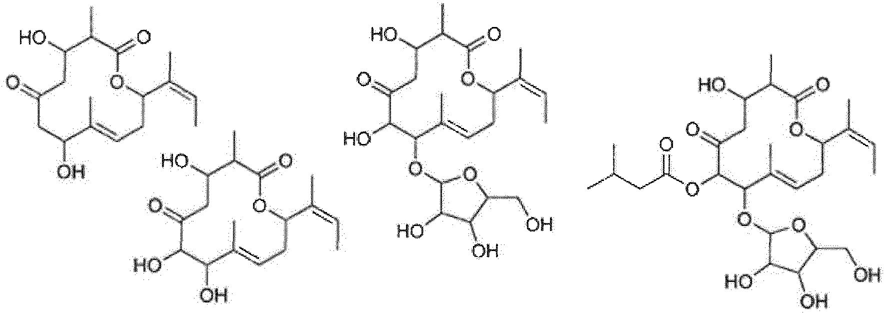 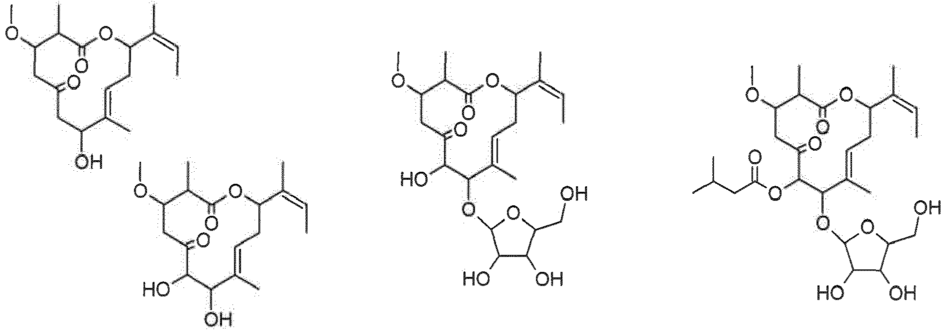 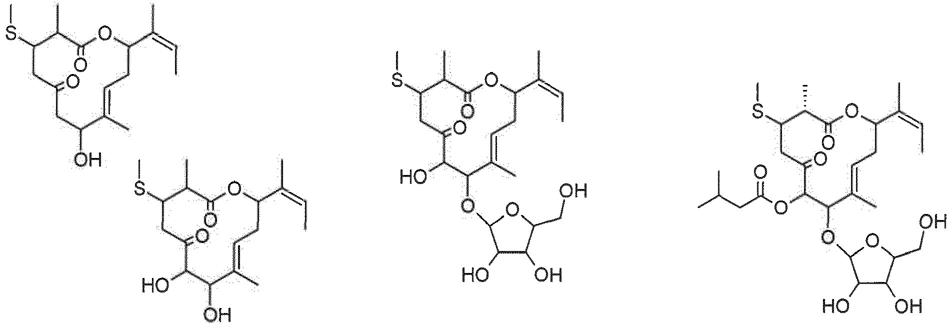 besteht.

8. Pharmazeutische Zusammensetzung, die mindestens eine Verbindung nach einem der Ansprüche 1 bis 7 und gegebenenfalls eine oder mehrere Trägersubstanz(en), (einen) Arzneistoffträger und / oder (einen) Hilfsstoff(e) umfasst.

9. Kombinationspräparat, das mindestens eine Verbindung nach einem der Ansprüche 1 bis 7 und mindestens einen weiteren pharmazeutisch aktiven Wirkstoff enthält.

10. Die Verbindung nach einem der Ansprüche 1 bis 7, die pharmazeutische Zusammensetzung nach Anspruch 8 oder das Kombinationspräparat nach Anspruch 9 zur Verwendung als Medikament.

11. Die Verbindung nach einem der Ansprüche 1 bis 7, die pharmazeutische Zusammensetzung nach Anspruch 8 oder das Kombinationspräparat nach Anspruch 9 zur Verwendung bei der Prävention und / oder Behandlung einer bakteriellen Infektion.

12. Rekombinante Polyketidsynthase (PKS), die in der Lage ist, eine Verbindung gemäß der allgemeinen Formel (I) zu synthetisieren, wobei die PKS mindestens ein Polypeptid, oder eine funktionelle Variante desselben, gemäß einer der SEQ ID NOs 11 bis 19 oder der SEQ ID NOs. 34 bis 46 umfasst.

13. Isolierte, synthetische oder rekombinante Nukleinsäure, welche umfasst:
(i) eine Sequenz, die für eine PKS der Erfindung codiert, wobei die Sequenz eine Sequenzidentität zu der Volllängen- Sequenz von SEQ ID NO. 1 oder SEQ ID NO. 20 von mindestens 85%, 90%, 95%, 96%, 97%, 98%, 98,5%, 99% oder 99,5% bis 100% aufweist;
(ii) eine Sequenz, die für einen Teil einer PKS der Erfindung codiert, wobei die Sequenz eine Sequenzidentität zu der Volllängen- Sequenz von irgendeiner der SEQ ID NOs. 2 bis 10 oder SEQ ID NOs. 21 bis 33 von mindestens 85%, 90%, 95%, 96%, 97%, 98%, 98,5%, 99% oder 99,5% bis 100% aufweist;
(iii) eine Sequenz, die zu irgendeiner Nukleinsäuresequenz gemäß (i) oder (ii) vollständig komplementär ist; oder
(iv) eine Sequenz, die für ein Polypeptid nach einer der SEQ ID NOs 11 bis 19 oder SEQ ID NOs. 34 bis 46 codiert.

14. Vektor, der mindestens eine Nukleinsäure gemäß Anspruch 13 enthält.

15. Wirtszelle, die mindestens eine Nukleinsäure gemäß Anspruch 13 oder einen Vektor gemäß Anspruch 14 umfasst.

16. Verfahren zur Herstellung einer Verbindung der Formel (I), wobei das Verfahren die Schritte umfasst:
(a) das Kultivieren einer Wirtszelle gemäß Anspruch 15; und
(b) das Abtrennen und Gewinnen der Verbindung aus der Kulturmedium.

## Revendications

1. Composé de formule générale (I) : 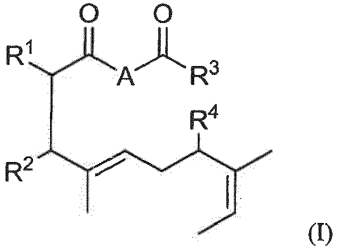 ou l'un de ses sels pharmacologiquement acceptables, dans lequel
A représente un groupe de formule : 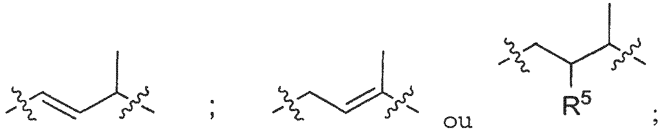
R¹, R², R³ et, si présent, R⁵ représentent chacun indépendamment un atome d'hydrogène ; un atome d'halogène ; un groupe hydroxyle ; un groupe amino ; un groupe mercapto ; un groupe alkyle en C₁ à C₁₂ ; un groupe alcényle en C₂ à C₁₂ ; un groupe alcynyle en C₂ à C₁₂ ; un groupe hétéroalkyle contenant 1 à 11 atomes de carbone ; un groupe cycloalkyle en C₃ à C₁₀, un groupe hétérocycloalkyle contenant 3 à 10 atomes de cycle, un groupe alkyl (en C₁ à C₆) -cycloalkyle (en C₃ à C₇), un groupe hétéroalkyl (en C₁ à C₆)-cycloalkyle (en C₃ à C₇), un groupe aryle en C₆ à C₁₄, un groupe hétéroaryle contenant 5 à 14 atomes de cycle, un groupe ar-alkyle (en C₁ à C₆), ou un groupe hétéroar-alkyle (en C₁ à C₆) contenant 5 à 10 atomes de cycle ;
R⁴ représente un atome d'hydrogène ; un atome d'halogène ; un groupe hydroxyle ; un groupe amino ; un groupe alkyle en C₁ à C₁₂ ; ou un groupe hétéroalkyle contenant 1 à 11 atomes de carbone ; ou
R⁴ et R³ sont pris ensemble pour former un atome d'oxygène ou de soufre, ou un groupe -NH-.

2. Composé selon la revendication 1, ou l'un de ses sels pharmacologiquement acceptables, dans lequel
R¹, R² et, si présent, R⁵ représentent chacun indépendamment un atome d'hydrogène ; un atome d'halogène ; un groupe hydroxyle ; un groupe amino ; un groupe mercapto ; un groupe alkyle en C₁ à C₁₂ ; un groupe alcényle en C₂ à C₁₂ ; un groupe alcynyle en C₂ à C₁₂ ; un groupe hétéroalkyle contenant 1 à 11 atomes de carbone ; un groupe cycloalkyle en C₃ à C₁₀, un groupe hétérocycloalkyle contenant 3 à 10 atomes de cycle, un groupe alkyl (en C₁ à C₆) -cycloalkyle (en C₃ à C₇), un groupe hétéroalkyl (en C₁ à C₆)-cycloalkyle (en C₃ à C₇), un groupe aryle en C₆ à C₁₄, un groupe hétéroaryle contenant 5 à 14 atomes de cycle, un groupe ar-alkyle (en C₁ à C₆), ou un groupe hétéroar-alkyle (en C₁ à C₆) contenant 5 à 10 atomes de cycle ; et
R³ et R⁴ sont pris ensemble pour former un atome d'oxygène.

3. Composé selon la revendication 1 ou 2, ou l'un de ses sels pharmacologiquement acceptables, dans lequel
R¹ et R² représentent chacun indépendamment un atome d'hydrogène ; un atome d'halogène ; un groupe hydroxyle ; un groupe hétéroalkyle contenant 1 à 11 atomes de carbone ; ou un groupe hétérocycloalkyle contenant 3 à 10 atomes de cycle.

4. Composé selon l'une quelconque des revendications 1 à 3, ou l'un de ses sels pharmacologiquement acceptables, dans lequel
R¹ représente un atome d'hydrogène ; un groupe hydroxyle ou un groupe hétéroalkyle contenant 1 à 11 atomes de carbone ; et
R² représente un groupe hydroxyle ; ou un groupe hétérocycloalkyle contenant 3 à 10 atomes de cycle.

5. Composé selon l'une quelconque des revendications 1 à 4, ou l'un de ses sels pharmacologiquement acceptables, dans lequel
R¹ représente un groupe hétéroalkyle contenant 1 à 11 atomes de carbone ; et
R² représente un groupe hétérocycloalkyle contenant 3 à 10 atomes de cycle.

6. Composé selon l'une quelconque des revendications 1 à 5, dans lequel R⁵ représente un atome d'hydrogène ; un atome d'halogène ; un groupe hydroxyle ; un groupe amino ; un groupe alkyle en C₁ à C₁₂ ; un groupe alcényle en C₂ à C₁₂ ; un groupe alcynyle en C₂ à C₁₂ ; ou un groupe hétéroalkyle contenant 1 à 11 atomes de carbone.

7. Composé selon l'une quelconque des revendications 1 à 6, dans lequel le composé est choisi dans le groupe constitué de :

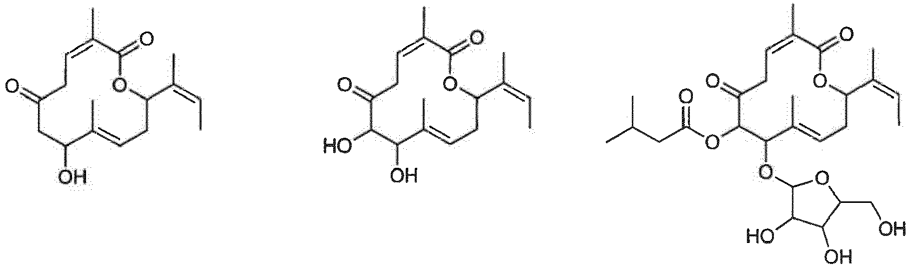

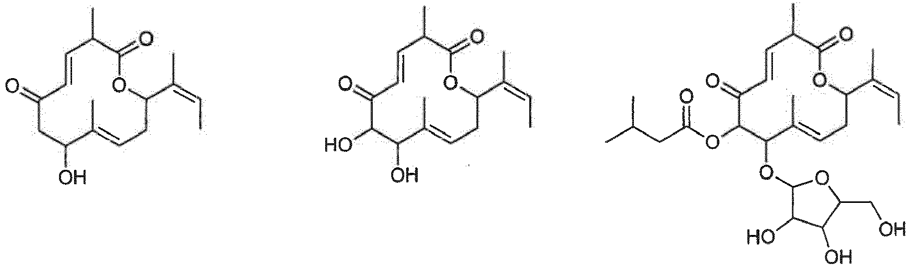

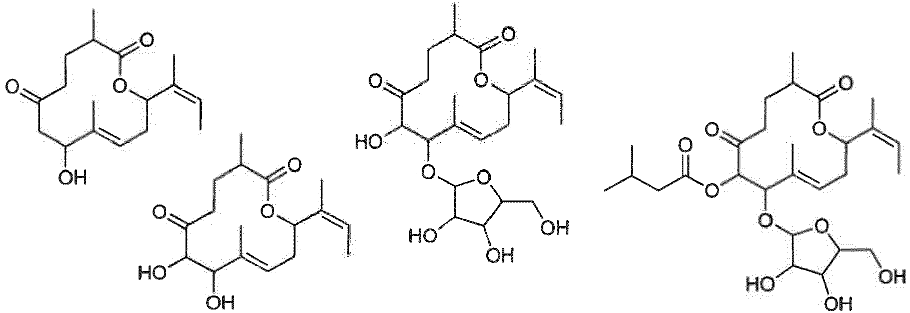

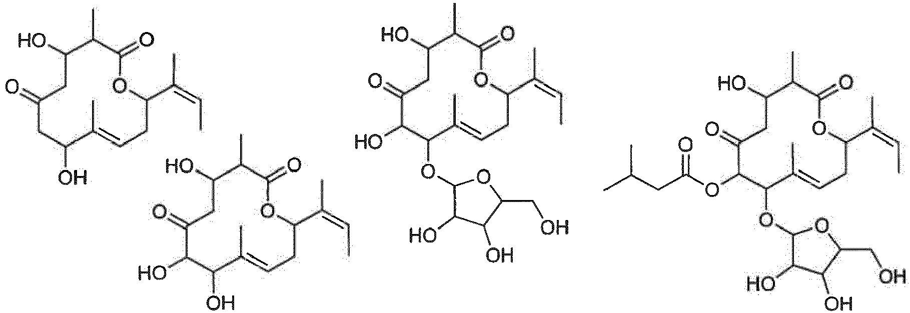

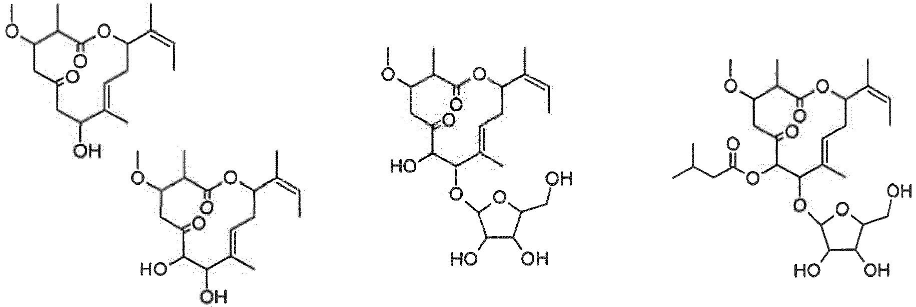

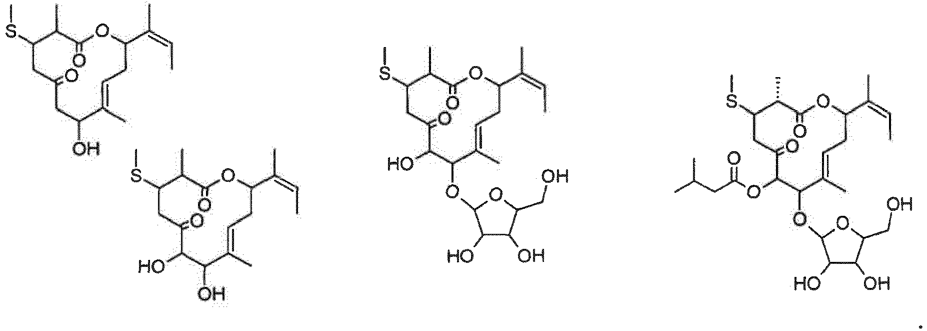

8. Composition pharmaceutique comprenant au moins un composé selon l'une quelconque des revendications 1 à 7 et, éventuellement, une ou plusieurs substance(s) de support, un ou plusieurs excipient(s) et/ou adjuvant(s).

9. Préparation combinée contenant au moins un composé selon l'une quelconque des revendications 1 à 7 et au moins un autre principe actif pharmaceutique.

10. Composé selon l'une quelconque des revendications 1 à 7, composition pharmaceutique selon la revendication 8, ou préparation combinée selon la revendication 9 destiné à être utilisé en tant que médicament.

11. Composé selon l'une quelconque des revendications 1 à 7, composition pharmaceutique selon la revendication 8, ou préparation combinée selon la revendication 9 destiné à être utilisé dans la prévention et/ou le traitement d'une infection bactérienne.

12. Polycétide synthase (PKS) recombinante capable de synthétiser un composé selon la formule générale (I), dans laquelle la PKS comprend au moins un polypeptide, ou l'un de ses variants fonctionnels, selon l'une quelconque de SEQ ID NO : 11 à 19 ou SEQ ID NO : 34 à 46.

13. Acide nucléique synthétique ou recombinant, isolé comprenant :
(i) une séquence codant pour une PKS de l'invention, dans lequel la séquence présente une identité de séquence avec la séquence pleine longueur de SEQ ID NO : 1 ou SEQ ID NO : 20 d'au moins 85 %, 90 %, 95 %, 96 %, 97 %, 98 %, 98,5 %, 99 %, ou 99,5 % à 100 % ;
(ii) une séquence codant pour une partie d'une PKS de l'invention, dans lequel la séquence présente une identité de séquence avec la séquence pleine longueur de l'une quelconque de SEQ ID NO : 2 à 10 ou SEQ ID NO : 21 à 33 d'au moins 85 %, 90 %, 95 %, 96 %, 97 %, 98 %, 98,5 %, 99 %, ou 99,5 % à 100 % ;
(iii) une séquence complètement complémentaire d'une séquence d'acide nucléique quelconque de (i) ou (ii) ; ou
(iv) une séquence codant pour un polypeptide selon l'une quelconque de SEQ ID NO : 11 à 19 ou SEQ ID NO : 34 à 46.

14. Vecteur comprenant au moins un acide nucléique selon la revendication 13.

15. Cellule hôte comprenant au moins un acide nucléique selon la revendication 13 ou un vecteur selon la revendication 14.

16. Procédé pour la préparation d'un composé de formule (I), le procédé comprenant les étapes de :
(a) culture d'une cellule hôte selon la revendication 15 ; et
(b) séparation et conservation du composé issu du bouillon de culture.


### SEQUENCE LISTING

<110> Helmholtz Zentrum fuer Infektionsforschung GmbH Leibniz institut für Naturstoff-Forschung und Infektionsbiologie e.v.-Hans-Knoell-Institut (HKI)
<120> Novel Macrolide Antibiotics
<130> 20676-EP
<140> EP 14002319
   <141> 2014-07-07
<160> 46
<170> PatentIn version 3.5
<210> 1
   <211> 37365
   <212> DNA
   <213> Angiococcus disciformis
<220>
   <221> misc_feature
   <222> (1)..(37365)
   <223> Disciformycin biosynthetic gene cluster
<220>
   <221> misc_feature
   <222> (20206)..(20215)
   <223> n is a, g, c or t
<400> 1
   tcagctggcc cggaacgtca tcggcatgct gaccatcccg tgcagcaggt agcccggcac      60
   gggctccagc tcggccccct ccaccatccg cagctccggc atgcggcgca ggatggccgg     120
   gatggcgata cgcgcctcca ggcggcccag cggagccccc atgcagtagt gggcgccgtg     180
   gccgaacgag acctgacgcg ccgccttgcg gctcacgtcc aggcgctccg gctcggggaa     240
   gacgtcggga tcgtggttgg ccgcgccgag ccacacgatg acgcgctggc cccgcttgag     300
   gagcttgccg ccgacctccg tgtcgtgccg cacccagcgg ggcgcggcga ggaccggtga     360
   gtcgtagcgg agcagctcct cgatggcccc cggcatctgg tccgggttcg cgaggaagcg     420
   cgccatctgg tccgggtgct tcaggagcgc gcggatggcg ttgccaatca tgtgggtggt     480
   ggagatgttg ccggcgatga gcagcagcat gcagaaggcg accaggtcga gctcctccag     540
   cctctcgccg tcgatctccg ccgccaggag gctgctgacg aggtcgtccg agggacggcg     600
   gcggcgctcg gcgatctgct gccgcacgta gtcctccatc ggcgtcagcg cctccgacag     660
   gagcctcacc ccgttctccg gatcgagcac cagggcattg gcccggacgc ccacctcgtc     720
   cgaccagcgc cggaaggact cctggtccga cgccggcacg cccagcatct cggcgatcac     780
   catcgtgggc agggggccgg cgatgtccgc cacgagctcg gtcctccccg tgccgaccac     840
   ggcgtcaatc agctgattga cgatggcggt gatccggtcc tgcatgcggg ccaccgccga     900
   ggccgtgaag gccctcatgg ccaggttgcg cagctgggta tggcggggcg ggtcaaaggc     960
   attcaggctg aagaggcggc gccactcggc ggggggcgcc ccgccatcat cgatggtcgc    1020
   cgtcgggaag ttcacgtggt cggagaggat ggccgtcacg tccgcgtggc ggtagacgga 1080
   ccagaggttg gccccgtcat cgaatgccac cgggtgatgc ctccgcatct cggcgaagaa 1140
   cggatagggg ttcaacagct ccggaccggc cgggaacgta cgcatggtgg ctcttccttc 1200
   cttgtgaact ggaaacgggt ttgcccacgg cttccacctg gtgaaaacca ggggcgggct 1260
   tctcgcgcgt ggatggcggg agaatctcgg gcgtctgatg aaacctgtcg gtcacggcgc 1320
   ggtgatggac agggccccta tggacggctg tatagataag caatagatgg cgcgcaaggg 1380
   ggcgagtcaa gtctcgcgcc gccgtatgaa gtcaaaacag attgcccctg tctcatgaca 1440
   gccgtgtgga cagacacacg cgacagtacg cgcgaggcca cgagcctggg cccgttcggc 1500
   acttctcacg acactggcga cgagcgcagc gaacacacgg ggagtgaaca gcctccgcgg 1560
   gggcgggctg ggccattccc tcgcccgtga cggggcggcc ccgggtccgc gcccggtccg 1620
   ccaggcgacc gccacagcat ccgcatccgc aaccgcaaga ggcggaattt ttacattgtg 1680
   cagtacttca cgtttttact tgacccgccg cattaacccg aaataagcat gaagccagaa 1740
   taatgagggg gcgttcatgt ttgtgatgag tgggatctac tgagaaagca ttcggtgtca 1800
   caggggagac accgatgtcg cagtcattgc cactggaagc agttggcgcc gcgacaccac 1860
   cccgaagcca gttccagcag tccgtccagc gcgctccgcg cggctgtctg ccattggttt 1920
   gttcagaaat ccctgccttc tctaccgaca cgtcgggtga cgtgctcgcc cgcgcggacg 1980
   tgtaacgact ggaagccatg actggaatca tcgacaagct gagccggctc gatgccgcga 2040
   gcaaggacct cctggcggag aagctgcgcc aggccaagga caagcacctg gcgcgagggg 2100
   cgcacgccga gcccatcgcg gtggttggca tggggtgccg ctttcccggt ggcgccagct 2160
   cgcccgagcg cttctggcag ttgctgatgc aggggcggag cggcgtcggc cgcgtccccg 2220
   cggatcgctt ccccatcgag gactactacg accccgaccc gagcgcgccg ggcgccatcc 2280
   tcagcgaccg ggcgcccttc gtggagggta tcgaccgctt cgacgctggc ttcttcggcg 2340
   tgtccccccg cgaggcacgg cgcctggacc ctcagcaccg cctcctcctg gaggtcacct 2400
   gggaggcgct ggagaacgcg ggcctgcccc agtcggcgct ccgcgagctc gttggcggcg 2460
   tgttcatcgg gttgatgggc aacgactact tctccaccgt ccaggagaac cccgccgaca 2520
   tcgacgccta caccagcacg ggcacgcact acagcttcgc ggccaaccgg ctgtcgttcc 2580
   atctggactt caagggcccg agcatggcca tcgactcggc ctgctcgtcg tcgctggtgg 2640
   ccgtccacca ggcctgccag agcctccgga cccgggactg cgacttcgcg gtggccggcg 2700
   gcgtcaacct cgtcctctcc cccgtcctga gcatctcgta ctcgaagtgg gggatgctca 2760
   gccccgacgg acagtgcaag accttcgacg cctccgcgaa cggcttcgtg cgcggcgagg 2820
   gcgccggcgt ggtcgtcctc aagcggctgg aggatgccgt ggcggccggg gaccccatcc 2880
   tcgccgtcgt gaggggctcg gccgtcaacc aggatggcgc caccagcgtc atcaccgcgc 2940
   ccaacgggct cgcccagcag gccgtcatcc gccgcgccct gaaggtggcg ggcgtggagc 3000
   ccgccgccat cggttacgtg gagacccacg ggacggggac gtcgctgggg gaccccatcg 3060
   aggtggaggc cctcaacgag acctacgggc aggagccggg cggccgcccg tgcgtgctcg 3120
   gggccgtcaa gacgaacgtg gggcacctcg agggagcggc cggcatcgcc ggcttcatca 3180
   aggcggtcct cgccgtacag cgcgggcgaa tccccccgaa cctccacttc cacgagctga 3240
   acccgctgat ccggctcgac ggctcgcggc tgtgtctggc cacggaggcc cgggagtggg 3300
   ccgtggcgag ccaggagcgc ttcgccgcca tcagctcgtt cggcgccgga ggcaccaacg 3360
   cccacgtcgt catccagggc ccccccgccg cgcgggcgcc cgcggccacc gggaaggcgc 3420
   ccgccttcgt ctgcctgtcg gcgaagagcg acgcctccct gaagcagcag gccgcgaggc 3480
   tgcgcgcctg gctcgggcag gacggccggg agtggagcgt cgccgacatc gcctccacga 3540
   gcctgcggcg caggaaccac ttcgaccacc gggcggcctt caccgccgac tcgccggagt 3600
   cgctcggtga gggcctggac gccttcctgt ccggccgggc cagcccgcgc tggtccgccg 3660
   gcagcggcac cctgaacgcc cagcggccgg tgttcgtgtt ctccggccag ggctcgcagt 3720
   ggcagggcat gggccgggag ctgctcaccc agagcccgcg cttcgcggcg gagatcgccg 3780
   ccatcgacgc gcggttcatc ccgctggccg gctggtccat cgccgaggtc ctcgcgcggg 3840
   acggcgacgg ggcgcggctg aacgccacgg aggtggcgca gccctgcctg ttcgccctgc 3900
   agctggggct cgtcgccgcg ctggacgagc tgggcatccg gcccgccgcg gtcgtcgggc 3960
   acagcctcgg cgagctcgcc gcggcgtgcg tggccggcct catggaccgc gaggccgcca 4020
   cggccctcgt gtaccaccgc gcgaagctca tgcagcggct gaccggcgcg gggaagatgg 4080
   ccgccgtcgg gctctcccgc gaggaggccg aggcacgcct cgcggcggag ccccggctgg 4140
   cgctcgcggc ggtgaacggg ccccgctcgg tcgtcgtggc cggtgagccg gaggccgtgg 4200
   acgccttcgt gcgggcgctg cgcgacgagg gagtcttcac gcggacgctg ccggtcgact 4260
   acgccttcca cagcccccag acggacccgc tcctggccga gctgcggcgg gccgtcgacg 4320
   gcatcttcgg cgcgggcgcg aggatggccg ccccccggct gccgatggtg tccacggtga 4380
   gcggcgccga gctcgatgcc tccgtcgacc acgccaccta ctgggcccgg aacatgcggc 4440
   agacggtgtg cttcgaggag gcggtgggcc agctgctgga gcagcggcac gacgccttcc 4500
   tggaggttgg cgcgcacccg gtcctgaacg tcgacctgcg ggagtcgttc cgcgcgcggg 4560
   aggtgacggc gcgggccgtc gccaccctcc ggagggagca gccccaggcc ctctgcctgc 4620
   gggcggtgcc cgccgcgctc tacgccgccg gcatggcccc cgaggcgctc cccggccagg 4680
   accagggcac cctcgtccca ctcccgaact acgcgtggga ccaccgccgc tactggattc 4740
   actccgcctc gcgggccgag gcgggctggg tctacgacct cgcctgggac gcggtgccgg 4800
   cacctgcgcc gggcgcccac cggcccgcgt cctggctcgt cctgggcgcc tccggcgggc 4860
   tgccggaggc gctggccgcc cggctgcgcg ccacgcccga cctggtgcgc cacgtgagcc 4920
   tggaggcgct gcgctccgtg cccgtgcccc cccacgggct ggtggacgcg gagcggctcg 4980
   agcgctggtt cacggacacg ctccaggtgg acctggcgga gttcgacttc ctcgtctacg 5040
   tgcccgcgtg cggcgccccc gaggacgcga gcgccgagcg ccaggtcagc gaggactgcg 5100
   ccatcctggt gagcctggcc caggccctgg agaggcggcc cggggcccgg ggcccggcgc 5160
   tctgggtcgt cacgagcggc gcgcaggccg tcgcgccgcg ggagcccgtc aatcccttcg 5220
   ccgcgcccgt gtggggcctc ggccgggtgc tcgccctgga ggccccgcga ggcctgggcg 5280
   gtctcgtcga cctgccgctc gcgtggtcgg agcaggacgt gggctgcctg ctcgaggcgc 5340
   tgcgccaccc cggcgaggag gaccagctcg cgctgcgctc gggccagcgc catgccctcc 5400
   ggctgcgccg ccgggcgggc cgcgccaccg acgcgggcgc ggcctggcag gtcgaggact 5460
   ccggcgtcta cctcatcacc ggtgggacgc ggggcctggg gctgctcgtg gcgaagcggc 5520
   tggccgagcg cggcgcccgt cacctggcgc tgctgggccg ccgggcgctg ccgccccgct 5580
   cgcggtgggc gacgctggcc gcggacgacg ccttcttccc ctccgtgcag gccatccagg 5640
   cgctggaggc ccagggcgtc accgtcacgc tccacgccgc cgacgtcagc gactcccacg 5700
   cgatgcggga cgtgttcgcg gagctcgagc ggcagcagcg ccccctcaag ggcgtcgtgc 5760
   acgcggcggg tatctcccgg gaggtccccc tggcctccat cgacgaggcg ctgctggagg 5820
   agaccctggc acccaaggtc gcgggcacct ggtggcttca ccagctctcg ctgcggcacc 5880
   cgctggactt cttcgtcgtc ttctcttcgg gggcctccgt ctggggctcc gcgggcctgg 5940
   cgccgtacgc cgccgcgaac gagttcctga acgccgtggc ggcccaccgc cgcgcctccg 6000
   gcctgcccgc cctcagcgtc tgctggggca tgtgggacgc ggacggcatg gccaccgacg 6060
   agatgcggga gaactggacg cgcctgggcg tcaaggcgat ggcccccgag cgcgccctca 6120
   acgcgctgga ggagctggtg gccgcggacg ccaccctcgc caccgtggcc gacgtgaagt 6180
   gggcccgctt ccggcccgtc tacgagctga agcgcaagcg tccgctgctg gcggcgctcg 6240
   aggacggcgc ggcggcaccg ccgcggcccg aggcggccgg ggcgccccag gccacctccc 6300
   tcaaggcgga cctccaggcg ctgccgcggg agcagcagcg cgagcggctg ctggccaccc 6360
   tccgcgacac ggccgcgctc gtgctcggcg ccgagccggg tgccgccatc aacccggacg 6420
   aggggctgtt ctcgatgggc ttcgactccg tcacggccgt cgagttcgcg gcgcacctga 6480
   gccgcctgtt cgagaagcgc tacgccgcga cgctggtctt cgaccacccc tccctcaagc 6540
   agatccgcga cttcctcatg caggactgct tcggctggac ggccgagccg tcccccgagc 6600
   ccgaggcggg tgacggccag gacctcgacg cgctcctcgc cttcgtcgag ggcgtggacg 6660
   aggccgaggc cgagcggctg ctctccgccg ggcagccccg gtcgaactga gccctccagc 6720
   gacgcgcgag acgtgccatg acctccgtcg acgaacgcat caagaatctc tccccgcaga 6780
   agcgcgccct gctcgagcag cggatgaacg ccgccgccgg cgccacccgg gacgcggagc 6840
   ccatcgccat catcgggatt ggctgccgct acccgggggg cgtgaaggac ctgaagagct 6900
   tcgcccagct gctgcgcgac ggcgccgacg ccaccgggga catccccgcg gaccgctggg 6960
   acgtggaggc ccactactcg cccgacccgg gcacccccgg gaagatgcgc acccggcgcg 7020
   gcggcttcct gcccgacgtg aagaccttcg acgcggactt cttcggcatc tctccgagag 7080
   aggcggagag catggacccc cagcagcggc tgctgctgga ggtgtcctac gaggccctcg 7140
   aggacgcggg gattcccacc gcctcgctga gcgggagcac caccggcgtc tacatcggta 7200
   tcaccctggc ggagtacgcc gaccggctgc cggacgaccc ccgcctcatc gacgtctact 7260
   cggggacggg cacctacctg aacgtggccg ccgggcgcct gtcctacgtc tacggcctcc 7320
   agggcccctg catggccgtg gacgcggcgt gcgcctcctc gctggtgacg gtccacctgg 7380
   cggcccaggc cctgcggcac cgcgagtgcg gcctggcgct ggcgggcggc gtgagcctga 7440
   tgctgtcgcc ggagaccacc atctacctgt cgcagacggg cgccctggcc cccgacggcc 7500
   gctgcaagac gttcgacgct cgcgccaacg gctacggccg cgccgagggg tgcggcatcc 7560
   tcgtcctcaa gcggctgtct gatgcgctgc gcgacaagga ccgcatccac gcgctcatcc 7620
   gcggcacggc ggtcagccag gacggcgcgt ccagcggcct gacggtgccc aatggccgcg 7680
   cccagcagaa ggtcatccag tccgcgctca aggacgcggc ggtgcggagc gaggacgtct 7740
   cgtacgtcga ggcccacggc acgggcacgc cgctcgggga cccgatagag ctcaacgccc 7800
   tccaggccgt ctacgggcag cgcgaggcgg actggccgct ccacgtcggc tccctgaaga 7860
   cgaacttcgg gcacgccgag gccgcggcgg gcgtgggcgg catcatcaag acggtggtgg 7920
   cgctccagga gaggcagatc cccgcccacc tccacttcga gaagctcaac ccgctcatca 7980
   ccatcgaccc cacgcgcgtc gtcatcccca cccggcccac gccgtggcaa acgcgggggg 8040
   agcggaagcg gctcgccggc gtcagctcgt tcgggttcag cggcgtgatt gcccacgccg 8100
   tcctggagga ggcgcccgcg ccggaagcgg ccgtcgagca gccggcgatg cccgccgggg 8160
   agcggccgct gcacgtcctg ccgctcaccg cgaagcaccc ccgggcgctg agcgagctca 8220
   tctcggccta ccggcacacc gtcggcgcgc tcgacgcggg ccggctcgcc gacctgggct 8280
   ataccgcgag ccggaagcgc ggccaccacg cgcaccgcgc ctggttcgcc ttccgcgaca 8340
   aggaggagct gctccagcag ctcgaggccg cgacccgggg ccacgagctg cccggcgccg 8400
   tgctccagcg gcaggcgcgc gacgacgtca aggtcgtcat ggtgttcccc ggccagggct 8460
   cccagcgggt cggcatgggc cagcagttga tgcggacctc cccggtgttc cgcgaggcca 8520
   tccacgagct ggaccgcgcg atgcggccct gggcggactt ctcgctgctg gaggtgctcg 8580
   gcgcccgcgg gggcgaggac cccttcaccc gcgtctccat cgtccagccg accctcttcg 8640
   ccgtccaggt ggccctggcc aggcagtggg aggcctgggg cgtcaggccg cacgcggtca 8700
   tcggtcacag catgggcgag gtcgcggccg cgtgcgtcgc gggcgcgctc agcatggagg 8760
   acgccgccgc ggtcatctgc cgccgcagcc agctcgtcca ggccaccagc ggccagggcg 8820
   ccatggcgct cgtcgagctg agccccgccg aggtcgaggc cgccatcgcg ccccacgggg 8880
   accgcgtcag catcgccgcg tacaacgggc cctccaccgt cctgctgtcg ggcgagccgg 8940
   ccgccgtcga cgaggtgctc accgccctcc aggagcgcgg ggcgttctgc cgccgcgtca 9000
   aggtggacta cgcctcgcac tgcgcgcaga tggacccgct gctgggccgc atccggagcg 9060
   agctggccca cgtgaagccg cgcaagggcg gcgtgcccat ctattcgacc gcgcgcggcg 9120
   aggtcatcga cggctcgcgg atggacgcgg gctactgggc ggacaacctg cggcgccccg 9180
   tcctcttcca cgcgcaggtc aaggcgctcc tcgcggacgg catgcacgcg ctgctggaga 9240
   tcagcccgca cccggtgctc ctgccgtcga tgcagaagac gatagacgag gccggcggac 9300
   agcagctcgt gctgcccagc ctgcgcaggg acgaggacga gccgcggagc ctcgcgacgt 9360
   cgctgggcgc cctcttcggc ctcggccacc cggtggactt ctccgccttc accccgaggt 9420
   ccggccggct cgtcgagacg ccgcgctacc cctggcagcg gcgcgagttc tggctgacgc 9480
   ggaaggcgtc gaagcgccgg cacgcgctgg gcggcgcgga gggacatccc ctcatcggtg 9540
   gcgccttccg cccggccgtg ggcccggagt cctactactg ggagttcgac gtcgacgagg 9600
   agcgcctcgc cttcatcgcc gaccaccgcg tgcgcggcat gacggtgctg ccggggacgg 9660
   cgtacctgga cctcgcgctc tcggcggcgc gggccgcctt cggcgacagg gccttccccc 9720
   tcagccaggt ctcgttccgg gaggcgctga ccctgctgcc cgagcagccg cggcgggtgc 9780
   agctctcgct cgtccgggag gcggagggcg cgttctccgt ctgcgtctcc agccgcgacg 9840
   cggcgtcccc ggccaccggg gagtggaccc tccacgcgac gatgcgactg gacgtcgagg 9900
   ccccggccac gggaggcgct cgcgccgagg gacaggtgac gccccccgcg ggcgccgagc 9960
   tccgggacgc caggacgcac taccgcctca tgaaggaggg cggcctcgcc tacggcccgc 10020
   gcttccaggg gctggcggag gtccactgcg gccagcacga ggccctgggg cgcctgcgcg 10080
   cgctgccgga gctcgggctc gacggcgcgg accacgtctt ccaccccgcc ctctgggacg 10140
   ccgcgcacca ggcgctggcg gccctgctcg gccgccagga cggccacgcc tccggcccgt 10200
   ccacctggat tccgcgcggc atcgagcggg tctcggtgtc cggcaccgtc ccggcccggg 10260
   aggcgctgtg ggtccgcgcc agcgcgcggg tggacgcgga cggcgccacg gcgcggggag 10320
   acctccaggt cctggatgac gccgggcggg tgctggtcga gacgcgcggg ctgacgctgc 10380
   tccggcgcga cgcggacaac ctcgacgacg tccgccagtg gttcttccgg ccggtgctcc 10440
   gcgaggcccc gctccgggag gaagcccccg cggacgggac gtggctcgtc ctcggcgcgc 10500
   ccgggcccct gcatgaccgg gtggtggcgg agctgcgcgg gcgcggcctc gacaccgtgc 10560
   aggcgttccc ggccaatacc tgggaggagc aggacgcggc gcgcttccac ctctcccccg 10620
   cggaccccga gcacctgcgc cggctgatgg agcgggtcag cgccgaccgt ccgctgcgcg 10680
   gcctcatcaa cctccagccg taccaggtgg cgcgcggcga cgcggcctcc tcggcgccgg 10740
   agggcgtgct gtccgccctc tacatggagg tgctcgacca ggcccgggcg ctccagggcg 10800
   tgagccggcg ggagcggccg aagctggtgc tcgtctcggg cgtctcccag ggcgcggagg 10860
   ccgagcccga cttggtcgcc gccccgctcc agggcctggg ccgcgtcatc gcggccgagc 10920
   tgccccggct ccactgcgtc caggtccagc tcggaggtca ggagcccgag gtggaggcgc 10980
   gcgacctcgt gcgagagctg tacgccaacg acggcgagga cgaggtcgtg ctgcgcgggg 11040
   gcgctcgcca cgtgcgccgg ctcgagcggc tcgtccccgg cgcggccccg gcggcgccca 11100
   tccacgcgga cggaacctac ctgctcacgg gcggcctggg cggcctgggg ctggccttcg 11160
   cgagctggct cgcggaccgc ggcgcgcggc acctggccct ggtcagccgg cgcccgccct 11220
   cgggggaggc ccgcgcgcgg ctcgacgcgc tccgcgcccg gggcgtcgag gtgctcgtgc 11280
   gcgaggtgga cctgtcccgg gaggactccg tcgccgccct cttccgggag ctggacgagc 11340
   gcatgccgcc cctgcggggc gtcctccacc tggcgggcgt cctggacgac gcgacgctgt 11400
   tccagcagaa cgagcagcgc ctcgcgaccg tgcgcggccc caaggtcacc gggacgcggc 11460
   tcctgcacgc gcacacccag caccggaagc tggacttctt cgtcctcttc tcggcgggcg 11520
   ccaccctcct cggctcgccg ggccaggcga actacgccgc ggtgaacgcg ttcatggatg 11580
   cgttcgcccc gcaccgccag cggctggggc tgcccgcgct ctccatcaac tggggcgtct 11640
   gggacgaggt cggcctcgcc gcggcgagcg cgcagcgagg ccagcgcctg cgggagcagg 11700
   ggctgttccc catccccccc gcgcagggcc tccaggcctt cgagctggcg ctgtcgctcg 11760
   cgggcgccaa cgtcgccatc ctgcccatcc gcctcgcccg ctggctgcgc gagcacccgg 11820
   acctggcggg cgcgtcgctg ttcaaggagc tggtccgcgg actggacgac tccgaggcgg 11880
   aggccgaggc gcccatcctg gagcagctgc gcgccacggt ggacccgcat gagcagcgcg 11940
   agtgcctgcg ccgctgggtc gggcgccagc tcgcggcggt gctgcgaagg gaccccgcgt 12000
   ccatgcagcg cgagcagaac ttcagcgcca tgggcgtcga ctcgctgatg gcgctcgagt 12060
   tccgcaaccg gctggagcgc tcgctccacg tccggctgcc gccgacgatg gtcttcaacc 12120
   atccgagcat cgaggcgctc tgcgggttcc tcgagccgaa gctgggcatg ggcgccccct 12180
   ccgctccggc gcagccggag cccgtgcctg ctcccgccgc gcccgtgcgc gtcgacgaga 12240
   aggacggcgc cgcgctgctc ggggccctgg cccggctgaa gaagctcacc cagcaccagg 12300
   cgtagttcga gccacctatc aatcctctgg agacgcaggc cgatgtcgat gttggatgag 12360
   ctcagggcag tgctggacag tctgtcgccg gaacagcggg cgacgctcgc ggagctgctg 12420
   agcgctgacg cgccccggaa ggccgacgag ccgatcgcca tcgtcggcat ggccggacgc 12480
   ttccccggag cacgcgacct ggcggagttc tggagcgtgc tcgccgaggg gcgggacgcc 12540
   gtctccgaga ttccaccgga gcgctggaac gcggacgccc tctatgaccc ggacaaggac 12600
   gtcccgggca agctggccac ccgctggggc ggcttcgtga aggacgtcga cctgttcgac 12660
   gcggagttct tcggcatcac tccgcgcgag gcggcggcca tggacccgca gcagcgcctg 12720
   ttcctcgaga cggcctggga ggccctcgag gacgcggggc tgcccgcggc ctccctcgcc 12780
   cggacgcaga cgggcgtctt cgtcggagtc tccaactccg actacatgtg gcaccagttc 12840
   ccccggctcg acaaggtgga cgcgtacgcg agctccgggt ccgcgcactg catcatcgcc 12900
   aaccggctgt cctacctgct ggacctcacc ggccccagcg tggcggtgga caccgcgtgc 12960
   tcctcgtcgc tggtgtcggt gcacctggcc tgccagagcc tgcgcaaccg ggagtgcgag 13020
   gcggcgattg ccggcggagt gaacgtcgtc ctgacgccgc acctgggggt gagcctctcc 13080
   aagtggggga tgatggcgtc ggacggccgc tgcaagacgt tcgacgcccg cgcggacggc 13140
   ttcgtccggg gcgagggcag cggcgtgctc gtgctcaagc ggctggcgga cgccgagaag 13200
   gcgggggacc gcgtcctggc cgtcatcctc ggcagcgccg tcaaccagga cgggaagacc 13260
   cacggcctga cggcgccgaa cggcgccgcc cagcaggccg tcatccggca ggcgctggcc 13320
   cgcgcggggc tgtctccctc gcaggtgtcc ttcctggaga cccacgggac ggggacgtcc 13380
   ctgggcgacc ccatcgaggt cgaggccatc aacgaggtgt tcggctccag ggtcgagcgc 13440
   tgcgtgctgg gcgcggtgaa gaccaacatc gggcacctgg agccggcggc aggtatcgcc 13500
   ggcctcatca aggtcgtgct cgcgctgcgc cacgggctca tcccgcgcaa cctgcactac 13560
   cagtccatga acccgcacct ccagctcgct ccgggcatcg agctggcgag cgaggcccgg 13620
   ccgtggaagc gcgacgccag gccccgcttc gccggggtga gcgccttcag cttcggcggc 13680
   accaacgcgc acatcgtcct cggagacgcg cccgcctacg cgccgccggc cgagtccgcg 13740
   cggcccgccc tgcccaccct cctccccctc tccgcgagcc aggagggggc gcggagccag 13800
   atggccgcgc tccacgccgg gctgctcgcc accgcggccc ccgagctcac ggtggacacc 13860
   gtggcgggac tgcgcgcgct gcaccgggac cacctggacc tgcgcctggc cgtcgtcgcc 13920
   tcggaccgcg cgacggccgc cgaggccctg aggtgcttcg ccgaggggcg gccccacgcg 13980
   ggcctggtcc acgggcgccg ccagccgaac ctgcggcccg tcttcatcta caccggccag 14040
   ggcaaccagt ggccgggcat gggccgcgag ctgatgcgca ccgagccggt gttcagggcc 14100
   gcggtggagg aggcggacgc cgcgttccag cgcctcacgg ggcggagcca cgccgccagc 14160
   ttccgcgagg acgcgtccga cgcggcgtcg ctggagcgca ccgacgccgc ccagccggcc 14220
   atcttcctcc tgcagtacgc gctcacgaag acgctggagc actggggcct gcgtcccggc 14280
   gcggtcgcgg gccacagcgt cggggaggtc gccgccgcct gggccgcggg catgctggac 14340
   ctggacgacg cggtccgggt ggtccaccac cgctcgcggc tcatgcagcg cgccaccggc 14400
   cagggggcga tggcctccgt cgcgctgtcg ccacgggagg cgcggcccct cctggagggg 14460
   cgcccctcgc tggccatcgc ggcgcacaac agccccgggg aggtggtgct ctcgggtgac 14520
   cccgccgagc tcgaggccgt gctccgcgag ctggaggggc gcggcgtccg ctgcaggaag 14580
   ctccccgtca actacgcctt ccacggcccg cagatggacc cgctccgcga cgagctgctg 14640
   cgggagctgt ccgtcctccg gccgaaggcg ccgcgcctgg cgttctactc gacggtgacg 14700
   ggccagcgcg agcggcaggc cgtggccgac gcgagccact gggtccgcaa catgagggac 14760
   cccgtcgcgt tccgcgaggt cgtcgagcgg ctcgcgaccg acagcccgag cttcttcctc 14820
   gagctcggcc cgcacccggc cctcgtgccc tcggtgatgg acacgctggc gcacctccgg 14880
   ctcgaggcgc gcacggcgca ctgcctgcgg cgcaaccgcc ccgaccggca cgagctgctg 14940
   tccgccgtgg cctcgctcta cgtccacggc atcgacccgg actggtcgcg actggtgcgc 15000
   cccccggacc gccggtgggc gctgcccgcc tacccctggc agcgccagcg cttctggctg 15060
   gaagcggcgc cccccccgga cgccacctgg agcgcgccga gcgcgcggcc cgccggccac 15120
   ctcgtctacc ggaccgagtg gctggccgac gcggcctccg cctccgggga caccgcgcgg 15180
   ccgaagggcc acctcgtgtt ctccgacggc agcgccctgg ccgaggccct ccgcgccgag 15240
   ctcgaggcgg ccggcgcgcg attcgggtgg gtgagccgcg gccaggactt cgccgcggac 15300
   ggcacgcgct gcaccgtgga cccggagaac cccgagcacc tcgagcggct cttcggccac 15360
   ctcgcggccg agtccttcga gttcgaccac gtcctgctgc tgttcacccc ggtggcggac 15420
   gaggcgcggc cggacggcgc ggcgccgtgg ttcgacacgg ccgtgcgccc cgtgcacgtc 15480
   ctgcgctgcc tgacccaggc cctgctcacg accgagcgcc ggccgaggat ggcggtcatc 15540
   acccgggcgg cgctgagcgc gggcggcgac accgcggcgg tgccccagca cacggcgctc 15600
   tggggcttcg ggcgcgcgct ggcgctggag gagccggggc tccgcctggc gctgctggac 15660
   cttccggcgg tgcccgacgg cctcgtggag gcccggcacg tcctggcgcg cctcgccagc 15720
   ccgacgcccg agcgccagtc ggcgtggcgg gacgggcggt ggcgcgtccc ccgcctcatg 15780
   ccccacgagg ccgggacggg cacgtccgcg ccgcccgtca tccagggcga gggcacctac 15840
   ctcatcaccg ggggcctcgg ctcgctgggg ctcgccaccg cccgccggct cgtccagcgc 15900
   ggcgcgagga gcgtggcgct cgtctcgcgc cgcccgccca ccgccgcgca gcaggccgag 15960
   ctcgcgcgcc tggaggaggc gggtgcccgg gtggtgacgg tgcagggcga cctggcggac 16020
   ctggcggagg ccacgcgcgt cttcgagacg ctcgcggcgt cggcgcctcc ggtccggggg 16080
   gtgattcacg cggccggcgt gctggaggac ggagtgctcg ggagcgcgac gaaggcgagc 16140
   ttcgaccgcg tgctgcggcc caaggtcgcc gggagctgga acctccacca gctcacgcag 16200
   tccctcccgc tggacttctt cgtgctgttc tcctccgcca ccgcgacgct gggcgccgcg 16260
   ggccagacga actacgccgc cgccaacgcc ttcctccagg gcctcgcgga gcaccgccgc 16320
   gcccgcggcc agcacgccct ctgcctgcac tggggcgcct ggcgcgagag caccatggcc 16380
   tccggcgccg tggtgacgaa gctgaaggcg ctgggcttcg acaccctggc gcccgaggac 16440
   gcgctggacg cgctggagcg cgtgctcctc gaaggggcga cggaggcgac catcatcgcc 16500
   gccgactggg aggtcttcgc ccagcggctc ccggacgaga gcctgggccg gttcctcgag 16560
   cccgtgctgc gcgcgagcgg ggccccgccg gccgtgaagc ccgaggccga ccccatcgtc 16620
   tcacgcctcg ccggcgcgcc ggcgagccag cgccagaagc tgctctacgg ccacgtccgc 16680
   gcggtggtgc tgtccaccat ggggatggac gcccgcaccg aggtccgccc ggaccagggg 16740
   ctcatcgagc tggggctgga ctcgctgctc gcggtgaaca tcgccaaccg gctctcgcgc 16800
   gacctggccc gccagctgcc caagacgctc atctacgacc acccgacgct gcacgccctg 16860
   acggaccacc tgctgggcgt gctcttcccc gagcccgccc aggccagcgt ccggcccgcc 16920
   gcctccggcg acgcgccctc ctacgaggac atcgccaacg cgagcgcgga caccatccag 16980
   agcctcctcg acgccgagct cgcgagcctc ctctcctccc gctgagcccc tccgcgggcg 17040
   tgcgccggac gcggtccccg cgggggtccg cgcccgcgcg cccggtgcgg ctccttcgca 17100
   cagaaagcac ttccgccgtg gatacgaacg ccctccttgc gcagaacaag aaagccctcc 17160
   tggccatcat ggagctcaag gcccagctcg cgcgggccga gtcgcggcag cgcgcgccca 17220
   tcgccatcgt cggcatggcc tgccgcttcc cgggtggctg cgccacgccc gagcagttct 17280
   gggagctgct gcgcgagggc cgcgacgcgg tcaccgaggc gttcccctcg cggtggccgg 17340
   agtcgctcta cgacgccaac cccgaggcgc ccggcaggat catggcgcgg ggcctgggcc 17400
   aggtggaggg cgtggccgac ttcgagccgg agttcttcca cgtctcgccg cgcgaggccg 17460
   cgagcatgga cccgcagcac cggctgctgc tcacggtcgc ctgggaggcg tgcgaggacg 17520
   ccgggctcac ccgggagctg ctgcacggca gccgcaccgg aatcttcgtc ggagcgggca 17580
   acaacgacta cggcgacttc gagctgcgca accccgacct gggcgcggtc agcgcctacg 17640
   cgggcacggg caacgccacc tcgctggtcg cggggcggct ctccttcttc ctcgggacgc 17700
   agggccccgc gctcgtggtc gacacggcgt gctcgtcctc cctcgtcgcc atccaccagg 17760
   cgtgcaccag cctgcgcctg ggtgagtcgg agctcgcgct cgctggcggc gtcaacctgg 17820
   tcctccgccc caacggcagc gtgttcctct cgcgcttcgg cgcgctctcg cccaccggcc 17880
   ggtgccgggc cttcgccgag gcggccgacg ggtacgtgcg cgcggacggc tgcggcgtca 17940
   tcgtgctcaa gcgcctggag gacgccctgc gggacgggga ccgggtgctg gccgtcatcc 18000
   ggggctcggc catcaaccac gacggccgca gcggcgggct caccgtcccc aacggggcgg 18060
   cgcagcggga gctgctcctg gccgccctga agaacgccgg ggtggagccg cggagcatcg 18120
   gctacctgga ggcccacggc acgggcacgc cgctcggcga cccgatagag atgggcgcca 18180
   tcgccgacgt cttcggcggg gacgcgcggc gcgagtccct gcgggtgggc tcggtgaaga 18240
   gcaacatcgg gcacaccgag gcggcggcgg gcatcgccgg catcatcaag gccgtcctca 18300
   gcctccagca ccgggaaatc cccgcccacc tccacttcga caagcccagc tcgcgcatcg 18360
   actggagcat ccccgtcgag gtccccacga gcacgcggcc ctggagcggg gacggccccc 18420
   ggctggcggg cgtgagctcg ttcggcttca gcggcacgaa cgcacacgtc atcctggagg 18480
   aggcgccgcg ccaggaggcc cggcactcgc cccggctcgg gacgcaggtg ctctgcctgt 18540
   cggcccgcac cgagggctcc ctcacgcggc tggccgcggc ctggagggag cagctccggc 18600
   ggagggattc ggcgcccctg gccgacctgt gccacgccgc cgcgcggcgc cgctcccacc 18660
   accgcgcccg gctggccgtc ctcggcgaca gcgccgacgc catcgcccag gcgctggagt 18720
   ccttcgccgc cgggacgccc caccccgccg tggtgagggg cgacgcggtg ctcgacggga 18780
   tgtcccgcac ggccttcgtc ttctccgggc agggcagcca gtggctcggc atgggacagg 18840
   agctgctcgc cagcgagccc ctcttccagc agagcctgcg cgaaatggac gggccgttcc 18900
   gcgccgagtt cggctggtcc ctcgaagagg cgctgcgggc cggggaggcc gagtcccagc 18960
   tcgagcggac gcgcgtcgcc cagccggtca tcgtggccgt ccagctggcg ctgagcgacc 19020
   tctggcgggc gtggggcgtg caccccgacg tgacgctggg gcacagcctc ggggagatct 19080
   ccgcggccca cgtcgccggc gtgctcgggc ggcacgaggc cctgcgcctg gcctgcatcc 19140
   gcggccggtt catggatggg gccaccgggc gcgggaagat ggccgagctc gcctgctccg 19200
   aggagcaggc gcggcgctac ctccagggct tcgaggccga ggtcgccatc gccgccctca 19260
   atggcgccat gtcgacggtg ctcgcgggcg cgccgggcgc cctggaggct gtcctccaga 19320
   aggtcgccag cgacggcatc cagcaccgcc tgctgcgcgt ggactacgcc ttccactcgc 19380
   cgcagatggc cgagttcgcg gcgcccttcc gcggcgcgct gggcgacatg aagccccgcg 19440
   gcgccgagcg ggacctctac agcagcgtgc tcggccgcgc ggtgcgcggc gaggcgctga 19500
   cggccgacta ctgggtgcgg aacatgacgg acaccgtgcg cttccacgag gcgctcgtgg 19560
   cgctcctgcg cgacggctgc cgcgccgtgg tggaggtctc cccccacccc gtcctgctct 19620
   ccgccatcca ggacaccctg agccgcgagg gcgtcgacgg cgtcgccgcg tactcgctcc 19680
   ggcgcggcaa cccggagcgc gccaccctgc tgtcgaacct cggcgccctg tacgccgccg 19740
   gccactccgt ggactggtcc gcgctctacc ccgggctggt ggaccacgtg gccctgccga 19800
   cctacccctg gcacgcccgg cgcttctgga cgaccgtcca gggcccggag gccaccaccg 19860
   gcgcggcccc cgccgtcacc gccccggccc cgaacgaggc cgcccccgcc gccgtcacct 19920
   cgaagctgtg ggacgagctc gtcaagctgg cggccgtcgc gcgcaggccg cgcctggagc 19980
   gccatgtccg cgagcagctc acgggcatcc tcaagctgga ctccggggac atcccctcga 20040
   agcgcacgcc gctgagcgag ctgggcatgg actccgtcgg cgccgtcgcg ctgtccaagg 20100
   cgctggccct cacccacgag caccggttcc ctcccaccgt gctcttcgag caccccacga 20160
   ttgaagcgct gaccgggttc ctcgccgagg agttcgagcg gctccnnnnn nnnnnccgcc 20220
   cccccccccc cccggccccc cgccgctccg gaggccgccg cgctcccggt gcccgactcg 20280
   gagctcgcca tcctggagaa gctcgccgcc ctcgagaagg agtttgcctg acatgtccac 20340
   accggagaat cccacgggcg accgcgcact cctccaccgg gcgctggccg ccatggagaa 20400
   gatgaagggc cgcatccagc agctcgaggg cgtgacgacc gagcccatcg ccatcatcgg 20460
   catgggctgc cgcttccccg gcggcgtcca cggccccgac gccttctggc ggttcctcgc 20520
   ggacggcgtc aacggcatcc gtgacacgcc gaaggaccgc tgggacaccg agcgctacct 20580
   cgacgccgag cagggcacgc cggggaagat gtacacgggc cacggcggct tcctggaccg 20640
   cgtggacggg ttcgacgccg cgttcttcgg catctcgccc cgcgaggcgc tgagcatgga 20700
   cccgcagcag cgcctcttcc tggagacgtg ctgggaggcc atcgagaacg cgggcctgtc 20760
   cgcggacgtg ctcgagggca gccagacggg cgtctacgcg ggcatcgtca accaggacta 20820
   cgccatgatg cagatgaagg acaccgcgca tggcgagatg gacacctact tcgccaccgg 20880
   acacccgcac tgcgtggtga gcggccgcgt gtcctaccag ttcggcctcc gcggccccac 20940
   gctgtccgtg gacaccgcgt gctcgtcgtc gctggtggcc ctgcacctgg cctgccagag 21000
   cctgcgcctg gggagctgcg acgtggccct ggctggcggc atcaacctgc tgctgatgcc 21060
   ggagatctac atgatccgct gccaggcgcg catgctgtcg cccagcgggc ggtgcgccac 21120
   gttcgacgcg tcggcggacg gatacatccc tggcgagggc gtgggcgtcg tcgtgctgaa 21180
   gcggctgcga gacgcggtcc gggatgggga ccgcgtcgac gccgtcatcc gcggcacgca 21240
   gatcaaccac gacggccgca gcagcgggct gaccgtgccg agcggggccg cgcagcggga 21300
   gctgctgcgc gccacgctgc gcgacgcccg cctggagccc cagcgcgtcg gctacctcga 21360
   ggcgcacggc accggcacga agctggggga ccccatcgag atggccgcca tccaggacgt 21420
   ctactgccag gaccgcgcgc ccggaaaccc gctggtcatc ggctcggtga agaccaacat 21480
   cggccacctg gagtccgccg ccggcatcag cggcctcatc aaggccgtcc tcaccctcaa 21540
   gcacggcgcc atcccgcgca acctccactt ctccgagctc aacccgcaca tcacgctgca 21600
   cccggcgacc cggattccca ccgagctcac cccgtggccg gcgggcagcg gcccgagggt 21660
   cgccgccgtc agctcgttcg gcatgagcgg caccaacgcc cacgccatcc tggaggaggc 21720
   ccccgcgcgc gaggccgcca gcgcccaggc gcccctccgc gtcacctccc ggccggcgac 21780
   gccgcggctc atcccgctca gcgcccacca ccccgaggtc ctccaggccc atgccgccgg 21840
   gctgaaggaa cacctggacg cccacccgga cgtcaccttc gacgacctgg cgcacacgct 21900
   gcgccggcgc accctccagc gccaccggct cgccctgccc gccagctcgc tggccgaggt 21960
   gaaggagcac ctggcggcgt tcacggccgg cgggcaggac ccccgcgtcc tccagacgga 22020
   ggacctcttc ttcaccgacc cgaaggtcgt cttcgtcttc ccgggccagg gctcgcagtg 22080
   ggtgcggatg gggctccagc tcatggagca ggagccggag ttcgccgcgg agctgcgcga 22140
   ggtggatgcg tggatccaga agtacgccgg ctggtccctg ctggacgagc tccgcgccga 22200
   cgcgagcgcg tcccggctga gccaggtcga cgtcgtgcag ccctgcatct tcgccgtgca 22260
   ggtcgccctc gcccggctgt ggcgctcatg gggcgtcgtc cccaacgccg tcatcggtca 22320
   cagcatgggt gaggtggcgg cggcctgcgt cgccggcatc ctgtcgctcg aggacgcggt 22380
   gtgcgtcatc acccgccgca gtcagatcgc gaagcgcacc agcggcaacg gcgccatggc 22440
   gctggtggag ctgacgcgcg agcaggccga gcgggccatc gccgcgcacc aggacgtggt 22500
   cgcggtcgcg gcccagaacg ggccccacac cgtcctcctc tccggcacgc cgggccccat 22560
   cgagcagatc ctccgcgagc tgcagcagca ggacgtcttc tgccgcgcca tcaaggtcga 22620
   ctacgcctcc cactccccgc agatggatcc gctccggccc gagctccagc gggcgctctc 22680
   cggcgtgaag ccccgggcgg ccaccgtcgc cttctactcg acggtgacgg gccaggtgga 22740
   ggacggcacg tcgcacgacg cgaactactg ggtgaacaac ctgcgcgacc ccgtcctctt 22800
   ctggccgggc atgcagaagc tcgtcgagga cggacacaac atcttcctgg agctcagccc 22860
   ccaccccgtc ctcatccccg ccatctccca gggcctgcag cacctgggcg cccgggcgct 22920
   gagcctcccg tcgctccacc gcgagaagcc ggaggccgag tgcctgttcg agtcgcgcgc 22980
   ccggctcttc tgcgccggcg gcaggctgga cctcgcgaag gaggacgtcc cgggcgcccg 23040
   ggtcctcgcc ctgcccggcc acccctggcg tgagcagcgc ttctggtaca cgcccagcgc 23100
   cccctcggcg cacccgcagg cccgcggcgg cagccggctg ctgccccgca tgtggacggc 23160
   gggctccggg gagcggcgcg tcttcgaggg cgacgtcggc cccggcgtca tgccctcgct 23220
   ggcggaccac cgcgtcaatg ggatgcagct cttccccggc gccggctatg tcgagctggt 23280
   gctcgaggcc gccgaggcgc tcgggctcga gggccggacg cacatcgact cgctcgacat 23340
   ccgcgagggg ctggtgctcc acgagggcgc ggcgcgcgtc gtccaggtgg agctgacgcc 23400
   cgtggccgcg cgctccttcg ggttcgtcat cagcagccgc gaggccgacg gcgccgcctg 23460
   gacgcgccat gccgacggcg tggtgacgcg cgaggaggcc ggggcggcgc cgggcgcgga 23520
   gcccggcgcg ctggaggcct tgcgcgcgcg gctcgcctcg cccctgtcgg cggaggacca 23580
   ctacctccgc acccgggagc gggggctgga gtacggcccc gcgtaccagg ccatcaagca 23640
   catcggccgc gggcgtggcg aggccctggc ggagctgcac gtgccccagg ccgtcgcgga 23700
   ggagctgagc gggtgccagc tccaccccag cctcctggac gccgggttcc agctcatcga 23760
   cacggcgctg gccgggtcgg gcctcgtgga ggagggagac gtctacctgc ccgccgccct 23820
   ccgcggggtg cggttccacc gcaagccggc ccccggacag acgctccatg cgcacctggg 23880
   gttgagcccg gagaagccgc gcgaggcgtc ggtgctggag gcgcggctgg cgctcctgga 23940
   tgaccagggc gctcccgtcc tcacggccga gcggttcatc atccgccgcg tcgagaccca 24000
   cgccgccgcc tgggaggggc tgccgggcga gtggttctac cgcctgcgct ggcagccgct 24060
   ggagggctgg cagccggagg ccgccggggc gaggcgcgac gaggcctggc tcctgttcgc 24120
   ggacgaccag ggcgtggccg cggcggtggc cgaccggctc gcgccctcgg gcgtgaggac 24180
   cacgctggtg cgcaaggcgc ccggcgccgt cacgcgggcg gggagcggct ggacgctcga 24240
   cccgagcacg ccggagtcct tccgcgagct gctcgccgcg gtgccgccgg gctccttcac 24300
   ccgcgtgctc tacttctggg gactggacgc gcccgcggtg gaccccgagc cggacgccct 24360
   ggacgcgcgg ctgcccgaga cggaggccct catctacctg gtccaggcgc tcgccgcccg 24420
   ggaggcgcag ccggggccgc gcctgtggct cgtcaccgcg ggcgccctcc ccggcgccga 24480
   cggcgaggcc gtgtcgccca tgcagacccc gctctgggcc ctgggccgcg tcctggacta 24540
   cgagcacccg gagctgaaga ccgtctcctg cgagctggag cgtcacgtca cggagaagca 24600
   cgtcgccgtg ctgacctcgc tgctcctcgc gcccgaggtg cccgaccagg tgcgcgtgcg 24660
   cgccgacggc ccggccgcgg cgcggctggc ccgctacgcg cccccggccc cccgggacac 24720
   gccgcaccgc gtgtcgccgc acgaggcggc cgtggtggtg tcgggcaacg gccccgcgcc 24780
   cgcggagctg tccgtcgcca ggcgacgggc ccccgcggcc cacgaggtcg aggtcctggt 24840
   cacccacgcg cacctcgcct gggcgcccgc gtcccccgac gcgggcggcc tcgtgccggg 24900
   agggttcttc gcgggccgcg tcctccggct cggaggcgag gtccgcgggc tctccgtggg 24960
   ccaggccatc gtggccccgg cgggcggcga gctccgctcg attcgcacgg tggacgccgg 25020
   tggcgtgcgc ccgctgccgg agcaggccga ccccgcgcgg gcgctctgcg cgctggggac 25080
   ccgggccctg gcgcggcacc tcctgcgcca gctcgccccc gtcgagcgag gccagacgct 25140
   gctgctgctc ggtggcgacc tccccctcct ccagtccctc gcgcgggtgg ccacgggcca 25200
   cggcgcgcgc gtcctcgccc tggtccggga cggcgcgcac gcgcccgcgc tccaggaggc 25260
   gggcgccgag gtggtgggcc ggctggacga gcccggcgcg ttcgagcggc tgcgcgagcg 25320
   cgccggtgcc tcgggcgtcg acgtcctcat caacctcgcg cccggcgccg cggtaccgcg 25380
   ggcgctcgac gtcttccggg cgggcggcgt cttcctggac ctgacgcgcg cgtccccggt 25440
   cggcccgaag gagctgcccg cctcggcgac gttcctctcc ctggggctcg caccgctcta 25500
   cgcgcgcctg gcgccgccca cgccggaagc catcgccgag gccagcgcgc ccgcggaggc 25560
   ccctgcgcgc gtgtggagcc tccaggagct gacgcagccg cgggcggagg cgccgctcga 25620
   cggccaggcc cacgtcctcg gcgtgtcgtc cgaacccgtg acggtggggg ccgggagcgc 25680
   cccgctcgcg ctctccgaca ccgcgacgta cctcatcacc ggcggcctcg gcggcctcgg 25740
   cctccgggtg gcgagcctgc tggtggacca cggcgtgcgc cacctggcgc tcgtcggccg 25800
   ctcgccgccg agcgcgcagg cccggagcac cctccgtgag ctggagcagc gcggcgtccg 25860
   ggtccaggtg ttcgccgccg acgtcgcccg gcgcgaggac ctgctcgggg tggtgaaggc 25920
   cctccaggcc ggcgacgccc cgctccgggg ggtcatccac tgcgccggcg tcctccagga 25980
   cggcgcgctc ctcggcctgg accgcgagcg gttccgctcg gtcctcggca gcaagctgag 26040
   cggcgcgtgg aacctgcacg ccctcacccg ggacctgccg ctcgaccact tcgtgctctt 26100
   ctcctcggtg tcctcgatgc tgggctcccc cggccagggc aactacgccg cggcgaacgc 26160
   gttcatggac ggcctcgccg cctggcggcg cgcccggggg ctgcccgcgc tcagcgtcaa 26220
   ctggggcgcc ttcagcgaca tcggcctggc ggccgtcggc gagcggcgcg gagaccgcct 26280
   ggccgacaac ggcatccccg gcatccctcc cgagaagggc atgaaggcgc tcgagctggc 26340
   gctgcgctgg ggcgagacgt ccctgggctt cttccccttc cccttcctca tctggcggaa 26400
   gctcaacccg cagattgccc gcaacacgct cttcaccgac atccgcgcgg agagccgcta 26460
   ccgcgaggag gccagcggcg agcagcccat cgccgaggcg ctcgccctcg cggacccggc 26520
   cgcccgccgg agccacctcg acggcttcct gaagcagcag ctctcgcgcg tactcaaggt 26580
   gccggcggag caggtccgcg acggcctcac cttcaaggag atgggcttcg actcgctgat 26640
   ggccatcgag ctgcgcaacc ggctggaggc cggcctcacg gtgtccctgt cgtcgacgct 26700
   cgtctggcgc tacccgacgc tcgaggagat gtcgcggttc ctgctccagc tcgtcggcgg 26760
   cgggagcgag acgccctcgg acgcacggcc ggcgtccgcc gccccggcgc gccggggccg 26820
   ctggctggtg cgtgagaagc cgagtccctc cgcgaagctc cggctgctct gcttcccgca 26880
   cggcggcgcc ggcgcctcgg ccttccaggg ctggtggcag cagttcccgg acacggtgga 26940
   ggtcctcacc ttccagccgc cggggcgcga ggagcggctc caggaggagc ccgagcgcga 27000
   catgggggcg tatgcctctg ccatcgtcga ggagctccag ccgctgctcg acctgccctt 27060
   cgccgtgttc ggctacagcc tcggcggcct caccgggttc gccaccgtgg ccgagctgcg 27120
   gcggcgcaac cgcgtcaccc cccggcacct gttcgtgtcg tccgccttcg ccccgcacat 27180
   ccccttctcc gagacgacgc tggcccgggt ctccacccag acctcggcgc tgaaggccat 27240
   ggccttctac cagtccacgc cggactcggt gttcaaggac ccggagatgc aggcgctgct 27300
   ggcgcgctcg atggacgcgg acaactccgt ggtcgagtcc taccgcttcg gcgcggagaa 27360
   gccgctggac tgccccatca ccgccttcgc gggccggcag gacgacctcg ctccgctcca 27420
   cgagcagctg cgctggaagg agctcaccac ccaggccttc gagctgcggc cgttcgaggg 27480
   cgaccacttc ttcttcctga gagacaagcg cccggtgatg gacgagctgc ggcgccacct 27540
   gctctccctc atcgcggact cccgggacgg ctcgtcccgc tgaccccgcg cctccccctt 27600
   cctgttcgaa gcgagaacac acccatggat gcctcctcct cccgcccctt cgagtcactg 27660
   gacgcccttc aggcgtgggt cgtcacctgg ctgtgcgagc atgcccggat tccccagcag 27720
   gccatcggcc tcgacgtccg cctgagggac ctcggcctcg actcggcccg ggccacgaag 27780
   ctggtggccg cgctctccga gcgcttcggc cggacgctcc cgctgacgct gctgtgggac 27840
   tacccgaccg tcacggagct gtcccggcac ctgatgaagc ccgcgacggc ggcggcggcc 27900
   ggcgccacgc cccgggccga gcgtcgcgcg agcggcgctc cggaggaccg cgccatcgcc 27960
   gtcatcggca tggcctgccg gttcccgggc ggcgcggaca cgcccgaggc cttctgggag 28020
   ctgctggcga aggggctcga tgtggtgggg ccggtgcccg cggaccgcta cgacgcggag 28080
   gccttctacg accgtgaccc gcgggcccgc ggcaaggccg tcacccggtg gggcggcttc 28140
   ctggagcgga ccgacacctt cgacccctac ttcttcggca tctctccgcg agaggccatc 28200
   gagatggacc cgcagcagcg catgctgctg gagctggcct gggaggcgtt cgaggcggcg 28260
   ggcctgacga aggacgggct ccgggaccgc gcggtcggca tcttcgtcgg cgccatgtgg 28320
   aatgactacg cccggctgtg gcggggcggc cccggcgcca tcgagcagtt caccgcgacc 28380
   ggccaggacg acagcatcat ctccgcccgc atctcctacg tcttcggcct gcgcggcccg 28440
   agcctgacgc tgaacaccgc ctgctcgtcc tcgctggtgt ccgtgcacat ggcctgccag 28500
   agcctgctgg cgggtgactc cgagctggcc ctggcgggtg gcgtcaacct catccacggc 28560
   ccggacagca ccatcgccat gtcgaagttc ggcggcatgg cgccggacgg gaagtgcaag 28620
   gccttcgacg cgcgtggcaa cggctacgtc cgcggcgagg gcgcgggcgt cgtgctgctc 28680
   aagccgctgc accgcgcgct cgaggacggc gacgtcatcc acgcggtcat ccgcggctcg 28740
   gccgtcaaca acgacggctt ctccaacggc ctgacctcgc ccagcccggc cgcccaggcc 28800
   gcgatgctgc gcgcggccta cgccagcgcg ggcgtgtccc cggcgcgggt ccactacgtg 28860
   gaggcgcacg gcaccggcac catgctgggt gaccccatcg aggcgggcgc gctcggcgag 28920
   gtgctcgggc agcaccccga gcgccagcag cccctgcgca tcgggtccgc gaagacgaac 28980
   atcggccacc tggaggccgc cgccggcatg gcgggcctca tcaagaccat cgaggcgatg 29040
   aagcaccggc gcctgccacc ctcgctccac ttcgagcagc ccaaccccaa catccacttc 29100
   gaggggctcc gcctgcgggt gcagaccgac ctctcaccgt ggcccaccga ggacgactcg 29160
   ctgctcgcgg gcgtcagctc cttcggcttc ggcggcacca acgcccacgt ggtgctcgag 29220
   tccttccagc ccgcccggcc ggtggccatc cccttctccg cgccggacgc ggaggcgctg 29280
   ggcgcccggc tcgtcgagct gcgggccttc gcggagcagc accccgaggc ctcgctggac 29340
   gacctccagc gcgcgtgggt ccagcgcctc accgaggggc cggtgcgcca ggtgctcctc 29400
   gcccgctccc ggaaggacct cctggagcag ctccgcgcgc cggcacggcc ggcggctccc 29460
   gcggggcgcg gcgcgacgcg gccggtcctc gtcttcaccg gcaacggcgc gcagtacccc 29520
   ggcatggcgc gggacctgat gctcggggac gcgaccttcc ggcaggcggt cctccgctgt 29580
   gacgaggcgc tgcgggcgca cggctgcgac ggggtggccc tggcgctcga gtccggtgac 29640
   ttcgacgtgc ggcgggtgga cctggtgcag ccgctgctgt gggcggtgca ggtcggcctc 29700
   gtctccctgt ggcgcgcgtg gggtgtggag cccgccgccg tcgtcggcca cagcgtcggc 29760
   gaggtggccg ccgcgtgggc cgcgggcgcg ctcgacctgg acgaggccgc gcgcgtcgtg 29820
   gtccaccgca gccggctgca gcaggagtgc gcggaccagg gcgggatggt gctcctcgag 29880
   accgacgccg agggcgcgcg ctccctcgtg ggcgccacgg cggcggaggg gctggccgtc 29940
   gccgccctca actcgcgcgg cagcaccgtc gtgagcggcc ccacggcgag cctccaggcg 30000
   ctcatcacga aggcggagga gcgcggggtg acggcgagga cggtccgcgt caacgtcgcg 30060
   taccacggcc ccgggatgga cgcgctgcgc gagcggctgc gggagtccct cgcggacctg 30120
   cgtccgtgcc ctccccgcgt cccgatgtcc tcctcggtga cggggggcgc ggtggggggc 30180
   gcgggcctgg acgccggcta ctgggtccgc aacatggccg accccgtgct gttcggtgac 30240
   gcggtcgacg cgctggtgag ggacggccac cgcctgttcc tggaggtggg cccgcacccc 30300
   acgctccacc gggccgtcca ggagcagctc cagcacggag gcgtggccgg gggcttcgtc 30360
   gcgacgagcc tggagcgcga gcagcacggc cgcgcccgcc tgctggagaa cctgggccac 30420
   ctgttcgagc gcggtgtccg cgtggcgtgg cgggcgctga cgcggggagg cgcgccggcc 30480
   tccgcgggcc aggcgcccgg ccccgtcctc ctccccctct ccgcccacac ggacgtggcg 30540
   ctccagcagc gcgtggaggg gctgcgcgcg tacctgcggg cgaacccgga ggcggagctg 30600
   gaggccctgg cccgtaccct gggccgggga cgcacccacc tgtcgcaccg ctgcgcgctc 30660
   gtggcctcct cggccggcga gctcgcggag gtcctggagc gcgcggccgg cgccgacggc 30720
   gcgcccgagc tcatccgccc cgcggcctcc ctcgacggcc gcgcgaaggt ggccttcgtg 30780
   ttccccgggc agggctccca gtggctcggc atgggccgcc agctcctgga gacggagccc 30840
   gtcttccgca aggcgctcca tgagtgggag gccgtgctgg gagagttcac cgactggtcg 30900
   ctgctggagc agctccacgc cgacgccgcg acgtcccgcc tgggcgaggt ggacgtcatc 30960
   cagccgaccc tcttcgccat ccaggtcgcg ctggcggcgc tgtggcgctc gtggggcgtg 31020
   cacccggacg ccgtcatcgg ccacagcatg ggcgaggtcg ccgcggccta cgtcgcgggg 31080
   gcgctggacg cgcgtgacgc ggcccgcatc atctgctcgc gaagccgcat cgcgaagaag 31140
   acgagcggcc gcggcagcat ggccgtggtc gagctgtccc ccgaggagtg ccagcgcgcc 31200
   atcggcgccc gcgccgccac ggtgagcctc gccgcctgca acgggcccgc ctcgtgcctg 31260
   ctgtcgggag agacggccgc ggtggaggag attggcaggg agctccaggc ccggggcgtc 31320
   ttcttccggc tcgtcaaggt ggactacgcg tcccacagcc ctcagatgga cgccatcaag 31380
   gacgagctgg cgcacgtcct tcgcgagacg cacccgcgcc cggccaccgt gccgttccac 31440
   tccaccgtgg agaacgccct cctcgacggg acacggctgg acgccggcta ctgggcgcgc 31500
   aacctgcgcg agccggtgct cttcttccag ggcatccagg cgctggaggc ccgcggcttc 31560
   aacgtgttcc tggagctcag cccgcacccc gtcctcacct actccatctc gcagacggtg 31620
   cgtccggaga gcgcgctcat cctcacctcg acgaagcgcg aggaggacga gtcgcgggcg 31680
   ctgcgcacgt cgctgggcgc gctcttcgcc gcgggcggcg ccgtggactg ggccgcgctg 31740
   tacccgggcg tggcgacgcc cgtcgagctg cccgcctatc ccttccagcg ggagcggttc 31800
   tggttcgacg aggcggaggt ccagcggtcc gcgcccgcgg gcaccggccg ccacgcgttc 31860
   ctcgagcgct tcctccgctc gccgtacccc caggagcgcc tcatctggga gacggacctg 31920
   agcctcgagc gcttcccgta cctcgtggac catcgcgtca gcggcctcgc cgtcttcccc 31980
   ggcaccggct acatcgagtg cgtcctggag gcggcgaagg acctgtacgg cgacgcaccc 32040
   gtcgagctgc tcgaggtctc cttcgagaag gccctcttca tcgaggacac cggcgcgaag 32100
   acgctgcagc tctgcatgga gccggacggc gccggccacc gcttctccct gtcgagccac 32160
   gacgcggccc agcagacgtg gacccgccac gtgaatggcc ggctcgtgcc cgccaccgcc 32220
   gagcaggccg cccacgcgcg ccagcaggtc caggacgtgc tggccggcga gccccacgcg 32280
   ccggtggacg tggggacgtt ctacgggaac ctcgagcgcg cggggaacac ctacggcccg 32340
   aagttccagg ccgtgaagtc cctgcgctcg aacgccaggg gcgtccgcgg cgaggtcgtc 32400
   atgggcgagg agctggccac ccggggcacc cgcatccacc ccgtcgtctt cgacgcctgc 32460
   ctccaggtga tggccacgcc cctctacatc gagaaggccg cggggggccg cgagagcgcc 32520
   ttcatgccga cgcgggcggc gcgcatccgc ttcttccgtg aggccgaccg ggccgtggtc 32580
   agccacgtgc gcttcctgaa cggggccacc gccgacgcgg ccggcgacct cctcgcggac 32640
   atcgacgtca cggacgggca gggccagctg ctggcgcggg tggagcgcat ctgcttccac 32700
   ctgctggagc acgccaccat ggcgcagcgc ccgtccaccg acatgctcta ccggctgacg 32760
   tgggaggagg ctccctcgcc ggaggtcggc gcctcgctgg cgcgccgctg gctcgtcttc 32820
   gcggacgacc agggcgtcgc cgaggcggcg gccgggctgc tgtccgcctc ggcgcatgcc 32880
   gtgcaccgcc tccacccggc ggacgccttc cggcgcgacg gcaacgactt ccacatccgc 32940
   ccgaacgagc ccgaggacta ccgcgccgtg ctccaggccc tgctggcctc gcgcggcccc 33000
   gcgctgccgc tcggcgtcct ccacctgtgg aacctccgcc tgcccgaggg cggcgccacg 33060
   gccgtgccgg acctgcggga gatggcgaag ctgggcaacc tcagcctcac ccacctgctg 33120
   aaggcgctcg acggggccgg cggctccgcg ccgccgcgcg tctggattgg cacgcggggc 33180
   gcgtgggccc tccccgggag cgagcagggg ctggcgccgg cccagtccgc ggtctggggg 33240
   ctcacccggg ccatcgcgca ggagcacccg gccctgtggg gcggcatcat cgacctggat 33300
   ccgctcgagg ccccgcgcct ccaggcggag cggctcgtca cggaggtctc cgtcccccaa 33360
   ccggcggagg accgcatcct ctaccggcag ggccggcgtc aggtgtcgcg gctggtccgc 33420
   gacacggccc ccgtcagcgc cgcgaccgcc accttccgcg gcgaccgcgg ctacctcatc 33480
   acagggggcc ttggtgagct gggcacgcgc ctggcggcgt ggatggtggc ccgtggcgcg 33540
   cgccacctca tcctggcggg gcgcaaggcg ctgccggccc ggcggacgtg gaagcaggac 33600
   gggcacgacg cggcgacccg ggcgaggatt gacgccgtcc tccacctgga gcgcctgggc 33660
   gctcacgtcc acacgcccgc gctggacgtg ggggacggcg cggcgctggc cgccttcctc 33720
   gccggctacg agcaggaggc gcggcccccc atcgccggtg tcgcccacct cgcgggggtc 33780
   atccggcccg agctgctggc ccggctccgc gaggagtccc tcgaggagtc cttccagtcg 33840
   aaggtctacg gcgcgtggaa cctgcaccag gccttcagcg ggcgcgggct ggacttcttc 33900
   acgctgttct catccgcctc ggccgtcctg aactcgcccc tgctggggag ctacgccgcg 33960
   gccaacgcct tcctggacgg cctggcgcac ttccgccgcg cgcggggcga gccggcccag 34020
   tccctgaact ggggcttctg gagcgaggcc ggcatggccg cgcgggacct ccacttcgac 34080
   gcgagcgcgt ccggccagca gggcatgagg agcttcagcc ccgcggtggg cctggacatc 34140
   ttcggcgggt tgctgagccg cgacccggtg cagtcggtgg tgatggacgt ggactggaag 34200
   ctctggctcg aggcctaccc ggacgccgcc gcgtcgccgc tgctgcgcac cctgcgcgcc 34260
   agcgagggcg tggccgcggg ctccgagcag gcggcgccgg ccgaggaggc ctcgcggagc 34320
   ctgagggacc tgccggcgga ggagcgccgg aaggccgtcg agggcatcct gctcaccgag 34380
   ctgtccgcgg tcctcaagat gccggccgcg aagatttccc ggacggcgct cttcgggagc 34440
   atgggcctgg actccatgct ctcgctggag ctgcgcaacc ggctggagcg gcgcacgggc 34500
   ctgacgctgc cgggcaccct ggcctggaac tacccctccc tcgagcggat ggccgcgttc 34560
   ttcgtcgaga agctcggcgg ggaaggcgct ggcgtggcgc ggtccgagcg ccagcaggcc 34620
   ggcgccgcga gcgatggcgt ggcgaaggag ccggacatct cggcggaggt catcgaggcc 34680
   ctgatgcagc tgggcgtcct cgaggcgaac gaccccgtgc tccaggatcc ggtcgcgctc 34740
   ggcaacctgg tgcgcgaggc ccagcagtac ctgaaggcgt gacgcggcgc tgacgggggg 34800
   cgctcccggc ggagcgcctc agggccgcgt cacgccctcc gaggagaccg gccaccgcgt 34860
   cgtcgcccac aggtacagca gcagcgtcag cggctcgaag cgggcgtcgg cggcctccgc 34920
   gggcgcgacg gtccgcgaga ggctggccag ccgggcgagg cgggtgaggt cggccagcgc 34980
   ggcccccagc gcgggaagct ccagcaggag gtgggcgcgc agcgccagcg cgagcaccag 35040
   cccgtcactc ggcgcgggct ccggctccgc gtccgcgtcc gccagcaggg cctccgcgag 35100
   ggacgcgacc cagcgcgtcc ccttcggctg cggcccggcc gggtcgagat agccctccag 35160
   ccgctcggcc tcggacagca tgcgggcgcg cagctcgccc agccacccgg gggtgagggc 35220
   cccgggagcc acgcgcaggc tctgggaggc gctgtccagg acgagggcgg gctcccccag 35280
   ccctggcgcg gacagccggt ccgccagggc ccgcacccac tggaggtaca tgccgcgcgc 35340
   gagggtccgc cgcccctcca ccaccaccga gtccgggacg cgcgggacgg acgtgagccc 35400
   ccgcagcggg tcctctggcc agtccccgcc ggagccgcgg gtcaccgtcc gggccgcgca 35460
   cgcgcactgc gggaggatgc tgacctcgaa gtggtctcca cagtggatga cctgcagcgg 35520
   gaactggcgg caggtccacg gcttcgcctc cgcgcccccg gccttgtgga cctggcaggc 35580
   cccctgggca aggaagggac agccatcgcc ctggaggatg ggcgacagga cgtcgcgccg 35640
   gccgggattc acgggggcga aggcgtcctc ggcccgcccg ggggccgccc cgagcgccag 35700
   ccggcgcagt gtctccacgg cgcgccggct ctcctccacc gtcgtgggga tgacgggata 35760
   ctgcgagcag cacgccccgg agaggttgca gccgaccgac agctcgctgg tggtgatggg 35820
   cagccccggc gccccgggac gcccccgctc ctcgggctga agggcgtgct ggaggaactg 35880
   gggtgtccgg ggcgcgaggt cccgctccgc gtcgtacggc gggagctcgg gcggatgcag 35940
   ggggtcgtcg acctcgacgc cggggccgaa gggccgcagc agcaggaagc ggcggagctg 36000
   gcctccgacc cggcgatgca ggagctgtgt gtacgtcatg tagggtgaaa cccccgagca 36060
   ggagcccctt ggtaaccacc ttcgcgggag gcgtccagag gctggccgcc ccccgggccg 36120
   cgctccgggc ggtgctcagg tgctcagggc ccgcacgcgc gcggcgaggt tctgcgtgaa 36180
   gagccggtag atgcgcagcg cggcggcctc gtgggtgtcg aggtagtgct cgaagtcggc 36240
   gcggctgatg cgcagcgcgc gcacggaggt gcgcgcgcgg acgtgcgcgg agacgggggc 36300
   gtcctgcacc agggagattt ccccgaggta cgcgccgggc cccagcgtgt tgaggtgacg 36360
   cgcgtcctgc tcggggccgc tgaagacctc cacggtgccg tccagcagga cgaagaggcc 36420
   cgtgccccgc gcgcccttct ccagcagcac cgtgcccggc gcgatgacga cctgccgcgc 36480
   gagccggtag aggtccttca tgtcctccag ggacagctcg ccgaagatgg ggatggcctt 36540
   gaggaagcgg tacccgttgg cggcgggcgt cgccgcggcc gtcgcgcgcc gcagcagcac 36600
   ggcctcggcc tccgcctcca tgcccatgcg ccgcagcagc cgcgccttct ccttcaccag 36660
   cgcctcgtcc gcccgcgcct cctcggagct gctcaccgcg tccgccagca ccgcgagcgc 36720
   ccggtgggtg aagccctcca cgtccagcag cgtgcagacg cgcagcaccg cctccacgta 36780
   ccgcgagtcc tccggcttca ccccgcccag cgcctccacc tcggcgtgcg cctggcccag 36840
   ctcgcggtag aggtccgccg cctcgaaggg gcggcccagg cggaccaggc actgcgccat 36900
   cgcctcgcgg gcgcccaggc gccggtacac gtccagcgcg cgctccagcg ctccggcccg 36960
   ctcgaaggcc gcggcggcgc gctccgcctc gccagcgcgc aggtacgcct ccgccgcctc 37020
   caggtactgc ccgctctgga agtagaggtc cgccacggtg gcgtcctcgc cgtcgccctc 37080
   catcaggcgg gcggcgccca ggaagtcgcg cgcccgccgc agcacctcca ccaggccccg 37140
   gcgctcccgg gccggacgcc gggccgcctc ccggcgcagg cgctcgcgct gcgcgtctcc 37200
   cagttcctcg tagagctgcg ccgcctgctc cgtctcctgg agctccacca gtgaccgcac 37260
   cgcgcgcagc gccccctccg gaggcgtgtc gatgcgcccg ccccccgccc cctggcctga 37320
   cgttcctgtc atgtgctctc cccttccggt cagaccgccc agcat 37365
<210> 2
   <211> 1110
   <212> DNA
   <213> Angiococcus disciformis
<400> 2
   gtggcattcg atgacggggc caacctctgg tccgtctacc gccacgcgga cgtgacggcc 60
   atcctctccg accacgtgaa cttcccgacg gcgaccatcg atgatggcgg ggcgcccccc 120
   gccgagtggc gccgcctctt cagcctgaat gcctttgacc cgccccgcca tacccagctg 180
   cgcaacctgg ccatgagggc cttcacggcc tcggcggtgg cccgcatgca ggaccggatc   240
   accgccatcg tcaatcagct gattgacgcc gtggtcggca cggggaggac cgagctcgtg   300
   gcggacatcg ccggccccct gcccacgatg gtgatcgccg agatgctggg cgtgccggcg   360
   tcggaccagg agtccttccg gcgctggtcg gacgaggtgg gcgtccgggc caatgccctg   420
   gtgctcgatc cggagaacgg ggtgaggctc ctgtcggagg cgctgacgcc gatggaggac   480
   tacgtgcggc agcagatcgc cgagcgccgc cgccgtccct cggacgacct cgtcagcagc   540
   ctcctggcgg cggagatcga cggcgagagg ctggaggagc tcgacctggt cgccttctgc   600
   atgctgctgc tcatcgccgg caacatctcc accacccaca tgattggcaa cgccatccgc   660
   gcgctcctga agcacccgga ccagatggcg cgcttcctcg cgaacccgga ccagatgccg   720
   ggggccatcg aggagctgct ccgctacgac tcaccggtcc tcgccgcgcc ccgctgggtg   780
   cggcacgaca cggaggtcgg cggcaagctc ctcaagcggg gccagcgcgt catcgtgtgg   840
   ctcggcgcgg ccaaccacga tcccgacgtc ttccccgagc cggagcgcct ggacgtgagc   900
   cgcaaggcgg cgcgtcaggt ctcgttcggc cacggcgccc actactgcat gggggctccg   960
   ctgggccgcc tggaggcgcg tatcgccatc ccggccatcc tgcgccgcat gccggagctg  1020
   cggatggtgg agggggccga gctggagccc gtgccgggct acctgctgca cgggatggtc  1080
   agcatgccga tgacgttccg ggccagctga                                    1110
<210> 3
   <211> 4713
   <212> DNA
   <213> Angiococcus disciformis
<400> 3
   atgactggaa tcatcgacaa gctgagccgg ctcgatgccg cgagcaagga cctcctggcg    60
   gagaagctgc gccaggccaa ggacaagcac ctggcgcgag gggcgcacgc cgagcccatc   120
   gcggtggttg gcatggggtg ccgctttccc ggtggcgcca gctcgcccga gcgcttctgg   180
   cagttgctga tgcaggggcg gagcggcgtc ggccgcgtcc ccgcggatcg cttccccatc   240
   gaggactact acgaccccga cccgagcgcg ccgggcgcca tcctcagcga ccggggcgcc   300
   ttcgtggagg gtatcgaccg cttcgacgct ggcttcttcg gcgtgtcccc ccgcgaggca   360
   cggcgcctgg accctcagca ccgcctcctc ctggaggtca cctgggaggc gctggagaac   420
   gcgggcctgc cccagtcggc gctccgcgag ctcgttggcg gcgtgttcat cgggttgatg   480
   ggcaacgact acttctccac cgtccaggag aaccccgccg acatcgacgc ctacaccagc   540
   acgggcacgc actacagctt cgcggccaac cggctgtcgt tccatctgga cttcaagggc   600
   ccgagcatgg ccatcgactc ggcctgctcg tcgtcgctgg tggccgtcca ccaggcctgc   660
   cagagcctcc ggacccggga ctgcgacttc gcggtggccg gcggcgtcaa cctcgtcctc   720
   tcccccgtcc tgagcatctc gtactcgaag tgggggatgc tcagccccga cggacagtgc   780
   aagaccttcg acgcctccgc gaacggcttc gtgcgcggcg agggcgccgg cgtggtcgtc   840
   ctcaagcggc tggaggatgc cgtggcggcc ggggacccca tcctcgccgt cgtgaggggc    900
   tcggccgtca accaggatgg cgccaccagc gtcatcaccg cgcccaacgg gctcgcccag    960
   caggccgtca tccgccgcgc cctgaaggtg gcgggcgtgg agcccgccgc catcggttac   1020
   gtggagaccc acgggacggg gacgtcgctg ggggacccca tcgaggtgga ggccctcaac   1080
   gagacctacg ggcaggagcc gggcggccgc ccgtgcgtgc tcggggccgt caagacgaac   1140
   gtggggcacc tcgagggagc ggccggcatc gccggcttca tcaaggcggt cctcgccgta   1200
   cagcgcgggc gaatcccccc gaacctccac ttccacgagc tgaacccgct gatccggctc   1260
   gacggctcgc ggctgtgtct ggccacggag gcccgggagt gggccgtggc gagccaggag   1320
   cgcttcgccg ccatcagctc gttcggcgcc ggaggcacca acgcccacgt cgtcatccag   1380
   ggcccccccg ccgcgcgggc gcccgcggcc accgggaagg cgcccgcctt cgtctgcctg   1440
   tcggcgaaga gcgacgcctc cctgaagcag caggccgcga ggctgcgcgc ctggctcggg   1500
   caggacggcc gggagtggag cgtcgccgac atcgcctcca cgagcctgcg gcgcaggaac   1560
   cacttcgacc accgggcggc cttcaccgcc gactcgccgg agtcgctcgg tgagggcctg   1620
   gacgccttcc tgtccggccg ggccagcccg cgctggtccg ccggcagcgg caccctgaac   1680
   gcccagcggc cggtgttcgt gttctccggc cagggctcgc agtggcaggg catgggccgg   1740
   gagctgctca cccagagccc gcgcttcgcg gcggagatcg ccgccatcga cgcgcggttc   1800
   atcccgctgg ccggctggtc catcgccgag gtcctcgcgc gggacggcga cggggcgcgg   1860
   ctgaacgcca cggaggtggc gcagccctgc ctgttcgccc tgcagctggg gctcgtcgcc   1920
   gcgctggacg agctgggcat ccggcccgcc gcggtcgtcg ggcacagcct cggcgagctc   1980
   gccgcggcgt gcgtggccgg cctcatggac cgcgaggccg ccacggccct cgtgtaccac   2040
   cgcgcgaagc tcatgcagcg gctgaccggc gcggggaaga tggccgccgt cgggctctcc   2100
   cgcgaggagg ccgaggcacg cctcgcggcg gagccccggc tggcgctcgc ggcggtgaac   2160
   gggccccgct cggtcgtcgt ggccggtgag ccggaggccg tggacgcctt cgtgcgggcg   2220
   ctgcgcgacg agggagtctt cacgcggacg ctgccggtcg actacgcctt ccacagcccc   2280
   cagacggacc cgctcctggc cgagctgcgg cgggccgtcg acggcatctt cggcgcgggc   2340
   gcgaggatgg ccgccccccg gctgccgatg gtgtccacgg tgagcggcgc cgagctcgat   2400
   gcctccgtcg accacgccac ctactgggcc cggaacatgc ggcagacggt gtgcttcgag   2460
   gaggcggtgg gccagctgct ggagcagcgg cacgacgcct tcctggaggt tggcgcgcac   2520
   ccggtcctga acgtcgacct gcgggagtcg ttccgcgcgc gggaggtgac ggcgcgggcc   2580
   gtcgccaccc tccggaggga gcagccccag gccctctgcc tgcgggcggt gcccgccgcg   2640
   ctctacgccg ccggcatggc ccccgaggcg ctccccggcc aggaccaggg caccctcgtc   2700
   ccactcccga actacgcgtg ggaccaccgc cgctactgga ttcactccgc ctcgcgggcc   2760
   gaggcgggct gggtctacga cctcgcctgg gacgcggtgc cggcacctgc gccgggcgcc   2820
   caccggcccg cgtcctggct cgtcctgggc gcctccggcg ggctgccgga ggcgctggcc   2880
   gcccggctgc gcgccacgcc cgacctggtg cgccacgtga gcctggaggc gctgcgctcc 2940
   gtgcccgtgc ccccccacgg gctggtggac gcggagcggc tcgagcgctg gttcacggac 3000
   acgctccagg tggacctggc ggagttcgac ttcctcgtct acgtgcccgc gtgcggcgcc 3060
   cccgaggacg cgagcgccga gcgccaggtc agcgaggact gcgccatcct ggtgagcctg 3120
   gcccaggccc tggagaggcg gcccggggcc cggggcccgg cgctctgggt cgtcacgagc 3180
   ggcgcgcagg ccgtcgcgcc gcgggagccc gtcaatccct tcgccgcgcc cgtgtggggc 3240
   ctcggccggg tgctcgccct ggaggccccg cgaggcctgg gcggtctcgt cgacctgccg 3300
   ctcgcgtggt cggagcagga cgtgggctgc ctgctcgagg cgctgcgcca ccccggcgag 3360
   gaggaccagc tcgcgctgcg ctcgggccag cgccatgccc tccggctgcg ccgccgggcg 3420
   ggccgcgcca ccgacgcggg cgcggcctgg caggtcgagg actccggcgt ctacctcatc 3480
   accggtggga cgcggggcct ggggctgctc gtggcgaagc ggctggccga gcgcggcgcc 3540
   cgtcacctgg cgctgctggg ccgccgggcg ctgccgcccc gctcgcggtg ggcgacgctg 3600
   gccgcggacg acgccttctt cccctccgtg caggccatcc aggcgctgga ggcccagggc 3660
   gtcaccgtca cgctccacgc cgccgacgtc agcgactccc acgcgatgcg ggacgtgttc 3720
   gcggagctcg agcggcagca gcgccccctc aagggcgtcg tgcacgcggc gggtatctcc 3780
   cgggaggtcc ccctggcctc catcgacgag gcgctgctgg aggagaccct ggcacccaag 3840
   gtcgcgggca cctggtggct tcaccagctc tcgctgcggc acccgctgga cttcttcgtc 3900
   gtcttctctt cgggggcctc cgtctggggc tccgcgggcc tggcgccgta cgccgccgcg 3960
   aacgagttcc tgaacgccgt ggcggcccac cgccgcgcct ccggcctgcc cgccctcagc 4020
   gtctgctggg gcatgtggga cgcggacggc atggccaccg acgagatgcg ggagaactgg 4080
   acgcgcctgg gcgtcaaggc gatggccccc gagcgcgccc tcaacgcgct ggaggagctg 4140
   gtggccgcgg acgccaccct cgccaccgtg gccgacgtga agtgggcccg cttccggccc 4200
   gtctacgagc tgaagcgcaa gcgtccgctg ctggcggcgc tcgaggacgg cgcggcggca 4260
   ccgccgcggc ccgaggcggc cggggcgccc caggccacct ccctcaaggc ggacctccag 4320
   gcgctgccgc gggagcagca gcgcgagcgg ctgctggcca ccctccgcga cacggccgcg 4380
   ctcgtgctcg gcgccgagcc gggtgccgcc atcaacccgg acgaggggct gttctcgatg 4440
   ggcttcgact ccgtcacggc cgtcgagttc gcggcgcacc tgagccgcct gttcgagaag 4500
   cgctacgccg cgacgctggt cttcgaccac ccctccctca agcagatccg cgacttcctc 4560
   atgcaggact gcttcggctg gacggccgag ccgtcccccg agcccgaggc gggtgacggc 4620
   caggacctcg acgcgctcct cgccttcgtc gagggcgtgg acgaggccga ggccgagcgg 4680
   ctgctctccg ccgggcagcc ccggtcgaac tga 4713
<210> 4
   <211> 5568
   <212> DNA
   <213> Angiococcus disciformis
<400> 4
   atgacctccg tcgacgaacg catcaagaat ctctccccgc agaagcgcgc cctgctcgag      60
   cagcggatga acgccgccgc cggcgccacc cgggacgcgg agcccatcgc catcatcggg     120
   attggctgcc gctacccggg gggcgtgaag gacctgaaga gcttcgccca gctgctgcgc     180
   gacggcgccg acgccaccgg ggacatcccc gcggaccgct gggacgtgga ggcccactac     240
   tcgcccgacc cgggcacccc cgggaagatg cgcacccggc gcggcggctt cctgcccgac     300
   gtgaagacct tcgacgcgga cttcttcggc atctctccga gagaggcgga gagcatggac     360
   ccccagcagc ggctgctgct ggaggtgtcc tacgaggccc tcgaggacgc ggggattccc     420
   accgcctcgc tgagcgggag caccaccggc gtctacatcg gtatcaccct ggcggagtac     480
   gccgaccggc tgccggacga cccccgcctc atcgacgtct actcggggac gggcacctac     540
   ctgaacgtgg ccgccgggcg cctgtcctac gtctacggcc tccagggccc ctgcatggcc     600
   gtggacgcgg cgtgcgcctc ctcgctggtg acggtccacc tggcggccca ggccctgcgg     660
   caccgcgagt gcggcctggc gctggcgggc ggcgtgagcc tgatgctgtc gccggagacc     720
   accatctacc tgtcgcagac gggcgccctg gcccccgacg gccgctgcaa gacgttcgac     780
   gctcgcgcca acggctacgg ccgcgccgag gggtgcggca tcctcgtcct caagcggctg      840
   tctgatgcgc tgcgcgacaa ggaccgcatc cacgcgctca tccgcggcac ggcggtcagc     900
   caggacggcg cgtccagcgg cctgacggtg cccaatggcc gcgcccagca gaaggtcatc     960
   cagtccgcgc tcaaggacgc ggcggtgcgg agcgaggacg tctcgtacgt cgaggcccac    1020
   ggcacgggca cgccgctcgg ggacccgata gagctcaacg ccctccaggc cgtctacggg    1080
   cagcgcgagg cggactggcc gctccacgtc ggctccctga agacgaactt cgggcacgcc    1140
   gaggccgcgg cgggcgtggg cggcatcatc aagacggtgg tggcgctcca ggagaggcag    1200
   atccccgccc acctccactt cgagaagctc aacccgctca tcaccatcga ccccacgcgc    1260
   gtcgtcatcc ccacccggcc cacgccgtgg caaacgcggg gggagcggaa gcggctcgcc    1320
   ggcgtcagct cgttcgggtt cagcggcgtg attgcccacg ccgtcctgga ggaggcgccc    1380
   gcgccggaag cggccgtcga gcagccggcg atgcccgccg gggagcggcc gctgcacgtc    1440
   ctgccgctca ccgcgaagca cccccgggcg ctgagcgagc tcatctcggc ctaccggcac    1500
   accgtcggcg cgctcgacgc gggccggctc gccgacctgg gctataccgc gagccggaag    1560
   cgcggccacc acgcgcaccg cgcctggttc gccttccgcg acaaggagga gctgctccag    1620
   cagctcgagg ccgcgacccg gggccacgag ctgcccggcg ccgtgctcca gcggcaggcg    1680
   cgcgacgacg tcaaggtcgt catggtgttc cccggccagg gctcccagcg ggtcggcatg    1740
   ggccagcagt tgatgcggac ctccccggtg ttccgcgagg ccatccacga gctggaccgc    1800
   gcgatgcggc cctgggcgga cttctcgctg ctggaggtgc tcggcgcccg cgggggcgag    1860
   gaccccttca cccgcgtctc catcgtccag ccgaccctct tcgccgtcca ggtggccctg    1920
   gccaggcagt gggaggcctg gggcgtcagg ccgcacgcgg tcatcggtca cagcatgggc    1980
   gaggtcgcgg ccgcgtgcgt cgcgggcgcg ctcagcatgg aggacgccgc cgcggtcatc    2040
   tgccgccgca gccagctcgt ccaggccacc agcggccagg gcgccatggc gctcgtcgag 2100
   ctgagccccg ccgaggtcga ggccgccatc gcgccccacg gggaccgcgt cagcatcgcc 2160
   gcgtacaacg ggccctccac cgtcctgctg tcgggcgagc cggccgccgt cgacgaggtg 2220
   ctcaccgccc tccaggagcg cggggcgttc tgccgccgcg tcaaggtgga ctacgcctcg 2280
   cactgcgcgc agatggaccc gctgctgggc cgcatccgga gcgagctggc ccacgtgaag 2340
   ccgcgcaagg gcggcgtgcc catctattcg accgcgcgcg gcgaggtcat cgacggctcg 2400
   cggatggacg cgggctactg ggcggacaac ctgcggcgcc ccgtcctctt ccacgcgcag 2460
   gtcaaggcgc tcctcgcgga cggcatgcac gcgctgctgg agatcagccc gcacccggtg 2520
   ctcctgccgt cgatgcagaa gacgatagac gaggccggcg gacagcagct cgtgctgccc 2580
   agcctgcgca gggacgagga cgagccgcgg agcctcgcga cgtcgctggg cgccctcttc 2640
   ggcctcggcc acccggtgga cttctccgcc ttcaccccga ggtccggccg gctcgtcgag 2700
   acgccgcgct acccctggca gcggcgcgag ttctggctga cgcggaaggc gtcgaagcgc 2760
   cggcacgcgc tgggcggcgc ggagggacat cccctcatcg gtggcgcctt ccgcccggcc 2820
   gtgggcccgg agtcctacta ctgggagttc gacgtcgacg aggagcgcct cgccttcatc 2880
   gccgaccacc gcgtgcgcgg catgacggtg ctgccgggga cggcgtacct ggacctcgcg 2940
   ctctcggcgg cgcgggccgc cttcggcgac agggccttcc ccctcagcca ggtctcgttc 3000
   cgggaggcgc tgaccctgct gcccgagcag ccgcggcggg tgcagctctc gctcgtccgg 3060
   gaggcggagg gcgcgttctc cgtctgcgtc tccagccgcg acgcggcgtc cccggccacc 3120
   ggggagtgga ccctccacgc gacgatgcga ctggacgtcg aggccccggc cacgggaggc 3180
   gctcgcgccg agggacaggt gacgcccccc gcgggcgccg agctccggga cgccaggacg 3240
   cactaccgcc tcatgaagga gggcggcctc gcctacggcc cgcgcttcca ggggctggcg 3300
   gaggtccact gcggccagca cgaggccctg gggcgcctgc gcgcgctgcc ggagctcggg 3360
   ctcgacggcg cggaccacgt cttccacccc gccctctggg acgccgcgca ccaggcgctg 3420
   gcggccctgc tcggccgcca ggacggccac gcctccggcc cgtccacctg gattccgcgc 3480
   ggcatcgagc gggtctcggt gtccggcacc gtcccggccc gggaggcgct gtgggtccgc 3540
   gccagcgcgc gggtggacgc ggacggcgcc acggcgcggg gagacctcca ggtcctggat 3600
   gacgccgggc gggtgctggt cgagacgcgc gggctgacgc tgctccggcg cgacgcggac 3660
   aacctcgacg acgtccgcca gtggttcttc cggccggtgc tccgcgaggc cccgctccgg 3720
   gaggaagccc ccgcggacgg gacgtggctc gtcctcggcg cgcccgggcc cctgcatgac 3780
   cgggtggtgg cggagctgcg cgggcgcggc ctcgacaccg tgcaggcgtt cccggccaat 3840
   acctgggagg agcaggacgc ggcgcgcttc cacctctccc ccgcggaccc cgagcacctg 3900
   cgccggctga tggagcgggt cagcgccgac cgtccgctgc gcggcctcat caacctccag 3960
   ccgtaccagg tggcgcgcgg cgacgcggcc tcctcggcgc cggagggcgt gctgtccgcc 4020
   ctctacatgg aggtgctcga ccaggcccgg gcgctccagg gcgtgagccg gcgggagcgg 4080
   ccgaagctgg tgctcgtctc gggcgtctcc cagggcgcgg aggccgagcc cgacttggtc 4140
   gccgccccgc tccagggcct gggccgcgtc atcgcggccg agctgccccg gctccactgc 4200
   gtccaggtcc agctcggagg tcaggagccc gaggtggagg cgcgcgacct cgtgcgagag 4260
   ctgtacgcca acgacggcga ggacgaggtc gtgctgcgcg ggggcgctcg ccacgtgcgc 4320
   cggctcgagc ggctcgtccc cggcgcggcc ccggcggcgc ccatccacgc ggacggaacc 4380
   tacctgctca cgggcggcct gggcggcctg gggctggcct tcgcgagctg gctcgcggac 4440
   cgcggcgcgc ggcacctggc cctggtcagc cggcgcccgc cctcggggga ggcccgcgcg 4500
   cggctcgacg cgctccgcgc ccggggcgtc gaggtgctcg tgcgcgaggt ggacctgtcc 4560
   cgggaggact ccgtcgccgc cctcttccgg gagctggacg agcgcatgcc gcccctgcgg 4620
   ggcgtcctcc acctggcggg cgtcctggac gacgcgacgc tgttccagca gaacgagcag 4680
   cgcctcgcga ccgtgcgcgg ccccaaggtc accgggacgc ggctcctgca cgcgcacacc 4740
   cagcaccgga agctggactt cttcgtcctc ttctcggcgg gcgccaccct cctcggctcg 4800
   ccgggccagg cgaactacgc cgcggtgaac gcgttcatgg atgcgttcgc cccgcaccgc 4860
   cagcggctgg ggctgcccgc gctctccatc aactggggcg tctgggacga ggtcggcctc 4920
   gccgcggcga gcgcgcagcg aggccagcgc ctgcgggagc aggggctgtt ccccatcccc 4980
   cccgcgcagg gcctccaggc cttcgagctg gcgctgtcgc tcgcgggcgc caacgtcgcc 5040
   atcctgccca tccgcctcgc ccgctggctg cgcgagcacc cggacctggc gggcgcgtcg 5100
   ctgttcaagg agctggtccg cggactggac gactccgagg cggaggccga ggcgcccatc 5160
   ctggagcagc tgcgcgccac ggtggacccg catgagcagc gcgagtgcct gcgccgctgg 5220
   gtcgggcgcc agctcgcggc ggtgctgcga agggaccccg cgtccatgca gcgcgagcag 5280
   aacttcagcg ccatgggcgt cgactcgctg atggcgctcg agttccgcaa ccggctggag 5340
   cgctcgctcc acgtccggct gccgccgacg atggtcttca accatccgag catcgaggcg 5400
   ctctgcgggt tcctcgagcc gaagctgggc atgggcgccc cctccgctcc ggcgcagccg 5460
   gagcccgtgc ctgctcccgc cgcgcccgtg cgcgtcgacg agaaggacgg cgccgcgctg 5520
   ctcggggccc tggcccggct gaagaagctc acccagcacc aggcgtag 5568
<210> 5
   <211> 4683
   <212> DNA
   <213> Angiococcus disciformis
<400> 5
   atgtcgatgt tggatgagct cagggcagtg ctggacagtc tgtcgccgga acagcgggcg 60
   acgctcgcgg agctgctgag cgctgacgcg ccccggaagg ccgacgagcc gatcgccatc 120
   gtcggcatgg ccggacgctt ccccggagca cgcgacctgg cggagttctg gagcgtgctc 180
   gccgaggggc gggacgccgt ctccgagatt ccaccggagc gctggaacgc ggacgccctc 240
   tatgacccgg acaaggacgt cccgggcaag ctggccaccc gctggggcgg cttcgtgaag 300
   gacgtcgacc tgttcgacgc ggagttcttc ggcatcactc cgcgcgaggc ggcggccatg 360
   gacccgcagc agcgcctgtt cctcgagacg gcctgggagg ccctcgagga cgcggggctg 420
   cccgcggcct ccctcgcccg gacgcagacg ggcgtcttcg tcggagtctc caactccgac 480
   tacatgtggc accagttccc ccggctcgac aaggtggacg cgtacgcgag ctccgggtcc 540
   gcgcactgca tcatcgccaa ccggctgtcc tacctgctgg acctcaccgg ccccagcgtg 600
   gcggtggaca ccgcgtgctc ctcgtcgctg gtgtcggtgc acctggcctg ccagagcctg 660
   cgcaaccggg agtgcgaggc ggcgattgcc ggcggagtga acgtcgtcct gacgccgcac 720
   ctgggggtga gcctctccaa gtgggggatg atggcgtcgg acggccgctg caagacgttc 780
   gacgcccgcg cggacggctt cgtccggggc gagggcagcg gcgtgctcgt gctcaagcgg 840
   ctggcggacg ccgagaaggc gggggaccgc gtcctggccg tcatcctcgg cagcgccgtc 900
   aaccaggacg ggaagaccca cggcctgacg gcgccgaacg gcgccgccca gcaggccgtc 960
   atccggcagg cgctggcccg cgcggggctg tctccctcgc aggtgtcctt cctggagacc 1020
   cacgggacgg ggacgtccct gggcgacccc atcgaggtcg aggccatcaa cgaggtgttc 1080
   ggctccaggg tcgagcgctg cgtgctgggc gcggtgaaga ccaacatcgg gcacctggag 1140
   ccggcggcag gtatcgccgg cctcatcaag gtcgtgctcg cgctgcgcca cgggctcatc 1200
   ccgcgcaacc tgcactacca gtccatgaac ccgcacctcc agctcgctcc gggcatcgag 1260
   ctggcgagcg aggcccggcc gtggaagcgc gacgccaggc cccgcttcgc cggggtgagc 1320
   gccttcagct tcggcggcac caacgcgcac atcgtcctcg gagacgcgcc cgcctacgcg 1380
   ccgccggccg agtccgcgcg gcccgccctg cccaccctcc tccccctctc cgcgagccag 1440
   gagggggcgc ggagccagat ggccgcgctc cacgccgggc tgctcgccac cgcggccccc 1500
   gagctcacgg tggacaccgt ggcgggactg cgcgcgctgc accgggacca cctggacctg 1560
   cgcctggccg tcgtcgcctc ggaccgcgcg acggccgccg aggccctgag gtgcttcgcc 1620
   gaggggcggc cccacgcggg cctggtccac gggcgccgcc agccgaacct gcggcccgtc 1680
   ttcatctaca ccggccaggg caaccagtgg ccgggcatgg gccgcgagct gatgcgcacc 1740
   gagccggtgt tcagggccgc ggtggaggag gcggacgccg cgttccagcg cctcacgggg 1800
   cggagccacg ccgccagctt ccgcgaggac gcgtccgacg cggcgtcgct ggagcgcacc 1860
   gacgccgccc agccggccat cttcctcctg cagtacgcgc tcacgaagac gctggagcac 1920
   tggggcctgc gtcccggcgc ggtcgcgggc cacagcgtcg ggaggtcgcc cgccgcctgg 1980
   gccgcgggca tgctggacct ggacgacgcg gtccgggtgg tccaccaccg ctcgcggctc 2040
   atgcagcgcg ccaccggcca gggggcgatg gcctccgtcg cgctgtcgcc acgggaggcg 2100
   cggcccctcc tggaggggcg cccctcgctg gccatcgcgg cgcacaacag ccccggggag 2160
   gtggtgctct cgggtgaccc cgccgagctc gaggccgtgc tccgcgagct ggaggggcgc 2220
   ggcgtccgct gcaggaagct ccccgtcaac tacgccttcc acggcccgca gatggacccg 2280
   ctccgcgacg agctgctgcg ggagctgtcc gtcctccggc cgaaggcgcc gcgcctggcg 2340
   ttctactcga cggtgacggg ccagcgcgag cggcaggccg tggccgacgc gagccactgg 2400
   gtccgcaaca tgagggaccc cgtcgcgttc cgcgaggtcg tcgagcggct cgcgaccgac 2460
   agcccgagct tcttcctcga gctcggcccg cacccggccc tcgtgccctc ggtgatggac 2520
   acgctggcgc acctccggct cgaggcgcgc acggcgcact gcctgcggcg caaccgcccc 2580
   gaccggcacg agctgctgtc cgccgtggcc tcgctctacg tccacggcat cgacccggac 2640
   tggtcgcgac tggtgcgccc cccggaccgc cggtgggcgc tgcccgccta cccctggcag 2700
   cgccagcgct tctggctgga agcggcgccc cccccggacg ccacctggag cgcgccgagc 2760
   gcgcggcccg ccggccacct cgtctaccgg accgagtggc tggccgacgc ggcctccgcc 2820
   tccggggaca ccgcgcggcc gaagggccac ctcgtgttct ccgacggcag cgccctggcc 2880
   gaggccctcc gcgccgagct cgaggcggcc ggcgcgcgat tcgggtgggt gagccgcggc 2940
   caggacttcg ccgcggacgg cacgcgctgc accgtggacc cggagaaccc cgagcacctc 3000
   gagcggctct tcggccacct cgcggccgag tccttcgagt tcgaccacgt cctgctgctg 3060
   ttcaccccgg tggcggacga ggcgcggccg gacggcgcgg cgccgtggtt cgacacggcc 3120
   gtgcgccccg tgcacgtcct gcgctgcctg acccaggccc tgctcacgac cgagcgccgg 3180
   ccgaggatgg cggtcatcac ccgggcggcg ctgagcgcgg gcggcgacac cgcggcggtg 3240
   ccccagcaca cggcgctctg ggcttcgggg cgcgcgctgg cgctggagga gccggggctc 3300
   cgcctggcgc tgctggacct tccggcggtg cccgacggcc tcgtggaggc ccggcacgtc 3360
   ctggcgcgcc tcgccagccc gacgcccgag cgccagtcgg cgtggcggga cgggcggtgg 3420
   cgcgtccccc gcctcatgcc ccacgaggcc gggacgggca cgtccgcgcc gcccgtcatc 3480
   cagggcgagg gcacctacct catcaccggg ggcctcggct cgctggggct cgccaccgcc 3540
   cgccggctcg tccagcgcgg cgcgaggagc gtggcgctcg tctcgcgccg cccgcccacc 3600
   gccgcgcagc aggccgagct cgcgcgcctg gaggaggcgg gtgcccgggt ggtgacggtg 3660
   cagggcgacc tggcggacct ggcggaggcc acgcgcgtct tcgagacgct cgcggcgtcg 3720
   gcgcctccgg tccggggggt gattcacgcg gccggcgtgc tggaggacgg agtgctcggg 3780
   agcgcgacga aggcgagctt cgaccgcgtg ctgcggccca aggtcgccgg gagctggaac 3840
   ctccaccagc tcacgcagtc cctcccgctg gacttcttcg tgctgttctc ctccgccacc 3900
   gcgacgctgg gcgccgcggg ccagacgaac tacgccgccg ccaacgcctt cctccagggc 3960
   ctcgcggagc accgccgcgc ccgcggccag cacgccctct gcctgcactg gggcgcctgg 4020
   cgcgagagca ccatggcctc cggcgccgtg gtgacgaagc tgaaggcgct gggcttcgac 4080
   accctggcgc ccgaggacgc gctggacgcg ctggagcgcg tgctcctcga aggggcgacg 4140
   gaggcgacca tcatcgccgc cgactgggag gtcttcgccc agcggctccc ggacgagagc 4200
   ctgggccggt tcctcgagcc cgtgctgcgc gcgagcgggg ccccgccggc cgtgaagccc 4260
   gaggccgacc ccatcgtctc acgcctcgcc ggcgcgccgg cgagccagcg ccagaagctg 4320
   ctctacggcc acgtccgcgc ggtggtgctg tccaccatgg ggatggacgc ccgcaccgag 4380
   gtccgcccgg accaggggct catcgagctg gggctggact cgctgctcgc ggtgaacatc 4440
   gccaaccggc tctcgcgcga cctggcccgc cagctgccca agacgctcat ctacgaccac 4500
   ccgacgctgc acgccctgac ggaccacctg ctgggcgtgc tcttccccga gcccgcccag 4560
   gccagcgtcc ggcccgccgc ctccggcgac gcgccctcct acgaggacat cgccaacgcg 4620
   agcgcggaca ccatccagag cctcctcgac gccgagctcg cgagcctcct ctcctcccgc 4680
   tga 4683
<210> 6
   <211> 3237
   <212> DNA
   <213> Angiococcus disciformis
<220>
   <221> misc_feature
   <222> (3038)..(3047)
   <223> n is a, c, g, or t
<400> 6
   atggagctca aggcccagct cgcgcgggcc gagtcgcggc agcgcgcgcc catcgccatc      60
   gtcggcatgg cctgccgctt cccgggtggc tgcgccacgc ccgagcagtt ctgggagctg     120
   ctgcgcgagg gccgcgacgc ggtcaccgag gcgttcccct cgcggtggcc ggagtcgctc     180
   tacgacgcca accccgaggc gcccggcagg atcatggcgc ggggcctggg ccaggtggag     240
   ggcgtggccg acttcgagcc ggagttcttc cacgtctcgc cgcgcgaggc cgcgagcatg     300
   gacccgcagc accggctgct gctcacggtc gcctgggagg cgtgcgagga cgccgggctc     360
   acccgggagc tgctgcacgg cagccgcacc ggaatcttcg tcggagcggg caacaacgac     420
   tacggcgact tcgagctgcg caaccccgac ctgggcgcgg tcagcgccta cgcgggcacg     480
   ggcaacgcca cctcgctggt cgcggggcgg ctctccttct tcctcgggac gcagggcccc     540
   gcgctcgtgg tcgacacggc gtgctcgtcc tccctcgtcg ccatccacca ggcgtgcacc     600
   agcctgcgcc tgggtgagtc ggagctcgcg ctcgctggcg gcgtcaacct ggtcctccgc     660
   cccaacggca gcgtgttcct ctcgcgcttc ggcgcgctct cgcccaccgg ccggtgccgg     720
   gccttcgccg aggcggccga cgggtacgtg cgcgcggacg gctgcggcgt catcgtgctc     780
   aagcgcctgg aggacgccct gcgggacggg gaccgggtgc tggccgtcat ccggggctcg     840
   gccatcaacc acgacggccg cagcggcggg ctcaccgtcc ccaacggggc ggcgcagcgg     900
   gagctgctcc tggccgccct gaagaacgcc ggggtggagc cgcggagcat cggctacctg     960
   gaggcccacg gcacgggcac gccgctcggc gacccgatag agatgggcgc catcgccgac    1020
   gtcttcggcg gggacgcgcg gcgcgagtcc ctgcgggtgg gctcggtgaa gagcaacatc    1080
   gggcacaccg aggcggcggc gggcatcgcc ggcatcatca aggccgtcct cagcctccag    1140
   caccgggaaa tccccgccca cctccacttc gacaagccca gctcgcgcat cgactggagc    1200
   atccccgtcg aggtccccac gagcacgcgg ccctggagcg gggacggccc ccggctggcg    1260
   ggcgtgagct cgttcggctt cagcggcacg aacgcacacg tcatcctgga ggaggcgccg    1320
   cgccaggagg cccggcactc gccccggctc gggacgcagg tgctctgcct gtcggcccgc 1380
   accgagggct ccctcacgcg gctggccgcg gcctggaggg agcagctccg gcggagggat 1440
   tcggcgcccc tggccgacct gtgccacgcc gccgcgcggc gccgctccca ccaccgcgcc 1500
   cggctggccg tcctcggcga cagcgccgac gccatcgccc aggcgctgga gtccttcgcc 1560
   gccgggacgc cccaccccgc cgtggtgagg ggcgacgcgg tgctcgacgg gatgtcccgc 1620
   acggccttcg tcttctccgg gcagggcagc cagtggctcg gcatgggaca ggagctgctc 1680
   gccagcgagc ccctcttcca gcagagcctg cgcgaaatgg acgggccgtt ccgcgccgag 1740
   ttcggctggt ccctcgaaga ggcgctgcgg gccggggagg ccgagtccca gctcgagcgg 1800
   acgcgcgtcg cccagccggt catcgtggcc gtccagctgg cgctgagcga cctctggcgg 1860
   gcgtggggcg tgcaccccga cgtgacgctg gggcacagcc tcggggagat ctccgcggcc 1920
   cacgtcgccg gcgtgctcgg gcggcacgag gccctgcgcc tggcctgcat ccgcggccgg 1980
   ttcatggatg gggccaccgg gcgcgggaag atggccgagc tcgcctgctc cgaggagcag 2040
   gcgcggcgct acctccaggg cttcgaggcc gaggtcgcca tcgccgccct caatggcgcc 2100
   atgtcgacgg tgctcgcggg cgcgccgggc gccctggagg ctgtcctcca gaaggtcgcc 2160
   agcgacggca tccagcaccg cctgctgcgc gtggactacg ccttccactc gccgcagatg 2220
   gccgagttcg cggcgccctt ccgcggcgcg ctgggcgaca tgaagccccg cggcgccgag 2280
   cgggacctct acagcagcgt gctcggccgc gcggtgcgcg gcgaggcgct gacggccgac 2340
   tactgggtgc ggaacatgac ggacaccgtg cgcttccacg aggcgctcgt ggcgctcctg 2400
   cgcgacggct gccgcgccgt ggtggaggtc tccccccacc ccgtcctgct ctccgccatc 2460
   caggacaccc tgagccgcga gggcgtcgac ggcgtcgccg cgtactcgct ccggcgcggc 2520
   aacccggagc gcgccaccct gctgtcgaac ctcggcgccc tgtacgccgc cggccactcc 2580
   gtggactggt ccgcgctcta ccccgggctg gtggaccacg tggccctgcc gacctacccc 2640
   tggcacgccc ggcgcttctg gacgaccgtc cagggcccgg aggccaccac cggcgcggcc 2700
   cccgccgtca ccgccccggc cccgaacgag gccgcccccg ccgccgtcac ctcgaagctg 2760
   tgggacgagc tcgtcaagct ggcggccgtc gcgcgcaggc cgcgcctgga gcgccatgtc 2820
   cgcgagcagc tcacgggcat cctcaagctg gactccgggg acatcccctc gaagcgcacg 2880
   ccgctgagcg agctgggcat ggactccgtc ggcgccgtcg cgctgtccaa ggcgctggcc 2940
   ctcacccacg agcaccggtt ccctcccacc gtgctcttcg agcaccccac gattgaagcg 3000
   ctgaccgggt tcctcgccga ggagttcgag cggctccnnn nnnnnnnccg cccccccccc 3060
   cccccggccc cccgccgctc cggaggccgc cgcgctcccg gtgcccgact cggagctcgc 3120
   catcctggag aagctcgccg ccctcgagaa ggagtttgcc tgacatgtcc acaccggaga 3180
   atcccacggg cgaccgcgca ctcctccacc gggcgctggc cgccatggag aagatga 3237
<210> 7
   <211> 7182
   <212> DNA
   <213> Angiococcus disciformis
<400> 7
   atgaagggcc gcatccagca gctcgagggc gtgacgaccg agcccatcgc catcatcggc     60
   atgggctgcc gcttccccgg cggcgtccac ggccccgacg ccttctggcg gttcctcgcg    120
   gacggcgtca acggcatccg tgacacgccg aaggaccgct gggacaccga gcgctacctc    180
   gacgccgagc agggcacgcc ggggaagatg tacacgggcc acggcggctt cctggaccgc    240
   gtggacgggt tcgacgccgc gttcttcggc atctcgcccc gcgaggcgct gagcatggac    300
   ccgcagcagc gcctcttcct ggagacgtgc tgggaggcca tcgagaacgc gggcctgtcc    360
   gcggacgtgc tcgagggcag ccagacgggc gtctacgcgg gcatcgtcaa ccaggactac    420
   gccatgatgc agatgaagga caccgcgcat ggcgagatgg acacctactt cgccaccgga    480
   cacccgcact gcgtggtgag cggccgcgtg tcctaccagt tcggcctccg cggccccacg    540
   ctgtccgtgg acaccgcgtg ctcgtcgtcg ctggtggccc tgcacctggc ctgccagagc    600
   ctgcgcctgg ggagctgcga cgtggccctg gctggcggca tcaacctgct gctgatgccg    660
   gagatctaca tgatccgctg ccaggcgcgc atgctgtcgc ccagcgggcg gtgcgccacg    720
   ttcgacgcgt cggcggacgg atacatccct ggcgagggcg tgggcgtcgt cgtgctgaag    780
   cggctgcgag acgcggtccg ggatggggac cgcgtcgacg ccgtcatccg cggcacgcag    840
   atcaaccacg acggccgcag cagcgggctg accgtgccga gcggggccgc gcagcgggag    900
   ctgctgcgcg ccacgctgcg cgacgcccgc ctggagcccc agcgcgtcgg ctacctcgag    960
   gcgcacggca ccggcacgaa gctgggggac cccatcgaga tggccgccat ccaggacgtc   1020
   tactgccagg accgcgcgcc cggaaacccg ctggtcatcg gctcggtgaa gaccaacatc   1080
   ggccacctgg agtccgccgc cggcatcagc ggcctcatca aggccgtcct caccctcaag   1140
   cacggcgcca tcccgcgcaa cctccacttc tccgagctca acccgcacat cacgctgcac   1200
   ccggcgaccc ggattcccac cgagctcacc ccgtggccgg cgggcagcgg cccgagggtc   1260
   gccgccgtca gctcgttcgg catgagcggc accaacgccc acgccatcct ggaggaggcc   1320
   cccgcgcgcg aggccgccag cgcccaggcg cccctccgcg tcacctcccg gccggcgacg   1380
   ccgcggctca tcccgctcag cgcccaccac cccgaggtcc tccaggccca tgccgccggg   1440
   ctgaaggaac acctggacgc ccacccggac gtcaccttcg acgacctggc gcacacgctg   1500
   cgccggcgca ccctccagcg ccaccggctc gccctgcccg ccagctcgct ggccgaggtg   1560
   aaggagcacc tggcggcgtt cacggccggc gggcaggacc cccgcgtcct ccagacggag   1620
   gacctcttct tcaccgaccc gaaggtcgtc ttcgtcttcc cgggccaggg ctcgcagtgg   1680
   gtgcggatgg ggctccagct catggagcag gagccggagt tcgccgcgga gctgcgcgag   1740
   gtggatgcgt ggatccagaa gtacgccggc tggtccctgc tggacgagct ccgcgccgac   1800
   gcgagcgcgt cccggctgag ccaggtcgac gtcgtgcagc cctgcatctt cgccgtgcag   1860
   gtcgccctcg cccggctgtg gcgctcatgg ggcgtcgtcc ccaacgccgt catcggtcac   1920
   agcatgggtg aggtggcggc ggcctgcgtc gccggcatcc tgtcgctcga ggacgcggtg 1980
   tgcgtcatca cccgccgcag tcagatcgcg aagcgcacca gcggcaacgg cgccatggcg 2040
   ctggtggagc tgacgcgcga gcaggccgag cgggccatcg ccgcgcacca ggacgtggtc 2100
   gcggtcgcgg cccagaacgg gccccacacc gtcctcctct ccggcacgcc gggccccatc 2160
   gagcagatcc tccgcgagct gcagcagcag gacgtcttct gccgcgccat caaggtcgac 2220
   tacgcctccc actccccgca gatggatccg ctccggcccg agctccagcg ggcgctctcc 2280
   ggcgtgaagc cccgggcggc caccgtcgcc ttctactcga cggtgacggg ccaggtggag 2340
   gacggcacgt cgcacgacgc gaactactgg gtgaacaacc tgcgcgaccc cgtcctcttc 2400
   tggccgggca tgcagaagct cgtcgaggac ggacacaaca tcttcctgga gctcagcccc 2460
   caccccgtcc tcatccccgc catctcccag ggcctgcagc acctgggcgc ccgggcgctg 2520
   agcctcccgt cgctccaccg cgagaagccg gaggccgagt gcctgttcga gtcgcgcgcc 2580
   cggctcttct gcgccggcgg caggctggac ctcgcgaagg aggacgtccc gggcgcccgg 2640
   gtcctcgccc tgcccggcca cccctggcgt gagcagcgct tctggtacac gcccagcgcc 2700
   ccctcggcgc acccgcaggc ccgcggcggc agccggctgc tgccccgcat gtggacggcg 2760
   ggctccgggg agcggcgcgt cttcgagggc gacgtcggcc ccggcgtcat gccctcgctg 2820
   gcggaccacc gcgtcaatgg gatgcagctc ttccccggcg ccggctatgt cgagctggtg 2880
   ctcgaggccg ccgaggcgct cgggctcgag ggccggacgc acatcgactc gctcgacatc 2940
   cgcgaggggc tggtgctcca cgagggcgcg gcgcgcgtcg tccaggtgga gctgacgccc 3000
   gtggccgcgc gctccttcgg gttcgtcatc agcagccgcg aggccgacgg cgccgcctgg 3060
   acgcgccatg ccgacggcgt ggtgacgcgc gaggaggccg gggcggcgcc gggcgcggag 3120
   cccggcgcgc tggaggcctt gcgcgcgcgg ctcgcctcgc ccctgtcggc ggaggaccac 3180
   tacctccgca cccgggagcg ggggctggag tacggccccg cgtaccaggc catcaagcac 3240
   atcggccgcg ggcgtggcga ggccctggcg gagctgcacg tgccccaggc cgtcgcggag 3300
   gagctgagcg ggtgccagct ccaccccagc ctcctggacg ccgggttcca gctcatcgac 3360
   acggcgctgg ccgggtcggg cctcgtggag gagggagacg tctacctgcc cgccgccctc 3420
   cgcggggtgc ggttccaccg caagccggcc cccggacaga cgctccatgc gcacctgggg 3480
   ttgagcccgg agaagccgcg cgaggcgtcg gtgctggagg cgcggctggc gctcctggat 3540
   gaccagggcg ctcccgtcct cacggccgag cggttcatca tccgccgcgt cgagacccac 3600
   gccgccgcct gggaggggct gccgggcgag tggttctacc gcctgcgctg gcagccgctg 3660
   gagggctggc agccggaggc cgccggggcg aggcgcgacg aggcctggct cctgttcgcg 3720
   gacgaccagg gcgtggccgc ggcggtggcc gaccggctcg cgccctcggg cgtgaggacc 3780
   acgctggtgc gcaaggcgcc cggcgccgtc acgcgggcgg ggagcggctg gacgctcgac 3840
   ccgagcacgc cggagtcctt ccgcgagctg ctcgccgcgg tgccgccggg ctccttcacc 3900
   cgcgtgctct acttctgggg actggacgcg cccgcggtgg accccgagcc ggacgccctg 3960
   gacgcgcggc tgcccgagac ggaggccctc atctacctgg tccaggcgct cgccgcccgg 4020
   gaggcgcagc cggggccgcg cctgtggctc gtcaccgcgg gcgccctccc cggcgccgac 4080
   ggcgaggccg tgtcgcccat gcagaccccg ctctgggccc tgggccgcgt cctggactac 4140
   gagcacccgg agctgaagac cgtctcctgc gagctggagc gtcacgtcac ggagaagcac 4200
   gtcgccgtgc tgacctcgct gctcctcgcg cccgaggtgc ccgaccaggt gcgcgtgcgc 4260
   gccgacggcc cggccgcggc gcggctggcc cgctacgcgc ccccggcccc ccgggacacg 4320
   ccgcaccgcg tgtcgccgca cgaggcggcc gtggtggtgt cgggcaacgg ccccgcgccc 4380
   gcggagctgt ccgtcgccag gcgacgggcc cccgcggccc acgaggtcga ggtcctggtc 4440
   acccacgcgc acctcgcctg ggcgcccgcg tcccccgacg cgggcggcct cgtgccggga 4500
   gggttcttcg cgggccgcgt cctccggctc ggaggcgagg tccgcgggct ctccgtgggc 4560
   caggccatcg tggccccggc gggcggcgag ctccgctcga ttcgcacggt ggacgccggt 4620
   ggcgtgcgcc cgctgccgga gcaggccgac cccgcgcggg cgctctgcgc gctggggacc 4680
   cgggccctgg cgcggcacct cctgcgccag ctcgcccccg tcgagcgagg ccagacgctg 4740
   ctgctgctcg gtggcgacct ccccctcctc cagtccctcg cgcgggtggc cacgggccac 4800
   ggcgcgcgcg tcctcgccct ggtccgggac ggcgcgcacg cgcccgcgct ccaggaggcg 4860
   ggcgccgagg tggtgggccg gctggacgag cccggcgcgt tcgagcggct gcgcgagcgc 4920
   gccggtgcct cgggcgtcga cgtcctcatc aacctcgcgc ccggcgccgc ggtaccgcgg 4980
   gcgctcgacg tcttccgggc gggcggcgtc ttcctggacc tgacgcgcgc gtccccggtc 5040
   ggcccgaagg agctgcccgc ctcggcgacg ttcctctccc tggggctcgc accgctctac 5100
   gcgcgcctgg cgccgcccac gccggaagcc atcgccgagg ccagcgcgcc cgcggaggcc 5160
   cctgcgcgcg tgtggagcct ccaggagctg acgcagccgc gggcggaggc gccgctcgac 5220
   ggccaggccc acgtcctcgg cgtgtcgtcc gaacccgtga cggtgggggc cgggagcgcc 5280
   ccgctcgcgc tctccgacac cgcgacgtac ctcatcaccg gcggcctcgg cggcctcggc 5340
   ctccgggtgg cgagcctgct ggtggaccac ggcgtgcgcc acctggcgct cgtcggccgc 5400
   tcgccgccga gcgcgcaggc ccggagcacc ctccgtgagc tggagcagcg cggcgtccgg 5460
   gtccaggtgt tcgccgccga cgtcgcccgg cgcgaggacc tgctcggggt ggtgaaggcc 5520
   ctccaggccg gcgacgcccc gctccggggg gtcatccact gcgccggcgt cctccaggac 5580
   ggcgcgctcc tcggcctgga ccgcgagcgg ttccgctcgg tcctcggcag caagctgagc 5640
   ggcgcgtgga acctgcacgc cctcacccgg gacctgccgc tcgaccactt cgtgctcttc 5700
   tcctcggtgt cctcgatgct gggctccccc ggccagggca actacgccgc ggcgaacgcg 5760
   ttcatggacg gcctcgccgc ctggcggcgc gcccgggggc tgcccgcgct cagcgtcaac 5820
   tggggcgcct tcagcgacat cggcctggcg gccgtcggcg agcggcgcgg agaccgcctg 5880
   gccgacaacg gcatccccgg catccctccc gagaagggca tgaaggcgct cgagctggcg 5940
   ctgcgctggg gcgagacgtc cctgggcttc ttccccttcc ccttcctcat ctggcggaag 6000
   ctcaacccgc agattgcccg caacacgctc ttcaccgaca tccgcgcgga gagccgctac 6060
   cgcgaggagg ccagcggcga gcagcccatc gccgaggcgc tcgccctcgc ggacccggcc 6120
   gcccgccgga gccacctcga cggcttcctg aagcagcagc tctcgcgcgt actcaaggtg 6180
   ccggcggagc aggtccgcga cggcctcacc ttcaaggaga tgggcttcga ctcgctgatg 6240
   gccatcgagc tgcgcaaccg gctggaggcc ggcctcacgg tgtccctgtc gtcgacgctc 6300
   gtctggcgct acccgacgct cgaggagatg tcgcggttcc tgctccagct cgtcggcggc 6360
   gggagcgaga cgccctcgga cgcacggccg gcgtccgccg ccccggcgcg ccggggccgc 6420
   tggctggtgc gtgagaagcc gagtccctcc gcgaagctcc ggctgctctg cttcccgcac 6480
   ggcggcgccg gcgcctcggc cttccagggc tggtggcagc agttcccgga cacggtggag 6540
   gtcctcacct tccagccgcc ggggcgcgag gagcggctcc aggaggagcc cgagcgcgac 6600
   atgggggcgt atgcctctgc catcgtcgag gagctccagc cgctgctcga cctgcccttc 6660
   gccgtgttcg gctacagcct cggcggcctc accgggttcg ccaccgtggc cgagctgcgg 6720
   cggcgcaacc gcgtcacccc ccggcacctg ttcgtgtcgt ccgccttcgc cccgcacatc 6780
   cccttctccg agacgacgct ggcccgggtc tccacccaga cctcggcgct gaaggccatg 6840
   gccttctacc agtccacgcc ggactcggtg ttcaaggacc cggagatgca ggcgctgctg 6900
   gcgcgctcga tggacgcgga caactccgtg gtcgagtcct accgcttcgg cgcggagaag 6960
   ccgctggact gccccatcac cgccttcgcg ggccggcagg acgacctcgc tccgctccac 7020
   gagcagctgc gctggaagga gctcaccacc caggccttcg agctgcggcc gttcgagggc 7080
   gaccacttct tcttcctgag agacaagcgc ccggtgatgg acgagctgcg gcgccacctg 7140
   ctctccctca tcgcggactc ccgggacggc tcgtcccgct ga 7182
<210> 8
   <211> 7158
   <212> DNA
   <213> Angiococcus disciformis
<400> 8
   atggatgcct cctcctcccg ccccttcgag tcactggacg cccttcaggc gtgggtcgtc      60
   acctggctgt gcgagcatgc ccggattccc cagcaggcca tcggcctcga cgtccgcctg     120
   agggacctcg gcctcgactc ggcccgggcc acgaagctgg tggccgcgct ctccgagcgc     180
   ttcggccgga cgctcccgct gacgctgctg tgggactacc cgaccgtcac ggagctgtcc     240
   cggcacctga tgaagcccgc gacggcggcg gcggccggcg ccacgccccg ggccgagcgt     300
   cgcgcgagcg gcgctccgga ggaccgcgcc atcgccgtca tcggcatggc ctgccggttc     360
   ccgggcggcg cggacacgcc cgaggccttc tgggagctgc tggcgaaggg gctcgatgtg     420
   gtggggccgg tgcccgcgga ccgctacgac gcggaggcct tctacgaccg tgacccgcgg     480
   gcccgcggca aggccgtcac ccggtggggc ggcttcctgg agcggaccga caccttcgac     540
   ccctacttct tcggcatctc tccgcgagag gccatcgaga tggacccgca gcagcgcatg     600
   ctgctggagc tggcctggga ggcgttcgag gcggcgggcc tgacgaagga cgggctccgg     660
   gaccgcgcgg tcggcatctt cgtcggcgcc atgtggaatg actacgcccg gctgtggcgg 720
   ggcggccccg gcgccatcga gcagttcacc gcgaccggcc aggacgacag catcatctcc 780
   gcccgcatct cctacgtctt cggcctgcgc ggcccgagcc tgacgctgaa caccgcctgc 840
   tcgtcctcgc tggtgtccgt gcacatggcc tgccagagcc tgctggcggg tgactccgag 900
   ctggccctgg cgggtggcgt caacctcatc cacggcccgg acagcaccat cgccatgtcg 960
   aagttcggcg gcatggcgcc ggacgggaag tgcaaggcct tcgacgcgcg tggcaacggc 1020
   tacgtccgcg gcgagggcgc gggcgtcgtg ctgctcaagc cgctgcaccg cgcgctcgag 1080
   gacggcgacg tcatccacgc ggtcatccgc ggctcggccg tcaacaacga cggcttctcc 1140
   aacggcctga cctcgcccag cccggccgcc caggccgcga tgctgcgcgc ggcctacgcc 1200
   agcgcgggcg tgtccccggc gcgggtccac tacgtggagg cgcacggcac cggcaccatg 1260
   ctgggtgacc ccatcgaggc gggcgcgctc ggcgaggtgc tcgggcagca ccccgagcgc 1320
   cagcagcccc tgcgcatcgg gtccgcgaag acgaacatcg gccacctgga ggccgccgcc 1380
   ggcatggcgg gcctcatcaa gaccatcgag gcgatgaagc accggcgcct gccaccctcg 1440
   ctccacttcg agcagcccaa ccccaacatc cacttcgagg ggctccgcct gcgggtgcag 1500
   accgacctct caccgtggcc caccgaggac gactcgctgc tcgcgggcgt cagctccttc 1560
   ggcttcggcg gcaccaacgc ccacgtggtg ctcgagtcct tccagcccgc ccggccggtg 1620
   gccatcccct tctccgcgcc ggacgcggag gcgctgggcg cccggctcgt cgagctgcgg 1680
   gccttcgcgg agcagcaccc cgaggcctcg ctggacgacc tccagcgcgc gtgggtccag 1740
   cgcctcaccg aggggccggt gcgccaggtg ctcctcgccc gctcccggaa ggacctcctg 1800
   gagcagctcc gcgcgccggc acggccggcg gctcccgcgg ggcgcggcgc gacgcggccg 1860
   gtcctcgtct tcaccggcaa cggcgcgcag taccccggca tggcgcggga cctgatgctc 1920
   ggggacgcga ccttccggca ggcggtcctc cgctgtgacg aggcgctgcg ggcgcacggc 1980
   tgcgacgggg tggccctggc gctcgagtcc ggtgacttcg acgtgcggcg ggtggacctg 2040
   gtgcagccgc tgctgtgggc ggtgcaggtc ggcctcgtct ccctgtggcg cgcgtggggt 2100
   gtggagcccg ccgccgtcgt cggccacagc gtcggcgagg tggccgccgc gtgggccgcg 2160
   ggcgcgctcg acctggacga ggccgcgcgc gtcgtggtcc accgcagccg gctgcagcag 2220
   gagtgcgcgg accagggcgg gatggtgctc ctcgagaccg acgccgaggg cgcgcgctcc 2280
   ctcgtgggcg ccacggcggc ggaggggctg gccgtcgccg ccctcaactc gcgcggcagc 2340
   accgtcgtga gcggccccac ggcgagcctc caggcgctca tcacgaaggc ggaggagcgc 2400
   ggggtgacgg cgaggacggt ccgcgtcaac gtcgcgtacc acggccccgg gatggacgcg 2460
   ctgcgcgagc ggctgcggga gtccctcgcg gacctgcgtc cgtgccctcc ccgcgtcccg 2520
   atgtcctcct cggtgacggg gggcgcggtg gggggcgcgg gcctggacgc cggctactgg 2580
   gtccgcaaca tggccgaccc cgtgctgttc ggtgacgcgg tcgacgcgct ggtgagggac 2640
   ggccaccgcc tgttcctgga ggtgggcccg caccccacgc tccaccgggc cgtccaggag 2700
   cagctccagc acggaggcgt ggccgggggc ttcgtcgcga cgagcctgga gcgcgagcag 2760
   cacggccgcg cccgcctgct ggagaacctg ggccacctgt tcgagcgcgg tgtccgcgtg 2820
   gcgtggcggg cgctgacgcg gggaggcgcg ccggcctccg cgggccaggc gcccggcccc 2880
   gtcctcctcc ccctctccgc ccacacggac gtggcgctcc agcagcgcgt ggaggggctg 2940
   cgcgcgtacc tgcgggcgaa cccggaggcg gagctggagg ccctggcccg taccctgggc 3000
   cggggacgca cccacctgtc gcaccgctgc gcgctcgtgg cctcctcggc cggcgagctc 3060
   gcggaggtcc tggagcgcgc ggccggcgcc gacggcgcgc ccgagctcat ccgccccgcg 3120
   gcctccctcg acggccgcgc gaaggtggcc ttcgtgttcc ccgggcaggg ctcccagtgg 3180
   ctcggcatgg gccgccagct cctggagacg gagcccgtct tccgcaaggc gctccatgag 3240
   tgggaggccg tgctgggaga gttcaccgac tggtcgctgc tggagcagct ccacgccgac 3300
   gccgcgacgt cccgcctggg cgaggtggac gtcatccagc cgaccctctt cgccatccag 3360
   gtcgcgctgg cggcgctgtg gcgctcgtgg ggcgtgcacc cggacgccgt catcggccac 3420
   agcatgggcg aggtcgccgc ggcctacgtc gcgggggcgc tggacgcgcg tgacgcggcc 3480
   cgcatcatct gctcgcgaag ccgcatcgcg aagaagacga gcggccgcgg cagcatggcc 3540
   gtggtcgagc tgtcccccga ggagtgccag cgcgccatcg gcgcccgcgc cgccacggtg 3600
   agcctcgccg cctgcaacgg gcccgcctcg tgcctgctgt cgggagagac ggccgcggtg 3660
   gaggagattg gcagggagct ccaggcccgg ggcgtcttct tccggctcgt caaggtggac 3720
   tacgcgtccc acagccctca gatggacgcc atcaaggacg agctggcgca cgtccttcgc 3780
   gagacgcacc cgcgcccggc caccgtgccg ttccactcca ccgtggagaa cgccctcctc 3840
   gacgggacac ggctggacgc cggctactgg gcgcgcaacc tgcgcgagcc ggtgctcttc 3900
   ttccagggca tccaggcgct ggaggcccgc ggcttcaacg tgttcctgga gctcagcccg 3960
   caccccgtcc tcacctactc catctcgcag acggtgcgtc cggagagcgc gctcatcctc 4020
   acctcgacga agcgcgagga ggacgagtcg cgggcgctgc gcacgtcgct gggcgcgctc 4080
   ttcgccgcgg gcggcgccgt ggactgggcc gcgctgtacc cgggcgtggc gacgcccgtc 4140
   gagctgcccg cctatccctt ccagcgggag cggttctggt tcgacgaggc ggaggtccag 4200
   cggtccgcgc ccgcgggcac cggccgccac gcgttcctcg agcgcttcct ccgctcgccg 4260
   tacccccagg agcgcctcat ctgggagacg gacctgagcc tcgagcgctt cccgtacctc 4320
   gtggaccatc gcgtcagcgg cctcgccgtc ttccccggca ccggctacat cgagtgcgtc 4380
   ctggaggcgg cgaaggacct gtacggcgac gcacccgtcg agctgctcga ggtctccttc 4440
   gagaaggccc tcttcatcga ggacaccggc gcgaagacgc tgcagctctg catggagccg 4500
   gacggcgccg gccaccgctt ctccctgtcg agccacgacg cggcccagca gacgtggacc 4560
   cgccacgtga atggccggct cgtgcccgcc accgccgagc aggccgccca cgcgcgccag 4620
   caggtccagg acgtgctggc cggcgagccc cacgcgccgg tggacgtggg gacgttctac 4680
   gggaacctcg agcgcgcggg gaacacctac ggcccgaagt tccaggccgt gaagtccctg 4740
   cgctcgaacg ccaggggcgt ccgcggcgag gtcgtcatgg gcgaggagct ggccacccgg 4800
   ggcacccgca tccaccccgt cgtcttcgac gcctgcctcc aggtgatggc cacgcccctc 4860
   tacatcgaga aggccgcggg gggccgcgag agcgccttca tgccgacgcg ggcggcgcgc 4920
   atccgcttct tccgtgaggc cgaccgggcc gtggtcagcc acgtgcgctt cctgaacggg 4980
   gccaccgccg acgcggccgg cgacctcctc gcggacatcg acgtcacgga cgggcagggc 5040
   cagctgctgg cgcgggtgga gcgcatctgc ttccacctgc tggagcacgc caccatggcg 5100
   cagcgcccgt ccaccgacat gctctaccgg ctgacgtggg aggaggctcc ctcgccggag 5160
   gtcggcgcct cgctggcgcg ccgctggctc gtcttcgcgg acgaccaggg cgtcgccgag 5220
   gcggcggccg ggctgctgtc cgcctcggcg catgccgtgc accgcctcca cccggcggac 5280
   gccttccggc gcgacggcaa cgacttccac atccgcccga acgagcccga ggactaccgc 5340
   gccgtgctcc aggccctgct ggcctcgcgc ggccccgcgc tgccgctcgg cgtcctccac 5400
   ctgtggaacc tccgcctgcc cgagggcggc gccacggccg tgccggacct gcgggagatg 5460
   gcgaagctgg gcaacctcag cctcacccac ctgctgaagg cgctcgacgg ggccggcggc 5520
   tccgcgccgc cgcgcgtctg gattggcacg cggggcgcgt gggccctccc cgggagcgag 5580
   caggggctgg cgccggccca gtccgcggtc tgggggctca cccgggccat cgcgcaggag 5640
   cacccggccc tgtggggcgg catcatcgac ctggatccgc tcgaggcccc gcgcctccag 5700
   gcggagcggc tcgtcacgga ggtctccgtc ccccaaccgg cggaggaccg catcctctac 5760
   cggcagggcc ggcgtcaggt gtcgcggctg gtccgcgaca cggcccccgt cagcgccgcg 5820
   accgccacct tccgcggcga ccgcggctac ctcatcacag gggccttggg tgagctgggc 5880
   acgcgcctgg cggcgtggat ggtggcccgt ggcgcgcgcc acctcatcct ggcggggcgc 5940
   aaggcgctgc cggcccggcg gacgtggaag caggacgggc acgacgcggc gacccgggcg 6000
   aggattgacg ccgtcctcca cctggagcgc ctgggcgctc acgtccacac gcccgcgctg 6060
   gacgtggggg acggcgcggc gctggccgcc ttcctcgccg gctacgagca ggaggcgcgg 6120
   ccccccatcg ccggtgtcgc ccacctcgcg ggggtcatcc ggcccgagct gctggcccgg 6180
   ctccgcgagg agtccctcga ggagtccttc cagtcgaagg tctacggcgc gtggaacctg 6240
   caccaggcct tcagcgggcg cgggctggac ttcttcacgc tgttctcatc cgcctcggcc 6300
   gtcctgaact cgcccctgct ggggagctac gccgcggcca acgccttcct ggacggcctg 6360
   gcgcacttcc gccgcgcgcg gggcgagccg gcccagtccc tgaactgggg cttctggagc 6420
   gaggccggca tggccgcgcg ggacctccac ttcgacgcga gcgcgtccgg ccagcagggc 6480
   atgaggagct tcagccccgc ggtgggcctg gacatcttcg gcgggttgct gagccgcgac 6540
   ccggtgcagt cggtggtgat ggacgtggac tggaagctct ggctcgaggc ctacccggac 6600
   gccgccgcgt cgccgctgct gcgcaccctg cgcgccagcg agggcgtggc cgcgggctcc 6660
   gagcaggcgg cgccggccga ggaggcctcg cggagcctga gggacctgcc ggcggaggag 6720
   cgccggaagg ccgtcgaggg catcctgctc accgagctgt ccgcggtcct caagatgccg 6780
   gccgcgaaga tttcccggac ggcgctcttc gggagcatgg gcctggactc catgctctcg 6840
   ctggagctgc gcaaccggct ggagcggcgc acgggcctga cgctgccggg caccctggcc 6900
   tggaactacc cctccctcga gcggatggcc gcgttcttcg tcgagaagct cggcggggaa 6960
   ggcgctggcg tggcgcggtc cgagcgccag caggccggcg ccgcgagcga tggcgtggcg 7020
   aaggagccgg acatctcggc ggaggtcatc gaggccctga tgcagctggg cgtcctcgag 7080
   gcgaacgacc ccgtgctcca ggatccggtc gcgctcggca acctggtgcg cgaggcccag 7140
   cagtacctga aggcgtga 7158
<210> 9
   <211> 1221
   <212> DNA
   <213> Angiococcus disciformis
<400> 9
   atgacgtaca cacagctcct gcatcgccgg gtcggaggcc agctccgccg cttcctgctg 60
   ctgcggccct tcggccccgg cgtcgaggtc gacgaccccc tgcatccgcc cgagctcccg 120
   ccgtacgacg cggagcggga cctcgcgccc cggacacccc agttcctcca gcacgccctt 180
   cagcccgagg agcgggggcg tcccggggcg ccggggctgc ccatcaccac cagcgagctg 240
   tcggtcggct gcaacctctc cggggcgtgc tgctcgcagt atcccgtcat ccccacgacg 300
   gtggaggaga gccggcgcgc cgtggagaca ctgcgccggc tggcgctcgg ggcggccccc 360
   gggcgggccg aggacgcctt cgcccccgtg aatcccggcc ggcgcgacgt cctgtcgccc 420
   atcctccagg gcgatggctg tcccttcctt gcccaggggg cctgccaggt ccacaaggcc 480
   gggggcgcgg aggcgaagcc gtggacctgc cgccagttcc cgctgcaggt catccactgt 540
   ggagaccact tcgaggtcag catcctcccg cagtgcgcgt gcgcggcccg gacggtgacc 600
   cgcggctccg gcggggactg gccagaggac ccgctgcggg ggctcacgtc cgtcccgcgc 660
   gtcccggact cggtggtggt ggaggggcgg cggaccctcg cgcgcggcat gtacctccag 720
   tgggtgcggg ccctggcgga ccggctgtcc gcgccagggc tgggggagcc cgccctcgtc 780
   ctggacagcg cctcccagag cctgcgcgtg gctcccgggg ccctcacccc cgggtggctg 840
   ggcgagctgc gcgcccgcat gctgtccgag gccgagcggc tggagggcta tctcgacccg 900
   gccgggccgc agccgaaggg gacgcgctgg gtcgcgtccc tcgcggaggc cctgctggcg 960
   gacgcggacg cggagccgga gcccgcgccg agtgacgggc tggtgctcgc gctggcgctg 1020
   cgcgcccacc tcctgctgga gcttcccgcg ctgggggccg cgctggccga cctcacccgc 1080
   ctcgcccggc tggccagcct ctcgcggacc gtcgcgcccg cggaggccgc cgacgcccgc 1140
   ttcgagccgc tgacgctgct gctgtacctg tgggcgacga cgcggtggcc ggtctcctcg 1200
   gagggcgtga cgcggccctg a 1221
<210> 10
   <211> 1230
   <212> DNA
   <213> Angiococcus disciformis
<400> 10
   atgctgggcg gtctgaccgg aaggggagag cacatgacag gaacgtcagg ccagggggcg      60
   gggggcgggc gcatcgacac gcctccggag ggggcgctgc gcgcggtgcg gtcactggtg     120
   gagctccagg agacggagca ggcggcgcag ctctacgagg aactgggaga cgcgcagcgc     180
   gagcgcctgc gccgggaggc ggcccggcgt ccggcccggg agcgccgggg cctggtggag     240
   gtgctgcggc gggcgcgcga cttcctgggc gccgcccgcc tgatggaggg cgacggcgag     300
   gacgccaccg tggcggacct ctacttccag agcgggcagt acctggaggc ggcggaggcg     360
   tacctgcgcg ctggcgaggc ggagcgcgcc gccgcggcct tcgagcgggc cggagcgctg     420
   gagcgcgcgc tggacgtgta ccggcgcctg ggcgcccgcg aggcgatggc gcagtgcctg     480
   gtccgcctgg gccgcccctt cgaggcggcg gacctctacc gcgagctggg ccaggcgcac     540
   gccgaggtgg aggcgctggg cggggtgaag ccggaggact cgcggtacgt ggaggcggtg     600
   ctgcgcgtct gcacgctgct ggacgtggag ggcttcaccc accgggcgct cgcggtgctg     660
   gcggacgcgg tgagcagctc cgaggaggcg cgggcggacg aggcgctggt gaaggagaag     720
   gcgcggctgc tgcggcgcat gggcatggag gcggaggccg aggccgtgct gctgcggcgc     780
   gcgacggccg cggcgacgcc cgccgccaac gggtaccgct tcctcaaggc catccccatc     840
   ttcggcgagc tgtccctgga ggacatgaag gacctctacc ggctcgcgcg gcaggtcgtc     900
   atcgcgccgg gcacggtgct gctggagaag ggcgcgcggg gcacgggcct cttcgtcctg     960
   ctggacggca ccgtggaggt cttcagcggc cccgagcagg acgcgcgtca cctcaacacg    1020
   ctggggcccg gcgcgtacct cggggaaatc tccctggtgc aggacgcccc cgtctccgcg    1080
   cacgtccgcg cgcgcacctc cgtgcgcgcg ctgcgcatca gccgcgccga cttcgagcac    1140
   tacctcgaca cccacgaggc cgccgcgctg cgcatctacc ggctcttcac gcagaacctc    1200
   gccgcgcgcg tgcgggccct gagcacctga                                     1230
<210> 11
   <211> 369
   <212> PRT
   <213> Angiococcus disciformis
<400> 11
   Val Ala Phe Asp Asp Gly Ala Asn Leu Trp Ser Val Tyr Arg His Ala
   1               5                   10                  15
   Asp Val Thr Ala Ile Leu Ser Asp His Val Asn Phe Pro Thr Ala Thr
               20                  25                  30
   Ile Asp Asp Gly Gly Ala Pro Pro Ala Glu Trp Arg Arg Leu Phe Ser
           35                  40                  45
   Leu Asn Ala Phe Asp Pro Pro Arg His Thr Gln Leu Arg Asn Leu Ala
       50                  55                  60
   Met Arg Ala Phe Thr Ala Ser Ala Val Ala Arg Met Gln Asp Arg Ile
   65 70 75 80
   Thr Ala Ile Val Asn Gln Leu Ile Asp Ala Val Val Gly Thr Gly Arg
   85 90 95
   Thr Glu Leu Val Ala Asp Ile Ala Gly Pro Leu Pro Thr Met Val Ile
   100 105 110
   Ala Glu Met Leu Gly Val Pro Ala Ser Asp Gln Glu Ser Phe Arg Arg
   115 120 125
   Trp Ser Asp Glu Val Gly Val Arg Ala Asn Ala Leu Val Leu Asp Pro
   130 135 140
   Glu Asn Gly Val Arg Leu Leu Ser Glu Ala Leu Thr Pro Met Glu Asp
   145 150 155 160
   Tyr Val Arg Gln Gln Ile Ala Glu Arg Arg Arg Arg Pro Ser Asp Asp
   165 170 175
   Leu Val Ser Ser Leu Leu Ala Ala Glu Ile Asp Gly Glu Arg Leu Glu
   180 185 190
   Glu Leu Asp Leu Val Ala Phe Cys Met Leu Leu Leu Ile Ala Gly Asn
   195 200 205
   Ile Ser Thr Thr His Met Ile Gly Asn Ala Ile Arg Ala Leu Leu Lys
   210 215 220
   His Pro Asp Gln Met Ala Arg Phe Leu Ala Asn Pro Asp Gln Met Pro
   225 230 235 240
   Gly Ala Ile Glu Glu Leu Leu Arg Tyr Asp Ser Pro Val Leu Ala Ala
   245 250 255
   Pro Arg Trp Val Arg His Asp Thr Glu Val Gly Gly Lys Leu Leu Lys
   260 265 270
   Arg Gly Gln Arg Val Ile Val Trp Leu Gly Ala Ala Asn His Asp Pro
   275 280 285
   Asp Val Phe Pro Glu Pro Glu Arg Leu Asp Val Ser Arg Lys Ala Ala
   290 295 300
   Arg Gln Val Ser Phe Gly His Gly Ala His Tyr Cys Met Gly Ala Pro
   305 310 315 320
   Leu Gly Arg Leu Glu Ala Arg Ile Ala Ile Pro Ala Ile Leu Arg Arg
   325 330 335
   Met Pro Glu Leu Arg Met Val Glu Gly Ala Glu Leu Glu Pro Val Pro
   340 345 350
   Gly Tyr Leu Leu His Gly Met Val Ser Met Pro Met Thr Phe Arg Ala
   355 360 365
   Ser
<210> 12
   <211> 1570
   <212> PRT
   <213> Angiococcus disciformis
<400> 12
   Met Thr Gly Ile Ile Asp Lys Leu Ser Arg Leu Asp Ala Ala Ser Lys
   1 5 10 15
   Asp Leu Leu Ala Glu Lys Leu Arg Gln Ala Lys Asp Lys His Leu Ala
   20 25 30
   Arg Gly Ala His Ala Glu Pro Ile Ala Val Val Gly Met Gly Cys Arg
   35 40 45
   Phe Pro Gly Gly Ala Ser Ser Pro Glu Arg Phe Trp Gln Leu Leu Met
   50 55 60
   Gln Gly Arg Ser Gly Val Gly Arg Val Pro Ala Asp Arg Phe Pro Ile
   65 70 75 80
   Glu Asp Tyr Tyr Asp Pro Asp Pro Ser Ala Pro Gly Ala Ile Leu Ser
   85 90 95
   Asp Arg Gly Ala Phe Val Glu Gly Ile Asp Arg Phe Asp Ala Gly Phe
   100 105 110
   Phe Gly Val Ser Pro Arg Glu Ala Arg Arg Leu Asp Pro Gln His Arg
   115 120 125
   Leu Leu Leu Glu Val Thr Trp Glu Ala Leu Glu Asn Ala Gly Leu Pro
   130 135 140
   Gln Ser Ala Leu Arg Glu Leu Val Gly Gly Val Phe Ile Gly Leu Met
   145 150 155 160
   Gly Asn Asp Tyr Phe Ser Thr Val Gln Glu Asn Pro Ala Asp Ile Asp
   165 170 175
   Ala Tyr Thr Ser Thr Gly Thr His Tyr Ser Phe Ala Ala Asn Arg Leu
   180 185 190
   Ser Phe His Leu Asp Phe Lys Gly Pro Ser Met Ala Ile Asp Ser Ala
   195 200 205
   Cys Ser Ser Ser Leu Val Ala Val His Gln Ala Cys Gln Ser Leu Arg
   210 215 220
   Thr Arg Asp Cys Asp Phe Ala Val Ala Gly Gly Val Asn Leu Val Leu
   225 230 235 240
   Ser Pro Val Leu Ser Ile Ser Tyr Ser Lys Trp Gly Met Leu Ser Pro
   245 250 255
   Asp Gly Gln Cys Lys Thr Phe Asp Ala Ser Ala Asn Gly Phe Val Arg
   260 265 270
   Gly Glu Gly Ala Gly Val Val Val Leu Lys Arg Leu Glu Asp Ala Val
   275 280 285
   Ala Ala Gly Asp Pro Ile Leu Ala Val Val Arg Gly Ser Ala Val Asn
   290 295 300
   Gln Asp Gly Ala Thr Ser Val Ile Thr Ala Pro Asn Gly Leu Ala Gln
   305 310 315 320
   Gln Ala Val Ile Arg Arg Ala Leu Lys Val Ala Gly Val Glu Pro Ala
   325 330 335
   Ala Ile Gly Tyr Val Glu Thr His Gly Thr Gly Thr Ser Leu Gly Asp
   340 345 350
   Pro Ile Glu Val Glu Ala Leu Asn Glu Thr Tyr Gly Gln Glu Pro Gly
   355 360 365
   Gly Arg Pro Cys Val Leu Gly Ala Val Lys Thr Asn Val Gly His Leu
   370 375 380
   Glu Gly Ala Ala Gly Ile Ala Gly Phe Ile Lys Ala Val Leu Ala Val
   385 390 395 400
   Gln Arg Gly Arg Ile Pro Pro Asn Leu His Phe His Glu Leu Asn Pro
   405 410 415
   Leu Ile Arg Leu Asp Gly Ser Arg Leu Cys Leu Ala Thr Glu Ala Arg
   420 425 430
   Glu Trp Ala Val Ala Ser Gln Glu Arg Phe Ala Ala Ile Ser Ser Phe
   435 440 445
   Gly Ala Gly Gly Thr Asn Ala His Val Val Ile Gln Gly Pro Pro Ala
   450 455 460
   Ala Arg Ala Pro Ala Ala Thr Gly Lys Ala Pro Ala Phe Val Cys Leu
   465 470 475 480
   Ser Ala Lys Ser Asp Ala Ser Leu Lys Gln Gln Ala Ala Arg Leu Arg
   485 490 495
   Ala Trp Leu Gly Gln Asp Gly Arg Glu Trp Ser Val Ala Asp Ile Ala
   500 505 510
   Ser Thr Ser Leu Arg Arg Arg Asn His Phe Asp His Arg Ala Ala Phe
   515 520 525
   Thr Ala Asp Ser Pro Glu Ser Leu Gly Glu Gly Leu Asp Ala Phe Leu
   530 535 540
   Ser Gly Arg Ala Ser Pro Arg Trp Ser Ala Gly Ser Gly Thr Leu Asn
   545 550 555 560
   Ala Gln Arg Pro Val Phe Val Phe Ser Gly Gln Gly Ser Gln Trp Gln
   565 570 575
   Gly Met Gly Arg Glu Leu Leu Thr Gln Ser Pro Arg Phe Ala Ala Glu
   580 585 590
   Ile Ala Ala Ile Asp Ala Arg Phe Ile Pro Leu Ala Gly Trp Ser Ile
   595 600 605
   Ala Glu Val Leu Ala Arg Asp Gly Asp Gly Ala Arg Leu Asn Ala Thr
   610 615 620
   Glu Val Ala Gln Pro Cys Leu Phe Ala Leu Gln Leu Gly Leu Val Ala
   625 630 635 640
   Ala Leu Asp Glu Leu Gly Ile Arg Pro Ala Ala Val Val Gly His Ser
   645 650 655
   Leu Gly Glu Leu Ala Ala Ala Cys Val Ala Gly Leu Met Asp Arg Glu
   660 665 670
   Ala Ala Thr Ala Leu Val Tyr His Arg Ala Lys Leu Met Gln Arg Leu
   675 680 685
   Thr Gly Ala Gly Lys Met Ala Ala Val Gly Leu Ser Arg Glu Glu Ala
   690 695 700
   Glu Ala Arg Leu Ala Ala Glu Pro Arg Leu Ala Leu Ala Ala Val Asn
   705 710 715 720
   Gly Pro Arg Ser Val Val Val Ala Gly Glu Pro Glu Ala Val Asp Ala
   725 730 735
   Phe Val Arg Ala Leu Arg Asp Glu Gly Val Phe Thr Arg Thr Leu Pro
   740 745 750
   Val Asp Tyr Ala Phe His Ser Pro Gln Thr Asp Pro Leu Leu Ala Glu
   755 760 765
   Leu Arg Arg Ala Val Asp Gly Ile Phe Gly Ala Gly Ala Arg Met Ala
   770 775 780
   Ala Pro Arg Leu Pro Met Val Ser Thr Val Ser Gly Ala Glu Leu Asp
   785 790 795 800
   Ala Ser Val Asp His Ala Thr Tyr Trp Ala Arg Asn Met Arg Gln Thr
   805 810 815
   Val Cys Phe Glu Glu Ala Val Gly Gln Leu Leu Glu Gln Arg His Asp
   820 825 830
   Ala Phe Leu Glu Val Gly Ala His Pro Val Leu Asn Val Asp Leu Arg
   835 840 845
   Glu Ser Phe Arg Ala Arg Glu Val Thr Ala Arg Ala Val Ala Thr Leu
   850 855 860
   Arg Arg Glu Gln Pro Gln Ala Leu Cys Leu Arg Ala Val Pro Ala Ala
   865 870 875 880
   Leu Tyr Ala Ala Gly Met Ala Pro Glu Ala Leu Pro Gly Gln Asp Gln
   885 890 895
   Gly Thr Leu Val Pro Leu Pro Asn Tyr Ala Trp Asp His Arg Arg Tyr
   900 905 910
   Trp Ile His Ser Ala Ser Arg Ala Glu Ala Gly Trp Val Tyr Asp Leu
   915 920 925
   Ala Trp Asp Ala Val Pro Ala Pro Ala Pro Gly Ala His Arg Pro Ala
   930 935 940
   Ser Trp Leu Val Leu Gly Ala Ser Gly Gly Leu Pro Glu Ala Leu Ala
   945 950 955 960
   Ala Arg Leu Arg Ala Thr Pro Asp Leu Val Arg His Val Ser Leu Glu
   965 970 975
   Ala Leu Arg Ser Val Pro Val Pro Pro His Gly Leu Val Asp Ala Glu
   980 985 990
   Arg Leu Glu Arg Trp Phe Thr Asp Thr Leu Gln Val Asp Leu Ala Glu
   995 1000 1005
   Phe Asp Phe Leu Val Tyr Val Pro Ala Cys Gly Ala Pro Glu Asp
   1010 1015 1020
   Ala Ser Ala Glu Arg Gln Val Ser Glu Asp Cys Ala Ile Leu Val
   1025 1030 1035
   Ser Leu Ala Gln Ala Leu Glu Arg Arg Pro Gly Ala Arg Gly Pro
   1040 1045 1050
   Ala Leu Trp Val Val Thr Ser Gly Ala Gln Ala Val Ala Pro Arg
   1055 1060 1065
   Glu Pro Val Asn Pro Phe Ala Ala Pro Val Trp Gly Leu Gly Arg
   1070 1075 1080
   Val Leu Ala Leu Glu Ala Pro Arg Gly Leu Gly Gly Leu Val Asp
   1085 1090 1095
   Leu Pro Leu Ala Trp Ser Glu Gln Asp Val Gly Cys Leu Leu Glu
   1100 1105 1110
   Ala Leu Arg His Pro Gly Glu Glu Asp Gln Leu Ala Leu Arg Ser
   1115 1120 1125
   Gly Gln Arg His Ala Leu Arg Leu Arg Arg Arg Ala Gly Arg Ala
   1130 1135 1140
   Thr Asp Ala Gly Ala Ala Trp Gln Val Glu Asp Ser Gly Val Tyr
   1145 1150 1155
   Leu Ile Thr Gly Gly Thr Arg Gly Leu Gly Leu Leu Val Ala Lys
   1160 1165 1170
   Arg Leu Ala Glu Arg Gly Ala Arg His Leu Ala Leu Leu Gly Arg
   1175 1180 1185
   Arg Ala Leu Pro Pro Arg Ser Arg Trp Ala Thr Leu Ala Ala Asp
   1190 1195 1200
   Asp Ala Phe Phe Pro Ser Val Gln Ala Ile Gln Ala Leu Glu Ala
   1205 1210 1215
   Gln Gly Val Thr Val Thr Leu His Ala Ala Asp Val Ser Asp Ser
   1220 1225 1230
   His Ala Met Arg Asp Val Phe Ala Glu Leu Glu Arg Gln Gln Arg
   1235 1240 1245
   Pro Leu Lys Gly Val Val His Ala Ala Gly Ile Ser Arg Glu Val
   1250 1255 1260
   Pro Leu Ala Ser Ile Asp Glu Ala Leu Leu Glu Glu Thr Leu Ala
   1265 1270 1275
   Pro Lys Val Ala Gly Thr Trp Trp Leu His Gln Leu Ser Leu Arg
   1280 1285 1290
   His Pro Leu Asp Phe Phe Val Val Phe Ser Ser Gly Ala Ser Val
   1295 1300 1305
   Trp Gly Ser Ala Gly Leu Ala Pro Tyr Ala Ala Ala Asn Glu Phe
   1310 1315 1320
   Leu Asn Ala Val Ala Ala His Arg Arg Ala Ser Gly Leu Pro Ala
   1325 1330 1335
   Leu Ser Val Cys Trp Gly Met Trp Asp Ala Asp Gly Met Ala Thr
   1340 1345 1350
   Asp Glu Met Arg Glu Asn Trp Thr Arg Leu Gly Val Lys Ala Met
   1355 1360 1365
   Ala Pro Glu Arg Ala Leu Asn Ala Leu Glu Glu Leu Val Ala Ala
   1370 1375 1380
   Asp Ala Thr Leu Ala Thr Val Ala Asp Val Lys Trp Ala Arg Phe
   1385 1390 1395
   Arg Pro Val Tyr Glu Leu Lys Arg Lys Arg Pro Leu Leu Ala Ala
   1400 1405 1410
   Leu Glu Asp Gly Ala Ala Ala Pro Pro Arg Pro Glu Ala Ala Gly
   1415 1420 1425
   Ala Pro Gln Ala Thr Ser Leu Lys Ala Asp Leu Gln Ala Leu Pro
   1430 1435 1440
   Arg Glu Gln Gln Arg Glu Arg Leu Leu Ala Thr Leu Arg Asp Thr
   1445 1450 1455
   Ala Ala Leu Val Leu Gly Ala Glu Pro Gly Ala Ala Ile Asn Pro
   1460 1465 1470
   Asp Glu Gly Leu Phe Ser Met Gly Phe Asp Ser Val Thr Ala Val
   1475 1480 1485
   Glu Phe Ala Ala His Leu Ser Arg Leu Phe Glu Lys Arg Tyr Ala
   1490 1495 1500
   Ala Thr Leu Val Phe Asp His Pro Ser Leu Lys Gln Ile Arg Asp
   1505 1510 1515
   Phe Leu Met Gln Asp Cys Phe Gly Trp Thr Ala Glu Pro Ser Pro
   1520 1525 1530
   Glu Pro Glu Ala Gly Asp Gly Gln Asp Leu Asp Ala Leu Leu Ala
   1535 1540 1545
   Phe Val Glu Gly Val Asp Glu Ala Glu Ala Glu Arg Leu Leu Ser
   1550 1555 1560
   Ala Gly Gln Pro Arg Ser Asn
   1565 1570
<210> 13
   <211> 1855
   <212> PRT
   <213> Angiococcus disciformis
<400> 13
   Met Thr Ser Val Asp Glu Arg Ile Lys Asn Leu Ser Pro Gln Lys Arg
   1 5 10 15
   Ala Leu Leu Glu Gln Arg Met Asn Ala Ala Ala Gly Ala Thr Arg Asp
   20 25 30
   Ala Glu Pro Ile Ala Ile Ile Gly Ile Gly Cys Arg Tyr Pro Gly Gly
   35 40 45
   Val Lys Asp Leu Lys Ser Phe Ala Gln Leu Leu Arg Asp Gly Ala Asp
   50 55 60
   Ala Thr Gly Asp Ile Pro Ala Asp Arg Trp Asp Val Glu Ala His Tyr
   65 70 75 80
   Ser Pro Asp Pro Gly Thr Pro Gly Lys Met Arg Thr Arg Arg Gly Gly
   85 90 95
   Phe Leu Pro Asp Val Lys Thr Phe Asp Ala Asp Phe Phe Gly Ile Ser
   100 105 110
   Pro Arg Glu Ala Glu Ser Met Asp Pro Gln Gln Arg Leu Leu Leu Glu
   115 120 125
   Val Ser Tyr Glu Ala Leu Glu Asp Ala Gly Ile Pro Thr Ala Ser Leu
   130 135 140
   Ser Gly Ser Thr Thr Gly Val Tyr Ile Gly Ile Thr Leu Ala Glu Tyr
   145 150 155 160
   Ala Asp Arg Leu Pro Asp Asp Pro Arg Leu Ile Asp Val Tyr Ser Gly
   165 170 175
   Thr Gly Thr Tyr Leu Asn Val Ala Ala Gly Arg Leu Ser Tyr Val Tyr
   180 185 190
   Gly Leu Gln Gly Pro Cys Met Ala Val Asp Ala Ala Cys Ala Ser Ser
   195 200 205
   Leu Val Thr Val His Leu Ala Ala Gln Ala Leu Arg His Arg Glu Cys
   210 215 220
   Gly Leu Ala Leu Ala Gly Gly Val Ser Leu Met Leu Ser Pro Glu Thr
   225 230 235 240
   Thr Ile Tyr Leu Ser Gln Thr Gly Ala Leu Ala Pro Asp Gly Arg Cys
   245 250 255
   Lys Thr Phe Asp Ala Arg Ala Asn Gly Tyr Gly Arg Ala Glu Gly Cys
   260 265 270
   Gly Ile Leu Val Leu Lys Arg Leu Ser Asp Ala Leu Arg Asp Lys Asp
   275 280 285
   Arg Ile His Ala Leu Ile Arg Gly Thr Ala Val Ser Gln Asp Gly Ala
   290 295 300
   Ser Ser Gly Leu Thr Val Pro Asn Gly Arg Ala Gln Gln Lys Val Ile
   305 310 315 320
   Gln Ser Ala Leu Lys Asp Ala Ala Val Arg Ser Glu Asp Val Ser Tyr
   325 330 335
   Val Glu Ala His Gly Thr Gly Thr Pro Leu Gly Asp Pro Ile Glu Leu
   340 345 350
   Asn Ala Leu Gln Ala Val Tyr Gly Gln Arg Glu Ala Asp Trp Pro Leu
   355 360 365
   His Val Gly Ser Leu Lys Thr Asn Phe Gly His Ala Glu Ala Ala Ala
   370 375 380
   Gly Val Gly Gly Ile Ile Lys Thr Val Val Ala Leu Gln Glu Arg Gln
   385 390 395 400
   Ile Pro Ala His Leu His Phe Glu Lys Leu Asn Pro Leu Ile Thr Ile
   405 410 415
   Asp Pro Thr Arg Val Val Ile Pro Thr Arg Pro Thr Pro Trp Gln Thr
   420 425 430
   Arg Gly Glu Arg Lys Arg Leu Ala Gly Val Ser Ser Phe Gly Phe Ser
   435 440 445
   Gly Val Ile Ala His Ala Val Leu Glu Glu Ala Pro Ala Pro Glu Ala
   450 455 460
   Ala Val Glu Gln Pro Ala Met Pro Ala Gly Glu Arg Pro Leu His Val
   465 470 475 480
   Leu Pro Leu Thr Ala Lys His Pro Arg Ala Leu Ser Glu Leu Ile Ser
   485 490 495
   Ala Tyr Arg His Thr Val Gly Ala Leu Asp Ala Gly Arg Leu Ala Asp
   500 505 510
   Leu Gly Tyr Thr Ala Ser Arg Lys Arg Gly His His Ala His Arg Ala
   515 520 525
   Trp Phe Ala Phe Arg Asp Lys Glu Glu Leu Leu Gln Gln Leu Glu Ala
   530 535 540
   Ala Thr Arg Gly His Glu Leu Pro Gly Ala Val Leu Gln Arg Gln Ala
   545 550 555 560
   Arg Asp Asp Val Lys Val Val Met Val Phe Pro Gly Gln Gly Ser Gln
   565 570 575
   Arg Val Gly Met Gly Gln Gln Leu Met Arg Thr Ser Pro Val Phe Arg
   580 585 590
   Glu Ala Ile His Glu Leu Asp Arg Ala Met Arg Pro Trp Ala Asp Phe
   595 600 605
   Ser Leu Leu Glu Val Leu Gly Ala Arg Gly Gly Glu Asp Pro Phe Thr
   610 615 620
   Arg Val Ser Ile Val Gln Pro Thr Leu Phe Ala Val Gln Val Ala Leu
   625 630 635 640
   Ala Arg Gln Trp Glu Ala Trp Gly Val Arg Pro His Ala Val Ile Gly
   645 650 655
   His Ser Met Gly Glu Val Ala Ala Ala Cys Val Ala Gly Ala Leu Ser
   660 665 670
   Met Glu Asp Ala Ala Ala Val Ile Cys Arg Arg Ser Gln Leu Val Gln
   675 680 685
   Ala Thr Ser Gly Gln Gly Ala Met Ala Leu Val Glu Leu Ser Pro Ala
   690 695 700
   Glu Val Glu Ala Ala Ile Ala Pro His Gly Asp Arg Val Ser Ile Ala
   705 710 715 720
   Ala Tyr Asn Gly Pro Ser Thr Val Leu Leu Ser Gly Glu Pro Ala Ala
   725 730 735
   Val Asp Glu Val Leu Thr Ala Leu Gln Glu Arg Gly Ala Phe Cys Arg
   740 745 750
   Arg Val Lys Val Asp Tyr Ala Ser His Cys Ala Gln Met Asp Pro Leu
   755 760 765
   Leu Gly Arg Ile Arg Ser Glu Leu Ala His Val Lys Pro Arg Lys Gly
   770 775 780
   Gly Val Pro Ile Tyr Ser Thr Ala Arg Gly Glu Val Ile Asp Gly Ser
   785 790 795 800
   Arg Met Asp Ala Gly Tyr Trp Ala Asp Asn Leu Arg Arg Pro Val Leu
   805 810 815
   Phe His Ala Gln Val Lys Ala Leu Leu Ala Asp Gly Met His Ala Leu
   820 825 830
   Leu Glu Ile Ser Pro His Pro Val Leu Leu Pro Ser Met Gln Lys Thr
   835 840 845
   Ile Asp Glu Ala Gly Gly Gln Gln Leu Val Leu Pro Ser Leu Arg Arg
   850 855 860
   Asp Glu Asp Glu Pro Arg Ser Leu Ala Thr Ser Leu Gly Ala Leu Phe
   865 870 875 880
   Gly Leu Gly His Pro Val Asp Phe Ser Ala Phe Thr Pro Arg Ser Gly
   885 890 895
   Arg Leu Val Glu Thr Pro Arg Tyr Pro Trp Gln Arg Arg Glu Phe Trp
   900 905 910
   Leu Thr Arg Lys Ala Ser Lys Arg Arg His Ala Leu Gly Gly Ala Glu
   915 920 925
   Gly His Pro Leu Ile Gly Gly Ala Phe Arg Pro Ala Val Gly Pro Glu
   930 935 940
   Ser Tyr Tyr Trp Glu Phe Asp Val Asp Glu Glu Arg Leu Ala Phe Ile
   945 950 955 960
   Ala Asp His Arg Val Arg Gly Met Thr Val Leu Pro Gly Thr Ala Tyr
   965 970 975
   Leu Asp Leu Ala Leu Ser Ala Ala Arg Ala Ala Phe Gly Asp Arg Ala
   980 985 990
   Phe Pro Leu Ser Gln Val Ser Phe Arg Glu Ala Leu Thr Leu Leu Pro
   995 1000 1005
   Glu Gln Pro Arg Arg Val Gln Leu Ser Leu Val Arg Glu Ala Glu
   1010 1015 1020
   Gly Ala Phe Ser Val Cys Val Ser Ser Arg Asp Ala Ala Ser Pro
   1025 1030 1035
   Ala Thr Gly Glu Trp Thr Leu His Ala Thr Met Arg Leu Asp Val
   1040 1045 1050
   Glu Ala Pro Ala Thr Gly Gly Ala Arg Ala Glu Gly Gln Val Thr
   1055 1060 1065
   Pro Pro Ala Gly Ala Glu Leu Arg Asp Ala Arg Thr His Tyr Arg
   1070 1075 1080
   Leu Met Lys Glu Gly Gly Leu Ala Tyr Gly Pro Arg Phe Gln Gly
   1085 1090 1095
   Leu Ala Glu Val His Cys Gly Gln His Glu Ala Leu Gly Arg Leu
   1100 1105 1110
   Arg Ala Leu Pro Glu Leu Gly Leu Asp Gly Ala Asp His Val Phe
   1115 1120 1125
   His Pro Ala Leu Trp Asp Ala Ala His Gln Ala Leu Ala Ala Leu
   1130 1135 1140
   Leu Gly Arg Gln Asp Gly His Ala Ser Gly Pro Ser Thr Trp Ile
   1145 1150 1155
   Pro Arg Gly Ile Glu Arg Val Ser Val Ser Gly Thr Val Pro Ala
   1160 1165 1170
   Arg Glu Ala Leu Trp Val Arg Ala Ser Ala Arg Val Asp Ala Asp
   1175 1180 1185
   Gly Ala Thr Ala Arg Gly Asp Leu Gln Val Leu Asp Asp Ala Gly
   1190 1195 1200
   Arg Val Leu Val Glu Thr Arg Gly Leu Thr Leu Leu Arg Arg Asp
   1205 1210 1215
   Ala Asp Asn Leu Asp Asp Val Arg Gln Trp Phe Phe Arg Pro Val
   1220 1225 1230
   Leu Arg Glu Ala Pro Leu Arg Glu Glu Ala Pro Ala Asp Gly Thr
   1235 1240 1245
   Trp Leu Val Leu Gly Ala Pro Gly Pro Leu His Asp Arg Val Val
   1250 1255 1260
   Ala Glu Leu Arg Gly Arg Gly Leu Asp Thr Val Gln Ala Phe Pro
   1265 1270 1275
   Ala Asn Thr Trp Glu Glu Gln Asp Ala Ala Arg Phe His Leu Ser
   1280 1285 1290
   Pro Ala Asp Pro Glu His Leu Arg Arg Leu Met Glu Arg Val Ser
   1295 1300 1305
   Ala Asp Arg Pro Leu Arg Gly Leu Ile Asn Leu Gln Pro Tyr Gln
   1310 1315 1320
   Val Ala Arg Gly Asp Ala Ala Ser Ser Ala Pro Glu Gly Val Leu
   1325 1330 1335
   Ser Ala Leu Tyr Met Glu Val Leu Asp Gln Ala Arg Ala Leu Gln
   1340 1345 1350
   Gly Val Ser Arg Arg Glu Arg Pro Lys Leu Val Leu Val Ser Gly
   1355 1360 1365
   Val Ser Gln Gly Ala Glu Ala Glu Pro Asp Leu Val Ala Ala Pro
   1370 1375 1380
   Leu Gln Gly Leu Gly Arg Val Ile Ala Ala Glu Leu Pro Arg Leu
   1385 1390 1395
   His Cys Val Gln Val Gln Leu Gly Gly Gln Glu Pro Glu Val Glu
   1400 1405 1410
   Ala Arg Asp Leu Val Arg Glu Leu Tyr Ala Asn Asp Gly Glu Asp
   1415 1420 1425
   Glu Val Val Leu Arg Gly Gly Ala Arg His Val Arg Arg Leu Glu
   1430 1435 1440
   Arg Leu Val Pro Gly Ala Ala Pro Ala Ala Pro Ile His Ala Asp
   1445 1450 1455
   Gly Thr Tyr Leu Leu Thr Gly Gly Leu Gly Gly Leu Gly Leu Ala
   1460 1465 1470
   Phe Ala Ser Trp Leu Ala Asp Arg Gly Ala Arg His Leu Ala Leu
   1475 1480 1485
   Val Ser Arg Arg Pro Pro Ser Gly Glu Ala Arg Ala Arg Leu Asp
   1490 1495 1500
   Ala Leu Arg Ala Arg Gly Val Glu Val Leu Val Arg Glu Val Asp
   1505 1510 1515
   Leu Ser Arg Glu Asp Ser Val Ala Ala Leu Phe Arg Glu Leu Asp
   1520 1525 1530
   Glu Arg Met Pro Pro Leu Arg Gly Val Leu His Leu Ala Gly Val
   1535 1540 1545
   Leu Asp Asp Ala Thr Leu Phe Gln Gln Asn Glu Gln Arg Leu Ala
   1550 1555 1560
   Thr Val Arg Gly Pro Lys Val Thr Gly Thr Arg Leu Leu His Ala
   1565 1570 1575
   His Thr Gln His Arg Lys Leu Asp Phe Phe Val Leu Phe Ser Ala
   1580 1585 1590
   Gly Ala Thr Leu Leu Gly Ser Pro Gly Gln Ala Asn Tyr Ala Ala
   1595 1600 1605
   Val Asn Ala Phe Met Asp Ala Phe Ala Pro His Arg Gln Arg Leu
   1610 1615 1620
   Gly Leu Pro Ala Leu Ser Ile Asn Trp Gly Val Trp Asp Glu Val
   1625 1630 1635
   Gly Leu Ala Ala Ala Ser Ala Gln Arg Gly Gln Arg Leu Arg Glu
   1640 1645 1650
   Gln Gly Leu Phe Pro Ile Pro Pro Ala Gln Gly Leu Gln Ala Phe
   1655 1660 1665
   Glu Leu Ala Leu Ser Leu Ala Gly Ala Asn Val Ala Ile Leu Pro
   1670 1675 1680
   Ile Arg Leu Ala Arg Trp Leu Arg Glu His Pro Asp Leu Ala Gly
   1685 1690 1695
   Ala Ser Leu Phe Lys Glu Leu Val Arg Gly Leu Asp Asp Ser Glu
   1700 1705 1710
   Ala Glu Ala Glu Ala Pro Ile Leu Glu Gln Leu Arg Ala Thr Val
   1715 1720 1725
   Asp Pro His Glu Gln Arg Glu Cys Leu Arg Arg Trp Val Gly Arg
   1730 1735 1740
   Gln Leu Ala Ala Val Leu Arg Arg Asp Pro Ala Ser Met Gln Arg
   1745 1750 1755
   Glu Gln Asn Phe Ser Ala Met Gly Val Asp Ser Leu Met Ala Leu
   1760 1765 1770
   Glu Phe Arg Asn Arg Leu Glu Arg Ser Leu His Val Arg Leu Pro
   1775 1780 1785
   Pro Thr Met Val Phe Asn His Pro Ser Ile Glu Ala Leu Cys Gly
   1790 1795 1800
   Phe Leu Glu Pro Lys Leu Gly Met Gly Ala Pro Ser Ala Pro Ala
   1805 1810 1815
   Gln Pro Glu Pro Val Pro Ala Pro Ala Ala Pro Val Arg Val Asp
   1820 1825 1830
   Glu Lys Asp Gly Ala Ala Leu Leu Gly Ala Leu Ala Arg Leu Lys
   1835 1840 1845
   Lys Leu Thr Gln His Gln Ala
   1850 1855
<210> 14
   <211> 1560
   <212> PRT
   <213> Angiococcus disciformis
<220>
   <221> SITE
   <222> (1)..(1560)
   <223> DifD
<400> 14
   Met Ser Met Leu Asp Glu Leu Arg Ala Val Leu Asp Ser Leu Ser Pro
   1 5 10 15
   Glu Gln Arg Ala Thr Leu Ala Glu Leu Leu Ser Ala Asp Ala Pro Arg
   20 25 30
   Lys Ala Asp Glu Pro Ile Ala Ile Val Gly Met Ala Gly Arg Phe Pro
   35 40 45
   Gly Ala Arg Asp Leu Ala Glu Phe Trp Ser Val Leu Ala Glu Gly Arg
   50 55 60
   Asp Ala Val Ser Glu Ile Pro Pro Glu Arg Trp Asn Ala Asp Ala Leu
   65 70 75 80
   Tyr Asp Pro Asp Lys Asp Val Pro Gly Lys Leu Ala Thr Arg Trp Gly
   85 90 95
   Gly Phe Val Lys Asp Val Asp Leu Phe Asp Ala Glu Phe Phe Gly Ile
   100 105 110
   Thr Pro Arg Glu Ala Ala Ala Met Asp Pro Gln Gln Arg Leu Phe Leu
   115 120 125
   Glu Thr Ala Trp Glu Ala Leu Glu Asp Ala Gly Leu Pro Ala Ala Ser
   130 135 140
   Leu Ala Arg Thr Gln Thr Gly Val Phe Val Gly Val Ser Asn Ser Asp
   145 150 155 160
   Tyr Met Trp His Gln Phe Pro Arg Leu Asp Lys Val Asp Ala Tyr Ala
   165 170 175
   Ser Ser Gly Ser Ala His Cys Ile Ile Ala Asn Arg Leu Ser Tyr Leu
   180 185 190
   Leu Asp Leu Thr Gly Pro Ser Val Ala Val Asp Thr Ala Cys Ser Ser
   195 200 205
   Ser Leu Val Ser Val His Leu Ala Cys Gln Ser Leu Arg Asn Arg Glu
   210 215 220
   Cys Glu Ala Ala Ile Ala Gly Gly Val Asn Val Val Leu Thr Pro His
   225 230 235 240
   Leu Gly Val Ser Leu Ser Lys Trp Gly Met Met Ala Ser Asp Gly Arg
   245 250 255
   Cys Lys Thr Phe Asp Ala Arg Ala Asp Gly Phe Val Arg Gly Glu Gly
   260 265 270
   Ser Gly Val Leu Val Leu Lys Arg Leu Ala Asp Ala Glu Lys Ala Gly
   275 280 285
   Asp Arg Val Leu Ala Val Ile Leu Gly Ser Ala Val Asn Gln Asp Gly
   290 295 300
   Lys Thr His Gly Leu Thr Ala Pro Asn Gly Ala Ala Gln Gln Ala Val
   305 310 315 320
   Ile Arg Gln Ala Leu Ala Arg Ala Gly Leu Ser Pro Ser Gln Val Ser
   325 330 335
   Phe Leu Glu Thr His Gly Thr Gly Thr Ser Leu Gly Asp Pro Ile Glu
   340 345 350
   Val Glu Ala Ile Asn Glu Val Phe Gly Ser Arg Val Glu Arg Cys Val
   355 360 365
   Leu Gly Ala Val Lys Thr Asn Ile Gly His Leu Glu Pro Ala Ala Gly
   370 375 380
   Ile Ala Gly Leu Ile Lys Val Val Leu Ala Leu Arg His Gly Leu Ile
   385 390 395 400
   Pro Arg Asn Leu His Tyr Gln Ser Met Asn Pro His Leu Gln Leu Ala
   405 410 415
   Pro Gly Ile Glu Leu Ala Ser Glu Ala Arg Pro Trp Lys Arg Asp Ala
   420 425 430
   Arg Pro Arg Phe Ala Gly Val Ser Ala Phe Ser Phe Gly Gly Thr Asn
   435 440 445
   Ala His Ile Val Leu Gly Asp Ala Pro Ala Tyr Ala Pro Pro Ala Glu
   450 455 460
   Ser Ala Arg Pro Ala Leu Pro Thr Leu Leu Pro Leu Ser Ala Ser Gln
   465 470 475 480
   Glu Gly Ala Arg Ser Gln Met Ala Ala Leu His Ala Gly Leu Leu Ala
   485 490 495
   Thr Ala Ala Pro Glu Leu Thr Val Asp Thr Val Ala Gly Leu Arg Ala
   500 505 510
   Leu His Arg Asp His Leu Asp Leu Arg Leu Ala Val Val Ala Ser Asp
   515 520 525
   Arg Ala Thr Ala Ala Glu Ala Leu Arg Cys Phe Ala Glu Gly Arg Pro
   530 535 540
   His Ala Gly Leu Val His Gly Arg Arg Gln Pro Asn Leu Arg Pro Val
   545 550 555 560
   Phe Ile Tyr Thr Gly Gln Gly Asn Gln Trp Pro Gly Met Gly Arg Glu
   565 570 575
   Leu Met Arg Thr Glu Pro Val Phe Arg Ala Ala Val Glu Glu Ala Asp
   580 585 590
   Ala Ala Phe Gln Arg Leu Thr Gly Arg Ser His Ala Ala Ser Phe Arg
   595 600 605
   Glu Asp Ala Ser Asp Ala Ala Ser Leu Glu Arg Thr Asp Ala Ala Gln
   610 615 620
   Pro Ala Ile Phe Leu Leu Gln Tyr Ala Leu Thr Lys Thr Leu Glu His
   625 630 635 640
   Trp Gly Leu Arg Pro Gly Ala Val Ala Gly His Ser Val Gly Glu Val
   645 650 655
   Ala Ala Ala Trp Ala Ala Gly Met Leu Asp Leu Asp Asp Ala Val Arg
               660                 665                 670
   Val Val His His Arg Ser Arg Leu Met Gln Arg Ala Thr Gly Gln Gly
           675                 680                 685
   Ala Met Ala Ser Val Ala Leu Ser Pro Arg Glu Ala Arg Pro Leu Leu
       690                 695                 700
   Glu Gly Arg Pro Ser Leu Ala Ile Ala Ala His Asn Ser Pro Gly Glu
   705                 710                 715                 720
   Val Val Leu Ser Gly Asp Pro Ala Glu Leu Glu Ala Val Leu Arg Glu
                   725                 730                 735
   Leu Glu Gly Arg Gly Val Arg Cys Arg Lys Leu Pro Val Asn Tyr Ala
               740                 745                 750
   Phe His Gly Pro Gln Met Asp Pro Leu Arg Asp Glu Leu Leu Arg Glu
           755                 760                 765
   Leu Ser Val Leu Arg Pro Lys Ala Pro Arg Leu Ala Phe Tyr Ser Thr
       770                 775                 780
   Val Thr Gly Gln Arg Glu Arg Gln Ala Val Ala Asp Ala Ser His Trp
   785                 790                 795                 800
   Val Arg Asn Met Arg Asp Pro Val Ala Phe Arg Glu Val Val Glu Arg
                   805                 810                 815
   Leu Ala Thr Asp Ser Pro Ser Phe Phe Leu Glu Leu Gly Pro His Pro
               820                 825                 830
   Ala Leu Val Pro Ser Val Met Asp Thr Leu Ala His Leu Arg Leu Glu
           835                 840                 845
   Ala Arg Thr Ala His Cys Leu Arg Arg Asn Arg Pro Asp Arg His Glu
       850                 855                 860
   Leu Leu Ser Ala Val Ala Ser Leu Tyr Val His Gly Ile Asp Pro Asp
   865                 870                 875                 880
   Trp Ser Arg Leu Val Arg Pro Pro Asp Arg Arg Trp Ala Leu Pro Ala
                   885                 890                 895
   Tyr Pro Trp Gln Arg Gln Arg Phe Trp Leu Glu Ala Ala Pro Pro Pro
               900                 905                 910
   Asp Ala Thr Trp Ser Ala Pro Ser Ala Arg Pro Ala Gly His Leu Val
           915                 920                 925
   Tyr Arg Thr Glu Trp Leu Ala Asp Ala Ala Ser Ala Ser Gly Asp Thr
   930 935 940
   Ala Arg Pro Lys Gly His Leu Val Phe Ser Asp Gly Ser Ala Leu Ala
   945 950 955 960
   Glu Ala Leu Arg Ala Glu Leu Glu Ala Ala Gly Ala Arg Phe Gly Trp
   965 970 975
   Val Ser Arg Gly Gln Asp Phe Ala Ala Asp Gly Thr Arg Cys Thr Val
   980 985 990
   Asp Pro Glu Asn Pro Glu His Leu Glu Arg Leu Phe Gly His Leu Ala
   995 1000 1005
   Ala Glu Ser Phe Glu Phe Asp His Val Leu Leu Leu Phe Thr Pro
   1010 1015 1020
   Val Ala Asp Glu Ala Arg Pro Asp Gly Ala Ala Pro Trp Phe Asp
   1025 1030 1035
   Thr Ala Val Arg Pro Val His Val Leu Arg Cys Leu Thr Gln Ala
   1040 1045 1050
   Leu Leu Thr Thr Glu Arg Arg Pro Arg Met Ala Val Ile Thr Arg
   1055 1060 1065
   Ala Ala Leu Ser Ala Gly Gly Asp Thr Ala Ala Val Pro Gln His
   1070 1075 1080
   Thr Ala Leu Trp Gly Phe Gly Arg Ala Leu Ala Leu Glu Glu Pro
   1085 1090 1095
   Gly Leu Arg Leu Ala Leu Leu Asp Leu Pro Ala Val Pro Asp Gly
   1100 1105 1110
   Leu Val Glu Ala Arg His Val Leu Ala Arg Leu Ala Ser Pro Thr
   1115 1120 1125
   Pro Glu Arg Gln Ser Ala Trp Arg Asp Gly Arg Trp Arg Val Pro
   1130 1135 1140
   Arg Leu Met Pro His Glu Ala Gly Thr Gly Thr Ser Ala Pro Pro
   1145 1150 1155
   Val Ile Gln Gly Glu Gly Thr Tyr Leu Ile Thr Gly Gly Leu Gly
   1160 1165 1170
   Ser Leu Gly Leu Ala Thr Ala Arg Arg Leu Val Gln Arg Gly Ala
   1175 1180 1185
   Arg Ser Val Ala Leu Val Ser Arg Arg Pro Pro Thr Ala Ala Gln
   1190 1195 1200
   Gln Ala Glu Leu Ala Arg Leu Glu Glu Ala Gly Ala Arg Val Val
   1205 1210 1215
   Thr Val Gln Gly Asp Leu Ala Asp Leu Ala Glu Ala Thr Arg Val
   1220 1225 1230
   Phe Glu Thr Leu Ala Ala Ser Ala Pro Pro Val Arg Gly Val Ile
   1235 1240 1245
   His Ala Ala Gly Val Leu Glu Asp Gly Val Leu Gly Ser Ala Thr
   1250 1255 1260
   Lys Ala Ser Phe Asp Arg Val Leu Arg Pro Lys Val Ala Gly Ser
   1265 1270 1275
   Trp Asn Leu His Gln Leu Thr Gln Ser Leu Pro Leu Asp Phe Phe
   1280 1285 1290
   Val Leu Phe Ser Ser Ala Thr Ala Thr Leu Gly Ala Ala Gly Gln
   1295 1300 1305
   Thr Asn Tyr Ala Ala Ala Asn Ala Phe Leu Gln Gly Leu Ala Glu
   1310 1315 1320
   His Arg Arg Ala Arg Gly Gln His Ala Leu Cys Leu His Trp Gly
   1325 1330 1335
   Ala Trp Arg Glu Ser Thr Met Ala Ser Gly Ala Val Val Thr Lys
   1340 1345 1350
   Leu Lys Ala Leu Gly Phe Asp Thr Leu Ala Pro Glu Asp Ala Leu
   1355 1360 1365
   Asp Ala Leu Glu Arg Val Leu Leu Glu Gly Ala Thr Glu Ala Thr
   1370 1375 1380
   Ile Ile Ala Ala Asp Trp Glu Val Phe Ala Gln Arg Leu Pro Asp
   1385 1390 1395
   Glu Ser Leu Gly Arg Phe Leu Glu Pro Val Leu Arg Ala Ser Gly
   1400 1405 1410
   Ala Pro Pro Ala Val Lys Pro Glu Ala Asp Pro Ile Val Ser Arg
   1415 1420 1425
   Leu Ala Gly Ala Pro Ala Ser Gln Arg Gln Lys Leu Leu Tyr Gly
   1430 1435 1440
   His Val Arg Ala Val Val Leu Ser Thr Met Gly Met Asp Ala Arg
   1445 1450 1455
   Thr Glu Val Arg Pro Asp Gln Gly Leu Ile Glu Leu Gly Leu Asp
   1460 1465 1470
   Ser Leu Leu Ala Val Asn Ile Ala Asn Arg Leu Ser Arg Asp Leu
   1475 1480 1485
   Ala Arg Gln Leu Pro Lys Thr Leu Ile Tyr Asp His Pro Thr Leu
   1490 1495 1500
   His Ala Leu Thr Asp His Leu Leu Gly Val Leu Phe Pro Glu Pro
   1505 1510 1515
   Ala Gln Ala Ser Val Arg Pro Ala Ala Ser Gly Asp Ala Pro Ser
   1520 1525 1530
   Tyr Glu Asp Ile Ala Asn Ala Ser Ala Asp Thr Ile Gln Ser Leu
   1535 1540 1545
   Leu Asp Ala Glu Leu Ala Ser Leu Leu Ser Ser Arg
   1550 1555 1560
<210> 15
   <211> 1074
   <212> PRT
   <213> Angiococcus disciformis
<400> 15
   Met Glu Leu Lys Ala Gln Leu Ala Arg Ala Glu Ser Arg Gln Arg Ala
   1 5 10 15
   Pro Ile Ala Ile Val Gly Met Ala Cys Arg Phe Pro Gly Gly Cys Ala
   20 25 30
   Thr Pro Glu Gln Phe Trp Glu Leu Leu Arg Glu Gly Arg Asp Ala Val
   35 40 45
   Thr Glu Ala Phe Pro Ser Arg Trp Pro Glu Ser Leu Tyr Asp Ala Asn
   50 55 60
   Pro Glu Ala Pro Gly Arg Ile Met Ala Arg Gly Leu Gly Gln Val Glu
   65 70 75 80
   Gly Val Ala Asp Phe Glu Pro Glu Phe Phe His Val Ser Pro Arg Glu
   85 90 95
   Ala Ala Ser Met Asp Pro Gln His Arg Leu Leu Leu Thr Val Ala Trp
   100 105 110
   Glu Ala Cys Glu Asp Ala Gly Leu Thr Arg Glu Leu Leu His Gly Ser
   115 120 125
   Arg Thr Gly Ile Phe Val Gly Ala Gly Asn Asn Asp Tyr Gly Asp Phe
   130 135 140
   Glu Leu Arg Asn Pro Asp Leu Gly Ala Val Ser Ala Tyr Ala Gly Thr
   145 150 155 160
   Gly Asn Ala Thr Ser Leu Val Ala Gly Arg Leu Ser Phe Phe Leu Gly
   165 170 175
   Thr Gln Gly Pro Ala Leu Val Val Asp Thr Ala Cys Ser Ser Ser Leu
   180 185 190
   Val Ala Ile His Gln Ala Cys Thr Ser Leu Arg Leu Gly Glu Ser Glu
   195 200 205
   Leu Ala Leu Ala Gly Gly Val Asn Leu Val Leu Arg Pro Asn Gly Ser
   210 215 220
   Val Phe Leu Ser Arg Phe Gly Ala Leu Ser Pro Thr Gly Arg Cys Arg
   225 230 235 240
   Ala Phe Ala Glu Ala Ala Asp Gly Tyr Val Arg Ala Asp Gly Cys Gly
   245 250 255
   Val Ile Val Leu Lys Arg Leu Glu Asp Ala Leu Arg Asp Gly Asp Arg
   260 265 270
   Val Leu Ala Val Ile Arg Gly Ser Ala Ile Asn His Asp Gly Arg Ser
   275 280 285
   Gly Gly Leu Thr Val Pro Asn Gly Ala Ala Gln Arg Glu Leu Leu Leu
   290 295 300
   Ala Ala Leu Lys Asn Ala Gly Val Glu Pro Arg Ser Ile Gly Tyr Leu
   305 310 315 320
   Glu Ala His Gly Thr Gly Thr Pro Leu Gly Asp Pro Ile Glu Met Gly
   325 330 335
   Ala Ile Ala Asp Val Phe Gly Gly Asp Ala Arg Arg Glu Ser Leu Arg
   340 345 350
   Val Gly Ser Val Lys Ser Asn Ile Gly His Thr Glu Ala Ala Ala Gly
   355 360 365
   Ile Ala Gly Ile Ile Lys Ala Val Leu Ser Leu Gln His Arg Glu Ile
   370 375 380
   Pro Ala His Leu His Phe Asp Lys Pro Ser Ser Arg Ile Asp Trp Ser
   385 390 395 400
   Ile Pro Val Glu Val Pro Thr Ser Thr Arg Pro Trp Ser Gly Asp Gly
   405 410 415
   Pro Arg Leu Ala Gly Val Ser Ser Phe Gly Phe Ser Gly Thr Asn Ala
   420 425 430
   His Val Ile Leu Glu Glu Ala Pro Arg Gln Glu Ala Arg His Ser Pro
   435 440 445
   Arg Leu Gly Thr Gln Val Leu Cys Leu Ser Ala Arg Thr Glu Gly Ser
   450 455 460
   Leu Thr Arg Leu Ala Ala Ala Trp Arg Glu Gln Leu Arg Arg Arg Asp
   465 470 475 480
   Ser Ala Pro Leu Ala Asp Leu Cys His Ala Ala Ala Arg Arg Arg Ser
   485 490 495
   His His Arg Ala Arg Leu Ala Val Leu Gly Asp Ser Ala Asp Ala Ile
   500 505 510
   Ala Gln Ala Leu Glu Ser Phe Ala Ala Gly Thr Pro His Pro Ala Val
   515 520 525
   Val Arg Gly Asp Ala Val Leu Asp Gly Met Ser Arg Thr Ala Phe Val
   530 535 540
   Phe Ser Gly Gln Gly Ser Gln Trp Leu Gly Met Gly Gln Glu Leu Leu
   545 550 555 560
   Ala Ser Glu Pro Leu Phe Gln Gln Ser Leu Arg Glu Met Asp Gly Pro
   565 570 575
   Phe Arg Ala Glu Phe Gly Trp Ser Leu Glu Glu Ala Leu Arg Ala Gly
   580 585 590
   Glu Ala Glu Ser Gln Leu Glu Arg Thr Arg Val Ala Gln Pro Val Ile
   595 600 605
   Val Ala Val Gln Leu Ala Leu Ser Asp Leu Trp Arg Ala Trp Gly Val
   610 615 620
   His Pro Asp Val Thr Leu Gly His Ser Leu Gly Glu Ile Ser Ala Ala
   625 630 635 640
   His Val Ala Gly Val Leu Gly Arg His Glu Ala Leu Arg Leu Ala Cys
   645 650 655
   Ile Arg Gly Arg Phe Met Asp Gly Ala Thr Gly Arg Gly Lys Met Ala
   660 665 670
   Glu Leu Ala Cys Ser Glu Glu Gln Ala Arg Arg Tyr Leu Gln Gly Phe
   675 680 685
   Glu Ala Glu Val Ala Ile Ala Ala Leu Asn Gly Ala Met Ser Thr Val
   690 695 700
   Leu Ala Gly Ala Pro Gly Ala Leu Glu Ala Val Leu Gln Lys Val Ala
   705 710 715 720
   Ser Asp Gly Ile Gln His Arg Leu Leu Arg Val Asp Tyr Ala Phe His
   725 730 735
   Ser Pro Gln Met Ala Glu Phe Ala Ala Pro Phe Arg Gly Ala Leu Gly
   740 745 750
   Asp Met Lys Pro Arg Gly Ala Glu Arg Asp Leu Tyr Ser Ser Val Leu
   755 760 765
   Gly Arg Ala Val Arg Gly Glu Ala Leu Thr Ala Asp Tyr Trp Val Arg
   770 775 780
   Asn Met Thr Asp Thr Val Arg Phe His Glu Ala Leu Val Ala Leu Leu
   785 790 795 800
   Arg Asp Gly Cys Arg Ala Val Val Glu Val Ser Pro His Pro Val Leu
   805 810 815
   Leu Ser Ala Ile Gln Asp Thr Leu Ser Arg Glu Gly Val Asp Gly Val
   820 825 830
   Ala Ala Tyr Ser Leu Arg Arg Gly Asn Pro Glu Arg Ala Thr Leu Leu
   835 840 845
   Ser Asn Leu Gly Ala Leu Tyr Ala Ala Gly His Ser Val Asp Trp Ser
   850 855 860
   Ala Leu Tyr Pro Gly Leu Val Asp His Val Ala Leu Pro Thr Tyr Pro
   865 870 875 880
   Trp His Ala Arg Arg Phe Trp Thr Thr Val Gln Gly Pro Glu Ala Thr
   885 890 895
   Thr Gly Ala Ala Pro Ala Val Thr Ala Pro Ala Pro Asn Glu Ala Ala
   900 905 910
   Pro Ala Ala Val Thr Ser Lys Leu Trp Asp Glu Leu Val Lys Leu Ala
   915 920 925
   Ala Val Ala Arg Arg Pro Arg Leu Glu Arg His Val Arg Glu Gln Leu
   930 935 940
   Thr Gly Ile Leu Lys Leu Asp Ser Gly Asp Ile Pro Ser Lys Arg Thr
   945 950 955 960
   Pro Leu Ser Glu Leu Gly Met Asp Ser Val Gly Ala Val Ala Leu Ser
   965 970 975
   Lys Ala Leu Ala Leu Thr His Glu His Arg Phe Pro Pro Thr Val Leu
   980 985 990
   Phe Glu His Pro Thr Ile Glu Ala Leu Thr Gly Phe Leu Ala Glu Glu
   995 1000 1005
   Phe Glu Arg Leu Arg Pro Pro Pro Pro Pro Ala Pro Arg Arg Ser
   1010 1015 1020
   Gly Gly Arg Arg Ala Pro Gly Ala Arg Leu Gly Ala Arg His Pro
   1025 1030 1035
   Gly Glu Ala Arg Arg Pro Arg Glu Gly Val Cys Leu Thr Cys Pro
   1040 1045 1050
   His Arg Arg Ile Pro Arg Ala Thr Ala His Ser Ser Thr Gly Arg
   1055 1060 1065
   Trp Pro Pro Trp Arg Arg
   1070
<210> 16
   <211> 2393
   <212> PRT
   <213> Angiococcus disciformis
<400> 16
   Met Lys Gly Arg Ile Gln Gln Leu Glu Gly Val Thr Thr Glu Pro Ile
   1 5 10 15
   Ala Ile Ile Gly Met Gly Cys Arg Phe Pro Gly Gly Val His Gly Pro
   20 25 30
   Asp Ala Phe Trp Arg Phe Leu Ala Asp Gly Val Asn Gly Ile Arg Asp
   35 40 45
   Thr Pro Lys Asp Arg Trp Asp Thr Glu Arg Tyr Leu Asp Ala Glu Gln
   50 55 60
   Gly Thr Pro Gly Lys Met Tyr Thr Gly His Gly Gly Phe Leu Asp Arg
   65 70 75 80
   Val Asp Gly Phe Asp Ala Ala Phe Phe Gly Ile Ser Pro Arg Glu Ala
   85 90 95
   Leu Ser Met Asp Pro Gln Gln Arg Leu Phe Leu Glu Thr Cys Trp Glu
               100                 105                 110
   Ala Ile Glu Asn Ala Gly Leu Ser Ala Asp Val Leu Glu Gly Ser Gln
           115                 120                 125
   Thr Gly Val Tyr Ala Gly Ile Val Asn Gln Asp Tyr Ala Met Met Gln
       130                 135                 140
   Met Lys Asp Thr Ala His Gly Glu Met Asp Thr Tyr Phe Ala Thr Gly
   145                 150                 155                 160
   His Pro His Cys Val Val Ser Gly Arg Val Ser Tyr Gln Phe Gly Leu
                   165                 170                 175
   Arg Gly Pro Thr Leu Ser Val Asp Thr Ala Cys Ser Ser Ser Leu Val
               180                 185                 190
   Ala Leu His Leu Ala Cys Gln Ser Leu Arg Leu Gly Ser Cys Asp Val
           195                 200                 205
   Ala Leu Ala Gly Gly Ile Asn Leu Leu Leu Met Pro Glu Ile Tyr Met
       210                 215                 220
   Ile Arg Cys Gln Ala Arg Met Leu Ser Pro Ser Gly Arg Cys Ala Thr
   225                 230                 235                 240
   Phe Asp Ala Ser Ala Asp Gly Tyr Ile Pro Gly Glu Gly Val Gly Val
                   245                 250                 255
   Val Val Leu Lys Arg Leu Arg Asp Ala Val Arg Asp Gly Asp Arg Val
               260                 265                 270
   Asp Ala Val Ile Arg Gly Thr Gln Ile Asn His Asp Gly Arg Ser Ser
           275                 280                 285
   Gly Leu Thr Val Pro Ser Gly Ala Ala Gln Arg Glu Leu Leu Arg Ala
       290                 295                 300
   Thr Leu Arg Asp Ala Arg Leu Glu Pro Gln Arg Val Gly Tyr Leu Glu
   305                 310                 315                 320
   Ala His Gly Thr Gly Thr Lys Leu Gly Asp Pro Ile Glu Met Ala Ala
                   325                 330                 335
   Ile Gln Asp Val Tyr Cys Gln Asp Arg Ala Pro Gly Asn Pro Leu Val
               340                 345                 350
   Ile Gly Ser Val Lys Thr Asn Ile Gly His Leu Glu Ser Ala Ala Gly
           355                 360                 365
   Ile Ser Gly Leu Ile Lys Ala Val Leu Thr Leu Lys His Gly Ala Ile
   370 375 380
   Pro Arg Asn Leu His Phe Ser Glu Leu Asn Pro His Ile Thr Leu His
   385 390 395 400
   Pro Ala Thr Arg Ile Pro Thr Glu Leu Thr Pro Trp Pro Ala Gly Ser
   405 410 415
   Gly Pro Arg Val Ala Ala Val Ser Ser Phe Gly Met Ser Gly Thr Asn
   420 425 430
   Ala His Ala Ile Leu Glu Glu Ala Pro Ala Arg Glu Ala Ala Ser Ala
   435 440 445
   Gln Ala Pro Leu Arg Val Thr Ser Arg Pro Ala Thr Pro Arg Leu Ile
   450 455 460
   Pro Leu Ser Ala His His Pro Glu Val Leu Gln Ala His Ala Ala Gly
   465 470 475 480
   Leu Lys Glu His Leu Asp Ala His Pro Asp Val Thr Phe Asp Asp Leu
   485 490 495
   Ala His Thr Leu Arg Arg Arg Thr Leu Gln Arg His Arg Leu Ala Leu
   500 505 510
   Pro Ala Ser Ser Leu Ala Glu Val Lys Glu His Leu Ala Ala Phe Thr
   515 520 525
   Ala Gly Gly Gln Asp Pro Arg Val Leu Gln Thr Glu Asp Leu Phe Phe
   530 535 540
   Thr Asp Pro Lys Val Val Phe Val Phe Pro Gly Gln Gly Ser Gln Trp
   545 550 555 560
   Val Arg Met Gly Leu Gln Leu Met Glu Gln Glu Pro Glu Phe Ala Ala
   565 570 575
   Glu Leu Arg Glu Val Asp Ala Trp Ile Gln Lys Tyr Ala Gly Trp Ser
   580 585 590
   Leu Leu Asp Glu Leu Arg Ala Asp Ala Ser Ala Ser Arg Leu Ser Gln
   595 600 605
   Val Asp Val Val Gln Pro Cys Ile Phe Ala Val Gln Val Ala Leu Ala
   610 615 620
   Arg Leu Trp Arg Ser Trp Gly Val Val Pro Asn Ala Val Ile Gly His
   625 630 635 640
   Ser Met Gly Glu Val Ala Ala Ala Cys Val Ala Gly Ile Leu Ser Leu
   645 650 655
   Glu Asp Ala Val Cys Val Ile Thr Arg Arg Ser Gln Ile Ala Lys Arg
   660 665 670
   Thr Ser Gly Asn Gly Ala Met Ala Leu Val Glu Leu Thr Arg Glu Gln
   675 680 685
   Ala Glu Arg Ala Ile Ala Ala His Gln Asp Val Val Ala Val Ala Ala
   690 695 700
   Gln Asn Gly Pro His Thr Val Leu Leu Ser Gly Thr Pro Gly Pro Ile
   705 710 715 720
   Glu Gln Ile Leu Arg Glu Leu Gln Gln Gln Asp Val Phe Cys Arg Ala
   725 730 735
   Ile Lys Val Asp Tyr Ala Ser His Ser Pro Gln Met Asp Pro Leu Arg
   740 745 750
   Pro Glu Leu Gln Arg Ala Leu Ser Gly Val Lys Pro Arg Ala Ala Thr
   755 760 765
   Val Ala Phe Tyr Ser Thr Val Thr Gly Gln Val Glu Asp Gly Thr Ser
   770 775 780
   His Asp Ala Asn Tyr Trp Val Asn Asn Leu Arg Asp Pro Val Leu Phe
   785 790 795 800
   Trp Pro Gly Met Gln Lys Leu Val Glu Asp Gly His Asn Ile Phe Leu
   805 810 815
   Glu Leu Ser Pro His Pro Val Leu Ile Pro Ala Ile Ser Gln Gly Leu
   820 825 830
   Gln His Leu Gly Ala Arg Ala Leu Ser Leu Pro Ser Leu His Arg Glu
   835 840 845
   Lys Pro Glu Ala Glu Cys Leu Phe Glu Ser Arg Ala Arg Leu Phe Cys
   850 855 860
   Ala Gly Gly Arg Leu Asp Leu Ala Lys Glu Asp Val Pro Gly Ala Arg
   865 870 875 880
   Val Leu Ala Leu Pro Gly His Pro Trp Arg Glu Gln Arg Phe Trp Tyr
   885 890 895
   Thr Pro Ser Ala Pro Ser Ala His Pro Gln Ala Arg Gly Gly Ser Arg
   900 905 910
   Leu Leu Pro Arg Met Trp Thr Ala Gly Ser Gly Glu Arg Arg Val Phe
   915 920 925
   Glu Gly Asp Val Gly Pro Gly Val Met Pro Ser Leu Ala Asp His Arg
   930 935 940
   Val Asn Gly Met Gln Leu Phe Pro Gly Ala Gly Tyr Val Glu Leu Val
   945 950 955 960
   Leu Glu Ala Ala Glu Ala Leu Gly Leu Glu Gly Arg Thr His Ile Asp
   965 970 975
   Ser Leu Asp Ile Arg Glu Gly Leu Val Leu His Glu Gly Ala Ala Arg
   980 985 990
   Val Val Gln Val Glu Leu Thr Pro Val Ala Ala Arg Ser Phe Gly Phe
   995 1000 1005
   Val Ile Ser Ser Arg Glu Ala Asp Gly Ala Ala Trp Thr Arg His
   1010 1015 1020
   Ala Asp Gly Val Val Thr Arg Glu Glu Ala Gly Ala Ala Pro Gly
   1025 1030 1035
   Ala Glu Pro Gly Ala Leu Glu Ala Leu Arg Ala Arg Leu Ala Ser
   1040 1045 1050
   Pro Leu Ser Ala Glu Asp His Tyr Leu Arg Thr Arg Glu Arg Gly
   1055 1060 1065
   Leu Glu Tyr Gly Pro Ala Tyr Gln Ala Ile Lys His Ile Gly Arg
   1070 1075 1080
   Gly Arg Gly Glu Ala Leu Ala Glu Leu His Val Pro Gln Ala Val
   1085 1090 1095
   Ala Glu Glu Leu Ser Gly Cys Gln Leu His Pro Ser Leu Leu Asp
   1100 1105 1110
   Ala Gly Phe Gln Leu Ile Asp Thr Ala Leu Ala Gly Ser Gly Leu
   1115 1120 1125
   Val Glu Glu Gly Asp Val Tyr Leu Pro Ala Ala Leu Arg Gly Val
   1130 1135 1140
   Arg Phe His Arg Lys Pro Ala Pro Gly Gln Thr Leu His Ala His
   1145 1150 1155
   Leu Gly Leu Ser Pro Glu Lys Pro Arg Glu Ala Ser Val Leu Glu
   1160 1165 1170
   Ala Arg Leu Ala Leu Leu Asp Asp Gln Gly Ala Pro Val Leu Thr
   1175 1180 1185
   Ala Glu Arg Phe Ile Ile Arg Arg Val Glu Thr His Ala Ala Ala
   1190 1195 1200
   Trp Glu Gly Leu Pro Gly Glu Trp Phe Tyr Arg Leu Arg Trp Gln
   1205 1210 1215
   Pro Leu Glu Gly Trp Gln Pro Glu Ala Ala Gly Ala Arg Arg Asp
   1220 1225 1230
   Glu Ala Trp Leu Leu Phe Ala Asp Asp Gln Gly Val Ala Ala Ala
   1235 1240 1245
   Val Ala Asp Arg Leu Ala Pro Ser Gly Val Arg Thr Thr Leu Val
   1250 1255 1260
   Arg Lys Ala Pro Gly Ala Val Thr Arg Ala Gly Ser Gly Trp Thr
   1265 1270 1275
   Leu Asp Pro Ser Thr Pro Glu Ser Phe Arg Glu Leu Leu Ala Ala
   1280 1285 1290
   Val Pro Pro Gly Ser Phe Thr Arg Val Leu Tyr Phe Trp Gly Leu
   1295 1300 1305
   Asp Ala Pro Ala Val Asp Pro Glu Pro Asp Ala Leu Asp Ala Arg
   1310 1315 1320
   Leu Pro Glu Thr Glu Ala Leu Ile Tyr Leu Val Gln Ala Leu Ala
   1325 1330 1335
   Ala Arg Glu Ala Gln Pro Gly Pro Arg Leu Trp Leu Val Thr Ala
   1340 1345 1350
   Gly Ala Leu Pro Gly Ala Asp Gly Glu Ala Val Ser Pro Met Gln
   1355 1360 1365
   Thr Pro Leu Trp Ala Leu Gly Arg Val Leu Asp Tyr Glu His Pro
   1370 1375 1380
   Glu Leu Lys Thr Val Ser Cys Glu Leu Glu Arg His Val Thr Glu
   1385 1390 1395
   Lys His Val Ala Val Leu Thr Ser Leu Leu Leu Ala Pro Glu Val
   1400 1405 1410
   Pro Asp Gln Val Arg Val Arg Ala Asp Gly Pro Ala Ala Ala Arg
   1415 1420 1425
   Leu Ala Arg Tyr Ala Pro Pro Ala Pro Arg Asp Thr Pro His Arg
   1430 1435 1440
   Val Ser Pro His Glu Ala Ala Val Val Val Ser Gly Asn Gly Pro
   1445 1450 1455
   Ala Pro Ala Glu Leu Ser Val Ala Arg Arg Arg Ala Pro Ala Ala
   1460 1465 1470
   His Glu Val Glu Val Leu Val Thr His Ala His Leu Ala Trp Ala
   1475 1480 1485
   Pro Ala Ser Pro Asp Ala Gly Gly Leu Val Pro Gly Gly Phe Phe
   1490 1495 1500
   Ala Gly Arg Val Leu Arg Leu Gly Gly Glu Val Arg Gly Leu Ser
   1505 1510 1515
   Val Gly Gln Ala Ile Val Ala Pro Ala Gly Gly Glu Leu Arg Ser
   1520 1525 1530
   Ile Arg Thr Val Asp Ala Gly Gly Val Arg Pro Leu Pro Glu Gln
   1535 1540 1545
   Ala Asp Pro Ala Arg Ala Leu Cys Ala Leu Gly Thr Arg Ala Leu
   1550 1555 1560
   Ala Arg His Leu Leu Arg Gln Leu Ala Pro Val Glu Arg Gly Gln
   1565 1570 1575
   Thr Leu Leu Leu Leu Gly Gly Asp Leu Pro Leu Leu Gln Ser Leu
   1580 1585 1590
   Ala Arg Val Ala Thr Gly His Gly Ala Arg Val Leu Ala Leu Val
   1595 1600 1605
   Arg Asp Gly Ala His Ala Pro Ala Leu Gln Glu Ala Gly Ala Glu
   1610 1615 1620
   Val Val Gly Arg Leu Asp Glu Pro Gly Ala Phe Glu Arg Leu Arg
   1625 1630 1635
   Glu Arg Ala Gly Ala Ser Gly Val Asp Val Leu Ile Asn Leu Ala
   1640 1645 1650
   Pro Gly Ala Ala Val Pro Arg Ala Leu Asp Val Phe Arg Ala Gly
   1655 1660 1665
   Gly Val Phe Leu Asp Leu Thr Arg Ala Ser Pro Val Gly Pro Lys
   1670 1675 1680
   Glu Leu Pro Ala Ser Ala Thr Phe Leu Ser Leu Gly Leu Ala Pro
       1685                1690                1695
   Leu Tyr Ala Arg Leu Ala Pro Pro Thr Pro Glu Ala Ile Ala Glu
       1700                1705                1710
   Ala Ser Ala Pro Ala Glu Ala Pro Ala Arg Val Trp Ser Leu Gln
       1715                1720                1725
   Glu Leu Thr Gln Pro Arg Ala Glu Ala Pro Leu Asp Gly Gln Ala
       1730                1735                1740
   His Val Leu Gly Val Ser Ser Glu Pro Val Thr Val Gly Ala Gly
       1745                1750                1755
   Ser Ala Pro Leu Ala Leu Ser Asp Thr Ala Thr Tyr Leu Ile Thr
       1760                1765                1770
   Gly Gly Leu Gly Gly Leu Gly Leu Arg Val Ala Ser Leu Leu Val
       1775                1780                1785
   Asp His Gly Val Arg His Leu Ala Leu Val Gly Arg Ser Pro Pro
       1790                1795                1800
   Ser Ala Gln Ala Arg Ser Thr Leu Arg Glu Leu Glu Gln Arg Gly
       1805                1810                1815
   Val Arg Val Gln Val Phe Ala Ala Asp Val Ala Arg Arg Glu Asp
       1820                1825                1830
   Leu Leu Gly Val Val Lys Ala Leu Gln Ala Gly Asp Ala Pro Leu
       1835                1840                1845
   Arg Gly Val Ile His Cys Ala Gly Val Leu Gln Asp Gly Ala Leu
       1850                1855                1860
   Leu Gly Leu Asp Arg Glu Arg Phe Arg Ser Val Leu Gly Ser Lys
       1865                1870                1875
   Leu Ser Gly Ala Trp Asn Leu His Ala Leu Thr Arg Asp Leu Pro
       1880                1885                1890
   Leu Asp His Phe Val Leu Phe Ser Ser Val Ser Ser Met Leu Gly
       1895                1900                1905
   Ser Pro Gly Gln Gly Asn Tyr Ala Ala Ala Asn Ala Phe Met Asp
       1910                1915                1920
   Gly Leu Ala Ala Trp Arg Arg Ala Arg Gly Leu Pro Ala Leu Ser
       1925                1930                1935
   Val Asn Trp Gly Ala Phe Ser Asp Ile Gly Leu Ala Ala Val Gly
   1940 1945 1950
   Glu Arg Arg Gly Asp Arg Leu Ala Asp Asn Gly Ile Pro Gly Ile
   1955 1960 1965
   Pro Pro Glu Lys Gly Met Lys Ala Leu Glu Leu Ala Leu Arg Trp
   1970 1975 1980
   Gly Glu Thr Ser Leu Gly Phe Phe Pro Phe Pro Phe Leu Ile Trp
   1985 1990 1995
   Arg Lys Leu Asn Pro Gln Ile Ala Arg Asn Thr Leu Phe Thr Asp
   2000 2005 2010
   Ile Arg Ala Glu Ser Arg Tyr Arg Glu Glu Ala Ser Gly Glu Gln
   2015 2020 2025
   Pro Ile Ala Glu Ala Leu Ala Leu Ala Asp Pro Ala Ala Arg Arg
   2030 2035 2040
   Ser His Leu Asp Gly Phe Leu Lys Gln Gln Leu Ser Arg Val Leu
   2045 2050 2055
   Lys Val Pro Ala Glu Gln Val Arg Asp Gly Leu Thr Phe Lys Glu
   2060 2065 2070
   Met Gly Phe Asp Ser Leu Met Ala Ile Glu Leu Arg Asn Arg Leu
   2075 2080 2085
   Glu Ala Gly Leu Thr Val Ser Leu Ser Ser Thr Leu Val Trp Arg
   2090 2095 2100
   Tyr Pro Thr Leu Glu Glu Met Ser Arg Phe Leu Leu Gln Leu Val
   2105 2110 2115
   Gly Gly Gly Ser Glu Thr Pro Ser Asp Ala Arg Pro Ala Ser Ala
   2120 2125 2130
   Ala Pro Ala Arg Arg Gly Arg Trp Leu Val Arg Glu Lys Pro Ser
   2135 2140 2145
   Pro Ser Ala Lys Leu Arg Leu Leu Cys Phe Pro His Gly Gly Ala
   2150 2155 2160
   Gly Ala Ser Ala Phe Gln Gly Trp Trp Gln Gln Phe Pro Asp Thr
   2165 2170 2175
   Val Glu Val Leu Thr Phe Gln Pro Pro Gly Arg Glu Glu Arg Leu
   2180 2185 2190
   Gln Glu Glu Pro Glu Arg Asp Met Gly Ala Tyr Ala Ser Ala Ile
   2195 2200 2205
   Val Glu Glu Leu Gln Pro Leu Leu Asp Leu Pro Phe Ala Val Phe
   2210 2215 2220
   Gly Tyr Ser Leu Gly Gly Leu Thr Gly Phe Ala Thr Val Ala Glu
   2225 2230 2235
   Leu Arg Arg Arg Asn Arg Val Thr Pro Arg His Leu Phe Val Ser
   2240 2245 2250
   Ser Ala Phe Ala Pro His Ile Pro Phe Ser Glu Thr Thr Leu Ala
   2255 2260 2265
   Arg Val Ser Thr Gln Thr Ser Ala Leu Lys Ala Met Ala Phe Tyr
   2270 2275 2280
   Gln Ser Thr Pro Asp Ser Val Phe Lys Asp Pro Glu Met Gln Ala
   2285 2290 2295
   Leu Leu Ala Arg Ser Met Asp Ala Asp Asn Ser Val Val Glu Ser
   2300 2305 2310
   Tyr Arg Phe Gly Ala Glu Lys Pro Leu Asp Cys Pro Ile Thr Ala
   2315 2320 2325
   Phe Ala Gly Arg Gln Asp Asp Leu Ala Pro Leu His Glu Gln Leu
   2330 2335 2340
   Arg Trp Lys Glu Leu Thr Thr Gln Ala Phe Glu Leu Arg Pro Phe
   2345 2350 2355
   Glu Gly Asp His Phe Phe Phe Leu Arg Asp Lys Arg Pro Val Met
   2360 2365 2370
   Asp Glu Leu Arg Arg His Leu Leu Ser Leu Ile Ala Asp Ser Arg
   2375 2380 2385
   Asp Gly Ser Ser Arg
   2390
<210> 17
   <211> 2385
   <212> PRT
   <213> Angiococcus disciformis
<400> 17
   Met Asp Ala Ser Ser Ser Arg Pro Phe Glu Ser Leu Asp Ala Leu Gln
   1 5 10 15
   Ala Trp Val Val Thr Trp Leu Cys Glu His Ala Arg Ile Pro Gln Gln
   20 25 30
   Ala Ile Gly Leu Asp Val Arg Leu Arg Asp Leu Gly Leu Asp Ser Ala
   35 40 45
   Arg Ala Thr Lys Leu Val Ala Ala Leu Ser Glu Arg Phe Gly Arg Thr
   50 55 60
   Leu Pro Leu Thr Leu Leu Trp Asp Tyr Pro Thr Val Thr Glu Leu Ser
   65 70 75 80
   Arg His Leu Met Lys Pro Ala Thr Ala Ala Ala Ala Gly Ala Thr Pro
   85 90 95
   Arg Ala Glu Arg Arg Ala Ser Gly Ala Pro Glu Asp Arg Ala Ile Ala
   100 105 110
   Val Ile Gly Met Ala Cys Arg Phe Pro Gly Gly Ala Asp Thr Pro Glu
   115 120 125
   Ala Phe Trp Glu Leu Leu Ala Lys Gly Leu Asp Val Val Gly Pro Val
   130 135 140
   Pro Ala Asp Arg Tyr Asp Ala Glu Ala Phe Tyr Asp Arg Asp Pro Arg
   145 150 155 160
   Ala Arg Gly Lys Ala Val Thr Arg Trp Gly Gly Phe Leu Glu Arg Thr
   165 170 175
   Asp Thr Phe Asp Pro Tyr Phe Phe Gly Ile Ser Pro Arg Glu Ala Ile
   180 185 190
   Glu Met Asp Pro Gln Gln Arg Met Leu Leu Glu Leu Ala Trp Glu Ala
   195 200 205
   Phe Glu Ala Ala Gly Leu Thr Lys Asp Gly Leu Arg Asp Arg Ala Val
   210 215 220
   Gly Ile Phe Val Gly Ala Met Trp Asn Asp Tyr Ala Arg Leu Trp Arg
   225 230 235 240
   Gly Gly Pro Gly Ala Ile Glu Gln Phe Thr Ala Thr Gly Gln Asp Asp
   245 250 255
   Ser Ile Ile Ser Ala Arg Ile Ser Tyr Val Phe Gly Leu Arg Gly Pro
   260 265 270
   Ser Leu Thr Leu Asn Thr Ala Cys Ser Ser Ser Leu Val Ser Val His
   275 280 285
   Met Ala Cys Gln Ser Leu Leu Ala Gly Asp Ser Glu Leu Ala Leu Ala
   290 295 300
   Gly Gly Val Asn Leu Ile His Gly Pro Asp Ser Thr Ile Ala Met Ser
   305 310 315 320
   Lys Phe Gly Gly Met Ala Pro Asp Gly Lys Cys Lys Ala Phe Asp Ala
   325 330 335
   Arg Gly Asn Gly Tyr Val Arg Gly Glu Gly Ala Gly Val Val Leu Leu
   340 345 350
   Lys Pro Leu His Arg Ala Leu Glu Asp Gly Asp Val Ile His Ala Val
   355 360 365
   Ile Arg Gly Ser Ala Val Asn Asn Asp Gly Phe Ser Asn Gly Leu Thr
   370 375 380
   Ser Pro Ser Pro Ala Ala Gln Ala Ala Met Leu Arg Ala Ala Tyr Ala
   385 390 395 400
   Ser Ala Gly Val Ser Pro Ala Arg Val His Tyr Val Glu Ala His Gly
   405 410 415
   Thr Gly Thr Met Leu Gly Asp Pro Ile Glu Ala Gly Ala Leu Gly Glu
   420 425 430
   Val Leu Gly Gln His Pro Glu Arg Gln Gln Pro Leu Arg Ile Gly Ser
   435 440 445
   Ala Lys Thr Asn Ile Gly His Leu Glu Ala Ala Ala Gly Met Ala Gly
   450 455 460
   Leu Ile Lys Thr Ile Glu Ala Met Lys His Arg Arg Leu Pro Pro Ser
   465 470 475 480
   Leu His Phe Glu Gln Pro Asn Pro Asn Ile His Phe Glu Gly Leu Arg
   485 490 495
   Leu Arg Val Gln Thr Asp Leu Ser Pro Trp Pro Thr Glu Asp Asp Ser
   500 505 510
   Leu Leu Ala Gly Val Ser Ser Phe Gly Phe Gly Gly Thr Asn Ala His
   515 520 525
   Val Val Leu Glu Ser Phe Gln Pro Ala Arg Pro Val Ala Ile Pro Phe
   530 535 540
   Ser Ala Pro Asp Ala Glu Ala Leu Gly Ala Arg Leu Val Glu Leu Arg
   545 550 555 560
   Ala Phe Ala Glu Gln His Pro Glu Ala Ser Leu Asp Asp Leu Gln Arg
                   565                 570                 575
   Ala Trp Val Gln Arg Leu Thr Glu Gly Pro Val Arg Gln Val Leu Leu
               580                 585                 590
   Ala Arg Ser Arg Lys Asp Leu Leu Glu Gln Leu Arg Ala Pro Ala Arg
           595                 600                 605
   Pro Ala Ala Pro Ala Gly Arg Gly Ala Thr Arg Pro Val Leu Val Phe
       610                 615                 620
   Thr Gly Asn Gly Ala Gln Tyr Pro Gly Met Ala Arg Asp Leu Met Leu
   625                 630                 635                 640
   Gly Asp Ala Thr Phe Arg Gln Ala Val Leu Arg Cys Asp Glu Ala Leu
                   645                 650                 655
   Arg Ala His Gly Cys Asp Gly Val Ala Leu Ala Leu Glu Ser Gly Asp
               660                 665                 670
   Phe Asp Val Arg Arg Val Asp Leu Val Gln Pro Leu Leu Trp Ala Val
           675                 680                 685
   Gln Val Gly Leu Val Ser Leu Trp Arg Ala Trp Gly Val Glu Pro Ala
       690                 695                 700
   Ala Val Val Gly His Ser Val Gly Glu Val Ala Ala Ala Trp Ala Ala
   705                 710                 715                 720
   Gly Ala Leu Asp Leu Asp Glu Ala Ala Arg Val Val Val His Arg Ser
                   725                 730                 735
   Arg Leu Gln Gln Glu Cys Ala Asp Gln Gly Gly Met Val Leu Leu Glu
               740                 745                 750
   Thr Asp Ala Glu Gly Ala Arg Ser Leu Val Gly Ala Thr Ala Ala Glu
           755                 760                 765
   Gly Leu Ala Val Ala Ala Leu Asn Ser Arg Gly Ser Thr Val Val Ser
       770                 775                 780
   Gly Pro Thr Ala Ser Leu Gln Ala Leu Ile Thr Lys Ala Glu Glu Arg
   785                 790                 795                 800
   Gly Val Thr Ala Arg Thr Val Arg Val Asn Val Ala Tyr His Gly Pro
                   805                 810                 815
   Gly Met Asp Ala Leu Arg Glu Arg Leu Arg Glu Ser Leu Ala Asp Leu
               820                 825                 830
   Arg Pro Cys Pro Pro Arg Val Pro Met Ser Ser Ser Val Thr Gly Gly
   835 840 845
   Ala Val Gly Gly Ala Gly Leu Asp Ala Gly Tyr Trp Val Arg Asn Met
   850 855 860
   Ala Asp Pro Val Leu Phe Gly Asp Ala Val Asp Ala Leu Val Arg Asp
   865 870 875 880
   Gly His Arg Leu Phe Leu Glu Val Gly Pro His Pro Thr Leu His Arg
   885 890 895
   Ala Val Gln Glu Gln Leu Gln His Gly Gly Val Ala Gly Gly Phe Val
   900 905 910
   Ala Thr Ser Leu Glu Arg Glu Gln His Gly Arg Ala Arg Leu Leu Glu
   915 920 925
   Asn Leu Gly His Leu Phe Glu Arg Gly Val Arg Val Ala Trp Arg Ala
   930 935 940
   Leu Thr Arg Gly Gly Ala Pro Ala Ser Ala Gly Gln Ala Pro Gly Pro
   945 950 955 960
   Val Leu Leu Pro Leu Ser Ala His Thr Asp Val Ala Leu Gln Gln Arg
   965 970 975
   Val Glu Gly Leu Arg Ala Tyr Leu Arg Ala Asn Pro Glu Ala Glu Leu
   980 985 990
   Glu Ala Leu Ala Arg Thr Leu Gly Arg Gly Arg Thr His Leu Ser His
   995 1000 1005
   Arg Cys Ala Leu Val Ala Ser Ser Ala Gly Glu Leu Ala Glu Val
   1010 1015 1020
   Leu Glu Arg Ala Ala Gly Ala Asp Gly Ala Pro Glu Leu Ile Arg
   1025 1030 1035
   Pro Ala Ala Ser Leu Asp Gly Arg Ala Lys Val Ala Phe Val Phe
   1040 1045 1050
   Pro Gly Gln Gly Ser Gln Trp Leu Gly Met Gly Arg Gln Leu Leu
   1055 1060 1065
   Glu Thr Glu Pro Val Phe Arg Lys Ala Leu His Glu Trp Glu Ala
   1070 1075 1080
   Val Leu Gly Glu Phe Thr Asp Trp Ser Leu Leu Glu Gln Leu His
   1085 1090 1095
   Ala Asp Ala Ala Thr Ser Arg Leu Gly Glu Val Asp Val Ile Gln
       1100                1105                1110
   Pro Thr Leu Phe Ala Ile Gln Val Ala Leu Ala Ala Leu Trp Arg
       1115                1120                1125
   Ser Trp Gly Val His Pro Asp Ala Val Ile Gly His Ser Met Gly
       1130                1135                1140
   Glu Val Ala Ala Ala Tyr Val Ala Gly Ala Leu Asp Ala Arg Asp
       1145                1150                1155
   Ala Ala Arg Ile Ile Cys Ser Arg Ser Arg Ile Ala Lys Lys Thr
       1160                1165                1170
   Ser Gly Arg Gly Ser Met Ala Val Val Glu Leu Ser Pro Glu Glu
       1175                1180                1185
   Cys Gln Arg Ala Ile Gly Ala Arg Ala Ala Thr Val Ser Leu Ala
       1190                1195                1200
   Ala Cys Asn Gly Pro Ala Ser Cys Leu Leu Ser Gly Glu Thr Ala
       1205                1210                1215
   Ala Val Glu Glu Ile Gly Arg Glu Leu Gln Ala Arg Gly Val Phe
       1220                1225                1230
   Phe Arg Leu Val Lys Val Asp Tyr Ala Ser His Ser Pro Gln Met
       1235                1240                1245
   Asp Ala Ile Lys Asp Glu Leu Ala His Val Leu Arg Glu Thr His
       1250                1255                1260
   Pro Arg Pro Ala Thr Val Pro Phe His Ser Thr Val Glu Asn Ala
       1265                1270                1275
   Leu Leu Asp Gly Thr Arg Leu Asp Ala Gly Tyr Trp Ala Arg Asn
       1280                1285                1290
   Leu Arg Glu Pro Val Leu Phe Phe Gln Gly Ile Gln Ala Leu Glu
       1295                1300                1305
   Ala Arg Gly Phe Asn Val Phe Leu Glu Leu Ser Pro His Pro Val
       1310                1315                1320
   Leu Thr Tyr Ser Ile Ser Gln Thr Val Arg Pro Glu Ser Ala Leu
       1325                1330                1335
   Ile Leu Thr Ser Thr Lys Arg Glu Glu Asp Glu Ser Arg Ala Leu
       1340                1345                1350
   Arg Thr  Ser Leu Gly Ala Leu  Phe Ala Ala Gly Gly  Ala Val Asp
       1355                 1360                 1365
   Trp Ala  Ala Leu Tyr Pro Gly  Val Ala Thr Pro Val  Glu Leu Pro
       1370                 1375                 1380
   Ala Tyr  Pro Phe Gln Arg Glu  Arg Phe Trp Phe Asp  Glu Ala Glu
       1385                 1390                 1395
   Val Gln  Arg Ser Ala Pro Ala  Gly Thr Gly Arg His  Ala Phe Leu
       1400                 1405                 1410
   Glu Arg  Phe Leu Arg Ser Pro  Tyr Pro Gln Glu Arg  Leu Ile Trp
       1415                 1420                 1425
   Glu Thr  Asp Leu Ser Leu Glu  Arg Phe Pro Tyr Leu  Val Asp His
       1430                 1435                 1440
   Arg Val  Ser Gly Leu Ala Val  Phe Pro Gly Thr Gly  Tyr Ile Glu
       1445                 1450                 1455
   Cys Val  Leu Glu Ala Ala Lys  Asp Leu Tyr Gly Asp  Ala Pro Val
       1460                 1465                 1470
   Glu Leu  Leu Glu Val Ser Phe  Glu Lys Ala Leu Phe  Ile Glu Asp
       1475                 1480                 1485
   Thr Gly  Ala Lys Thr Leu Gln  Leu Cys Met Glu Pro  Asp Gly Ala
       1490                 1495                 1500
   Gly His  Arg Phe Ser Leu Ser  Ser His Asp Ala Ala  Gln Gln Thr
       1505                 1510                 1515
   Trp Thr  Arg His Val Asn Gly  Arg Leu Val Pro Ala  Thr Ala Glu
       1520                 1525                 1530
   Gln Ala  Ala His Ala Arg Gln  Gln Val Gln Asp Val  Leu Ala Gly
       1535                 1540                 1545
   Glu Pro  His Ala Pro Val Asp  Val Gly Thr Phe Tyr  Gly Asn Leu
       1550                 1555                 1560
   Glu Arg  Ala Gly Asn Thr Tyr  Gly Pro Lys Phe Gln  Ala Val Lys
       1565                 1570                 1575
   Ser Leu  Arg Ser Asn Ala Arg  Gly Val Arg Gly Glu  Val Val Met
       1580                 1585                 1590
   Gly Glu  Glu Leu Ala Thr Arg  Gly Thr Arg Ile His  Pro Val Val
       1595                 1600                 1605
   Phe Asp Ala Cys Leu Gln Val Met Ala Thr Pro Leu Tyr Ile Glu
   1610 1615 1620
   Lys Ala Ala Gly Gly Arg Glu Ser Ala Phe Met Pro Thr Arg Ala
   1625 1630 1635
   Ala Arg Ile Arg Phe Phe Arg Glu Ala Asp Arg Ala Val Val Ser
   1640 1645 1650
   His Val Arg Phe Leu Asn Gly Ala Thr Ala Asp Ala Ala Gly Asp
   1655 1660 1665
   Leu Leu Ala Asp Ile Asp Val Thr Asp Gly Gln Gly Gln Leu Leu
   1670 1675 1680
   Ala Arg Val Glu Arg Ile Cys Phe His Leu Leu Glu His Ala Thr
   1685 1690 1695
   Met Ala Gln Arg Pro Ser Thr Asp Met Leu Tyr Arg Leu Thr Trp
   1700 1705 1710
   Glu Glu Ala Pro Ser Pro Glu Val Gly Ala Ser Leu Ala Arg Arg
   1715 1720 1725
   Trp Leu Val Phe Ala Asp Asp Gln Gly Val Ala Glu Ala Ala Ala
   1730 1735 1740
   Gly Leu Leu Ser Ala Ser Ala His Ala Val His Arg Leu His Pro
   1745 1750 1755
   Ala Asp Ala Phe Arg Arg Asp Gly Asn Asp Phe His Ile Arg Pro
   1760 1765 1770
   Asn Glu Pro Glu Asp Tyr Arg Ala Val Leu Gln Ala Leu Leu Ala
   1775 1780 1785
   Ser Arg Gly Pro Ala Leu Pro Leu Gly Val Leu His Leu Trp Asn
   1790 1795 1800
   Leu Arg Leu Pro Glu Gly Gly Ala Thr Ala Val Pro Asp Leu Arg
   1805 1810 1815
   Glu Met Ala Lys Leu Gly Asn Leu Ser Leu Thr His Leu Leu Lys
   1820 1825 1830
   Ala Leu Asp Gly Ala Gly Gly Ser Ala Pro Pro Arg Val Trp Ile
   1835 1840 1845
   Gly Thr Arg Gly Ala Trp Ala Leu Pro Gly Ser Glu Gln Gly Leu
   1850 1855 1860
   Ala Pro Ala Gln Ser Ala Val Trp Gly Leu Thr Arg Ala Ile Ala
   1865 1870 1875
   Gln Glu His Pro Ala Leu Trp Gly Gly Ile Ile Asp Leu Asp Pro
   1880 1885 1890
   Leu Glu Ala Pro Arg Leu Gln Ala Glu Arg Leu Val Thr Glu Val
   1895 1900 1905
   Ser Val Pro Gln Pro Ala Glu Asp Arg Ile Leu Tyr Arg Gln Gly
   1910 1915 1920
   Arg Arg Gln Val Ser Arg Leu Val Arg Asp Thr Ala Pro Val Ser
   1925 1930 1935
   Ala Ala Thr Ala Thr Phe Arg Gly Asp Arg Gly Tyr Leu Ile Thr
   1940 1945 1950
   Gly Gly Leu Gly Glu Leu Gly Thr Arg Leu Ala Ala Trp Met Val
   1955 1960 1965
   Ala Arg Gly Ala Arg His Leu Ile Leu Ala Gly Arg Lys Ala Leu
   1970 1975 1980
   Pro Ala Arg Arg Thr Trp Lys Gln Asp Gly His Asp Ala Ala Thr
   1985 1990 1995
   Arg Ala Arg Ile Asp Ala Val Leu His Leu Glu Arg Leu Gly Ala
   2000 2005 2010
   His Val His Thr Pro Ala Leu Asp Val Gly Asp Gly Ala Ala Leu
   2015 2020 2025
   Ala Ala Phe Leu Ala Gly Tyr Glu Gln Glu Ala Arg Pro Pro Ile
   2030 2035 2040
   Ala Gly Val Ala His Leu Ala Gly Val Ile Arg Pro Glu Leu Leu
   2045 2050 2055
   Ala Arg Leu Arg Glu Glu Ser Leu Glu Glu Ser Phe Gln Ser Lys
   2060 2065 2070
   Val Tyr Gly Ala Trp Asn Leu His Gln Ala Phe Ser Gly Arg Gly
   2075 2080 2085
   Leu Asp Phe Phe Thr Leu Phe Ser Ser Ala Ser Ala Val Leu Asn
   2090 2095 2100
   Ser Pro Leu Leu Gly Ser Tyr Ala Ala Ala Asn Ala Phe Leu Asp
   2105 2110 2115
   Gly Leu Ala His Phe Arg Arg Ala Arg Gly Glu Pro Ala Gln Ser
   2120 2125 2130
   Leu Asn Trp Gly Phe Trp Ser Glu Ala Gly Met Ala Ala Arg Asp
   2135 2140 2145
   Leu His Phe Asp Ala Ser Ala Ser Gly Gln Gln Gly Met Arg Ser
   2150 2155 2160
   Phe Ser Pro Ala Val Gly Leu Asp Ile Phe Gly Gly Leu Leu Ser
   2165 2170 2175
   Arg Asp Pro Val Gln Ser Val Val Met Asp Val Asp Trp Lys Leu
   2180 2185 2190
   Trp Leu Glu Ala Tyr Pro Asp Ala Ala Ala Ser Pro Leu Leu Arg
   2195 2200 2205
   Thr Leu Arg Ala Ser Glu Gly Val Ala Ala Gly Ser Glu Gln Ala
   2210 2215 2220
   Ala Pro Ala Glu Glu Ala Ser Arg Ser Leu Arg Asp Leu Pro Ala
   2225 2230 2235
   Glu Glu Arg Arg Lys Ala Val Glu Gly Ile Leu Leu Thr Glu Leu
   2240 2245 2250
   Ser Ala Val Leu Lys Met Pro Ala Ala Lys Ile Ser Arg Thr Ala
   2255 2260 2265
   Leu Phe Gly Ser Met Gly Leu Asp Ser Met Leu Ser Leu Glu Leu
   2270 2275 2280
   Arg Asn Arg Leu Glu Arg Arg Thr Gly Leu Thr Leu Pro Gly Thr
   2285 2290 2295
   Leu Ala Trp Asn Tyr Pro Ser Leu Glu Arg Met Ala Ala Phe Phe
   2300 2305 2310
   Val Glu Lys Leu Gly Gly Glu Gly Ala Gly Val Ala Arg Ser Glu
   2315 2320 2325
   Arg Gln Gln Ala Gly Ala Ala Ser Asp Gly Val Ala Lys Glu Pro
   2330 2335 2340
   Asp Ile Ser Ala Glu Val Ile Glu Ala Leu Met Gln Leu Gly Val
   2345 2350 2355
   Leu Glu Ala Asn Asp Pro Val Leu Gln Asp Pro Val Ala Leu Gly
   2360 2365 2370
   Asn Leu Val Arg Glu Ala Gln Gln Tyr Leu Lys Ala
   2375 2380 2385
<210> 18
   <211> 406
   <212> PRT
   <213> Angiococcus disciformis
<400> 18
   Met Thr Tyr Thr Gln Leu Leu His Arg Arg Val Gly Gly Gln Leu Arg
   1               5                   10                  15
   Arg Phe Leu Leu Leu Arg Pro Phe Gly Pro Gly Val Glu Val Asp Asp
               20                  25                  30
   Pro Leu His Pro Pro Glu Leu Pro Pro Tyr Asp Ala Glu Arg Asp Leu
           35                  40                  45
   Ala Pro Arg Thr Pro Gln Phe Leu Gln His Ala Leu Gln Pro Glu Glu
       50                  55                  60
   Arg Gly Arg Pro Gly Ala Pro Gly Leu Pro Ile Thr Thr Ser Glu Leu
   65                  70                  75                  80
   Ser Val Gly Cys Asn Leu Ser Gly Ala Cys Cys Ser Gln Tyr Pro Val
                   85                  90                  95
   Ile Pro Thr Thr Val Glu Glu Ser Arg Arg Ala Val Glu Thr Leu Arg
               100                 105                 110
   Arg Leu Ala Leu Gly Ala Ala Pro Gly Arg Ala Glu Asp Ala Phe Ala
           115                 120                 125
   Pro Val Asn Pro Gly Arg Arg Asp Val Leu Ser Pro Ile Leu Gln Gly
       130                 135                 140
   Asp Gly Cys Pro Phe Leu Ala Gln Gly Ala Cys Gln Val His Lys Ala
   145                 150                 155                 160
   Gly Gly Ala Glu Ala Lys Pro Trp Thr Cys Arg Gln Phe Pro Leu Gln
                   165                 170                 175
   Val Ile His Cys Gly Asp His Phe Glu Val Ser Ile Leu Pro Gln Cys
               180                 185                 190
   Ala Cys Ala Ala Arg Thr Val Thr Arg Gly Ser Gly Gly Asp Trp Pro
           195                 200                 205
   Glu Asp Pro Leu Arg Gly Leu Thr Ser Val Pro Arg Val Pro Asp Ser
       210                 215                 220
   Val Val Val Glu Gly Arg Arg Thr Leu Ala Arg Gly Met Tyr Leu Gln
   225 230 235 240
   Trp Val Arg Ala Leu Ala Asp Arg Leu Ser Ala Pro Gly Leu Gly Glu
   245 250 255
   Pro Ala Leu Val Leu Asp Ser Ala Ser Gln Ser Leu Arg Val Ala Pro
   260 265 270
   Gly Ala Leu Thr Pro Gly Trp Leu Gly Glu Leu Arg Ala Arg Met Leu
   275 280 285
   Ser Glu Ala Glu Arg Leu Glu Gly Tyr Leu Asp Pro Ala Gly Pro Gln
   290 295 300
   Pro Lys Gly Thr Arg Trp Val Ala Ser Leu Ala Glu Ala Leu Leu Ala
   305 310 315 320
   Asp Ala Asp Ala Glu Pro Glu Pro Ala Pro Ser Asp Gly Leu Val Leu
   325 330 335
   Ala Leu Ala Leu Arg Ala His Leu Leu Leu Glu Leu Pro Ala Leu Gly
   340 345 350
   Ala Ala Leu Ala Asp Leu Thr Arg Leu Ala Arg Leu Ala Ser Leu Ser
   355 360 365
   Arg Thr Val Ala Pro Ala Glu Ala Ala Asp Ala Arg Phe Glu Pro Leu
   370 375 380
   Thr Leu Leu Leu Tyr Leu Trp Ala Thr Thr Arg Trp Pro Val Ser Ser
   385 390 395 400
   Glu Gly Val Thr Arg Pro
   405
<210> 19
   <211> 409
   <212> PRT
   <213> Angiococcus disciformis
<400> 19
   Met Leu Gly Gly Leu Thr Gly Arg Gly Glu His Met Thr Gly Thr Ser
   1 5 10 15
   Gly Gln Gly Ala Gly Gly Gly Arg Ile Asp Thr Pro Pro Glu Gly Ala
   20 25 30
   Leu Arg Ala Val Arg Ser Leu Val Glu Leu Gln Glu Thr Glu Gln Ala
   35 40 45
   Ala Gln Leu Tyr Glu Glu Leu Gly Asp Ala Gln Arg Glu Arg Leu Arg
   50 55 60
   Arg Glu Ala Ala Arg Arg Pro Ala Arg Glu Arg Arg Gly Leu Val Glu
   65 70 75 80
   Val Leu Arg Arg Ala Arg Asp Phe Leu Gly Ala Ala Arg Leu Met Glu
   85 90 95
   Gly Asp Gly Glu Asp Ala Thr Val Ala Asp Leu Tyr Phe Gln Ser Gly
   100 105 110
   Gln Tyr Leu Glu Ala Ala Glu Ala Tyr Leu Arg Ala Gly Glu Ala Glu
   115 120 125
   Arg Ala Ala Ala Ala Phe Glu Arg Ala Gly Ala Leu Glu Arg Ala Leu
   130 135 140
   Asp Val Tyr Arg Arg Leu Gly Ala Arg Glu Ala Met Ala Gln Cys Leu
   145 150 155 160
   Val Arg Leu Gly Arg Pro Phe Glu Ala Ala Asp Leu Tyr Arg Glu Leu
   165 170 175
   Gly Gln Ala His Ala Glu Val Glu Ala Leu Gly Gly Val Lys Pro Glu
   180 185 190
   Asp Ser Arg Tyr Val Glu Ala Val Leu Arg Val Cys Thr Leu Leu Asp
   195 200 205
   Val Glu Gly Phe Thr His Arg Ala Leu Ala Val Leu Ala Asp Ala Val
   210 215 220
   Ser Ser Ser Glu Glu Ala Arg Ala Asp Glu Ala Leu Val Lys Glu Lys
   225 230 235 240
   Ala Arg Leu Leu Arg Arg Met Gly Met Glu Ala Glu Ala Glu Ala Val
   245 250 255
   Leu Leu Arg Arg Ala Thr Ala Ala Ala Thr Pro Ala Ala Asn Gly Tyr
   260 265 270
   Arg Phe Leu Lys Ala Ile Pro Ile Phe Gly Glu Leu Ser Leu Glu Asp
   275 280 285
   Met Lys Asp Leu Tyr Arg Leu Ala Arg Gln Val Val Ile Ala Pro Gly
   290 295 300
   Thr Val Leu Leu Glu Lys Gly Ala Arg Gly Thr Gly Leu Phe Val Leu
   305 310 315 320
   Leu Asp Gly Thr Val Glu Val Phe Ser Gly Pro Glu Gln Asp Ala Arg
                   325                 330                 335
   His Leu Asn Thr Leu Gly Pro Gly Ala Tyr Leu Gly Glu Ile Ser Leu
               340                 345                 350
   Val Gln Asp Ala Pro Val Ser Ala His Val Arg Ala Arg Thr Ser Val
           355                 360                 365
   Arg Ala Leu Arg Ile Ser Arg Ala Asp Phe Glu His Tyr Leu Asp Thr
       370                 375                 380
   His Glu Ala Ala Ala Leu Arg Ile Tyr Arg Leu Phe Thr Gln Asn Leu
   385                 390                 395                 400
   Ala Ala Arg Val Arg Ala Leu Ser Thr
                   405
<210> 20
   <211> 40784
   <212> DNA
   <213> Pyxidicoccus fallax
<220>
   <221> misc_feature
   <222> (1)..(40784)
   <223> Gulmirecin biosynthetic gene cluster
<400> 20
<210> 21
   <211> 1185
   <212> DNA
   <213> Pyxidicoccus fallax
<400> 21
   atgcgtacgt tcccggccgg tccggagctg ttgaacccct atccattctt cgccgagatg 60
   cggcggcatc acccggtggc attcgatgac ggggccaacc tctggtccat ctaccgccac 120
   gcggacgtga cggccatcct ctccgaccac gtgaacttcc cgacggcgac catcgatgat 180
   ggcggggcgc ctcccgccga gtggcgccgc ctcttcagcc tgaatgcctt tgacccgccc 240
   cgccataccc agctgcgcaa cctggccatg agggccttca cggcctcggc ggtggcccgc 300
   atgcaggacc ggatcaccgc catcgtcaat cagctgattg acgccgtggt cggcacgggg 360
   aggaccgagc tcgtggcgga catcgccggc cccctgccca cgatggtgat cgccgagatg 420
   ctgggcgtgc cggcgtcgga ccaggagtcc ttccggcgct ggtcggacga ggtgggcgtc 480
   cgggccaatg ccctggtgct cgatccggag aacggggtga ggctcctgtc ggaggcgctg 540
   acgccgatgg aggactacgt gcggcagcag atcgccgagc gccgccgccg tccctcggac 600
   gacctcgtca gcagcctcct ggcggcggag atcgacggcg agaggctgga ggagctcgac 660
   ctggtcgcct tctgcatgct gctgctcatc gccggcaaca tctccaccac ccacatgatt 720
   ggcaacgcca tccgcgcgct cctgaagcac ccggaccaga tggcgcgctt cctcgcgaac 780
   ccggaccaga tgccgggggc catcgaggag ctgctccgct acgactcacc ggtcctcgcc 840
   gcgccccgct gggtgcggca cgacacggag gtcggcggca agctcctcaa gcggggccag 900
   cgcgtcatcg tgtggctcgg cgcggccaac cacgatcccg acgtcttccc cgagccggag 960
   cgcctggacg tgagccgcaa ggcggcgcgt caggtctcgt tcggccacgg cgcccactac 1020
   tgcatggggg ctccgctggg ccgcctggag gcgcgtatcg ccatcccggc catcctgcgc 1080
   cgcatgccgg agctgcggat ggtggagggg gccgagctgg agcccgtgcc gggctacctg 1140
   ctgcacggga tggtcagcat gccgatgacg ttccgggcca gctga 1185
<210> 22
   <211> 7158
   <212> DNA
   <213> Pyxidicoccus fallax
<400> 22
   atggatgcct cctcctcccg ccccttcgag tcactggacg cccttcaggc gtgggtcgtc 60
   acctggctgt gcgagcatgc ccggattccc cagcaggcca tcggcctcga cgtccgcctg 120
   agggacctcg gcctcgactc ggcccgggcc acgaagctgg tggccgcgct ctccgagcgc 180
   ttcggccgga cgctcccgct gacgctgctg tgggactacc cgaccgtcac ggagctgtcc 240
   cggcacctga tgaagcccgc gacggcggcg gcggccggcg ccacgccccg ggccgagcgt 300
   cgcgcgagcg gcgctccgga ggaccgcgcc atcgccgtca tcggcatggc ctgccggttc 360
   ccgggcggcg cggacacgcc cgaggccttc tgggagctgc tggcgaaggg gctcgacgtg 420
   gtggggccgg tgcccgcgga ccgctacgac gcggaggcct tctacgaccg tgacccgcgg 480
   gcccgcggca aggccgtcac ccggtggggc ggcttcctgg agcggaccga caccttcgac 540
   ccctacttct tcggcatctc tccgcgagag gccatcgaga tggacccgca gcagcgcatg 600
   ctgctggagc tggcctggga ggcgttcgag gcggcgggcc tgacgaagga cgggctccgg 660
   gaccgcgcgg tcggcgtctt cgtcggcgcc atgtggaatg actacgcccg gctgtggcgg 720
   ggcggccccg gcgccatcga gcagttcacc gcgaccggcc aggacgacag catcatctcc 780
   gcccgcatct cctacgtctt cggcctgcgc ggcccgagcc tgacgctgaa caccgcctgc 840
   tcgtcctcgc tggtgtccgt gcacatggcc tgccagagcc tgctggcggg tgactccgag 900
   ctggccctgg cgggtggcgt caacctcatc cacggcccgg acagcaccat cgccatgtcg 960
   aagttcggcg gcatggcgcc ggacgggaag tgcaaggcct tcgacgcgcg tggcaacggc 1020
   tacgtccgcg gcgagggcgc gggcgtcgtg ctgctcaagc cgctgcaccg cgcgctcgag 1080
   gacggcgacg tcatccacgc ggtcatccgc ggctcggccg tcaacaacga cggcttctcc 1140
   aacggactga cctcgcccag cccggccgcc caggccgcga tgctgcgcgc ggcctacgcc 1200
   agcgccggcg tgtccccggc gcgggtccac tacgtggagg cgcacggcac cggcaccatg 1260
   ctgggtgacc ccatcgaggc gggcgcgctc ggcgaggtgc tcgggcagca ccccgagcgc 1320
   cagcagcccc tgcgcatcgg gtccgcgaag acgaacatcg gtcacctgga ggccgccgcg 1380
   ggcatggcgg gcctcatcaa gaccatcgag gcgatgaagc accggcgcct gccgccctcg 1440
   ctccacttcg agcagcccaa ccccaacatc cacttcgagg ggctccgcct gcgggtgcag 1500
   accgacctct caccgtggcc caccgaggac gactcgctgc tcgcgggcgt cagctccttc 1560
   ggcttcggcg gcaccaacgc ccacgtggtg ctcgagtcct tccagcccgc ccggccggtg 1620
   gccatccccc tctccgcgcc ggacgcggag gcgctgggcg cccggctcgt cgagctgcgg 1680
   gccttcgcgg agcagcaccc cgaggcctcg ctggacgacc tccagcgcgc gtgggtccag 1740
   cgcctcaccg aggagccggt gcgccaggtg ctcctcgccc gctcccggga ggacctcctg 1800
   gagcagctcc gcgcgccgtc acggccggcg gctcccgcgg ggggcggcgc ggcgcggccg 1860
   gtcctcgtct tcaccggcaa cggcgcgcag taccccggca tggcgcgaga cctgatgctc 1920
   ggggacgcga ccttccggca ggcggtcctc cgctgtgacg aggcgctgcg ggcgcacggc 1980
   tgcgacgggg tggcccgggc gctcgagtcc ggtgacttcg acgtgcggcg ggtggacctg 2040
   gtgcagccgc tgctgtgggc ggtgcaggtc ggcctcgtct ccctgtggcg cgcgtggggt 2100
   gtggagcccg ccgccgtcgt cggccacagc gtcggcgagg tggccgccgc gtgggccgcg 2160
   ggcgcgctcg acctggacga ggccgcgcgc gtcgtggtcc accgcagccg gctgcagcag 2220
   gagtgcgcgg accagggcgg gatggtgctc ctcgagaccg acgccgaggg cgcgcgctcc 2280
   ctcgtgggcg ccacggcggc ggaggggctg gccgtcgccg ccctcaactc gcgcggcagc 2340
   accgtcgtga gcggccccac ggcgagcctc caggcgctca tcacgaaggc ggaggagcgc 2400
   ggggtgacgg cgaggacggt ccgcgtcaac gtcgcgtacc acggccccgg gatggacgcg 2460
   ctgcgcgagc ggctgcggga gtccctcgcg gacctgcgtc cgtgccctcc ccgcgtcccg 2520
   atgtcctcct cggtgacggg gggcgcggtg gggggcgcgg gcctggacgc cggctactgg 2580
   gtccgcaaca tggccgaccc cgtgctgttc ggtgacgcgg tcgacgcgct ggtgagggac 2640
   ggccaccgcc tgttcctgga ggtgggcccg caccccacgc tccaccgggc cgtccaggag 2700
   cagctccagc acggaggcgt ggccgggggc ttcgtcgcga cgagcctgga gcgcgagcag 2760
   cacggccgcg cccgcctgct ggagaacctg ggccacctgt tcgagcgcgg tgtccgcgtg 2820
   gcgtggcggg cgctgacgcg gggaggcgcg ccggcctccg cgggccaggc gccccgcccc 2880
   gtcctcctcc ccctctccgc ccacacggac gcggcgctcc agcagcgcgt ggaggggctg 2940
   cgcgcgtacc tgcgggcgaa cccggaggcg gagctggaag ccctggcccg taccctgggc 3000
   cggggacgca cccacctgtc gcaccgctgc gcgctcgtgg cctcctcggc cggcgagctc 3060
   gcggaggtcc tggagcgcgc ggccggcgcc gacggcgcgc ccgagctcat ccgccccgcg 3120
   gcctccctcg acggccgcgc gaaggtggcc ttcgtgttcc ccgggcaggg ctcccagtgg 3180
   ctcggcatgg gccgccagct cctggagacg gagcccgtct tccgcaaggc gctccatgag 3240
   tgggaggccg tgctgggaga gttcaccgac tggtcgctgc tggagcagct ccacgccgac 3300
   gccgcgacgt cccgcctggg cgaggtggac gtcatccagc cgaccctctt cgccatccag 3360
   gtcgcgctgg cggcgctgtg gcgctcgtgg ggcgtgcacc cggacgccgt catcggccac 3420
   agcatgggcg aggtcgccgc ggcctacgtc gcgggggcgc tggacgcgcg agacgcggcc 3480
   cgcatcatct gctcgcgaag ccgcatcgcg aagaagacga gcggccgcgg cagcatggcc 3540
   gttgtcgagc tgtcccccga ggagtgccag cgcgccatcg gcgcccgcgc cgccacggtg 3600
   agcctcgccg cctgcaacgg gcccgcctcg tgcctgctgt cgggagagac ggccgcggtg 3660
   gaggagattg gcagggagct ccaggcccgg ggcgtcttct tccggctcgt caaggtggac 3720
   tacgcgtccc acagccctca gatggacgcc atcaaggacg agctggcgca cgtccttcgc 3780
   gagacgcacc cgcgcccggc caccgtgccg ttccactcca ccgtcgagaa cgccctcctc 3840
   gacgggacgc ggctggacgc cggctactgg gcgcgcaacc tgcgcgagcc ggtgctcttc 3900
   ttccagggca tccaggcgct ggaggcccgc ggcttcaacg tgttcctgga gctcagcccg 3960
   caccccgtcc tcacctactc catctcgcag acggtgcgtc cggagagcgc gctcatcctc 4020
   acctcgacga agcgcgagga ggacgagtcg cgggcgctgc gcacgtcgct gggcgcgctc 4080
   ttcgccgcgg gcggcgccgt ggactgggcc gcgctgtacc cgggcgtggc gacgcccgtc 4140
   gagctgcccg cctatccctt ccagcgggag cggttctggt tcgacgaggc ggaggtccag 4200
   cggtccgcgc ccgcgggcac cggccgccac gcgttcctcg agcgcttcct ccgctcgccg 4260
   tacccccagg agcgcctcat ctgggagacg gacctgagcc tcgagcgctt cccgtacctc 4320
   gtggaccatc gcgtcagcgg cctcgccgtc ttccccggca ccggctacat cgagtgcgtc 4380
   ctggaggcgg cgaaggacct gtacggcgac gcacccgtcg agctgctcga ggtctccttc 4440
   gagaaggccc tcttcatcga ggacaccggc gcgaagacgc tgcagctctg catggagccg 4500
   gacggcgccg gccaccgctt ctccctgtcg agccacgacg cggcccagca gacgtggacc 4560
   cgccacgtga atggccggct cgtgcccgcc accgccgagc aggccgccca cgcgcgccag 4620
   caggtccagg acgtgctggc cggcgagccc cacgcgccgg tggacgtggg gacgttctac 4680
   gggaacctcg agcgcgcggg gaacacctac ggcccgaagt tccaggccgt gaagtccctg 4740
   cgctcgaacg ccaggggcgt ccgcggcgag gtcgtcatgg gcgaggagct ggccacccgg 4800
   ggcacccgca tccaccccgt cgtcttcgac gcctgcctcc aggtgatggc cacgcccctc 4860
   tacatcgaga aggccgcggg gggccgcgag agcgccttca tgccgacgcg ggcggcgcgc 4920
   atccgcttct tccgtgaggc cgaccgggcc gtggtcagcc acgtgcgctt cctgaacggg 4980
   gccaccgccg acgcggccgg cgacctcctc gcggacatcg acgtcacgga cgggcagggc 5040
   cagctgctgg cgcgggtgga gcgcatctgc ttccacctgc tggagcacgc caccatggcg 5100
   cagcgcccgt ccaccgacat gctctaccgg ctgacgtggg aggaggctcc ctcgccggag 5160
   gtcggcgcct cgctggcgcg ccgctggctc gtcttcgcgg acgaccaggg cgtcgccgag 5220
   gcggcggccg ggctgctgtc cgcctcggcg catgccgtgc accgcctcca cccggcggac 5280
   gccttccggc gcgacggcaa cgacttccac atccgcccga acgagcccga ggactaccgc 5340
   gccgtgctcc aggccctgct ggcctcgcgc ggccccgcgc tgccgctcgg cgtcctccac 5400
   ctgtggaacc tccgcctgcc cgagggcggc gccacggccg tgccggacct gcgggagatg 5460
   gcgaagctgg gcaacctcag cctcacccac ctgctgaagg cgctcgacgg ggccggcggc 5520
   tccgcgccgc cgcgcgtctg gattggcacg cggggcgcgt gggccctccc cgggagcgag 5580
   caggggctgg cgccggccca gtccgcggtc tgggggctca cccgggccat cgcgcaggag 5640
   cacccggccc tgtggggcgg catcatcgac ctggatccgc tcgaggcgcc gcgcctccag 5700
   gcggagcggc tcgtcacgga ggtctccgtc ccccaaccgg cggaggaccg catcctctac 5760
   cggcagggcc ggcgtcaggt gtcgcggctg gtccgcgaca cggcccccgt cagcgccgcg 5820
   accgccacct tccgcggcga ccgcggctac ctcatcacgg ggggcctcgg tgagctgggc 5880
   acgcgcctgg cggcgtggat ggtggcccgt ggcgcgcgcc acctcatcct ggcggggcgc 5940
   aaggcgctgc cggcccggcg gacgtggaag caggacgggc acgacgcggc gacccgggcg 6000
   aggattgacg ccgtcctcca cctggagcgc ctgggcgctc acgtccacac gcccgcgctg 6060
   gacgtggggg acggcgcggc gctggccgcc ttcctcgccg gctacgagca ggaggcgcgg 6120
   ccccccatcg ccggtgtcgc ccacctcgcg ggggtcatcc ggcccgagct gctggcccgg 6180
   ctccgcgagg agtccctcga ggagtccttc cagtcgaagg tctacggcgc gtggaacctg 6240
   caccaggcct tcagcgggcg agggctggac ttcttcacgc tgttctcatc cgcctcggcc 6300
   gtcctgaact cgcccctgct ggggagctac gccgcggcca acgccttcct ggacggcctg 6360
   gcgcacttcc gccgcgcgcg gggcgagccg gcccagtccc tgaactgggg cttctggagc 6420
   gaggccggca tggccgcgcg ggacctccac ttcgacgcga gcgcgtccgg ccagcagggc 6480
   atgaggagct tcagccccgc ggtgggcctg gacatcttcg gcgggttgct gagccgcgac 6540
   ccggtgcagt cggtggtgat ggacgtggac tggaagctct ggctcgaggc ctacccggac 6600
   gccgccgcgt cgccgctgct gcgcaccctg cgcgccagcg agggcgtggc cgcgggctcc 6660
   gagcaggcgg cgccggccga ggaggcctcg cggagcctga gggacctgcc ggcggaggag 6720
   cgccggaagg ccgtcgaggg catcctgctc accgagctgt ccgcggtcct caagatgccg 6780
   gccgcgaaga tttcccggac ggcgctcttc gggagcatgg gcctggactc catgctctcg 6840
   ctggagctgc gcaaccggct ggagcggcgc acgggcctga cgctgccggg caccctggcc 6900
   tggaactacc cctccctcga gcggatggcc gcgttcttcg tcgagaagct cggcggggaa 6960
   ggcgctggcg tggcgcggtc cgagcgccag caggccggcg ccgcgagcga tggcgtggcg 7020
   aaggagccgg acatctcggc ggaggtcatc gaggccctga tgcagctggg cgtcctcgag 7080
   gcgaacgacc ccgtgctcca ggatccggtc gcgctcggca acctggtgcg cgaggcccag 7140
   cagtacctga aggcgtga 7158
<210> 23
   <211> 4713
   <212> DNA
   <213> Pyxidicoccus fallax
<400> 23
   atgactggaa tcatcgacaa gctgagccgg ctcgatgccg cgagcaagga cctcctggcg 60
   gagaagctgc gccaggccaa ggacaagcac ctggcgcgag gggcgcacgc cgagcccatc 120
   gcggtggttg gcatggggtg ccgctttccc ggtggcgcca gctctcccga gcgcttctgg      180
   cagttgctga cgcaggggcg gagcggcgtc ggccgcgtcc ccgcggatcg ctttcccatc      240
   gaggactact acgaccccga cccgagcgcg ccgggcgcca tcctcagtga ccggggcgcc      300
   ttcgtggagg gtatcgaccg cttcgacgct ggcttcttcg gcgtgtcccc ccgcgaggca      360
   cggcgcctgg accctcagca ccgcctcctc ctggaggtca cctgggaggc gctggagaac      420
   gcgggcctgc ctcagtcggc gctccgcgag ctcgttggcg gcgtgttcat cgggttgatg      480
   ggcaacgact acttctccac cgtccaggag aaccccgccg acatcgacgc ctacaccagc      540
   acgggcacgc actacagctt cgcggccaac cggctctcgt tccatctgga cttcaagggc      600
   ccgagcatgg ccatcgactc ggcctgctcg tcgtcgctgg tggccgtcca ccaggcctgc      660
   cagagcctcc ggacccggga ctgcgacttc gcggtggccg gcggcgtcaa cctcgtcctc      720
   tcccccgtcc tgagcatctc gtattcgaag tgggggatgc tcagccccga cgggcagtgc      780
   aagaccttcg acgcctcggc gaacggcttc gtgcgcggcg agggcgccgg cgtggtcgtc      840
   ctcaagcggc tggaggacgc cgtggcggcc ggggacccca tcctcgccgt cgtgaggggc      900
   tcggccgtca accaggatgg cgccaccagc gtcatcaccg cgcccagcgg gctcgcccaa      960
   caggccgtca tccgccgcgc cctgaaggtg gcgggcgtgg agccctccgc catcggctac     1020
   gtggagaccc acgggacggg gacgtcgctg gggaccccca tcgaggtgga ggccctcaac     1080
   gagacctacg ggcaggcgcc gggcggccgc ccgtgcgtgc tcggggccgt caagacgaac     1140
   gtgggacacc tcgagggagc ggccggcatc gccggcttca tcaaggcggt cctcgccgta     1200
   cagcacgggc gaatccctcc gaacctccac ttccacgagc tgaacccgct gatccggctc     1260
   gacggctcgc ggctgtgcct ggccacggag gcccgggagt gggccgtggc gagccaggag     1320
   cgcttcgccg ccatcagctc gttcggcgcc ggaggcacca acgcccacgt cgtcatccag     1380
   ggcccccccg cggcgcgggc gcccgcggcc accgggaagg cgcccgcctt cgtctgcctg     1440
   tcggcgaaga gcgacgcctc cctgaagcag caggccgcga ggctgcgcgc ctggctcggg     1500
   caggacggcc gggagtggag cgtcgccgac atcgcctcca cgagcctgcg gcgcaggaac     1560
   cacttcgacc accgggcggc cttcaccgcc gactcgccgg agtcgctcgg cgagggcctg     1620
   gacgccttcc tgtccggccg ggccagcccg cgctggtccg ccggcagcgg caccctgaac     1680
   gcccagcggc cggtgttcgt gttctccggc cagggctcgc agtggcaggg catgggccgg     1740
   gagctgctca cccagagccc gcgcttcgcg gcggagatcg ccgccatcga cgcgcggttc     1800
   atcccgctgg ccggctggtc catcgccgag gtcctcgcgc gggacggcga cggggcgcgg     1860
   ctgaacgcca cggaggtggc gcagccctgc ctgttcgccc tgcagctggg gctcgtcgcc     1920
   gcgctggacg agctgggcat ccggcccgcc gcggtcgtcg gccacagcct cggcgagctc     1980
   gccgcggcgt gcgtggccgg cctcatggac cgcgaggccg ccacggccct cgtgtaccac     2040
   cgtgcgaagc tcatgcagcg gctgaccggc gcggggaaga tggccgccgt cggactctcc     2100
   cgcgacgagg ccgaggcacg cctcgcggcg gagccccggc tggcgctcgc ggcggtgaac     2160
   ggaccccgct cggtcgtcgt ggccggcgag ccggaggccg tggacgcctt cgtgcggacg 2220
   ctgcgcgacg agggggtctt cacgcggacg ctgccggtcg actacgcctt ccacagcccc 2280
   cagacggacc cgctcctggc cgagctgcgg cgggccgtcg acggcatctt cggcgcgggc 2340
   gcgaggatgg ccgccccccg gctgccgatg gtgtccacgg tgagcggcgc cgagctcgat 2400
   gcctccgtcg accacgccac ctactgggcc cggaacatgc ggcagacggt gtgcttcgag 2460
   gaggcggtgg gccagctgct ggagcagcgg cacgacgcct tcctggaggt tggcgcgcac 2520
   ccggtcctga acgtcgacct gcgggagtcg ttccgcgcgc gggaggtgac ggcgcgggcc 2580
   gtcgccaccc tccggaggga acagccccag gccctctgcc tgcgggcggt gcccgccgcg 2640
   ctctacgccg ctggcatggc ccccgaggcg ctccccggcc aggaccaggg caccctcgtc 2700
   ccgctcccga actacgcgtg ggaccaccgc cgctactgga ttcactccgc ctcgcgggcc 2760
   gaggcgggct gggtctacga cctcgcctgg gacgcggtgc cggcacctgc gccgggcgcc 2820
   caccggcccg cgtcctggct cgtcctgggc gcctccggcg ggctgccgga ggcgctggcc 2880
   gcccggctgc gcgccacgcc cgacctggtg cgccacgtga gcctggaggc gctgcgctcc 2940
   gtgcccgtgc ccccccacgg gctggtggac gcggagcggc tcgagcgctg gttcacggac 3000
   acgctccagg tggacctggc ggagttcgac ttcctcgtct acgtgcccgc gtgcggcgcc 3060
   cccgaggacg cgagcgccga gcgccaggtc agcgaggact gcgccatcct ggtgagcctg 3120
   gcccaggccc tggagaggcg gcccggggcc cggggcccgg cgctctgggt cgtcacgagc 3180
   ggcgcgcagg ccgtcgcgcc gcgggagccc gtcaatccct tcgccgcgcc cgtgtggggc 3240
   ctcggccggg tgctcgccct ggaagccccg cgaggcctgg gcggtctcgt cgacctgccg 3300
   ctcgcatggt cggagcagga cgtgggctgc ctgctcgagg cgctgcgcca ccccggcgag 3360
   gaggaccagc tcgcgctgcg ctcgggccag cgccatgccc tccggctgcg ccgccgggcg 3420
   ggccgcgcca ccgacgcggc ctcgacctgg caggtcgagg actccggcgt ctacctcatc 3480
   accggtggga cgcggggcct ggggctgctc gtggcgaagc ggctggccga gcgcggcgcc 3540
   cgtcacctgg cgctgctggg ccgccgggcg ctgccgcccc gctcgcggtg ggcgacgctg 3600
   gccgcggacg acgccttctt cccctccgtg caggccatcc aggcgctgga ggcccagggc 3660
   gtcaccgtca cgctccacgc cgccgacgtc agcgactccc acgcgatgcg ggacgtgttc 3720
   gcggagctcg agcgtcagca gcgccccctc aagggcgtcg tgcacgctgc gggcatctcc 3780
   cgggaggtcc ccctggcctc catcgatgag gcgctgctgg aggagaccct ggcgcccaag 3840
   gtcgcgggca cctggtggct tcaccagctc tcgctgcggc acccgctgga cttcttcgtc 3900
   gtcttctctt cgggggcctc cgtctggggc tccgcgggcc tggcgccgta cgccgccgcg 3960
   aacgagttcc tgaacgccgt ggcggcccac cgccgcgcct ccggcctgcc cgccctcagc 4020
   gtctgctggg gcatgtggga cgcggacggc atggccaccg acgagatgcg ggagaactgg 4080
   acgcgcctgg gcgtcaaggc gatggccccc gagcgcgccc tcaacgcgct ggaggagctg 4140
   gtggccgcgg acgccaccct cgccaccgtg gccgacgtga agtgggcccg cttccggccc 4200
   gtctacgagc tgaagcgcaa gcgtccgctg ctggcggcgc tcgaggacgg cgcggcggcg 4260
   gcgccccggg ccgaggcggc cggggcgctc caggccgcct ccctcaaggc ggagctccag 4320
   gcgctgccgc gggagcagca gcgcgagcgg ctgctggcca ccctccgcga cgcggccgcg 4380
   ctcgtgctcg gcgccgagcc gggtgccgcc atcaacccgg acgaggggct gttctcgatg 4440
   ggcttcgact ccgtcacggc cgtcgagttc gcggcgcacc tgagccgcct gttcgagaag 4500
   cgctacgccg cgacgctggt cttcgaccac ccctccctca agcagatccg cgacttcctc 4560
   atgcaggact gcttcggctg gacggccgag ccgtcccccg agcccgaggc gggtgacggc 4620
   caggacctcg acgcgctcct cgccttcgtc gagggcgtgg acgaggccga ggccgagcgg 4680
   ctgctctccg ccggtcagcc ccggtcgaac tga 4713
<210> 24
   <211> 5571
   <212> DNA
   <213> Pyxidicoccus fallax
<400> 24
   atgacctccg tcgacgaacg catcaagaac ctctccccgc agaagcgcgc cctgctcgag      60
   cagcggatga acgccgccgc cgccagcgcc acccgggacg cggagcccat cgccatcatc     120
   gggattggtt gccgctaccc ggggggcgtg aaggacctga agagcttcgc ccagctgctg     180
   cgcgacggcg ccgacgccac cggggacatc cccgcggacc gctgggacgt ggaggcccac     240
   tactcgcccg acccgggcac ccccgggaag atgcgcaccc ggcgcggcgg cttcctgccc     300
   gacgtgaaga ccttcgacgc ggacttcttc ggcatctctc cgcgagaggc gcagagcatg     360
   gacccccagc agcggctgct gctggaggtg tcctacgagg ccctcgagga cgcggggatt     420
   cccaccgcct cgctgagcgg gagcaccacc ggcgtctaca tcggtatcac cctggcggag     480
   tacgccgacc ggctgccgga cgacccccgc ctcatcgacg tctactcggg gacgggcacc     540
   tacctgaacg tggccgccgg gcgcctgtcc tacgtctacg gcctccaggg cccctgcatg     600
   gccgtggacg cggcctgcgc ctcctcgctg gtgacggtcc acctggcggc ccaggccctg     660
   cggcaccgcg agtgcggcct ggcgctggcg ggcggcgtga gcctgatgct gtcgccggag     720
   accaccatct acctgtcgca gacgggcgcc ctggcccccg acggccgctg caagacgttc     780
   gacgctcgcg ccaacggcta cggccgcgcc gaggggtgcg gcatcctcgt cctcaagcgg     840
   ctgtctgatg cgctgcgcga caaggaccgc atccacgcgc tcatccgcgg cacggcggtc     900
   agccaggacg gcgcgtccag cggcctgacg gtgcccaatg gccgcgccca gcagaaggtc     960
   atccagtccg cgctcaagga cgcggcggtg cggagcgagg acgtctcgta cgtcgaggcc    1020
   cacggcacgg gcacgccgct cggggacccg atagagctca acgccctcca ggccgtctac    1080
   gggcagcgcg aggcggactg gccgctccat gtcggctccc tgaagacgaa cttcgggcac    1140
   gccgaggccg cggcgggcgt gggcggcatc atcaagacgg tggtggcgct ccaggagagg    1200
   cagatccccg cccacctcca cttcgagaag ctcaacccgc tcatcaccat cgaccccacg    1260
   cgcgtcgtca tccccacccg gcccacgccg tggcaaacgc ggggggagcg gaagcggctc     1320
   gccggcgtca gctcgttcgg gttcagcggc gtgattgccc acgccgtcct ggaggaggcg 1380
   cccgcgccgg aagcggccgt cgagcagccg gcgatgcccg ccggggagcg gccgctgcac 1440
   gtcctgccgc tcaccgcgaa gcacccccgg gcgctgagcg agctcatctc ggcctaccgg 1500
   cacaccgtcg gcgcgctcga cgcgggccgg ctcgccgacc tgggctatac cgcgagccgg 1560
   aagcgcggcc accacgcgca ccgcgcctgg ttcgccttcc gcgacaagga ggagctgctc 1620
   cagcagctcg aggccgcgac ccggggccac gagctgcccg gcgccgtgct ccagcggcag 1680
   gcgcgcgacg acgtcaaggt cgtcatggtg ttccccggcc agggctccca gtgggtcggc 1740
   atgggccagc agttgatgcg gacctccccg gtgttccgcg aggccatcca cgagctggac 1800
   cgcgcgatgc ggccctgggc ggacttctcg ctgctggagg tgctcggcgc ccgcgggggg 1860
   gaggatccgt tcacccgcgt ctccatcgtc cagccgaccc tcttcgccgt ccaggtggcc 1920
   ctggccaggc agtgggaggc ctggggcgtc aggccgcacg cggtcatcgg tcacagcatg 1980
   ggcgaggtcg cggccgcgtg cgtcgcgggc gcgctcagca tggaggacgc cgccgcggtc 2040
   atctgccgcc gcagccagct cgtccaggcc accagcggcc agggcgccat ggcgctcgtc 2100
   gagctgagcc ccgccgaggt cgaggccgcc atcgcgcccc acggggaccg cgtcagcatc 2160
   gccgcgtaca acgggccctc caccgtcctg ctgtcgggcg agccggccgc cgtcgacgag 2220
   gtgctcaccg ccctccagga gcgcggggcg ttctgccgcc gcgtcaaggt ggactacgcc 2280
   tcgcactgcg cgcagatgga cccgctgctg ggccgcatcc gcagcgagct ggcccacgtg 2340
   aagccgcgca agggcggcgt gcccatctat tcgaccgcgc gcggcgaggt catcgacggc 2400
   tcgcggatgg acgcgggcta ctgggcggac aacctgcggc gccccgtcct cttccacgcg 2460
   caggtcaagg cgctcctcgc ggacggcatg agcgcgctgc tggagatcag cccgcacccg 2520
   gtgctcctgc cgtcaatgca gaagaccctg gacgaggccg gcggacagca gctcgtgctg 2580
   cccagcctgc gcagggacga ggacgagccg cggagcctcg cgacgtcgct gggcgccctc 2640
   ttcggcctcg gccaccctgt ggacttctcc gccttcaccc cgaggtccgg ccggctcgtc 2700
   gagacgccgc gctacccctg gcagcggcgc gagttctggc tgacgcggaa ggcgtcgaag 2760
   cgccggcacg cgctgggcgg cgcggaggga catcccctca tcgggggcgc cttccgcccg 2820
   gccgtgggcc cggagtccta ctactgggag ttcgacgtcg acgaggagcg cctcgccttc 2880
   atcgccgacc accgcgtgcg cggcatgacg gtgctgccgg ggacggcgta cctggacctc 2940
   gcgctctcgg cggcgcgggc cgccttcggc gacaagggct tccccctcag ccaggtctcg 3000
   ttccgggagg cgctgaccct gctgcccgag cagccgcggc gggtgcagct ctcgctcgtc 3060
   cgggaggcgg agggcgcgtt ctccgtctgc gtctccagcc gcgacgcggc gtccccggcc 3120
   accggggagt ggaccctcca cgcgacgatg cgactggacg tcgaggcccc ggccaccgga 3180
   ggcgctcgcg ccgagggaca ggtgacgccc cccgcgggcg ccgagctccg ggacgccagg 3240
   acgcactacc gcctcatgaa ggagggcggc ctcgcctatg gcccgcgctt ccaggggctg 3300
   gcggaggtcc actgcggcca gcacgaggcc ctggggcgcc tgcgcgcgct gccggagctc 3360
   gggctcgacg gcgcggacca cgtcttccac cccgccctct gggacgccgc gcaccaggcg 3420
   ctggcggccc tgctcggccg ccaggacggc cacgcctccg gcccgtccac ctggattccg 3480
   cgcggcatcg agcgggtctc ggtgtccggc accgtcccgg cccgagaggc gctgtgggtc 3540
   cgcgccagcg cgcgggtgga cgcggacggc gccacggcgc ggggggacct ccaggtcctg 3600
   gatgacgccg ggcgggtgct ggtcgagacg cgcgggctga cgctgctccg gcgcgacgcg 3660
   gacaacctcg acgacgtccg ccagtggttc ttccggccgg tgctccgcga ggccccgctc 3720
   cgggaggaag cccccgcgga cgggacgtgg ctcgtcctcg gcgcgccggg gcccctgcat 3780
   gaccgggtgg tggcggagct gcgcgggcgc ggcctcgaca ccgtgcaggc gttcccggcc 3840
   aattcctggg aggagcagga cgcggcgcgc ttccacctct cccccgcgga ccccgagcac 3900
   ctgcgccggc tgatggagcg ggtcagcgcc gaccgtccgc tgcgcggcct catcaacctc 3960
   cagccgtacc aggtggcgcg cggcgacgcg gcctcctcgg cgccggaggg cgtgctgtcc 4020
   gccctctaca tggaggtgct cgaccaggcc cgggcgctcc agggcgtgag ccggcgggag 4080
   cggccgaagc tggtgctcgt ctcgggcgtc tcccagggcg cggaggccga gcccgacctg 4140
   gtcgccgccc cgctccaggg cctgggccgc gtcatcgcgg ccgagctgcc ccggctccat 4200
   tgcgtccagg tccagctcgg aggccaggag cccgaggtgg aggcgcgaga cctcgtgcgc 4260
   gagctgtacg ccaacgacgg cgaggacgag gtcgtgctgc gcgggggcgc tcgccacgtg 4320
   cgccggctcg agcggctcgt ccccggcgcg gccccggcgg cgcccatcca cgcggacgga 4380
   acctacctgc tcacaggcgg cctgggcggc ctggggctgg ccttcgcgag ctggctcgcg 4440
   gaccgcggcg cgcggcacct ggccctggtc agccggcgcc cgccctcgga ggaggcccgc 4500
   gcgcggctcg acgcgctccg cgcccggggc gtcgaggtgc tcgtgcgcga ggtggacctg 4560
   tcccgggagg actccgtcgc cgccctcttc cgggagctgg acgagcgcat gccgcccctg 4620
   cggggcgtcc tccacctggc gggcgtcctg gacgacgcga cgctgttcca gcagaacgag 4680
   cagcgcctcg cggccgtgcg cggccccaag gtcaccggga cgcggctcct gcacgcgcac 4740
   acccagcacc ggaagctgga cttcttcgtc ctcttctcgg cgggcgccac cctcctcggc 4800
   tctccgggcc aggcgaacta cgccgcggtg aacgcgttca tggatgcgtt cgccccgcac 4860
   cgccagcggc tggggctgcc cgcgctctcc atcaactggg gcgtctggga cgaggtcggc 4920
   ctcgccgcgg cgagcgcgca gcgaggccag cgcctgcggg agcaggggct gttccccatc 4980
   ccccccgcgc agggcctcca ggccttcgag ctggcgctgt cgctcgcggg cgccaacgtc 5040
   gccatcctgc ccatccgcct cgcccgctgg ctgcgcgagc acccggacct ggcgggcgcg 5100
   tcgctgttca aggagctggt ccgcggactg gacgactccg aggcggaggc cgaggcgccc 5160
   atcctggagc agctgcgcgc cacggtggac ccgcatgagc agcgcgagtg cctgcgccgc 5220
   tgggtcgggc gccagctcgc ggcggtgctg cgaagggacc ccgcgtccat gcagcgcgag 5280
   cagaacttca gcgccatggg cgtcgactcg ctgatggcgc tcgagttccg caaccggctg 5340
   gagcgctcgc tccacgtccg gctgccgccg acgatggtct tcaaccatcc gagcatcgag 5400
   gcgctctgcg ggttcctcga gccgaagctg ggcatgggcg ccccctccgc tccggcgcag 5460
   ccggagcccg tgccggctcc cgccgcgccc gtgcgcgtcg acgagaagga cggcgccgcg 5520
   ctgctcgggg ccctggcccg gctgaagaag ctcacccagc aacaggcgta g 5571
<210> 25
   <211> 4686
   <212> DNA
   <213> Pyxidicoccus fallax
<400> 25
   atgttggatg agctcagggc agtgctggac agtctgtcgc ctgaacagcg ggcgacgctc 60
   gcggagctgc tgagcgcgga cgcgccccgg aaggccgacg agccgattgc catcgtcggc 120
   atggccggac gcttccccgg agcacgcgac ctggcggagt tctggagcgt gctcgccgag 180
   gggcgggacg ccgtctcgga gattccaccg gaccgctgga acgcggacgc cctctatgac 240
   ccggacaagg acgtcccggg caagctggcc acccgctggg gcggcttcgt gaaggacgtc 300
   gacctgttcg acgcggagtt cttcggcatc actccgcgcg aggccgcggc catggacccg 360
   cagcagcgcc tgttcctcga gacggcctgg gaggccctcg aggacgcggg gctgcccgcg 420
   gcctccctcg cccggacgca gacgggcgtc ttcgtcggag tctccaactc cgactacatg 480
   tggcaccagt tcccccggct cgacaaggtg gacgcgtacg cgagctccgg gtccgcgcac 540
   tgcatcatcg ccaaccggct gtcctacctg ctggacctca ccggccccag cgtggcggtg 600
   gacaccgcgt gctcctcgtc gctggtgtcg gtgcacctgg cctgccagag cctgcgcaac 660
   cgggagtgcg aggcggcgat tgccggcgga gtgaacgccg tcctgacgcc gcacctgggg 720
   gtgagcctct ccaagtgggg gatgatggcg tcggacggcc gctgcaagac gttcgacgcc 780
   cgcgcggacg gcttcgtccg gggcgagggc agcggcgtgc tcgtgctcaa gcggctggcg 840
   gacgccgaga aggccgggga ccgcgtcctg gccgtcatcc tcggcagcgc cgtcaaccag 900
   gacgggaaga cccacggcct gacggcgccg aacggcgccg cccagcaggc cgtcatccgg 960
   caggcgctgg cccgcgcggg gctgtctccc tcgcaggtgt ccttcctgga gacgcacggg 1020
   acggggacgt ccctgggtga ccccatcgag gtcgaggcca tcaacgaggt gttcggctcc 1080
   agggtcgagc gctgcgtgct gggcgcggtg aagaccaaca tcgggcacct ggagccggcg 1140
   gcgggtatcg ccggcctcat caaggtcgtg ctcgcgctgc gccacgggct catcccgcgc 1200
   aacctgcact accagtccat gaacccgcac ctccagctcg ctccgggcat cgagctggcg 1260
   aacgaggccc ggccctggaa gcgcgacgcc aggccccgct tcgccggggt gagcgccttc 1320
   agcttcggcg gcaccaacgc gcacatcgtc ctcggagacg cgcccgccta cgcgccgccg 1380
   gccgagtccg cgcggcccgc ccagcccacc ctcctccccc tctccgcgag ccaggagggg 1440
   gcgcggagcc agatggccgc gctccacgcc gagctgctcg ccaccgcggc ccccgagctc 1500
   acggtggaca ccgtggcggg actgcgcgcg ctgcaccggg accacctgga cctgcgcctg 1560
   gccgtcgtcg cctcggaccg cgcgacggcc gccgaggccc tgaagtgctt cgccgagggg 1620
   cggccccacg cgggactggt ccacgggcgc cgccagccga acctgcggcc cgtcttcatc 1680
   tacaccggcc agggcaacca gtggccgggc atgggccgcg agctgatgcg caccgagccg 1740
   gtgttcaggg ccgcggtgga ggaggcggac gccgcgttcc agcgcctcac ggggcggagc 1800
   cacgccgcca gcttccgcga ggacgcgtcc gacgcggcgt cgctggagcg caccgacgcc 1860
   gcccagccgg ccatcttcct cctgcagtac gcgctcacga agacgctgga gcactggggc 1920
   atccgtcccg gcgcggtcgc ggggcacagc gtcggtgagg tcgccgccgc ctgggccgcg 1980
   ggcatgctgg acctggacga cgcggtccgg gtggtccacc accgctcgcg gctcatgcag 2040
   cgcgccaccg gccagggggc gatggcctcc gtcgcgctgt cgccacggga ggcgcggccc 2100
   ctcctggagg ggcgcccctc gctggccatc gcggcgcaca acagccccgg ggaggtggtg 2160
   ctctcgggag accccgccga gctcgaggcc gtgctccgcg agctggaggg gcgtggcgtc 2220
   cgctgcagga agctccccgt caactacgcc ttccacggcc cgcagatgga cccgctccgc 2280
   gacgagctgc tgcgggagct gtccgtcctc cggccgaagg cgccgcgcct ggcgttctac 2340
   tcgacggtga cgggccagcg cgagcggcag gccgtggccg acgcgagcca ctgggtccgc 2400
   aacatgaggg accccgtcgc gttccgcgag gtcgtcgagc gcctcgcgac cgacagcccg 2460
   ggcttcttcc tcgagctcgg cccgcatccg gccctcgtgc cctcggtgat ggacacgctg 2520
   gcgcacctcc ggcttgaggc gcgcacggcg cactgcctgc ggcgcaaccg ccccgaccgg 2580
   cacgagctgc tgtccgccgt ggcatcgctc tacgtccacg gcatcgaccc ggactggtca 2640
   cgcctggtgc gcccccggga ccgccggtgg gcgctgcccg cctacccctg gcagcgccag 2700
   cgcttctggc tggaagcggc ggcctccccg gacgccacct ggagcgctcc gagcgcgcgg 2760
   cccgccggcc acctcgtcta ccggaccgag tggctggccg acgcggcctc cacctccggg 2820
   gacaccgcgc ggccgaaggg ccacctcgtg ttctccgacg gcagcgccct ggccgaggcc 2880
   ctccgcgccg agctcgaggc ggccggctcg cgattcgggt gggtgagccg cggccaggac 2940
   ttcgccgcgg acggcacgcg ctgcaccgtg gacccggaga accccgagca cctcgagcgg 3000
   ctcttcggcc acctcgcggc cgagtccttc gacttcgacc acgtcctgct gctgttcacc 3060
   cgggtggagg acgaggcgcg gccggacggc gcggcgccgt ggttcgacac ggccgtgcgc 3120
   cccgtgcacg tcctgcgctg cctgacccag gccctgctca cgaccgagcg ccggccgagg 3180
   atggcggtca tcacccgggc ggcgctgagc gcgggcggcg gcaccgaggc ggtgccccag 3240
   cacacggcgc tctggggctt cggccgcgcg ctggcgctgg aggagccggg actctccctg 3300
   gccctgctgg accttccggc ggtgcccgac ggcctcgtcg aggcccggca cgtcctggcg 3360
   cgcctcgcca gcccgacgcc cgagcgtcag tcggcatggc gggacgggcg gtggcgcgtc 3420
   ccccgcctca tgccccacga agccgggacg ggcacgtccg cgccgcccgt catccagggc 3480
   gagggcacct acctcatcac cgggggcctc ggctcgctgg ggctcgccac cgcccggcgg 3540
   ctcgtccagc gcggcgcgag gagcgtggcg ctcgtctcgc gccgcccgcc caccgccgcg 3600
   cagcaggccg agctcgcgcg cctggaggag gcgggtgccc gcgtggtggc ggtgcagggc 3660
   gacctggcgg acctggcgga ggccacgcgc gtcttcgaga cgctcgcggc gtcggcgcct 3720
   ccggtccggg gcgtgattca cgcggccggc gtgctggagg acggcgtgct cgggagcgcg 3780
   acgaaggcga gcttcgaccg cgtgctgcgg cccaaggtcg ccgggagctg gaacctccac 3840
   ctgctcacgc aggccctccc gctggacttc ttcgtgctgt tctcctccgc caccgcgacg 3900
   ctgggcgccg cgggccagac gaactacgcc gccgccaacg ccttcctcca gggcctcgcg 3960
   gagcaccgcc gcgcccgcgg ccagcacgcc ctctgcctgc actggggcgc ctggcgcgag 4020
   agcaccatgg cctccggcgc cgtggtgacg aagctgaagg cgctgggctt cgacaccatg 4080
   gcgcccgagg acgcgctgga cgcgctggag cgcgtgctcc tcgagggggc gacggaggcg 4140
   accatcatcg ccgccgactg ggaggtcttc gcccggcggc tcccggacga gggcctgggc 4200
   cggttcctcg agcccgtgcg gcgcgcgagc ggggccccgc cggccgtgaa gcccggggtg 4260
   cccgaggccg accccatcgt ctcacgcctc gccggcgcgc cggcgagcca gcgccagaag 4320
   ctgctctacg gccacgtccg cgcggtggtg ctgtccacca tggggatgga cgcccgcacc 4380
   gaggtccgcc cggaccaggg gctcatcgag ctgggactgg actcgctgct cgcggtgaac 4440
   atcgccaacc ggctctcgcg cgacctggcc cgccagctgc ccaagacgct catctacgac 4500
   cacccgacgc tgcacgccct gacggaccac ctgctgggcg tgctcttccc cgagcccgcc 4560
   caggccagcg tccggcccgc cgcctccggc gacgcgccct cctacgagga catcgccaac 4620
   gcgagcgcgg acaccatcca gagcctcctc gacgccgagc tcgcgagcct cctctcctcc 4680
   cgctga 4686
<210> 26
   <211> 3315
   <212> DNA
   <213> Pyxidicoccus fallax
<400> 26
   gtgcgccgga cgcggtcccc gcgggggtcc gcgcccgcgc gcccggagcg gctccttcgc 60
   ccagaaagca cttccgccgt ggatacgaac gccctccttg cgcagaacaa gaaagccctc 120
   ctggccatca tggagctcaa ggcccagctc gcgcgggccg agtcgcggca gcgcgcgccc 180
   atcgccatcg tcggcatggc ctgccgcttc ccgggtggct gcgccacgcc cgagcagttc 240
   tgggagctgc tgcgcgaggg ccgtgacgcg gtcaccgagg cgttcccctc gcggtggccg 300
   gagtcgctct acgacgccaa ccccgaggcg cccggcagga tcatggcgcg gggcctgggc 360
   caggtggagg gcgtggccga cttcgagccg gagttcttcc acgtctcgcc gcgcgaggcc 420
   gcgagcatgg acccgcagca ccggctgctg ctcacggtcg cctgggaggc atgcgaggac 480
   gccgggctca cccgggagct gctgcacggc agccgcaccg gcatcttcgt cggagcgggc 540
   aacaacgact acggcgactt cgagctgcgc aaccccgacc tgggcgcggt cagcgcctac 600
   gcgggcacgg gcaacgccac ctcgctggtc gcggggcggc tctccttctt cctcgggacg 660
   cagggccccg cgctcgtggt cgacacggcg tgctcgtcct ccctcgtcgc catccaccag 720
   gcgtgcacca gcctgcgcct gggtgagtcg gagctcgcgc tcgctggcgg cgtcaacctg 780
   gtcctccgcc ccaacggcag cgtgttcctc tcgcgcttcg gcgcgctctc gcccaccggc 840
   cggtgccggg ccttcgccga ggcggccgac gggtacgtgc gcgcggacgg ctgcggcgtc 900
   atcgtgctca agcgcctgga ggacgccctg cgggacgggg accgggtgct ggcggtcatc 960
   cggggctcgg ccatcaacca cgacggccgc agcggcgggc tcaccgtccc caacggggcg 1020
   gcgcagcggg agctgctcct ggccgccctg aagaacgccg gggtggagcc gcggagcgtc 1080
   ggctacctgg aggcccacgg cacgggcacg ccgctcggcg acccgataga gatgggcgcc 1140
   atcgccgacg tcttcggcgg ggacgcgcgg cgcgaggccc ttcgggtggg ctcggtgaag 1200
   agcaacatcg ggcacaccga ggcggcggcg ggcatcgccg gcatcatcaa ggccgtcctc 1260
   agcctccagc accgggaaat ccccgcccac ctccacttcg acaagcccag ctcgcgcatc 1320
   gactggagca tccccgtcga ggtccccacg agcacgcggc cctggagcgg ggacggcccc 1380
   cggctggcgg gcgtgagctc gttcggcttc agcggcacga acgcacacgt catcctggag 1440
   gaggcgccgc gccaggaggc ccggcactcg ccccggctcg ggacgcaggt gctctgcctg 1500
   tcggcccgca ccgagggctc cctcacgcgg ctggccgcgg cctggaggga gcagctccgg 1560
   cggagggatt cggcgcccct cgcggacctg tgccacgccg ccgcgcggcg ccgctcccac 1620
   caccgcgccc ggctggccgt cctcggcgac agcgccgacg ccatcgccca ggcgctggag 1680
   tccttcgccg ccggggcgcc ccaccccgcc gtggtgaggg gcgacgcggt gctcgacggg 1740
   atgtcccgca cggccttcgt cttctccggg cagggcagcc agtggctcgg catgggccag 1800
   gagctgctcg cgagcgagcc cctcttccag cagagcctgc gcgaaatgga cgggccgttc 1860
   cgcgacgagt tcggctggtc cctcgaagag gcgctgcggg ccggggaggc cgagtcccag 1920
   ctcgagcgga cgcgcgtcgc ccagcccgtc atcgtggccg tccagctggc gctgagcgac 1980
   ctctggcggg cgtggggcgt gcaccccgac gtgacgctgg ggcacagcct cggggagatc 2040
   tccgcggccc acgtcgccgg cgtcctcggg cggcacgagg ccctgcgcct ggcctgcatc 2100
   cgcggccggt tcatggatgg ggccaccggg cgcgggaaga tggccgagct cgcctgctcc 2160
   gaggagcagg cgcggcgcta cctccagggc ctcgaggccg aggtcgccat cgccgccctc 2220
   aatggcgcca tgtcgacggt gctcgcgggc gcgccgggcg ccctggagtc cgtcctccag 2280
   aaggtcgcca gcgacggcat ccagcaccgc ctgctgcgcg tggactacgc cttccactcg 2340
   ccgcagatgg ccgagttcgc ggcgcccttc cgcggcgcgc tgggcgacat gaagccccgc 2400
   gccgccgagc gggacctcta cagcagcgtg ctcggccgcg cggtgcgcgg cgaggcgctg 2460
   acggccgact actgggtgcg gaacatgacg gacaccgtgc gcttccacga ggcgctcgtg 2520
   gcgctcctgc gtgacggctg ccgcgccgtg gtggaggtct ctccccaccc cgtcctgctc 2580
   tccgccatcc aggacaccct gagccgcgag ggcgtcgacg gcgtcgccgc gtactcgctc 2640
   cggcgcggga acccggagcg cgccaccctg ctgtcgaacc tcggcgccct gtacgccgcc 2700
   ggccaccccg tggactggtc cgcgctctac cccgggctgg tggaccacgt ggccctgcct 2760
   gcctacccct ggcacgcgcg gcgcttctgg acgaccgtcc agggcccgga ggccaccacc 2820
   ggcgcggccc ccgccgtcac cgccccggcc ccggacgagg ccgcccccgc cgccgtcacc 2880
   tcgaaggtgt gggacgagct cgtcaagctg gcggccgtcg cgcgcaggcc gcgcctggag 2940
   cgccatgtcc gcgagcagct cacgggcatc ctcaagctgg actccgggga catcccctcg 3000
   aagcgcacgc cgctgagcga gctgggcatg gactccgtcg gcgccgtcgc gctgtccaag 3060
   gcgctggccc tcacccacga gcaccggttc cctcccaccg tgctcttcga gcaccccacg 3120
   attgaagcgc tgaccgggtt cctcgccgag gagttcgagc ggctccacgc ggcccgaggc 3180
   ggcgcggcgg cccctggcga cagcgcgcca ccgcgccagg cgcccgccgc tccggaggcc 3240
   gccgcgctcc cggtgcccga ctcggagctc gccatcctgg agaagctcgc cgccctcgag 3300
   aaggagtttg cctga 3315
<210> 27
   <211> 7251
   <212> DNA
   <213> Pyxidicoccus fallax
<400> 27
   atgtccacac cggagaatcc cacgggcgac cgcgcactcc tccaccgggc gctggccgcc 60
   atggagaaga tgaagggccg catccagcag ctcgagggcg tgacgaccga gcccatcgcc 120
   atcatcggca tgggctgccg cttccccggc ggcgtccacg gccccgacgc cttctggcgg 180
   ttcctcgcgg acggcgtcaa cggcatccgt gacacgccga aggaccgctg ggacaccgag 240
   cgctacctcg acgccgagca gggcacgccg gggaagatgt acacgggcca cggcggcttc 300
   ctggaccgcg tggacgggtt cgacgccgcg ttcttcggca tctcgccccg cgaggcgctg 360
   agcatggacc cgcagcagcg cctcttcctg gagacgtgct gggaggccat cgagaacgcg 420
   ggcctgtccg cggacgtgct cgagggcagc cagacgggcg tctacgcggg catcgtcaac 480
   caggactacg ccatgatgca aatgaaggac accgcgcatg gcgagatgga cacctacttc 540
   gccaccggcc acccgcactg cgtggtgagc ggccgcgtgt cctaccagtt cggcctccgc 600
   ggccccacgc tgtccgtgga caccgcgtgc tcgtcgtcgc tggtggccct gcacctggcc 660
   tgccagagcc tgcgcctggg gagctgcgac gtggccctgg ccggcggcat caacctgctg 720
   ctgatgccgg agatctacat gatccgctgc caggcgcgca tgctgtcgcc cagcgggcgg 780
   tgcgccacgt tcgacgcgtc ggcggacgga tacatccctg gcgagggcgt gggcgtcgtc 840
   gtgctgaagc ggctgcgaga cgcggtccgg gatggggacc gcgtcgacgc cgtcatccgc 900
   ggcacgcaga tcaaccacga cggccgcagc agcgggctga ccgtgccgag cggggccgcg 960
   cagcgggagc tgctgcgcgc cacgctgcgc gacgcccgcc tggagcccca gcgcgtcggc 1020
   tacctcgagg cgcacggcac cggcacgaag ctgggggacc ccatcgagat ggccgccatc 1080
   caggacgtct actgccagga ccgcgcgccc ggaaacccgc tggtcatcgg ctcggtgaag 1140
   accaacatcg gccacctgga gtccgccgcc ggcatcagcg gcctcatcaa ggccgtcctc 1200
   accctcaagc acggcgccat cccgcgcaac ctccacttct ccgagctcaa cccgcacatc 1260
   acgctgcacc cggcgacccg gattcccacc gagctcaccc cgtggccggc gggcagcggc 1320
   ccgagggtcg ccgccgtcag ctcgttcggc atgagcggca ccaacgccca cgccatcctg 1380
   gaggaggccc ccgcgcgcga ggccgccagc acggaggcgc ccctccgcgt cacctcccgg 1440
   ccggcgacgc cgcggctcgt cccgctcagc gcccaccacc ccgaggtcct ccaggcccac 1500
   gccgccgggc tgaaggaaca cctggacgcc cacccggacg tcaccttcga cgacctggcg 1560
   cacacgctgc gccggcgcac cctccagcgc caccggctcg ccctgcccgc cagctcgctg 1620
   gccgaggtga aggagcatct ggcggcgttc gcggccggcg ggcaggaccc ccgcgtcctc 1680
   cagacggagg acctcttctt caccgacccg aaggtcgtct tcgtcttccc gggccagggc 1740
   tcgcagtggg tgcggatggg gctccagctc atggagcagg agccggagtt cgccgcggag 1800
   ctgcgcgagg tggatgcgtg gatccagaag tacgctggct ggtccctgct ggacgagctc 1860
   cgcgccgacg cgagcgcgtc ccggctgagc caggtcgacg tcgtgcagcc ctgcatcttc 1920
   gccgtgcagg tcgccctcgc ccggctgtgg cgctcgtggg gtgtcgtccc caacgccgtc 1980
   atcgggcaca gcatgggtga ggtggcggcg gcctgcgtcg ccggcatcct gtcgctcgag 2040
   gacgcggtgt gcgtcatcac ccgccgcagt cagatcgcga agcgcaccag cggcaacggc 2100
   gccatggcgc tggtggagct gacgcgcgag caggccgagc gggccatcgc cgcgcaccag 2160
   gacgtggtcg cggtcgcggc ccagaacggg ccccacaccg tcctcctctc cggcacgccg 2220
   ggccccatcg agcagatcct ccgcgagctc cagcagcagg acgtcttctg ccgcgccatc 2280
   aaggtcgact acgcctccca ctccccgcag atggatccgc tccggcccga gctgcagcgg 2340
   gcgctctccg gcgtgaagcc ccgggcggcc accgtcgcct tctactcgac ggtgacgggc 2400
   caggtggagg acggcacggc gcacgacgcg aactactggg tgaacaacct gcgcgacccc 2460
   gtcctcttct ggccgggcat gcagaagctc gtcgaggacg gacacaacat cttcctggag 2520
   ctcagccccc accccgtcct catccccgcc atctcccagg gcctgcagca cctgggcgcc 2580
   agggcgctga gcctcccgtc gctccaccgc gagaagccgg aggccgagtg cctgttcgag 2640
   tcgcgcgccc ggctcttctg cgccggcggc aggctggacc tcgcgaagga ggacgtcccg 2700
   ggcgcccggg tcctcgccct gcccggccac ccctggcgtg agcagcgctt ctggtacacg 2760
   cccagcgccc cctcggcgca cccgcaggcc cgcggcggca gccggctgct gccccgcatg 2820
   tggacggcgg gctccgggga gcggcgcgtc ttcgagggcg acgtcggccc cggcgtcatg 2880
   ccctcgctgg cggaccaccg cgtcaatggg atgcagctct tccccggcgc cggctatgtc 2940
   gagctggtgc tcgaggccgc cgaggcgctc gggctcgcgg gccggacgca catcgactcg 3000
   ctcgacatcc gcgaggggct ggtgctccac gagggcgcgg cgcgcgtcgt ccaggtggag 3060
   ctgacgcccg tggccgcgcg ctccttcggg ttcgtcatca gcagccgcga ggccgacggc 3120
   gccgcctgga cgcgccatgc cgacggcgtg gtgacgcgcg aggaggccgg ggcggcgccg 3180
   ggcgcggagc ccggcgcgct ggaggcgctg cgcgcgcgtc tcgcctcgcc cctgtcggcg 3240
   gaggaccact acctccgcac ccgggagcgg gggctggagt acggccccgc gtaccaggcc 3300
   atcaagcaca tcggccgcgg gcgcggcgag gccctggcgg agctgcacgt gccccaggcc 3360
   gtcgcggagg agctgagcgg gtgccagctc caccccagcc tcctggacgc cgggttccag 3420
   ctcatcgaca cggcgctggc cgggtcgggg ctcgtggagg agggagacgt ctacctgccc 3480
   gccgccctcc gcggggtgcg cttccaccgc aagccggccc ctggacagac gctccatgcg 3540
   cacctggggt tgagcccgga gaagccgcgc gaggcgtcgg tgctggaggc gcggctggcg 3600
   ctcctggatg accagggcgc tcccgtcctc acggccgagc ggttcatcat ccgccgcgtc 3660
   gagacccacg ccgccgcctg ggaggggctg ccgggcgagt ggttctaccg cctgcgctgg 3720
   cagccgctgg agggctggaa gccggaggcc gccggggcga agcgcgacga ggcctggctc 3780
   ctgttcgcgg acgaccaggg cgtggccgcg gcggtggccg accggctcgc gccctcgggc 3840
   gtgcggacca cgctggtgcg caaggcgccc ggcgccgtca cgcgggcggg gagcggctgg 3900
   acgctcgacc cgggcacgcc ggagtccttc cgcgagctgc tcgccgcggt gtcgccgggc 3960
   tccttcacgc gcgtgctcta cttctgggga ctggacgcgc ccgcggtgga ccccgagccg 4020
   gacgccctgg acgcgcggct gcccgagacg gaggccctca tctacctggt ccaggcgctc 4080
   gccgcccggg aggcgcagcc ggggccgcgc ctgtggctcg tcaccgcggg cgccctcccc 4140
   ggcgccgacg gcgaggccgt gtcgcccatg cagaccccgc tctgggccct gggccgcgtc 4200
   ctggactacg agcacccgga gctgaagacc gtctcctgcg agctggagcg tcacgtcacg 4260
   gagaagcacg tcgccgtgct gacctcgctg ctcctcgcgc ccgaggtgcc cgaccaggtg 4320
   cgcgtgcgcg ccgacggccc ggccgcggcg cggctggccc gctactcgcc cccggccccc 4380
   cgggacacgc cgcaccgcgt gtcgccgcac gaggccgtcg tggtggtgtc ggacaacggc 4440
   cccgcgcccg cggagctgtc cgtcgccagg cggcgggccc ccgcggccca cgaggtcgag 4500
   gtcctggtca cccacgcgca cctcgcctgg gcgcccgcgt ccccggacgc gggcggcctc 4560
   gtgccgggag ggtacttcgc gggccgcgtc ctccggctcg gaggcgaggt ccgcgggctc 4620
   tccgtgggcc aggacatcgt ggccccggcg ggcggcgagc tccgctcgat tcgcacggtg 4680
   gacgccggtg gcgtgcgccc gctgccggag caggccgacc ccgcgcgggc gctctgcgcg 4740
   ctggggaccc gggccctggc gcggcacctc ctgcgccagc tcgcccccgt cgagcgaggc 4800
   cagacgctgc tgctgctcgg tggcgacctc cccctcctcc agtccctcgc gcgggtggcc 4860
   acgggccacg gcgcgcgcgt gctcgccctg gtccgggacg gcgcgcacgc gcccgcgctc 4920
   caggaggcgg gcgccgaggt ggtgggacgg ctggacgagc ccggcgcgtt cgagcggctg 4980
   cgcgagcgcg ccggtgcctc gggcgtcgac gtcctcatca acctcgcgcc cggcgccgcg 5040
   gtgccgcggg cgctcgacgt cttccgggcg ggcggcgtct tcctggacct gacgcgcgcg 5100
   tccccggtcg gcccgaagga gctgcccgcc tcggcgacgt tcctctccct ggggctcgca 5160
   ccgctctacg cgcgcctggc gccgcccacg ccggaagcca tcgccgaggc cagcgcgccc 5220
   gcggaggccc ctgcgcgcgt gtggagcctc caggagctga cgcagccgcg ggcggaggcg 5280
   ccgctcgacg gccaggccca cgtcctcggc gtgtcgtccg agcccgtggc ggtgggggcc 5340
   gggagcgccc cgctcgcgct ctccgacacc gcgacgtacc tcatcaccgg cggcctcggc 5400
   ggcctcggcc tccgggtggc gagcctgctg gtggaccacg gcgtgcgcca cctggcgctc 5460
   gtcggccgct cgccgccgag cgcgcaggcc cggagcaccc tccgtgagct ggagcagcgc 5520
   ggcgtccggg tccaggtgtt cgccgccgac gtcgcccggc gcgaggacct gctcggggtg 5580
   gtgaaggccc tccaggccgg cgacgccccg ctccgggggg tcatccactg cgccggcgtc 5640
   ctccaggacg gcgcgctcct cggcatggac cgcgagcggt tccgctcggt cctcggcagc 5700
   aagctgagcg gcgcgtggaa cctgcacgcc ctcacccggg acctgccgct cgaccacttc 5760
   gtgctcttct cctcggtgtc ctcgatgctg ggctcccccg gccagggcaa ctacgccgcg 5820
   gcgaacgcgt tcatggacgg cctcgccgcc tggcggcgcg cccgggggct gcccgcgctc 5880
   agcgtcaact ggggcgcctt cagcgacatc ggcctggcgg ccgtcggcga gcggcgcgga 5940
   gaccgcctgg ccgacaacgg catccccggc atccctcccg agaagggcat gaaggcgctc 6000
   gagctggcgc tgcgctgggg cgagacgtcc ctgggcttct tccccttccc cttcctcatc 6060
   tggcggaagc tcaacccgca gattgcccgc aacacgctct tcaccgacat ccgcgcggag 6120
   agccgctacc gcgaggaggc cagcggcgag cagcccatcg ccgaggcgct cgccctcgcg 6180
   gacccggccg cccgccggag ccacctcgac ggcttcctga agcagcagct ctcgcgcgta 6240
   ctcaaggtgc cggcggagca ggtccgcgac ggcctcacct tcaaggagat gggcttcgac 6300
   tcgctgatgg ccatcgagct gcgcaaccgg ctggaggccg gcctcacggt gtccctgtcg 6360
   tcgacgctcg tctggcgcta cccgacgctc gaggagatgt cgcggttcct gctccagctc 6420
   gtcggcggcg ggagcgagac gccctcggac gcacggccgg cgtccgccgc cccggcgcgc 6480
   cggggccgct ggctggtgcg tgagaagccg agtccctccg cgaagctccg gctgctctgc 6540
   ttcccgcacg gcggcgccgg cgcctcggcc ttccagggct ggtggcagca gttcccggac 6600
   acggtggagg tcctcacctt ccagccgccg gggcgcgagg agcggctcca ggaggagccc 6660
   gagcgcgaca tgggggcgta tgcctctgcc atcgtcgagg agctccagcc gctgctcgac 6720
   ctgcccttcg ccgtgttcgg ctacagcctc ggcggcctca ccgggttcgc caccgtggcc 6780
   gagctgcggc ggcgcaaccg cgtcaccccc cggcacctgt tcgtgtcgtc cgccttcgcc 6840
   ccgcacatcc ccttctccga gacgacgctg gcccgggtct ccacccagac ctcggcgctg 6900
   aaggccatgg ccttctacca gtccacgccg gactcggtgt tcaaggaccc ggagatgcag 6960
   gcgctgctgg cgcgctcgat ggacgcggac aactccgtgg tcgagtccta ccgcttcggc 7020
   gcggagaagc cgctggactg ccccatcacc gccttcgcgg gccggcagga cgacctcgct 7080
   ccgctccacg agcagctgcg ctggaaggag ctcaccaccc aggccttcga gctgcggccg 7140
   ttcgagggcg accacttctt cttcctgaga gacaagcgcc cggtgatgga cgagctgcgg 7200
   cgccacctgc tctccctcat cgcggactcc cgggacggct cgtcccgctg a 7251
<210> 28
   <211> 1221
   <212> DNA
   <213> Pyxidicoccus fallax
<400> 28
   atgacgtaca cacagctcct gcatcgccgg gttggaggcc agctccgccg cttcctgctg     60
   ctgcggccct tcggccccgg cgtcgaggtc gacgaccccc tgcatccgcc cgagctcccg    120
   ccgtacgacg cggagcggga cctcgcgccc cggacacccc agttcctcca gcacgccctt    180
   cagcccgagg agcgggggcg tcccggggcg ccggggctgc ccgtcaccac cagcgagctg    240
   tcggtcggct gcaacctctc cggggcgtgc tgctcgcagt atcccgtcat ccccacgacg    300
   gtggaggaga gccggcgcgc cgtggagaca ctgcgccggc tggcgcccgg ggcggccccc    360
   gggcgggccg aggacgcctt cgcccccgtg aatcccggcc ggcgcgacgt cctgtcgccc    420
   atcctccagg gcgatggctg tcccttcctc gcccaggggg cctgccaggt ccacaaggcc    480
   gggggcgcgg aggcgaagcc gtggacctgc cgccagttcc cgctgcaggt catccactgt    540
   ggagaccact tcgaggtcag catcctcccg cagtgcgcgt gcgcggcccg gacggtgacc    600
   cgcggctccg gcggggactg gccagaggac ccgctgcggg ggctcacgtc cgtcccgcgc    660
   gtcccggact cggtggtggt ggaggggcgg cggaccctcg cgcgcggcat gtatctccag    720
   tgggtgcggg ccctggcgga ccggctgtcc gcgccagggc tgggggagcc cgccctcgtc    780
   ctggacagcg cctcccagag cctgcgcgtg gctcccgggg ccctcacccc cgggtggctg    840
   ggcgagctgc gcgcccgcat gctgtccgag gccgagcggc tggagggcta tctcgacccg    900
   gccgggccgc agccgaaggg gacgcgctgg gtcgcgtccc tcgcggaggc cctgctggcg    960
   gacgcggacg cggagccgga gcccgcgccg agtgacgggc tggtgctcgc gctggcgctg   1020
   cgcgcccacc tcctgctgga gcttcccgcg ctgggggccg cgctggccga cctcacccgc   1080
   ctcgcccggc tggccagcct ctcgcggacc gtcgcgcccg cggaggccgc cgacgcccgc   1140
   ttcgagccgc tgacgctgct gctgtacctg tgggcgacga cgcggtggcc ggtctcctcg   1200
   gagggcgtga cgcggccctg a                                             1221
<210> 29
   <211> 1197
   <212> DNA
   <213> Pyxidicoccus fallax
<400> 29
   atgacaggaa cgtcaggcca gggggcgggg ggcgggcgca tcgacacgcc tccggagggg     60
   gcgctgcgcg cggtgcggtc actggtggag ctccaggaga cggagcaggc ggcgcagctc    120
   tacgaggagc tgggagacgc gcagcgcgag cgcctgcgcc gggaggcggc ccggcgtccg    180
   gcccgggagc gccggggcct ggtggaggtg ctgcggcggg cgcgcgactt cctgggcgcc    240
   gcccgcctga tggagggcga cggcgaggac gccaccgtgg cggacctcta cttccagagc    300
   gggcagtacc tggaggcggc ggaggcgtac ctgcgcgccg gcgaggcgga gcgcgccgcc    360
   gcggccttcg agcgggccgg agcgctggag cgcgcgctgg acgtgtaccg gcgcctgggc    420
   gcccgcgagg cgatggcgca gtgcctggtc cgcctgggcc gccccttcga ggcggcggac    480
   ctctaccgcg agctgggcca ggcgcacgcc gaggtagagg cgctgggcgg ggtgaagccg 540
   gaggactcgc ggtacgtgga ggcggtgctg cgcgtctgca cgctgctgga cgtggagggc 600
   ttcacccacc gggcgctcgc ggtgctggcg gacgcggtga gcagctccga ggaggcgcgg 660
   gcggacgagg cgctggtgaa ggagaaggcg cggctgctgc ggcgcatggg catggaggcg 720
   gaggccgagg ccgtgctgct gcggcgcgcg acggccgcgg cgacgcccgc cgccaacggg 780
   taccgcttcc tcaaggccat ccccatcttc ggcgagctgt ccctggagga catgaaggac 840
   ctctaccggc tcgcgcggca ggtcgtcatc gcgccgggca cggtgctgct ggagaagggc 900
   gcgcggggca cgggcctctt cgtcctgctg gacggcaccg tggaggtctt cagcggcccc 960
   gagcaggacg cgcgtcacct caacacgctg gggcccggcg cgtacctcgg ggaaatctcc 1020
   ctggtgcagg acgcccccgt ctccgcgcac gtccgcgcgc gcacctccgt gcgcgcgctg 1080
   cgcatcagcc gcgccgactt cgagcactac ctcgacaccc acgaggccgc cgcgctgcgc 1140
   atctaccggc tcttcacgca gaacctcgcc gcgcgcgtgc gggccctgag cacctga 1197
<210> 30
   <211> 633
   <212> DNA
   <213> Pyxidicoccus fallax
<400> 30
   atggccagcg cacgccgccc ggagaaggac ctccaggcac tcgccaggaa gtcgaagaag 60
   ctctcatggt tgttgcgcca tggcgcccgg gagatgggcc tcgccatgga ctccgcgggc 120
   ttcgcccaca tcggcgaggt gctgcggatg acgggcctgt ctcgcgacga gctcgatgag 180
   gtggtcgcgg agaacaacaa gtcgcgctac gaggtgcggg gcaagcaggt gcgcgccgtg 240
   caggggcact ccctggacgg cactcccgtg acgctcgacg ggctggagcg cagctgggag 300
   gaggtgctcg cggacgcgcc cctctaccac ggcacgtccg tggtcgccgc gcgtgccatc 360
   ctctccgggg aaggcatcca ctccgcggcg cgcacccatg tccacctggc cgccgcggtg 420
   gatgcgaagg tgggcaagcg cgccggggtg gaggtgctgc tggtcatctc cgccgcgcgc 480
   ctccgggcct cggggctgcg cgtcttccgc gcacccaacg gggtgttgct cgcgcgcgcc 540
   atccccgccg cctccatcgt ggacgtgctc gcgtgcaacg gcccgggaaa cgccgcgctc 600
   gcggagctgc agcggttttt ccgggaagcg tag 633
<210> 31
   <211> 855
   <212> DNA
   <213> Pyxidicoccus fallax
<400> 31
   atggagacgg atggacttcg cgccctggtg gcgttcgcgg aggctggctg caacctgacg 60
   ctcgcggggc gggccatcgg gctgtcccag cccgcggtgc atgcgcggct gcaaggggtg 120
   gcgcgggcgc tggagacctc cctctacgag cggcggggcc ggcgcctcca gctcaccccg 180
   gagggagcgc gcgtgctggc ctgggcgagg gacgtgctcg agcgggagcg cgccctgcgc 240
   gtggagctgc gacagggaca cgcggaggag cgggtggtgc tcgcctgcgg cgagggggcc 300
   ctggtgcacg tggtggccga gcgcgtcgcg ccgctccttc gcgagcggac cggcgtgctc 360
   tccttcctgg tgctggatgg ccccgcggcg gtcgccgccg tggagcgcgg ctcggcacac 420
   ctggcggtgg tggccgggcc cgcatccagc cccccggggc tgcgctcaca gccgctcgtc 480
   accgcctccc tgctggccgt ggctccccgg cagcacccgc tggcacgggc gggacgggag 540
   gtggaggtgg gggcgctgct cgagcaccgg ctcctggtgc ctccgctcgg ccgcgccctg 600
   cgcgccaccc tggaggacgc cgccgcggcc catggacgga cgctcgaggt cgccgcggag 660
   gtgacgggct gggaggcggt ctgccggctc gcggcgttgg gggtgggcgt gggcgtcatc 720
   aacgacgtgg tggccacgcc gggcctcgcg cgcctctccg tgcgcggcct tcccccggtg 780
   acctaccggc tgctgatgcg ccggggacgc gggacgcggc tcgcggagga ggtcgtgcgg 840
   gcgctgctcg gctga 855
<210> 32
   <211> 420
   <212> DNA
   <213> Pyxidicoccus fallax
<400> 32
   atggcacgtc cccctcgcaa aggtcccccg gagccgaagc ccgcgggcac gccgcccggg 60
   aagtcctcct cgggtggatt caacagtccc ttcgccgcgc tcgcgggcct gcgggagtcg 120
   ctcccgggca ggacgccgca gctccgcgcg gaggacttcc ccgccgagcc gcctccggac 180
   ggccccgagc gggcagtggt gcggctgacg cggcgcgagc gtgacggcgc cgaggtgacg 240
   cgggtggagg agctggggct ggctcccgac gcgctcacgg cctggagtca ggcgctgcaa 300
   cgggggctcg cctgccgtgt cacggtggaa ggggaggccc tggtgctgga gggggaccag 360
   cgccgaagcg tgccggacct cctcctgcgg cggggcgtgc ggcgcgtcac ccaggggtga 420
<210> 33
   <211> 714
   <212> DNA
   <213> Pyxidicoccus fallax
<400> 33
   atggcgcgtg cacgcagcgg agcgaaggcc tcggccaccc caccggaacc cgacgcccgg 60
   gcgcggctga ttgcggctgg cctgcgggtg ctggccgagc gcggctacga cgcgacgacg 120
   gtgaaggagg tggcgcgcga ggccggggtg aaccaggggc tggtgcacta ctacttcggc 180
   agcaaggacg cgctgctgct ggcggtggcc caggaggcgc ggcagcagta catggccgag 240
   ctgcggaagc tgcgcgagga gacgccgcgg gagcggctcg ccgccgcgag cttcgcgtgg 300
   ggggagcagc tgctgaggga gacgccggag cagttccgcg tgcgctacga gctgttcgcc 360
   atcgggctgc acaaccagga gctgaagccg gcggtggcgg agtcgctgcg ctgcgtggac 420
   gaggaggtgg cgctgacggt ggcgcgcatc cggcacggag acgacgcgcc gccggagccg 480
   ctggaccagc actatgccgc catcatgaag gcgtgcatcg acgggctctc gctgcaccac 540
   ctgctggatg actcgttcga ccccgcgccc gtgtaccagc tgctgagccg catcgtcctc 600
   gcgagcctgg agcccccgga ggggaagaag cccccggggc gcaaggggaa ggcgcaggcc 660
   cggcggggga gggtgccggt gccgcgcggg gggaggggtg cgcgtcggcg gtga 714
<210> 34
   <211> 394
   <212> PRT
   <213> Pyxidicoccus fallax
<400> 34
   Met Arg Thr Phe Pro Ala Gly Pro Glu Leu Leu Asn Pro Tyr Pro Phe
   1 5 10 15
   Phe Ala Glu Met Arg Arg His His Pro Val Ala Phe Asp Asp Gly Ala
   20 25 30
   Asn Leu Trp Ser Ile Tyr Arg His Ala Asp Val Thr Ala Ile Leu Ser
   35 40 45
   Asp His Val Asn Phe Pro Thr Ala Thr Ile Asp Asp Gly Gly Ala Pro
   50 55 60
   Pro Ala Glu Trp Arg Arg Leu Phe Ser Leu Asn Ala Phe Asp Pro Pro
   65 70 75 80
   Arg His Thr Gln Leu Arg Asn Leu Ala Met Arg Ala Phe Thr Ala Ser
   85 90 95
   Ala Val Ala Arg Met Gln Asp Arg Ile Thr Ala Ile Val Asn Gln Leu
   100 105 110
   Ile Asp Ala Val Val Gly Thr Gly Arg Thr Glu Leu Val Ala Asp Ile
   115 120 125
   Ala Gly Pro Leu Pro Thr Met Val Ile Ala Glu Met Leu Gly Val Pro
   130 135 140
   Ala Ser Asp Gln Glu Ser Phe Arg Arg Trp Ser Asp Glu Val Gly Val
   145 150 155 160
   Arg Ala Asn Ala Leu Val Leu Asp Pro Glu Asn Gly Val Arg Leu Leu
   165 170 175
   Ser Glu Ala Leu Thr Pro Met Glu Asp Tyr Val Arg Gln Gln Ile Ala
   180 185 190
   Glu Arg Arg Arg Arg Pro Ser Asp Asp Leu Val Ser Ser Leu Leu Ala
   195 200 205
   Ala Glu Ile Asp Gly Glu Arg Leu Glu Glu Leu Asp Leu Val Ala Phe
   210 215 220
   Cys Met Leu Leu Leu Ile Ala Gly Asn Ile Ser Thr Thr His Met Ile
   225 230 235 240
   Gly Asn Ala Ile Arg Ala Leu Leu Lys His Pro Asp Gln Met Ala Arg
   245 250 255
   Phe Leu Ala Asn Pro Asp Gln Met Pro Gly Ala Ile Glu Glu Leu Leu
   260 265 270
   Arg Tyr Asp Ser Pro Val Leu Ala Ala Pro Arg Trp Val Arg His Asp
   275 280 285
   Thr Glu Val Gly Gly Lys Leu Leu Lys Arg Gly Gln Arg Val Ile Val
   290 295 300
   Trp Leu Gly Ala Ala Asn His Asp Pro Asp Val Phe Pro Glu Pro Glu
   305 310 315 320
   Arg Leu Asp Val Ser Arg Lys Ala Ala Arg Gln Val Ser Phe Gly His
   325 330 335
   Gly Ala His Tyr Cys Met Gly Ala Pro Leu Gly Arg Leu Glu Ala Arg
   340 345 350
   Ile Ala Ile Pro Ala Ile Leu Arg Arg Met Pro Glu Leu Arg Met Val
   355 360 365
   Glu Gly Ala Glu Leu Glu Pro Val Pro Gly Tyr Leu Leu His Gly Met
   370 375 380
   Val Ser Met Pro Met Thr Phe Arg Ala Ser
   385 390
<210> 35
   <211> 2385
   <212> PRT
   <213> Pyxidicoccus fallax
<400> 35
   Met Asp Ala Ser Ser Ser Arg Pro Phe Glu Ser Leu Asp Ala Leu Gln
   1 5 10 15
   Ala Trp Val Val Thr Trp Leu Cys Glu His Ala Arg Ile Pro Gln Gln
   20 25 30
   Ala Ile Gly Leu Asp Val Arg Leu Arg Asp Leu Gly Leu Asp Ser Ala
   35 40 45
   Arg Ala Thr Lys Leu Val Ala Ala Leu Ser Glu Arg Phe Gly Arg Thr
   50 55 60
   Leu Pro Leu Thr Leu Leu Trp Asp Tyr Pro Thr Val Thr Glu Leu Ser
   65                  70                  75                  80
   Arg His Leu Met Lys Pro Ala Thr Ala Ala Ala Ala Gly Ala Thr Pro
                   85                  90                  95
   Arg Ala Glu Arg Arg Ala Ser Gly Ala Pro Glu Asp Arg Ala Ile Ala
               100                 105                 110
   Val Ile Gly Met Ala Cys Arg Phe Pro Gly Gly Ala Asp Thr Pro Glu
           115                 120                 125
   Ala Phe Trp Glu Leu Leu Ala Lys Gly Leu Asp Val Val Gly Pro Val
       130                 135                 140
   Pro Ala Asp Arg Tyr Asp Ala Glu Ala Phe Tyr Asp Arg Asp Pro Arg
   145                 150                 155                 160
   Ala Arg Gly Lys Ala Val Thr Arg Trp Gly Gly Phe Leu Glu Arg Thr
                   165                 170                 175
   Asp Thr Phe Asp Pro Tyr Phe Phe Gly Ile Ser Pro Arg Glu Ala Ile
               180                 185                 190
   Glu Met Asp Pro Gln Gln Arg Met Leu Leu Glu Leu Ala Trp Glu Ala
           195                 200                 205
   Phe Glu Ala Ala Gly Leu Thr Lys Asp Gly Leu Arg Asp Arg Ala Val
       210                 215                 220
   Gly Val Phe Val Gly Ala Met Trp Asn Asp Tyr Ala Arg Leu Trp Arg
   225                 230                 235                 240
   Gly Gly Pro Gly Ala Ile Glu Gln Phe Thr Ala Thr Gly Gln Asp Asp
                   245                 250                 255
   Ser Ile Ile Ser Ala Arg Ile Ser Tyr Val Phe Gly Leu Arg Gly Pro
               260                 265                 270
   Ser Leu Thr Leu Asn Thr Ala Cys Ser Ser Ser Leu Val Ser Val His
           275                 280                 285
   Met Ala Cys Gln Ser Leu Leu Ala Gly Asp Ser Glu Leu Ala Leu Ala
       290                 295                 300
   Gly Gly Val Asn Leu Ile His Gly Pro Asp Ser Thr Ile Ala Met Ser
   305                 310                 315                 320
   Lys Phe Gly Gly Met Ala Pro Asp Gly Lys Cys Lys Ala Phe Asp Ala
                   325                 330                 335
   Arg Gly Asn Gly Tyr Val Arg Gly Glu Gly Ala Gly Val Val Leu Leu
   340 345 350
   Lys Pro Leu His Arg Ala Leu Glu Asp Gly Asp Val Ile His Ala Val
   355 360 365
   Ile Arg Gly Ser Ala Val Asn Asn Asp Gly Phe Ser Asn Gly Leu Thr
   370 375 380
   Ser Pro Ser Pro Ala Ala Gln Ala Ala Met Leu Arg Ala Ala Tyr Ala
   385 390 395 400
   Ser Ala Gly Val Ser Pro Ala Arg Val His Tyr Val Glu Ala His Gly
   405 410 415
   Thr Gly Thr Met Leu Gly Asp Pro Ile Glu Ala Gly Ala Leu Gly Glu
   420 425 430
   Val Leu Gly Gln His Pro Glu Arg Gln Gln Pro Leu Arg Ile Gly Ser
   435 440 445
   Ala Lys Thr Asn Ile Gly His Leu Glu Ala Ala Ala Gly Met Ala Gly
   450 455 460
   Leu Ile Lys Thr Ile Glu Ala Met Lys His Arg Arg Leu Pro Pro Ser
   465 470 475 480
   Leu His Phe Glu Gln Pro Asn Pro Asn Ile His Phe Glu Gly Leu Arg
   485 490 495
   Leu Arg Val Gln Thr Asp Leu Ser Pro Trp Pro Thr Glu Asp Asp Ser
   500 505 510
   Leu Leu Ala Gly Val Ser Ser Phe Gly Phe Gly Gly Thr Asn Ala His
   515 520 525
   Val Val Leu Glu Ser Phe Gln Pro Ala Arg Pro Val Ala Ile Pro Leu
   530 535 540
   Ser Ala Pro Asp Ala Glu Ala Leu Gly Ala Arg Leu Val Glu Leu Arg
   545 550 555 560
   Ala Phe Ala Glu Gln His Pro Glu Ala Ser Leu Asp Asp Leu Gln Arg
   565 570 575
   Ala Trp Val Gln Arg Leu Thr Glu Glu Pro Val Arg Gln Val Leu Leu
   580 585 590
   Ala Arg Ser Arg Glu Asp Leu Leu Glu Gln Leu Arg Ala Pro Ser Arg
   595 600 605
   Pro Ala Ala Pro Ala Gly Gly Gly Ala Ala Arg Pro Val Leu Val Phe
   610 615 620
   Thr Gly Asn Gly Ala Gln Tyr Pro Gly Met Ala Arg Asp Leu Met Leu
   625 630 635 640
   Gly Asp Ala Thr Phe Arg Gln Ala Val Leu Arg Cys Asp Glu Ala Leu
   645 650 655
   Arg Ala His Gly Cys Asp Gly Val Ala Arg Ala Leu Glu Ser Gly Asp
   660 665 670
   Phe Asp Val Arg Arg Val Asp Leu Val Gln Pro Leu Leu Trp Ala Val
   675 680 685
   Gln Val Gly Leu Val Ser Leu Trp Arg Ala Trp Gly Val Glu Pro Ala
   690 695 700
   Ala Val Val Gly His Ser Val Gly Glu Val Ala Ala Ala Trp Ala Ala
   705 710 715 720
   Gly Ala Leu Asp Leu Asp Glu Ala Ala Arg Val Val Val His Arg Ser
   725 730 735
   Arg Leu Gln Gln Glu Cys Ala Asp Gln Gly Gly Met Val Leu Leu Glu
   740 745 750
   Thr Asp Ala Glu Gly Ala Arg Ser Leu Val Gly Ala Thr Ala Ala Glu
   755 760 765
   Gly Leu Ala Val Ala Ala Leu Asn Ser Arg Gly Ser Thr Val Val Ser
   770 775 780
   Gly Pro Thr Ala Ser Leu Gln Ala Leu Ile Thr Lys Ala Glu Glu Arg
   785 790 795 800
   Gly Val Thr Ala Arg Thr Val Arg Val Asn Val Ala Tyr His Gly Pro
   805 810 815
   Gly Met Asp Ala Leu Arg Glu Arg Leu Arg Glu Ser Leu Ala Asp Leu
   820 825 830
   Arg Pro Cys Pro Pro Arg Val Pro Met Ser Ser Ser Val Thr Gly Gly
   835 840 845
   Ala Val Gly Gly Ala Gly Leu Asp Ala Gly Tyr Trp Val Arg Asn Met
   850 855 860
   Ala Asp Pro Val Leu Phe Gly Asp Ala Val Asp Ala Leu Val Arg Asp
   865 870 875 880
   Gly His Arg Leu Phe Leu Glu Val Gly Pro His Pro Thr Leu His Arg
   885 890 895
   Ala Val Gln Glu Gln Leu Gln His Gly Gly Val Ala Gly Gly Phe Val
   900 905 910
   Ala Thr Ser Leu Glu Arg Glu Gln His Gly Arg Ala Arg Leu Leu Glu
   915 920 925
   Asn Leu Gly His Leu Phe Glu Arg Gly Val Arg Val Ala Trp Arg Ala
   930 935 940
   Leu Thr Arg Gly Gly Ala Pro Ala Ser Ala Gly Gln Ala Pro Arg Pro
   945 950 955 960
   Val Leu Leu Pro Leu Ser Ala His Thr Asp Ala Ala Leu Gln Gln Arg
   965 970 975
   Val Glu Gly Leu Arg Ala Tyr Leu Arg Ala Asn Pro Glu Ala Glu Leu
   980 985 990
   Glu Ala Leu Ala Arg Thr Leu Gly Arg Gly Arg Thr His Leu Ser His
   995 1000 1005
   Arg Cys Ala Leu Val Ala Ser Ser Ala Gly Glu Leu Ala Glu Val
   1010 1015 1020
   Leu Glu Arg Ala Ala Gly Ala Asp Gly Ala Pro Glu Leu Ile Arg
   1025 1030 1035
   Pro Ala Ala Ser Leu Asp Gly Arg Ala Lys Val Ala Phe Val Phe
   1040 1045 1050
   Pro Gly Gln Gly Ser Gln Trp Leu Gly Met Gly Arg Gln Leu Leu
   1055 1060 1065
   Glu Thr Glu Pro Val Phe Arg Lys Ala Leu His Glu Trp Glu Ala
   1070 1075 1080
   Val Leu Gly Glu Phe Thr Asp Trp Ser Leu Leu Glu Gln Leu His
   1085 1090 1095
   Ala Asp Ala Ala Thr Ser Arg Leu Gly Glu Val Asp Val Ile Gln
   1100 1105 1110
   Pro Thr Leu Phe Ala Ile Gln Val Ala Leu Ala Ala Leu Trp Arg
   1115 1120 1125
   Ser Trp Gly Val His Pro Asp Ala Val Ile Gly His Ser Met Gly
   1130 1135 1140
   Glu Val Ala Ala Ala Tyr Val Ala Gly Ala Leu Asp Ala Arg Asp
   1145 1150 1155
   Ala Ala Arg Ile Ile Cys Ser Arg Ser Arg Ile Ala Lys Lys Thr
   1160 1165 1170
   Ser Gly Arg Gly Ser Met Ala Val Val Glu Leu Ser Pro Glu Glu
   1175 1180 1185
   Cys Gln Arg Ala Ile Gly Ala Arg Ala Ala Thr Val Ser Leu Ala
   1190 1195 1200
   Ala Cys Asn Gly Pro Ala Ser Cys Leu Leu Ser Gly Glu Thr Ala
   1205 1210 1215
   Ala Val Glu Glu Ile Gly Arg Glu Leu Gln Ala Arg Gly Val Phe
   1220 1225 1230
   Phe Arg Leu Val Lys Val Asp Tyr Ala Ser His Ser Pro Gln Met
   1235 1240 1245
   Asp Ala Ile Lys Asp Glu Leu Ala His Val Leu Arg Glu Thr His
   1250 1255 1260
   Pro Arg Pro Ala Thr Val Pro Phe His Ser Thr Val Glu Asn Ala
   1265 1270 1275
   Leu Leu Asp Gly Thr Arg Leu Asp Ala Gly Tyr Trp Ala Arg Asn
   1280 1285 1290
   Leu Arg Glu Pro Val Leu Phe Phe Gln Gly Ile Gln Ala Leu Glu
   1295 1300 1305
   Ala Arg Gly Phe Asn Val Phe Leu Glu Leu Ser Pro His Pro Val
   1310 1315 1320
   Leu Thr Tyr Ser Ile Ser Gln Thr Val Arg Pro Glu Ser Ala Leu
   1325 1330 1335
   Ile Leu Thr Ser Thr Lys Arg Glu Glu Asp Glu Ser Arg Ala Leu
   1340 1345 1350
   Arg Thr Ser Leu Gly Ala Leu Phe Ala Ala Gly Gly Ala Val Asp
   1355 1360 1365
   Trp Ala Ala Leu Tyr Pro Gly Val Ala Thr Pro Val Glu Leu Pro
   1370 1375 1380
   Ala Tyr Pro Phe Gln Arg Glu Arg Phe Trp Phe Asp Glu Ala Glu
   1385 1390 1395
   Val Gln Arg Ser Ala Pro Ala Gly Thr Gly Arg His Ala Phe Leu
   1400 1405 1410
   Glu Arg Phe Leu Arg Ser Pro Tyr Pro Gln Glu Arg Leu Ile Trp
   1415 1420 1425
   Glu Thr Asp Leu Ser Leu Glu Arg Phe Pro Tyr Leu Val Asp His
   1430 1435 1440
   Arg Val Ser Gly Leu Ala Val Phe Pro Gly Thr Gly Tyr Ile Glu
   1445 1450 1455
   Cys Val Leu Glu Ala Ala Lys Asp Leu Tyr Gly Asp Ala Pro Val
   1460 1465 1470
   Glu Leu Leu Glu Val Ser Phe Glu Lys Ala Leu Phe Ile Glu Asp
   1475 1480 1485
   Thr Gly Ala Lys Thr Leu Gln Leu Cys Met Glu Pro Asp Gly Ala
   1490 1495 1500
   Gly His Arg Phe Ser Leu Ser Ser His Asp Ala Ala Gln Gln Thr
   1505 1510 1515
   Trp Thr Arg His Val Asn Gly Arg Leu Val Pro Ala Thr Ala Glu
   1520 1525 1530
   Gln Ala Ala His Ala Arg Gln Gln Val Gln Asp Val Leu Ala Gly
   1535 1540 1545
   Glu Pro His Ala Pro Val Asp Val Gly Thr Phe Tyr Gly Asn Leu
   1550 1555 1560
   Glu Arg Ala Gly Asn Thr Tyr Gly Pro Lys Phe Gln Ala Val Lys
   1565 1570 1575
   Ser Leu Arg Ser Asn Ala Arg Gly Val Arg Gly Glu Val Val Met
   1580 1585 1590
   Gly Glu Glu Leu Ala Thr Arg Gly Thr Arg Ile His Pro Val Val
   1595 1600 1605
   Phe Asp Ala Cys Leu Gln Val Met Ala Thr Pro Leu Tyr Ile Glu
   1610 1615 1620
   Lys Ala Ala Gly Gly Arg Glu Ser Ala Phe Met Pro Thr Arg Ala
   1625 1630 1635
   Ala Arg Ile Arg Phe Phe Arg Glu Ala Asp Arg Ala Val Val Ser
   1640 1645 1650
   His Val Arg Phe Leu Asn Gly Ala Thr Ala Asp Ala Ala Gly Asp
   1655 1660 1665
   Leu Leu Ala Asp Ile Asp Val Thr Asp Gly Gln Gly Gln Leu Leu
   1670 1675 1680
   Ala Arg Val Glu Arg Ile Cys Phe His Leu Leu Glu His Ala Thr
   1685 1690 1695
   Met Ala Gln Arg Pro Ser Thr Asp Met Leu Tyr Arg Leu Thr Trp
   1700 1705 1710
   Glu Glu Ala Pro Ser Pro Glu Val Gly Ala Ser Leu Ala Arg Arg
   1715 1720 1725
   Trp Leu Val Phe Ala Asp Asp Gln Gly Val Ala Glu Ala Ala Ala
   1730 1735 1740
   Gly Leu Leu Ser Ala Ser Ala His Ala Val His Arg Leu His Pro
   1745 1750 1755
   Ala Asp Ala Phe Arg Arg Asp Gly Asn Asp Phe His Ile Arg Pro
   1760 1765 1770
   Asn Glu Pro Glu Asp Tyr Arg Ala Val Leu Gln Ala Leu Leu Ala
   1775 1780 1785
   Ser Arg Gly Pro Ala Leu Pro Leu Gly Val Leu His Leu Trp Asn
   1790 1795 1800
   Leu Arg Leu Pro Glu Gly Gly Ala Thr Ala Val Pro Asp Leu Arg
   1805 1810 1815
   Glu Met Ala Lys Leu Gly Asn Leu Ser Leu Thr His Leu Leu Lys
   1820 1825 1830
   Ala Leu Asp Gly Ala Gly Gly Ser Ala Pro Pro Arg Val Trp Ile
   1835 1840 1845
   Gly Thr Arg Gly Ala Trp Ala Leu Pro Gly Ser Glu Gln Gly Leu
   1850 1855 1860
   Ala Pro Ala Gln Ser Ala Val Trp Gly Leu Thr Arg Ala Ile Ala
   1865 1870 1875
   Gln Glu His Pro Ala Leu Trp Gly Gly Ile Ile Asp Leu Asp Pro
   1880 1885 1890
   Leu Glu Ala Pro Arg Leu Gln Ala Glu Arg Leu Val Thr Glu Val
   1895 1900 1905
   Ser Val Pro Gln Pro Ala Glu Asp Arg Ile Leu Tyr Arg Gln Gly
       1910                1915                1920
   Arg Arg Gln Val Ser Arg Leu Val Arg Asp Thr Ala Pro Val Ser
       1925                1930                1935
   Ala Ala Thr Ala Thr Phe Arg Gly Asp Arg Gly Tyr Leu Ile Thr
       1940                1945                1950
   Gly Gly Leu Gly Glu Leu Gly Thr Arg Leu Ala Ala Trp Met Val
       1955                1960                1965
   Ala Arg Gly Ala Arg His Leu Ile Leu Ala Gly Arg Lys Ala Leu
       1970                1975                1980
   Pro Ala Arg Arg Thr Trp Lys Gln Asp Gly His Asp Ala Ala Thr
       1985                1990                1995
   Arg Ala Arg Ile Asp Ala Val Leu His Leu Glu Arg Leu Gly Ala
       2000                2005                2010
   His Val His Thr Pro Ala Leu Asp Val Gly Asp Gly Ala Ala Leu
       2015                2020                2025
   Ala Ala Phe Leu Ala Gly Tyr Glu Gln Glu Ala Arg Pro Pro Ile
       2030                2035                2040
   Ala Gly Val Ala His Leu Ala Gly Val Ile Arg Pro Glu Leu Leu
       2045                2050                2055
   Ala Arg Leu Arg Glu Glu Ser Leu Glu Glu Ser Phe Gln Ser Lys
       2060                2065                2070
   Val Tyr Gly Ala Trp Asn Leu His Gln Ala Phe Ser Gly Arg Gly
       2075                2080                2085
   Leu Asp Phe Phe Thr Leu Phe Ser Ser Ala Ser Ala Val Leu Asn
       2090                2095                2100
   Ser Pro Leu Leu Gly Ser Tyr Ala Ala Ala Asn Ala Phe Leu Asp
       2105                2110                2115
   Gly Leu Ala His Phe Arg Arg Ala Arg Gly Glu Pro Ala Gln Ser
       2120                2125                2130
   Leu Asn Trp Gly Phe Trp Ser Glu Ala Gly Met Ala Ala Arg Asp
       2135                2140                2145
   Leu His Phe Asp Ala Ser Ala Ser Gly Gln Gln Gly Met Arg Ser
       2150                2155                2160
   Phe Ser Pro Ala Val Gly Leu Asp Ile Phe Gly Gly Leu Leu Ser
   2165 2170 2175
   Arg Asp Pro Val Gln Ser Val Val Met Asp Val Asp Trp Lys Leu
   2180 2185 2190
   Trp Leu Glu Ala Tyr Pro Asp Ala Ala Ala Ser Pro Leu Leu Arg
   2195 2200 2205
   Thr Leu Arg Ala Ser Glu Gly Val Ala Ala Gly Ser Glu Gln Ala
   2210 2215 2220
   Ala Pro Ala Glu Glu Ala Ser Arg Ser Leu Arg Asp Leu Pro Ala
   2225 2230 2235
   Glu Glu Arg Arg Lys Ala Val Glu Gly Ile Leu Leu Thr Glu Leu
   2240 2245 2250
   Ser Ala Val Leu Lys Met Pro Ala Ala Lys Ile Ser Arg Thr Ala
   2255 2260 2265
   Leu Phe Gly Ser Met Gly Leu Asp Ser Met Leu Ser Leu Glu Leu
   2270 2275 2280
   Arg Asn Arg Leu Glu Arg Arg Thr Gly Leu Thr Leu Pro Gly Thr
   2285 2290 2295
   Leu Ala Trp Asn Tyr Pro Ser Leu Glu Arg Met Ala Ala Phe Phe
   2300 2305 2310
   Val Glu Lys Leu Gly Gly Glu Gly Ala Gly Val Ala Arg Ser Glu
   2315 2320 2325
   Arg Gln Gln Ala Gly Ala Ala Ser Asp Gly Val Ala Lys Glu Pro
   2330 2335 2340
   Asp Ile Ser Ala Glu Val Ile Glu Ala Leu Met Gln Leu Gly Val
   2345 2350 2355
   Leu Glu Ala Asn Asp Pro Val Leu Gln Asp Pro Val Ala Leu Gly
   2360 2365 2370
   Asn Leu Val Arg Glu Ala Gln Gln Tyr Leu Lys Ala
   2375 2380 2385
<210> 36
   <211> 1570
   <212> PRT
   <213> Pyxidicoccus fallax
<400> 36
   Met Thr Gly Ile Ile Asp Lys Leu Ser Arg Leu Asp Ala Ala Ser Lys
   1               5                   10                  15
   Asp Leu Leu Ala Glu Lys Leu Arg Gln Ala Lys Asp Lys His Leu Ala
               20                  25                  30
   Arg Gly Ala His Ala Glu Pro Ile Ala Val Val Gly Met Gly Cys Arg
           35                  40                  45
   Phe Pro Gly Gly Ala Ser Ser Pro Glu Arg Phe Trp Gln Leu Leu Thr
       50                  55                  60
   Gln Gly Arg Ser Gly Val Gly Arg Val Pro Ala Asp Arg Phe Pro Ile
   65                  70                  75                  80
   Glu Asp Tyr Tyr Asp Pro Asp Pro Ser Ala Pro Gly Ala Ile Leu Ser
                   85                  90                  95
   Asp Arg Gly Ala Phe Val Glu Gly Ile Asp Arg Phe Asp Ala Gly Phe
               100                 105                 110
   Phe Gly Val Ser Pro Arg Glu Ala Arg Arg Leu Asp Pro Gln His Arg
           115                 120                 125
   Leu Leu Leu Glu Val Thr Trp Glu Ala Leu Glu Asn Ala Gly Leu Pro
       130                 135                 140
   Gln Ser Ala Leu Arg Glu Leu Val Gly Gly Val Phe Ile Gly Leu Met
   145                 150                 155                 160
   Gly Asn Asp Tyr Phe Ser Thr Val Gln Glu Asn Pro Ala Asp Ile Asp
                   165                 170                 175
   Ala Tyr Thr Ser Thr Gly Thr His Tyr Ser Phe Ala Ala Asn Arg Leu
               180                 185                 190
   Ser Phe His Leu Asp Phe Lys Gly Pro Ser Met Ala Ile Asp Ser Ala
           195                 200                 205
   Cys Ser Ser Ser Leu Val Ala Val His Gln Ala Cys Gln Ser Leu Arg
       210                 215                 220
   Thr Arg Asp Cys Asp Phe Ala Val Ala Gly Gly Val Asn Leu Val Leu
   225                 230                 235                 240
   Ser Pro Val Leu Ser Ile Ser Tyr Ser Lys Trp Gly Met Leu Ser Pro
                   245                 250                 255
   Asp Gly Gln Cys Lys Thr Phe Asp Ala Ser Ala Asn Gly Phe Val Arg
               260                 265                 270
   Gly Glu Gly Ala Gly Val Val Val Leu Lys Arg Leu Glu Asp Ala Val
   275 280 285
   Ala Ala Gly Asp Pro Ile Leu Ala Val Val Arg Gly Ser Ala Val Asn
   290 295 300
   Gln Asp Gly Ala Thr Ser Val Ile Thr Ala Pro Ser Gly Leu Ala Gln
   305 310 315 320
   Gln Ala Val Ile Arg Arg Ala Leu Lys Val Ala Gly Val Glu Pro Ser
   325 330 335
   Ala Ile Gly Tyr Val Glu Thr His Gly Thr Gly Thr Ser Leu Gly Asp
   340 345 350
   Pro Ile Glu Val Glu Ala Leu Asn Glu Thr Tyr Gly Gln Ala Pro Gly
   355 360 365
   Gly Arg Pro Cys Val Leu Gly Ala Val Lys Thr Asn Val Gly His Leu
   370 375 380
   Glu Gly Ala Ala Gly Ile Ala Gly Phe Ile Lys Ala Val Leu Ala Val
   385 390 395 400
   Gln His Gly Arg Ile Pro Pro Asn Leu His Phe His Glu Leu Asn Pro
   405 410 415
   Leu Ile Arg Leu Asp Gly Ser Arg Leu Cys Leu Ala Thr Glu Ala Arg
   420 425 430
   Glu Trp Ala Val Ala Ser Gln Glu Arg Phe Ala Ala Ile Ser Ser Phe
   435 440 445
   Gly Ala Gly Gly Thr Asn Ala His Val Val Ile Gln Gly Pro Pro Ala
   450 455 460
   Ala Arg Ala Pro Ala Ala Thr Gly Lys Ala Pro Ala Phe Val Cys Leu
   465 470 475 480
   Ser Ala Lys Ser Asp Ala Ser Leu Lys Gln Gln Ala Ala Arg Leu Arg
   485 490 495
   Ala Trp Leu Gly Gln Asp Gly Arg Glu Trp Ser Val Ala Asp Ile Ala
   500 505 510
   Ser Thr Ser Leu Arg Arg Arg Asn His Phe Asp His Arg Ala Ala Phe
   515 520 525
   Thr Ala Asp Ser Pro Glu Ser Leu Gly Glu Gly Leu Asp Ala Phe Leu
   530 535 540
   Ser Gly Arg Ala Ser Pro Arg Trp Ser Ala Gly Ser Gly Thr Leu Asn
   545 550 555 560
   Ala Gln Arg Pro Val Phe Val Phe Ser Gly Gln Gly Ser Gln Trp Gln
   565 570 575
   Gly Met Gly Arg Glu Leu Leu Thr Gln Ser Pro Arg Phe Ala Ala Glu
   580 585 590
   Ile Ala Ala Ile Asp Ala Arg Phe Ile Pro Leu Ala Gly Trp Ser Ile
   595 600 605
   Ala Glu Val Leu Ala Arg Asp Gly Asp Gly Ala Arg Leu Asn Ala Thr
   610 615 620
   Glu Val Ala Gln Pro Cys Leu Phe Ala Leu Gln Leu Gly Leu Val Ala
   625 630 635 640
   Ala Leu Asp Glu Leu Gly Ile Arg Pro Ala Ala Val Val Gly His Ser
   645 650 655
   Leu Gly Glu Leu Ala Ala Ala Cys Val Ala Gly Leu Met Asp Arg Glu
   660 665 670
   Ala Ala Thr Ala Leu Val Tyr His Arg Ala Lys Leu Met Gln Arg Leu
   675 680 685
   Thr Gly Ala Gly Lys Met Ala Ala Val Gly Leu Ser Arg Asp Glu Ala
   690 695 700
   Glu Ala Arg Leu Ala Ala Glu Pro Arg Leu Ala Leu Ala Ala Val Asn
   705 710 715 720
   Gly Pro Arg Ser Val Val Val Ala Gly Glu Pro Glu Ala Val Asp Ala
   725 730 735
   Phe Val Arg Thr Leu Arg Asp Glu Gly Val Phe Thr Arg Thr Leu Pro
   740 745 750
   Val Asp Tyr Ala Phe His Ser Pro Gln Thr Asp Pro Leu Leu Ala Glu
   755 760 765
   Leu Arg Arg Ala Val Asp Gly Ile Phe Gly Ala Gly Ala Arg Met Ala
   770 775 780
   Ala Pro Arg Leu Pro Met Val Ser Thr Val Ser Gly Ala Glu Leu Asp
   785 790 795 800
   Ala Ser Val Asp His Ala Thr Tyr Trp Ala Arg Asn Met Arg Gln Thr
   805 810 815
   Val Cys Phe Glu Glu Ala Val Gly Gln Leu Leu Glu Gln Arg His Asp
   820 825 830
   Ala Phe Leu Glu Val Gly Ala His Pro Val Leu Asn Val Asp Leu Arg
   835 840 845
   Glu Ser Phe Arg Ala Arg Glu Val Thr Ala Arg Ala Val Ala Thr Leu
   850 855 860
   Arg Arg Glu Gln Pro Gln Ala Leu Cys Leu Arg Ala Val Pro Ala Ala
   865 870 875 880
   Leu Tyr Ala Ala Gly Met Ala Pro Glu Ala Leu Pro Gly Gln Asp Gln
   885 890 895
   Gly Thr Leu Val Pro Leu Pro Asn Tyr Ala Trp Asp His Arg Arg Tyr
   900 905 910
   Trp Ile His Ser Ala Ser Arg Ala Glu Ala Gly Trp Val Tyr Asp Leu
   915 920 925
   Ala Trp Asp Ala Val Pro Ala Pro Ala Pro Gly Ala His Arg Pro Ala
   930 935 940
   Ser Trp Leu Val Leu Gly Ala Ser Gly Gly Leu Pro Glu Ala Leu Ala
   945 950 955 960
   Ala Arg Leu Arg Ala Thr Pro Asp Leu Val Arg His Val Ser Leu Glu
   965 970 975
   Ala Leu Arg Ser Val Pro Val Pro Pro His Gly Leu Val Asp Ala Glu
   980 985 990
   Arg Leu Glu Arg Trp Phe Thr Asp Thr Leu Gln Val Asp Leu Ala Glu
   995 1000 1005
   Phe Asp Phe Leu Val Tyr Val Pro Ala Cys Gly Ala Pro Glu Asp
   1010 1015 1020
   Ala Ser Ala Glu Arg Gln Val Ser Glu Asp Cys Ala Ile Leu Val
   1025 1030 1035
   Ser Leu Ala Gln Ala Leu Glu Arg Arg Pro Gly Ala Arg Gly Pro
   1040 1045 1050
   Ala Leu Trp Val Val Thr Ser Gly Ala Gln Ala Val Ala Pro Arg
   1055 1060 1065
   Glu Pro Val Asn Pro Phe Ala Ala Pro Val Trp Gly Leu Gly Arg
   1070 1075 1080
   Val Leu Ala Leu Glu Ala Pro Arg Gly Leu Gly Gly Leu Val Asp
   1085 1090 1095
   Leu Pro Leu Ala Trp Ser Glu Gln Asp Val Gly Cys Leu Leu Glu
   1100 1105 1110
   Ala Leu Arg His Pro Gly Glu Glu Asp Gln Leu Ala Leu Arg Ser
   1115 1120 1125
   Gly Gln Arg His Ala Leu Arg Leu Arg Arg Arg Ala Gly Arg Ala
   1130 1135 1140
   Thr Asp Ala Ala Ser Thr Trp Gln Val Glu Asp Ser Gly Val Tyr
   1145 1150 1155
   Leu Ile Thr Gly Gly Thr Arg Gly Leu Gly Leu Leu Val Ala Lys
   1160 1165 1170
   Arg Leu Ala Glu Arg Gly Ala Arg His Leu Ala Leu Leu Gly Arg
   1175 1180 1185
   Arg Ala Leu Pro Pro Arg Ser Arg Trp Ala Thr Leu Ala Ala Asp
   1190 1195 1200
   Asp Ala Phe Phe Pro Ser Val Gln Ala Ile Gln Ala Leu Glu Ala
   1205 1210 1215
   Gln Gly Val Thr Val Thr Leu His Ala Ala Asp Val Ser Asp Ser
   1220 1225 1230
   His Ala Met Arg Asp Val Phe Ala Glu Leu Glu Arg Gln Gln Arg
   1235 1240 1245
   Pro Leu Lys Gly Val Val His Ala Ala Gly Ile Ser Arg Glu Val
   1250 1255 1260
   Pro Leu Ala Ser Ile Asp Glu Ala Leu Leu Glu Glu Thr Leu Ala
   1265 1270 1275
   Pro Lys Val Ala Gly Thr Trp Trp Leu His Gln Leu Ser Leu Arg
   1280 1285 1290
   His Pro Leu Asp Phe Phe Val Val Phe Ser Ser Gly Ala Ser Val
   1295 1300 1305
   Trp Gly Ser Ala Gly Leu Ala Pro Tyr Ala Ala Ala Asn Glu Phe
   1310 1315 1320
   Leu Asn Ala Val Ala Ala His Arg Arg Ala Ser Gly Leu Pro Ala
   1325 1330 1335
   Leu Ser Val Cys Trp Gly Met Trp Asp Ala Asp Gly Met Ala Thr
   1340 1345 1350
   Asp Glu Met Arg Glu Asn Trp Thr Arg Leu Gly Val Lys Ala Met
   1355 1360 1365
   Ala Pro Glu Arg Ala Leu Asn Ala Leu Glu Glu Leu Val Ala Ala
   1370 1375 1380
   Asp Ala Thr Leu Ala Thr Val Ala Asp Val Lys Trp Ala Arg Phe
   1385 1390 1395
   Arg Pro Val Tyr Glu Leu Lys Arg Lys Arg Pro Leu Leu Ala Ala
   1400 1405 1410
   Leu Glu Asp Gly Ala Ala Ala Ala Pro Arg Ala Glu Ala Ala Gly
   1415 1420 1425
   Ala Leu Gln Ala Ala Ser Leu Lys Ala Glu Leu Gln Ala Leu Pro
   1430 1435 1440
   Arg Glu Gln Gln Arg Glu Arg Leu Leu Ala Thr Leu Arg Asp Ala
   1445 1450 1455
   Ala Ala Leu Val Leu Gly Ala Glu Pro Gly Ala Ala Ile Asn Pro
   1460 1465 1470
   Asp Glu Gly Leu Phe Ser Met Gly Phe Asp Ser Val Thr Ala Val
   1475 1480 1485
   Glu Phe Ala Ala His Leu Ser Arg Leu Phe Glu Lys Arg Tyr Ala
   1490 1495 1500
   Ala Thr Leu Val Phe Asp His Pro Ser Leu Lys Gln Ile Arg Asp
   1505 1510 1515
   Phe Leu Met Gln Asp Cys Phe Gly Trp Thr Ala Glu Pro Ser Pro
   1520 1525 1530
   Glu Pro Glu Ala Gly Asp Gly Gln Asp Leu Asp Ala Leu Leu Ala
   1535 1540 1545
   Phe Val Glu Gly Val Asp Glu Ala Glu Ala Glu Arg Leu Leu Ser
   1550 1555 1560
   Ala Gly Gln Pro Arg Ser Asn
   1565 1570
<210> 37
   <211> 1856
   <212> PRT
   <213> Pyxidicoccus fallax
<400> 37
   Met Thr Ser Val Asp Glu Arg Ile Lys Asn Leu Ser Pro Gln Lys Arg
   1 5 10 15
   Ala Leu Leu Glu Gln Arg Met Asn Ala Ala Ala Ala Ser Ala Thr Arg
   20 25 30
   Asp Ala Glu Pro Ile Ala Ile Ile Gly Ile Gly Cys Arg Tyr Pro Gly
   35 40 45
   Gly Val Lys Asp Leu Lys Ser Phe Ala Gln Leu Leu Arg Asp Gly Ala
   50 55 60
   Asp Ala Thr Gly Asp Ile Pro Ala Asp Arg Trp Asp Val Glu Ala His
   65 70 75 80
   Tyr Ser Pro Asp Pro Gly Thr Pro Gly Lys Met Arg Thr Arg Arg Gly
   85 90 95
   Gly Phe Leu Pro Asp Val Lys Thr Phe Asp Ala Asp Phe Phe Gly Ile
   100 105 110
   Ser Pro Arg Glu Ala Gln Ser Met Asp Pro Gln Gln Arg Leu Leu Leu
   115 120 125
   Glu Val Ser Tyr Glu Ala Leu Glu Asp Ala Gly Ile Pro Thr Ala Ser
   130 135 140
   Leu Ser Gly Ser Thr Thr Gly Val Tyr Ile Gly Ile Thr Leu Ala Glu
   145 150 155 160
   Tyr Ala Asp Arg Leu Pro Asp Asp Pro Arg Leu Ile Asp Val Tyr Ser
   165 170 175
   Gly Thr Gly Thr Tyr Leu Asn Val Ala Ala Gly Arg Leu Ser Tyr Val
   180 185 190
   Tyr Gly Leu Gln Gly Pro Cys Met Ala Val Asp Ala Ala Cys Ala Ser
   195 200 205
   Ser Leu Val Thr Val His Leu Ala Ala Gln Ala Leu Arg His Arg Glu
   210 215 220
   Cys Gly Leu Ala Leu Ala Gly Gly Val Ser Leu Met Leu Ser Pro Glu
   225 230 235 240
   Thr Thr Ile Tyr Leu Ser Gln Thr Gly Ala Leu Ala Pro Asp Gly Arg
   245 250 255
   Cys Lys Thr Phe Asp Ala Arg Ala Asn Gly Tyr Gly Arg Ala Glu Gly
   260 265 270
   Cys Gly Ile Leu Val Leu Lys Arg Leu Ser Asp Ala Leu Arg Asp Lys
   275 280 285
   Asp Arg Ile His Ala Leu Ile Arg Gly Thr Ala Val Ser Gln Asp Gly
   290 295 300
   Ala Ser Ser Gly Leu Thr Val Pro Asn Gly Arg Ala Gln Gln Lys Val
   305 310 315 320
   Ile Gln Ser Ala Leu Lys Asp Ala Ala Val Arg Ser Glu Asp Val Ser
   325 330 335
   Tyr Val Glu Ala His Gly Thr Gly Thr Pro Leu Gly Asp Pro Ile Glu
   340 345 350
   Leu Asn Ala Leu Gln Ala Val Tyr Gly Gln Arg Glu Ala Asp Trp Pro
   355 360 365
   Leu His Val Gly Ser Leu Lys Thr Asn Phe Gly His Ala Glu Ala Ala
   370 375 380
   Ala Gly Val Gly Gly Ile Ile Lys Thr Val Val Ala Leu Gln Glu Arg
   385 390 395 400
   Gln Ile Pro Ala His Leu His Phe Glu Lys Leu Asn Pro Leu Ile Thr
   405 410 415
   Ile Asp Pro Thr Arg Val Val Ile Pro Thr Arg Pro Thr Pro Trp Gln
   420 425 430
   Thr Arg Gly Glu Arg Lys Arg Leu Ala Gly Val Ser Ser Phe Gly Phe
   435 440 445
   Ser Gly Val Ile Ala His Ala Val Leu Glu Glu Ala Pro Ala Pro Glu
   450 455 460
   Ala Ala Val Glu Gln Pro Ala Met Pro Ala Gly Glu Arg Pro Leu His
   465 470 475 480
   Val Leu Pro Leu Thr Ala Lys His Pro Arg Ala Leu Ser Glu Leu Ile
   485 490 495
   Ser Ala Tyr Arg His Thr Val Gly Ala Leu Asp Ala Gly Arg Leu Ala
   500 505 510
   Asp Leu Gly Tyr Thr Ala Ser Arg Lys Arg Gly His His Ala His Arg
   515 520 525
   Ala Trp Phe Ala Phe Arg Asp Lys Glu Glu Leu Leu Gln Gln Leu Glu
   530 535 540
   Ala Ala Thr Arg Gly His Glu Leu Pro Gly Ala Val Leu Gln Arg Gln
   545 550 555 560
   Ala Arg Asp Asp Val Lys Val Val Met Val Phe Pro Gly Gln Gly Ser
   565 570 575
   Gln Trp Val Gly Met Gly Gln Gln Leu Met Arg Thr Ser Pro Val Phe
   580 585 590
   Arg Glu Ala Ile His Glu Leu Asp Arg Ala Met Arg Pro Trp Ala Asp
   595 600 605
   Phe Ser Leu Leu Glu Val Leu Gly Ala Arg Gly Gly Glu Asp Pro Phe
   610 615 620
   Thr Arg Val Ser Ile Val Gln Pro Thr Leu Phe Ala Val Gln Val Ala
   625 630 635 640
   Leu Ala Arg Gln Trp Glu Ala Trp Gly Val Arg Pro His Ala Val Ile
   645 650 655
   Gly His Ser Met Gly Glu Val Ala Ala Ala Cys Val Ala Gly Ala Leu
   660 665 670
   Ser Met Glu Asp Ala Ala Ala Val Ile Cys Arg Arg Ser Gln Leu Val
   675 680 685
   Gln Ala Thr Ser Gly Gln Gly Ala Met Ala Leu Val Glu Leu Ser Pro
   690 695 700
   Ala Glu Val Glu Ala Ala Ile Ala Pro His Gly Asp Arg Val Ser Ile
   705 710 715 720
   Ala Ala Tyr Asn Gly Pro Ser Thr Val Leu Leu Ser Gly Glu Pro Ala
   725 730 735
   Ala Val Asp Glu Val Leu Thr Ala Leu Gln Glu Arg Gly Ala Phe Cys
   740 745 750
   Arg Arg Val Lys Val Asp Tyr Ala Ser His Cys Ala Gln Met Asp Pro
   755 760 765
   Leu Leu Gly Arg Ile Arg Ser Glu Leu Ala His Val Lys Pro Arg Lys
   770 775 780
   Gly Gly Val Pro Ile Tyr Ser Thr Ala Arg Gly Glu Val Ile Asp Gly
   785 790 795 800
   Ser Arg Met Asp Ala Gly Tyr Trp Ala Asp Asn Leu Arg Arg Pro Val
   805 810 815
   Leu Phe His Ala Gln Val Lys Ala Leu Leu Ala Asp Gly Met Ser Ala
   820 825 830
   Leu Leu Glu Ile Ser Pro His Pro Val Leu Leu Pro Ser Met Gln Lys
   835 840 845
   Thr Leu Asp Glu Ala Gly Gly Gln Gln Leu Val Leu Pro Ser Leu Arg
   850 855 860
   Arg Asp Glu Asp Glu Pro Arg Ser Leu Ala Thr Ser Leu Gly Ala Leu
   865 870 875 880
   Phe Gly Leu Gly His Pro Val Asp Phe Ser Ala Phe Thr Pro Arg Ser
   885 890 895
   Gly Arg Leu Val Glu Thr Pro Arg Tyr Pro Trp Gln Arg Arg Glu Phe
   900 905 910
   Trp Leu Thr Arg Lys Ala Ser Lys Arg Arg His Ala Leu Gly Gly Ala
   915 920 925
   Glu Gly His Pro Leu Ile Gly Gly Ala Phe Arg Pro Ala Val Gly Pro
   930 935 940
   Glu Ser Tyr Tyr Trp Glu Phe Asp Val Asp Glu Glu Arg Leu Ala Phe
   945 950 955 960
   Ile Ala Asp His Arg Val Arg Gly Met Thr Val Leu Pro Gly Thr Ala
   965 970 975
   Tyr Leu Asp Leu Ala Leu Ser Ala Ala Arg Ala Ala Phe Gly Asp Lys
   980 985 990
   Gly Phe Pro Leu Ser Gln Val Ser Phe Arg Glu Ala Leu Thr Leu Leu
   995 1000 1005
   Pro Glu Gln Pro Arg Arg Val Gln Leu Ser Leu Val Arg Glu Ala
   1010 1015 1020
   Glu Gly Ala Phe Ser Val Cys Val Ser Ser Arg Asp Ala Ala Ser
   1025 1030 1035
   Pro Ala Thr Gly Glu Trp Thr Leu His Ala Thr Met Arg Leu Asp
   1040 1045 1050
   Val Glu Ala Pro Ala Thr Gly Gly Ala Arg Ala Glu Gly Gln Val
   1055 1060 1065
   Thr Pro Pro Ala Gly Ala Glu Leu Arg Asp Ala Arg Thr His Tyr
   1070 1075 1080
   Arg Leu Met Lys Glu Gly Gly Leu Ala Tyr Gly Pro Arg Phe Gln
   1085 1090 1095
   Gly Leu Ala Glu Val His Cys Gly Gln His Glu Ala Leu Gly Arg
   1100 1105 1110
   Leu Arg Ala Leu Pro Glu Leu Gly Leu Asp Gly Ala Asp His Val
   1115 1120 1125
   Phe His Pro Ala Leu Trp Asp Ala Ala His Gln Ala Leu Ala Ala
   1130 1135 1140
   Leu Leu Gly Arg Gln Asp Gly His Ala Ser Gly Pro Ser Thr Trp
   1145 1150 1155
   Ile Pro Arg Gly Ile Glu Arg Val Ser Val Ser Gly Thr Val Pro
   1160 1165 1170
   Ala Arg Glu Ala Leu Trp Val Arg Ala Ser Ala Arg Val Asp Ala
   1175 1180 1185
   Asp Gly Ala Thr Ala Arg Gly Asp Leu Gln Val Leu Asp Asp Ala
   1190 1195 1200
   Gly Arg Val Leu Val Glu Thr Arg Gly Leu Thr Leu Leu Arg Arg
   1205 1210 1215
   Asp Ala Asp Asn Leu Asp Asp Val Arg Gln Trp Phe Phe Arg Pro
   1220 1225 1230
   Val Leu Arg Glu Ala Pro Leu Arg Glu Glu Ala Pro Ala Asp Gly
   1235 1240 1245
   Thr Trp Leu Val Leu Gly Ala Pro Gly Pro Leu His Asp Arg Val
   1250 1255 1260
   Val Ala Glu Leu Arg Gly Arg Gly Leu Asp Thr Val Gln Ala Phe
   1265 1270 1275
   Pro Ala Asn Ser Trp Glu Glu Gln Asp Ala Ala Arg Phe His Leu
   1280 1285 1290
   Ser Pro Ala Asp Pro Glu His Leu Arg Arg Leu Met Glu Arg Val
   1295 1300 1305
   Ser Ala Asp Arg Pro Leu Arg Gly Leu Ile Asn Leu Gln Pro Tyr
   1310 1315 1320
   Gln Val Ala Arg Gly Asp Ala Ala Ser Ser Ala Pro Glu Gly Val
   1325 1330 1335
   Leu Ser Ala Leu Tyr Met Glu Val Leu Asp Gln Ala Arg Ala Leu
   1340 1345 1350
   Gln Gly Val Ser Arg Arg Glu Arg Pro Lys Leu Val Leu Val Ser
   1355 1360 1365
   Gly Val Ser Gln Gly Ala Glu Ala Glu Pro Asp Leu Val Ala Ala
   1370 1375 1380
   Pro Leu Gln Gly Leu Gly Arg Val Ile Ala Ala Glu Leu Pro Arg
   1385 1390 1395
   Leu His Cys Val Gln Val Gln Leu Gly Gly Gln Glu Pro Glu Val
   1400 1405 1410
   Glu Ala Arg Asp Leu Val Arg Glu Leu Tyr Ala Asn Asp Gly Glu
   1415 1420 1425
   Asp Glu Val Val Leu Arg Gly Gly Ala Arg His Val Arg Arg Leu
   1430 1435 1440
   Glu Arg Leu Val Pro Gly Ala Ala Pro Ala Ala Pro Ile His Ala
   1445 1450 1455
   Asp Gly Thr Tyr Leu Leu Thr Gly Gly Leu Gly Gly Leu Gly Leu
   1460 1465 1470
   Ala Phe Ala Ser Trp Leu Ala Asp Arg Gly Ala Arg His Leu Ala
   1475 1480 1485
   Leu Val Ser Arg Arg Pro Pro Ser Glu Glu Ala Arg Ala Arg Leu
   1490 1495 1500
   Asp Ala Leu Arg Ala Arg Gly Val Glu Val Leu Val Arg Glu Val
   1505 1510 1515
   Asp Leu Ser Arg Glu Asp Ser Val Ala Ala Leu Phe Arg Glu Leu
   1520 1525 1530
   Asp Glu Arg Met Pro Pro Leu Arg Gly Val Leu His Leu Ala Gly
   1535 1540 1545
   Val Leu Asp Asp Ala Thr Leu Phe Gln Gln Asn Glu Gln Arg Leu
   1550 1555 1560
   Ala Ala Val Arg Gly Pro Lys Val Thr Gly Thr Arg Leu Leu His
   1565 1570 1575
   Ala His Thr Gln His Arg Lys Leu Asp Phe Phe Val Leu Phe Ser
   1580 1585 1590
   Ala Gly Ala Thr Leu Leu Gly Ser Pro Gly Gln Ala Asn Tyr Ala
   1595 1600 1605
   Ala Val Asn Ala Phe Met Asp Ala Phe Ala Pro His Arg Gln Arg
   1610 1615 1620
   Leu Gly Leu Pro Ala Leu Ser Ile Asn Trp Gly Val Trp Asp Glu
   1625 1630 1635
   Val Gly Leu Ala Ala Ala Ser Ala Gln Arg Gly Gln Arg Leu Arg
   1640 1645 1650
   Glu Gln Gly Leu Phe Pro Ile Pro Pro Ala Gln Gly Leu Gln Ala
   1655 1660 1665
   Phe Glu Leu Ala Leu Ser Leu Ala Gly Ala Asn Val Ala Ile Leu
   1670 1675 1680
   Pro Ile Arg Leu Ala Arg Trp Leu Arg Glu His Pro Asp Leu Ala
   1685 1690 1695
   Gly Ala Ser Leu Phe Lys Glu Leu Val Arg Gly Leu Asp Asp Ser
   1700 1705 1710
   Glu Ala Glu Ala Glu Ala Pro Ile Leu Glu Gln Leu Arg Ala Thr
   1715 1720 1725
   Val Asp Pro His Glu Gln Arg Glu Cys Leu Arg Arg Trp Val Gly
   1730 1735 1740
   Arg Gln Leu Ala Ala Val Leu Arg Arg Asp Pro Ala Ser Met Gln
   1745 1750 1755
   Arg Glu Gln Asn Phe Ser Ala Met Gly Val Asp Ser Leu Met Ala
   1760 1765 1770
   Leu Glu Phe Arg Asn Arg Leu Glu Arg Ser Leu His Val Arg Leu
   1775 1780 1785
   Pro Pro Thr Met Val Phe Asn His Pro Ser Ile Glu Ala Leu Cys
   1790 1795 1800
   Gly Phe Leu Glu Pro Lys Leu Gly Met Gly Ala Pro Ser Ala Pro
   1805 1810 1815
   Ala Gln Pro Glu Pro Val Pro Ala Pro Ala Ala Pro Val Arg Val
   1820 1825 1830
   Asp Glu Lys Asp Gly Ala Ala Leu Leu Gly Ala Leu Ala Arg Leu
   1835 1840 1845
   Lys Lys Leu Thr Gln Gln Gln Ala
   1850 1855
<210> 38
   <211> 1561
   <212> PRT
   <213> Pyxidicoccus fallax
<400> 38
   Met Leu Asp Glu Leu Arg Ala Val Leu Asp Ser Leu Ser Pro Glu Gln
   1 5 10 15
   Arg Ala Thr Leu Ala Glu Leu Leu Ser Ala Asp Ala Pro Arg Lys Ala
   20 25 30
   Asp Glu Pro Ile Ala Ile Val Gly Met Ala Gly Arg Phe Pro Gly Ala
   35 40 45
   Arg Asp Leu Ala Glu Phe Trp Ser Val Leu Ala Glu Gly Arg Asp Ala
   50 55 60
   Val Ser Glu Ile Pro Pro Asp Arg Trp Asn Ala Asp Ala Leu Tyr Asp
   65 70 75 80
   Pro Asp Lys Asp Val Pro Gly Lys Leu Ala Thr Arg Trp Gly Gly Phe
   85 90 95
   Val Lys Asp Val Asp Leu Phe Asp Ala Glu Phe Phe Gly Ile Thr Pro
   100 105 110
   Arg Glu Ala Ala Ala Met Asp Pro Gln Gln Arg Leu Phe Leu Glu Thr
   115 120 125
   Ala Trp Glu Ala Leu Glu Asp Ala Gly Leu Pro Ala Ala Ser Leu Ala
   130 135 140
   Arg Thr Gln Thr Gly Val Phe Val Gly Val Ser Asn Ser Asp Tyr Met
   145 150 155 160
   Trp His Gln Phe Pro Arg Leu Asp Lys Val Asp Ala Tyr Ala Ser Ser
   165 170 175
   Gly Ser Ala His Cys Ile Ile Ala Asn Arg Leu Ser Tyr Leu Leu Asp
   180 185 190
   Leu Thr Gly Pro Ser Val Ala Val Asp Thr Ala Cys Ser Ser Ser Leu
   195 200 205
   Val Ser Val His Leu Ala Cys Gln Ser Leu Arg Asn Arg Glu Cys Glu
   210 215 220
   Ala Ala Ile Ala Gly Gly Val Asn Ala Val Leu Thr Pro His Leu Gly
   225                 230                 235                 240
   Val Ser Leu Ser Lys Trp Gly Met Met Ala Ser Asp Gly Arg Cys Lys
                   245                 250                 255
   Thr Phe Asp Ala Arg Ala Asp Gly Phe Val Arg Gly Glu Gly Ser Gly
               260                 265                 270
   Val Leu Val Leu Lys Arg Leu Ala Asp Ala Glu Lys Ala Gly Asp Arg
           275                 280                 285
   Val Leu Ala Val Ile Leu Gly Ser Ala Val Asn Gln Asp Gly Lys Thr
       290                 295                 300
   His Gly Leu Thr Ala Pro Asn Gly Ala Ala Gln Gln Ala Val Ile Arg
   305                 310                 315                 320
   Gln Ala Leu Ala Arg Ala Gly Leu Ser Pro Ser Gln Val Ser Phe Leu
                   325                 330                 335
   Glu Thr His Gly Thr Gly Thr Ser Leu Gly Asp Pro Ile Glu Val Glu
               340                 345                 350
   Ala Ile Asn Glu Val Phe Gly Ser Arg Val Glu Arg Cys Val Leu Gly
           355                 360                 365
   Ala Val Lys Thr Asn Ile Gly His Leu Glu Pro Ala Ala Gly Ile Ala
       370                 375                 380
   Gly Leu Ile Lys Val Val Leu Ala Leu Arg His Gly Leu Ile Pro Arg
   385                 390                 395                 400
   Asn Leu His Tyr Gln Ser Met Asn Pro His Leu Gln Leu Ala Pro Gly
                   405                 410                 415
   Ile Glu Leu Ala Asn Glu Ala Arg Pro Trp Lys Arg Asp Ala Arg Pro
               420                 425                 430
   Arg Phe Ala Gly Val Ser Ala Phe Ser Phe Gly Gly Thr Asn Ala His
           435                 440                 445
   Ile Val Leu Gly Asp Ala Pro Ala Tyr Ala Pro Pro Ala Glu Ser Ala
       450                 455                 460
   Arg Pro Ala Gln Pro Thr Leu Leu Pro Leu Ser Ala Ser Gln Glu Gly
   465                 470                 475                 480
   Ala Arg Ser Gln Met Ala Ala Leu His Ala Glu Leu Leu Ala Thr Ala
                   485                 490                 495
   Ala Pro Glu Leu Thr Val Asp Thr Val Ala Gly Leu Arg Ala Leu His
   500 505 510
   Arg Asp His Leu Asp Leu Arg Leu Ala Val Val Ala Ser Asp Arg Ala
   515 520 525
   Thr Ala Ala Glu Ala Leu Lys Cys Phe Ala Glu Gly Arg Pro His Ala
   530 535 540
   Gly Leu Val His Gly Arg Arg Gln Pro Asn Leu Arg Pro Val Phe Ile
   545 550 555 560
   Tyr Thr Gly Gln Gly Asn Gln Trp Pro Gly Met Gly Arg Glu Leu Met
   565 570 575
   Arg Thr Glu Pro Val Phe Arg Ala Ala Val Glu Glu Ala Asp Ala Ala
   580 585 590
   Phe Gln Arg Leu Thr Gly Arg Ser His Ala Ala Ser Phe Arg Glu Asp
   595 600 605
   Ala Ser Asp Ala Ala Ser Leu Glu Arg Thr Asp Ala Ala Gln Pro Ala
   610 615 620
   Ile Phe Leu Leu Gln Tyr Ala Leu Thr Lys Thr Leu Glu His Trp Gly
   625 630 635 640
   Ile Arg Pro Gly Ala Val Ala Gly His Ser Val Gly Glu Val Ala Ala
   645 650 655
   Ala Trp Ala Ala Gly Met Leu Asp Leu Asp Asp Ala Val Arg Val Val
   660 665 670
   His His Arg Ser Arg Leu Met Gln Arg Ala Thr Gly Gln Gly Ala Met
   675 680 685
   Ala Ser Val Ala Leu Ser Pro Arg Glu Ala Arg Pro Leu Leu Glu Gly
   690 695 700
   Arg Pro Ser Leu Ala Ile Ala Ala His Asn Ser Pro Gly Glu Val Val
   705 710 715 720
   Leu Ser Gly Asp Pro Ala Glu Leu Glu Ala Val Leu Arg Glu Leu Glu
   725 730 735
   Gly Arg Gly Val Arg Cys Arg Lys Leu Pro Val Asn Tyr Ala Phe His
   740 745 750
   Gly Pro Gln Met Asp Pro Leu Arg Asp Glu Leu Leu Arg Glu Leu Ser
   755 760 765
   Val Leu Arg Pro Lys Ala Pro Arg Leu Ala Phe Tyr Ser Thr Val Thr
       770                 775                 780
   Gly Gln Arg Glu Arg Gln Ala Val Ala Asp Ala Ser His Trp Val Arg
   785                 790                 795                 800
   Asn Met Arg Asp Pro Val Ala Phe Arg Glu Val Val Glu Arg Leu Ala
                   805                 810                 815
   Thr Asp Ser Pro Gly Phe Phe Leu Glu Leu Gly Pro His Pro Ala Leu
               820                 825                 830
   Val Pro Ser Val Met Asp Thr Leu Ala His Leu Arg Leu Glu Ala Arg
           835                 840                 845
   Thr Ala His Cys Leu Arg Arg Asn Arg Pro Asp Arg His Glu Leu Leu
       850                 855                 860
   Ser Ala Val Ala Ser Leu Tyr Val His Gly Ile Asp Pro Asp Trp Ser
   865                 870                 875                 880
   Arg Leu Val Arg Pro Pro Asp Arg Arg Trp Ala Leu Pro Ala Tyr Pro
                   885                 890                 895
   Trp Gln Arg Gln Arg Phe Trp Leu Glu Ala Ala Ala Ser Pro Asp Ala
               900                 905                 910
   Thr Trp Ser Ala Pro Ser Ala Arg Pro Ala Gly His Leu Val Tyr Arg
           915                 920                 925
   Thr Glu Trp Leu Ala Asp Ala Ala Ser Thr Ser Gly Asp Thr Ala Arg
       930                 935                 940
   Pro Lys Gly His Leu Val Phe Ser Asp Gly Ser Ala Leu Ala Glu Ala
   945                 950                 955                 960
   Leu Arg Ala Glu Leu Glu Ala Ala Gly Ser Arg Phe Gly Trp Val Ser
                   965                 970                 975
   Arg Gly Gln Asp Phe Ala Ala Asp Gly Thr Arg Cys Thr Val Asp Pro
               980                 985                 990
   Glu Asn Pro Glu His Leu Glu Arg Leu Phe Gly His Leu Ala Ala Glu
           995                 1000                1005
   Ser Phe Asp Phe Asp His Val Leu Leu Leu Phe Thr Arg Val Glu
       1010                1015                1020
   Asp Glu Ala Arg Pro Asp Gly Ala Ala Pro Trp Phe Asp Thr Ala
       1025                1030                1035
   Val Arg Pro Val His Val Leu Arg Cys Leu Thr Gln Ala Leu Leu
   1040 1045 1050
   Thr Thr Glu Arg Arg Pro Arg Met Ala Val Ile Thr Arg Ala Ala
   1055 1060 1065
   Leu Ser Ala Gly Gly Gly Thr Glu Ala Val Pro Gln His Thr Ala
   1070 1075 1080
   Leu Trp Gly Phe Gly Arg Ala Leu Ala Leu Glu Glu Pro Gly Leu
   1085 1090 1095
   Ser Leu Ala Leu Leu Asp Leu Pro Ala Val Pro Asp Gly Leu Val
   1100 1105 1110
   Glu Ala Arg His Val Leu Ala Arg Leu Ala Ser Pro Thr Pro Glu
   1115 1120 1125
   Arg Gln Ser Ala Trp Arg Asp Gly Arg Trp Arg Val Pro Arg Leu
   1130 1135 1140
   Met Pro His Glu Ala Gly Thr Gly Thr Ser Ala Pro Pro Val Ile
   1145 1150 1155
   Gln Gly Glu Gly Thr Tyr Leu Ile Thr Gly Gly Leu Gly Ser Leu
   1160 1165 1170
   Gly Leu Ala Thr Ala Arg Arg Leu Val Gln Arg Gly Ala Arg Ser
   1175 1180 1185
   Val Ala Leu Val Ser Arg Arg Pro Pro Thr Ala Ala Gln Gln Ala
   1190 1195 1200
   Glu Leu Ala Arg Leu Glu Glu Ala Gly Ala Arg Val Val Ala Val
   1205 1210 1215
   Gln Gly Asp Leu Ala Asp Leu Ala Glu Ala Thr Arg Val Phe Glu
   1220 1225 1230
   Thr Leu Ala Ala Ser Ala Pro Pro Val Arg Gly Val Ile His Ala
   1235 1240 1245
   Ala Gly Val Leu Glu Asp Gly Val Leu Gly Ser Ala Thr Lys Ala
   1250 1255 1260
   Ser Phe Asp Arg Val Leu Arg Pro Lys Val Ala Gly Ser Trp Asn
   1265 1270 1275
   Leu His Leu Leu Thr Gln Ala Leu Pro Leu Asp Phe Phe Val Leu
   1280 1285 1290
   Phe Ser Ser Ala Thr Ala Thr Leu Gly Ala Ala Gly Gln Thr Asn
   1295 1300 1305
   Tyr Ala Ala Ala Asn Ala Phe Leu Gln Gly Leu Ala Glu His Arg
   1310 1315 1320
   Arg Ala Arg Gly Gln His Ala Leu Cys Leu His Trp Gly Ala Trp
   1325 1330 1335
   Arg Glu Ser Thr Met Ala Ser Gly Ala Val Val Thr Lys Leu Lys
   1340 1345 1350
   Ala Leu Gly Phe Asp Thr Met Ala Pro Glu Asp Ala Leu Asp Ala
   1355 1360 1365
   Leu Glu Arg Val Leu Leu Glu Gly Ala Thr Glu Ala Thr Ile Ile
   1370 1375 1380
   Ala Ala Asp Trp Glu Val Phe Ala Arg Arg Leu Pro Asp Glu Gly
   1385 1390 1395
   Leu Gly Arg Phe Leu Glu Pro Val Arg Arg Ala Ser Gly Ala Pro
   1400 1405 1410
   Pro Ala Val Lys Pro Gly Val Pro Glu Ala Asp Pro Ile Val Ser
   1415 1420 1425
   Arg Leu Ala Gly Ala Pro Ala Ser Gln Arg Gln Lys Leu Leu Tyr
   1430 1435 1440
   Gly His Val Arg Ala Val Val Leu Ser Thr Met Gly Met Asp Ala
   1445 1450 1455
   Arg Thr Glu Val Arg Pro Asp Gln Gly Leu Ile Glu Leu Gly Leu
   1460 1465 1470
   Asp Ser Leu Leu Ala Val Asn Ile Ala Asn Arg Leu Ser Arg Asp
   1475 1480 1485
   Leu Ala Arg Gln Leu Pro Lys Thr Leu Ile Tyr Asp His Pro Thr
   1490 1495 1500
   Leu His Ala Leu Thr Asp His Leu Leu Gly Val Leu Phe Pro Glu
   1505 1510 1515
   Pro Ala Gln Ala Ser Val Arg Pro Ala Ala Ser Gly Asp Ala Pro
   1520 1525 1530
   Ser Tyr Glu Asp Ile Ala Asn Ala Ser Ala Asp Thr Ile Gln Ser
   1535 1540 1545
   Leu Leu Asp Ala Glu Leu Ala Ser Leu Leu Ser Ser Arg
   1550 1555 1560
<210> 39
   <211> 1104
   <212> PRT
   <213> Pyxidicoccus fallax
<400> 39
   Val Arg Arg Thr Arg Ser Pro Arg Gly Ser Ala Pro Ala Arg Pro Glu
   1               5                   10                  15
   Arg Leu Leu Arg Pro Glu Ser Thr Ser Ala Val Asp Thr Asn Ala Leu
               20                  25                  30
   Leu Ala Gln Asn Lys Lys Ala Leu Leu Ala Ile Met Glu Leu Lys Ala
           35                  40                  45
   Gln Leu Ala Arg Ala Glu Ser Arg Gln Arg Ala Pro Ile Ala Ile Val
       50                  55                  60
   Gly Met Ala Cys Arg Phe Pro Gly Gly Cys Ala Thr Pro Glu Gln Phe
   65                  70                  75                  80
   Trp Glu Leu Leu Arg Glu Gly Arg Asp Ala Val Thr Glu Ala Phe Pro
                   85                  90                  95
   Ser Arg Trp Pro Glu Ser Leu Tyr Asp Ala Asn Pro Glu Ala Pro Gly
               100                 105                 110
   Arg Ile Met Ala Arg Gly Leu Gly Gln Val Glu Gly Val Ala Asp Phe
           115                 120                 125
   Glu Pro Glu Phe Phe His Val Ser Pro Arg Glu Ala Ala Ser Met Asp
       130                 135                 140
   Pro Gln His Arg Leu Leu Leu Thr Val Ala Trp Glu Ala Cys Glu Asp
   145                 150                 155                 160
   Ala Gly Leu Thr Arg Glu Leu Leu His Gly Ser Arg Thr Gly Ile Phe
                   165                 170                 175
   Val Gly Ala Gly Asn Asn Asp Tyr Gly Asp Phe Glu Leu Arg Asn Pro
               180                 185                 190
   Asp Leu Gly Ala Val Ser Ala Tyr Ala Gly Thr Gly Asn Ala Thr Ser
           195                 200                 205
   Leu Val Ala Gly Arg Leu Ser Phe Phe Leu Gly Thr Gln Gly Pro Ala
       210                 215                 220
   Leu Val Val Asp Thr Ala Cys Ser Ser Ser Leu Val Ala Ile His Gln
   225                 230                 235                 240
   Ala Cys Thr Ser Leu Arg Leu Gly Glu Ser Glu Leu Ala Leu Ala Gly
                   245                 250                 255
   Gly Val Asn Leu Val Leu Arg Pro Asn Gly Ser Val Phe Leu Ser Arg
               260                 265                 270
   Phe Gly Ala Leu Ser Pro Thr Gly Arg Cys Arg Ala Phe Ala Glu Ala
           275                 280                 285
   Ala Asp Gly Tyr Val Arg Ala Asp Gly Cys Gly Val Ile Val Leu Lys
       290                 295                 300
   Arg Leu Glu Asp Ala Leu Arg Asp Gly Asp Arg Val Leu Ala Val Ile
   305                 310                 315                 320
   Arg Gly Ser Ala Ile Asn His Asp Gly Arg Ser Gly Gly Leu Thr Val
                   325                 330                 335
   Pro Asn Gly Ala Ala Gln Arg Glu Leu Leu Leu Ala Ala Leu Lys Asn
               340                 345                 350
   Ala Gly Val Glu Pro Arg Ser Val Gly Tyr Leu Glu Ala His Gly Thr
           355                 360                 365
   Gly Thr Pro Leu Gly Asp Pro Ile Glu Met Gly Ala Ile Ala Asp Val
       370                 375                 380
   Phe Gly Gly Asp Ala Arg Arg Glu Ala Leu Arg Val Gly Ser Val Lys
   385                 390                 395                 400
   Ser Asn Ile Gly His Thr Glu Ala Ala Ala Gly Ile Ala Gly Ile Ile
                   405                 410                 415
   Lys Ala Val Leu Ser Leu Gln His Arg Glu Ile Pro Ala His Leu His
               420                 425                 430
   Phe Asp Lys Pro Ser Ser Arg Ile Asp Trp Ser Ile Pro Val Glu Val
           435                 440                 445
   Pro Thr Ser Thr Arg Pro Trp Ser Gly Asp Gly Pro Arg Leu Ala Gly
       450                 455                 460
   Val Ser Ser Phe Gly Phe Ser Gly Thr Asn Ala His Val Ile Leu Glu
   465                 470                 475                 480
   Glu Ala Pro Arg Gln Glu Ala Arg His Ser Pro Arg Leu Gly Thr Gln
                   485                 490                 495
   Val Leu Cys Leu Ser Ala Arg Thr Glu Gly Ser Leu Thr Arg Leu Ala
   500 505 510
   Ala Ala Trp Arg Glu Gln Leu Arg Arg Arg Asp Ser Ala Pro Leu Ala
   515 520 525
   Asp Leu Cys His Ala Ala Ala Arg Arg Arg Ser His His Arg Ala Arg
   530 535 540
   Leu Ala Val Leu Gly Asp Ser Ala Asp Ala Ile Ala Gln Ala Leu Glu
   545 550 555 560
   Ser Phe Ala Ala Gly Ala Pro His Pro Ala Val Val Arg Gly Asp Ala
   565 570 575
   Val Leu Asp Gly Met Ser Arg Thr Ala Phe Val Phe Ser Gly Gln Gly
   580 585 590
   Ser Gln Trp Leu Gly Met Gly Gln Glu Leu Leu Ala Ser Glu Pro Leu
   595 600 605
   Phe Gln Gln Ser Leu Arg Glu Met Asp Gly Pro Phe Arg Asp Glu Phe
   610 615 620
   Gly Trp Ser Leu Glu Glu Ala Leu Arg Ala Gly Glu Ala Glu Ser Gln
   625 630 635 640
   Leu Glu Arg Thr Arg Val Ala Gln Pro Val Ile Val Ala Val Gln Leu
   645 650 655
   Ala Leu Ser Asp Leu Trp Arg Ala Trp Gly Val His Pro Asp Val Thr
   660 665 670
   Leu Gly His Ser Leu Gly Glu Ile Ser Ala Ala His Val Ala Gly Val
   675 680 685
   Leu Gly Arg His Glu Ala Leu Arg Leu Ala Cys Ile Arg Gly Arg Phe
   690 695 700
   Met Asp Gly Ala Thr Gly Arg Gly Lys Met Ala Glu Leu Ala Cys Ser
   705 710 715 720
   Glu Glu Gln Ala Arg Arg Tyr Leu Gln Gly Leu Glu Ala Glu Val Ala
   725 730 735
   Ile Ala Ala Leu Asn Gly Ala Met Ser Thr Val Leu Ala Gly Ala Pro
   740 745 750
   Gly Ala Leu Glu Ser Val Leu Gln Lys Val Ala Ser Asp Gly Ile Gln
   755 760 765
   His Arg Leu Leu Arg Val Asp Tyr Ala Phe His Ser Pro Gln Met Ala
       770                 775                 780
   Glu Phe Ala Ala Pro Phe Arg Gly Ala Leu Gly Asp Met Lys Pro Arg
   785                 790                 795                 800
   Ala Ala Glu Arg Asp Leu Tyr Ser Ser Val Leu Gly Arg Ala Val Arg
                   805                 810                 815
   Gly Glu Ala Leu Thr Ala Asp Tyr Trp Val Arg Asn Met Thr Asp Thr
               820                 825                 830
   Val Arg Phe His Glu Ala Leu Val Ala Leu Leu Arg Asp Gly Cys Arg
           835                 840                 845
   Ala Val Val Glu Val Ser Pro His Pro Val Leu Leu Ser Ala Ile Gln
       850                 855                 860
   Asp Thr Leu Ser Arg Glu Gly Val Asp Gly Val Ala Ala Tyr Ser Leu
   865                 870                 875                 880
   Arg Arg Gly Asn Pro Glu Arg Ala Thr Leu Leu Ser Asn Leu Gly Ala
                   885                 890                 895
   Leu Tyr Ala Ala Gly His Pro Val Asp Trp Ser Ala Leu Tyr Pro Gly
               900                 905                 910
   Leu Val Asp His Val Ala Leu Pro Ala Tyr Pro Trp His Ala Arg Arg
           915                 920                 925
   Phe Trp Thr Thr Val Gln Gly Pro Glu Ala Thr Thr Gly Ala Ala Pro
       930                 935                 940
   Ala Val Thr Ala Pro Ala Pro Asp Glu Ala Ala Pro Ala Ala Val Thr
   945                 950                 955                 960
   Ser Lys Val Trp Asp Glu Leu Val Lys Leu Ala Ala Val Ala Arg Arg
                   965                 970                 975
   Pro Arg Leu Glu Arg His Val Arg Glu Gln Leu Thr Gly Ile Leu Lys
               980                 985                 990
   Leu Asp Ser Gly Asp Ile Pro Ser  Lys Arg Thr Pro Leu  Ser Glu Leu
           995                 1000                1005
   Gly Met  Asp Ser Val Gly Ala  Val Ala Leu Ser Lys  Ala Leu Ala
       1010                1015                1020
   Leu Thr  His Glu His Arg Phe  Pro Pro Thr Val Leu  Phe Glu His
       1025                1030                1035
   Pro Thr Ile Glu Ala Leu Thr Gly Phe Leu Ala Glu Glu Phe Glu
   1040 1045 1050
   Arg Leu His Ala Ala Arg Gly Gly Ala Ala Ala Pro Gly Asp Ser
   1055 1060 1065
   Ala Pro Pro Arg Gln Ala Pro Ala Ala Pro Glu Ala Ala Ala Leu
   1070 1075 1080
   Pro Val Pro Asp Ser Glu Leu Ala Ile Leu Glu Lys Leu Ala Ala
   1085 1090 1095
   Leu Glu Lys Glu Phe Ala
   1100
<210> 40
   <211> 2416
   <212> PRT
   <213> Pyxidicoccus fallax
<400> 40
   Met Ser Thr Pro Glu Asn Pro Thr Gly Asp Arg Ala Leu Leu His Arg
   1 5 10 15
   Ala Leu Ala Ala Met Glu Lys Met Lys Gly Arg Ile Gln Gln Leu Glu
   20 25 30
   Gly Val Thr Thr Glu Pro Ile Ala Ile Ile Gly Met Gly Cys Arg Phe
   35 40 45
   Pro Gly Gly Val His Gly Pro Asp Ala Phe Trp Arg Phe Leu Ala Asp
   50 55 60
   Gly Val Asn Gly Ile Arg Asp Thr Pro Lys Asp Arg Trp Asp Thr Glu
   65 70 75 80
   Arg Tyr Leu Asp Ala Glu Gln Gly Thr Pro Gly Lys Met Tyr Thr Gly
   85 90 95
   His Gly Gly Phe Leu Asp Arg Val Asp Gly Phe Asp Ala Ala Phe Phe
   100 105 110
   Gly Ile Ser Pro Arg Glu Ala Leu Ser Met Asp Pro Gln Gln Arg Leu
   115 120 125
   Phe Leu Glu Thr Cys Trp Glu Ala Ile Glu Asn Ala Gly Leu Ser Ala
   130 135 140
   Asp Val Leu Glu Gly Ser Gln Thr Gly Val Tyr Ala Gly Ile Val Asn
   145 150 155 160
   Gln Asp Tyr Ala Met Met Gln Met Lys Asp Thr Ala His Gly Glu Met
   165 170 175
   Asp Thr Tyr Phe Ala Thr Gly His Pro His Cys Val Val Ser Gly Arg
   180 185 190
   Val Ser Tyr Gln Phe Gly Leu Arg Gly Pro Thr Leu Ser Val Asp Thr
   195 200 205
   Ala Cys Ser Ser Ser Leu Val Ala Leu His Leu Ala Cys Gln Ser Leu
   210 215 220
   Arg Leu Gly Ser Cys Asp Val Ala Leu Ala Gly Gly Ile Asn Leu Leu
   225 230 235 240
   Leu Met Pro Glu Ile Tyr Met Ile Arg Cys Gln Ala Arg Met Leu Ser
   245 250 255
   Pro Ser Gly Arg Cys Ala Thr Phe Asp Ala Ser Ala Asp Gly Tyr Ile
   260 265 270
   Pro Gly Glu Gly Val Gly Val Val Val Leu Lys Arg Leu Arg Asp Ala
   275 280 285
   Val Arg Asp Gly Asp Arg Val Asp Ala Val Ile Arg Gly Thr Gln Ile
   290 295 300
   Asn His Asp Gly Arg Ser Ser Gly Leu Thr Val Pro Ser Gly Ala Ala
   305 310 315 320
   Gln Arg Glu Leu Leu Arg Ala Thr Leu Arg Asp Ala Arg Leu Glu Pro
   325 330 335
   Gln Arg Val Gly Tyr Leu Glu Ala His Gly Thr Gly Thr Lys Leu Gly
   340 345 350
   Asp Pro Ile Glu Met Ala Ala Ile Gln Asp Val Tyr Cys Gln Asp Arg
   355 360 365
   Ala Pro Gly Asn Pro Leu Val Ile Gly Ser Val Lys Thr Asn Ile Gly
   370 375 380
   His Leu Glu Ser Ala Ala Gly Ile Ser Gly Leu Ile Lys Ala Val Leu
   385 390 395 400
   Thr Leu Lys His Gly Ala Ile Pro Arg Asn Leu His Phe Ser Glu Leu
   405 410 415
   Asn Pro His Ile Thr Leu His Pro Ala Thr Arg Ile Pro Thr Glu Leu
   420 425 430
   Thr Pro Trp Pro Ala Gly Ser Gly Pro Arg Val Ala Ala Val Ser Ser
   435 440 445
   Phe Gly Met Ser Gly Thr Asn Ala His Ala Ile Leu Glu Glu Ala Pro
   450 455 460
   Ala Arg Glu Ala Ala Ser Thr Glu Ala Pro Leu Arg Val Thr Ser Arg
   465 470 475 480
   Pro Ala Thr Pro Arg Leu Val Pro Leu Ser Ala His His Pro Glu Val
   485 490 495
   Leu Gln Ala His Ala Ala Gly Leu Lys Glu His Leu Asp Ala His Pro
   500 505 510
   Asp Val Thr Phe Asp Asp Leu Ala His Thr Leu Arg Arg Arg Thr Leu
   515 520 525
   Gln Arg His Arg Leu Ala Leu Pro Ala Ser Ser Leu Ala Glu Val Lys
   530 535 540
   Glu His Leu Ala Ala Phe Ala Ala Gly Gly Gln Asp Pro Arg Val Leu
   545 550 555 560
   Gln Thr Glu Asp Leu Phe Phe Thr Asp Pro Lys Val Val Phe Val Phe
   565 570 575
   Pro Gly Gln Gly Ser Gln Trp Val Arg Met Gly Leu Gln Leu Met Glu
   580 585 590
   Gln Glu Pro Glu Phe Ala Ala Glu Leu Arg Glu Val Asp Ala Trp Ile
   595 600 605
   Gln Lys Tyr Ala Gly Trp Ser Leu Leu Asp Glu Leu Arg Ala Asp Ala
   610 615 620
   Ser Ala Ser Arg Leu Ser Gln Val Asp Val Val Gln Pro Cys Ile Phe
   625 630 635 640
   Ala Val Gln Val Ala Leu Ala Arg Leu Trp Arg Ser Trp Gly Val Val
   645 650 655
   Pro Asn Ala Val Ile Gly His Ser Met Gly Glu Val Ala Ala Ala Cys
   660 665 670
   Val Ala Gly Ile Leu Ser Leu Glu Asp Ala Val Cys Val Ile Thr Arg
   675 680 685
   Arg Ser Gln Ile Ala Lys Arg Thr Ser Gly Asn Gly Ala Met Ala Leu
   690 695 700
   Val Glu Leu Thr Arg Glu Gln Ala Glu Arg Ala Ile Ala Ala His Gln
   705 710 715 720
   Asp Val Val Ala Val Ala Ala Gln Asn Gly Pro His Thr Val Leu Leu
   725 730 735
   Ser Gly Thr Pro Gly Pro Ile Glu Gln Ile Leu Arg Glu Leu Gln Gln
   740 745 750
   Gln Asp Val Phe Cys Arg Ala Ile Lys Val Asp Tyr Ala Ser His Ser
   755 760 765
   Pro Gln Met Asp Pro Leu Arg Pro Glu Leu Gln Arg Ala Leu Ser Gly
   770 775 780
   Val Lys Pro Arg Ala Ala Thr Val Ala Phe Tyr Ser Thr Val Thr Gly
   785 790 795 800
   Gln Val Glu Asp Gly Thr Ala His Asp Ala Asn Tyr Trp Val Asn Asn
   805 810 815
   Leu Arg Asp Pro Val Leu Phe Trp Pro Gly Met Gln Lys Leu Val Glu
   820 825 830
   Asp Gly His Asn Ile Phe Leu Glu Leu Ser Pro His Pro Val Leu Ile
   835 840 845
   Pro Ala Ile Ser Gln Gly Leu Gln His Leu Gly Ala Arg Ala Leu Ser
   850 855 860
   Leu Pro Ser Leu His Arg Glu Lys Pro Glu Ala Glu Cys Leu Phe Glu
   865 870 875 880
   Ser Arg Ala Arg Leu Phe Cys Ala Gly Gly Arg Leu Asp Leu Ala Lys
   885 890 895
   Glu Asp Val Pro Gly Ala Arg Val Leu Ala Leu Pro Gly His Pro Trp
   900 905 910
   Arg Glu Gln Arg Phe Trp Tyr Thr Pro Ser Ala Pro Ser Ala His Pro
   915 920 925
   Gln Ala Arg Gly Gly Ser Arg Leu Leu Pro Arg Met Trp Thr Ala Gly
   930 935 940
   Ser Gly Glu Arg Arg Val Phe Glu Gly Asp Val Gly Pro Gly Val Met
   945 950 955 960
   Pro Ser Leu Ala Asp His Arg Val Asn Gly Met Gln Leu Phe Pro Gly
   965 970 975
   Ala Gly Tyr Val Glu Leu Val Leu Glu Ala Ala Glu Ala Leu Gly Leu
   980 985 990
   Ala Gly Arg Thr His Ile Asp Ser Leu Asp Ile Arg Glu Gly Leu Val
   995 1000 1005
   Leu His Glu Gly Ala Ala Arg Val Val Gln Val Glu Leu Thr Pro
   1010 1015 1020
   Val Ala Ala Arg Ser Phe Gly Phe Val Ile Ser Ser Arg Glu Ala
   1025 1030 1035
   Asp Gly Ala Ala Trp Thr Arg His Ala Asp Gly Val Val Thr Arg
   1040 1045 1050
   Glu Glu Ala Gly Ala Ala Pro Gly Ala Glu Pro Gly Ala Leu Glu
   1055 1060 1065
   Ala Leu Arg Ala Arg Leu Ala Ser Pro Leu Ser Ala Glu Asp His
   1070 1075 1080
   Tyr Leu Arg Thr Arg Glu Arg Gly Leu Glu Tyr Gly Pro Ala Tyr
   1085 1090 1095
   Gln Ala Ile Lys His Ile Gly Arg Gly Arg Gly Glu Ala Leu Ala
   1100 1105 1110
   Glu Leu His Val Pro Gln Ala Val Ala Glu Glu Leu Ser Gly Cys
   1115 1120 1125
   Gln Leu His Pro Ser Leu Leu Asp Ala Gly Phe Gln Leu Ile Asp
   1130 1135 1140
   Thr Ala Leu Ala Gly Ser Gly Leu Val Glu Glu Gly Asp Val Tyr
   1145 1150 1155
   Leu Pro Ala Ala Leu Arg Gly Val Arg Phe His Arg Lys Pro Ala
   1160 1165 1170
   Pro Gly Gln Thr Leu His Ala His Leu Gly Leu Ser Pro Glu Lys
   1175 1180 1185
   Pro Arg Glu Ala Ser Val Leu Glu Ala Arg Leu Ala Leu Leu Asp
   1190 1195 1200
   Asp Gln Gly Ala Pro Val Leu Thr Ala Glu Arg Phe Ile Ile Arg
   1205 1210 1215
   Arg Val Glu Thr His Ala Ala Ala Trp Glu Gly Leu Pro Gly Glu
   1220 1225 1230
   Trp Phe Tyr Arg Leu Arg Trp Gln Pro Leu Glu Gly Trp Lys Pro
   1235 1240 1245
   Glu Ala Ala Gly Ala Lys Arg Asp Glu Ala Trp Leu Leu Phe Ala
   1250 1255 1260
   Asp Asp Gln Gly Val Ala Ala Ala Val Ala Asp Arg Leu Ala Pro
   1265 1270 1275
   Ser Gly Val Arg Thr Thr Leu Val Arg Lys Ala Pro Gly Ala Val
   1280 1285 1290
   Thr Arg Ala Gly Ser Gly Trp Thr Leu Asp Pro Gly Thr Pro Glu
   1295 1300 1305
   Ser Phe Arg Glu Leu Leu Ala Ala Val Ser Pro Gly Ser Phe Thr
   1310 1315 1320
   Arg Val Leu Tyr Phe Trp Gly Leu Asp Ala Pro Ala Val Asp Pro
   1325 1330 1335
   Glu Pro Asp Ala Leu Asp Ala Arg Leu Pro Glu Thr Glu Ala Leu
   1340 1345 1350
   Ile Tyr Leu Val Gln Ala Leu Ala Ala Arg Glu Ala Gln Pro Gly
   1355 1360 1365
   Pro Arg Leu Trp Leu Val Thr Ala Gly Ala Leu Pro Gly Ala Asp
   1370 1375 1380
   Gly Glu Ala Val Ser Pro Met Gln Thr Pro Leu Trp Ala Leu Gly
   1385 1390 1395
   Arg Val Leu Asp Tyr Glu His Pro Glu Leu Lys Thr Val Ser Cys
   1400 1405 1410
   Glu Leu Glu Arg His Val Thr Glu Lys His Val Ala Val Leu Thr
   1415 1420 1425
   Ser Leu Leu Leu Ala Pro Glu Val Pro Asp Gln Val Arg Val Arg
   1430 1435 1440
   Ala Asp Gly Pro Ala Ala Ala Arg Leu Ala Arg Tyr Ser Pro Pro
   1445 1450 1455
   Ala Pro Arg Asp Thr Pro His Arg Val Ser Pro His Glu Ala Val
   1460 1465 1470
   Val Val Val Ser Asp Asn Gly Pro Ala Pro Ala Glu Leu Ser Val
   1475 1480 1485
   Ala Arg Arg Arg Ala Pro Ala Ala His Glu Val Glu Val Leu Val
   1490 1495 1500
   Thr His Ala His Leu Ala Trp Ala Pro Ala Ser Pro Asp Ala Gly
   1505 1510 1515
   Gly Leu Val Pro Gly Gly Tyr Phe Ala Gly Arg Val Leu Arg Leu
   1520 1525 1530
   Gly Gly Glu Val Arg Gly Leu Ser Val Gly Gln Asp Ile Val Ala
   1535 1540 1545
   Pro Ala Gly Gly Glu Leu Arg Ser Ile Arg Thr Val Asp Ala Gly
   1550 1555 1560
   Gly Val Arg Pro Leu Pro Glu Gln Ala Asp Pro Ala Arg Ala Leu
   1565 1570 1575
   Cys Ala Leu Gly Thr Arg Ala Leu Ala Arg His Leu Leu Arg Gln
   1580 1585 1590
   Leu Ala Pro Val Glu Arg Gly Gln Thr Leu Leu Leu Leu Gly Gly
   1595 1600 1605
   Asp Leu Pro Leu Leu Gln Ser Leu Ala Arg Val Ala Thr Gly His
   1610 1615 1620
   Gly Ala Arg Val Leu Ala Leu Val Arg Asp Gly Ala His Ala Pro
   1625 1630 1635
   Ala Leu Gln Glu Ala Gly Ala Glu Val Val Gly Arg Leu Asp Glu
   1640 1645 1650
   Pro Gly Ala Phe Glu Arg Leu Arg Glu Arg Ala Gly Ala Ser Gly
   1655 1660 1665
   Val Asp Val Leu Ile Asn Leu Ala Pro Gly Ala Ala Val Pro Arg
   1670 1675 1680
   Ala Leu Asp Val Phe Arg Ala Gly Gly Val Phe Leu Asp Leu Thr
   1685 1690 1695
   Arg Ala Ser Pro Val Gly Pro Lys Glu Leu Pro Ala Ser Ala Thr
   1700 1705 1710
   Phe Leu Ser Leu Gly Leu Ala Pro Leu Tyr Ala Arg Leu Ala Pro
   1715 1720 1725
   Pro Thr Pro Glu Ala Ile Ala Glu Ala Ser Ala Pro Ala Glu Ala
   1730 1735 1740
   Pro Ala Arg Val Trp Ser Leu Gln Glu Leu Thr Gln Pro Arg Ala
   1745 1750 1755
   Glu Ala Pro Leu Asp Gly Gln Ala His Val Leu Gly Val Ser Ser
   1760 1765 1770
   Glu Pro Val Ala Val Gly Ala Gly Ser Ala Pro Leu Ala Leu Ser
   1775 1780 1785
   Asp Thr Ala Thr Tyr Leu Ile Thr Gly Gly Leu Gly Gly Leu Gly
   1790 1795 1800
   Leu Arg Val Ala Ser Leu Leu Val Asp His Gly Val Arg His Leu
   1805 1810 1815
   Ala Leu Val Gly Arg Ser Pro Pro Ser Ala Gln Ala Arg Ser Thr
   1820 1825 1830
   Leu Arg Glu Leu Glu Gln Arg Gly Val Arg Val Gln Val Phe Ala
   1835 1840 1845
   Ala Asp Val Ala Arg Arg Glu Asp Leu Leu Gly Val Val Lys Ala
   1850 1855 1860
   Leu Gln Ala Gly Asp Ala Pro Leu Arg Gly Val Ile His Cys Ala
   1865 1870 1875
   Gly Val Leu Gln Asp Gly Ala Leu Leu Gly Met Asp Arg Glu Arg
   1880 1885 1890
   Phe Arg Ser Val Leu Gly Ser Lys Leu Ser Gly Ala Trp Asn Leu
   1895 1900 1905
   His Ala Leu Thr Arg Asp Leu Pro Leu Asp His Phe Val Leu Phe
   1910 1915 1920
   Ser Ser Val Ser Ser Met Leu Gly Ser Pro Gly Gln Gly Asn Tyr
   1925 1930 1935
   Ala Ala Ala Asn Ala Phe Met Asp Gly Leu Ala Ala Trp Arg Arg
   1940 1945 1950
   Ala Arg Gly Leu Pro Ala Leu Ser Val Asn Trp Gly Ala Phe Ser
   1955 1960 1965
   Asp Ile Gly Leu Ala Ala Val Gly Glu Arg Arg Gly Asp Arg Leu
   1970 1975 1980
   Ala Asp Asn Gly Ile Pro Gly Ile Pro Pro Glu Lys Gly Met Lys
   1985 1990 1995
   Ala Leu Glu Leu Ala Leu Arg Trp Gly Glu Thr Ser Leu Gly Phe
   2000 2005 2010
   Phe Pro Phe Pro Phe Leu Ile Trp Arg Lys Leu Asn Pro Gln Ile
   2015 2020 2025
   Ala Arg Asn Thr Leu Phe Thr Asp Ile Arg Ala Glu Ser Arg Tyr
   2030 2035 2040
   Arg Glu Glu Ala Ser Gly Glu Gln Pro Ile Ala Glu Ala Leu Ala
   2045 2050 2055
   Leu Ala Asp Pro Ala Ala Arg Arg Ser His Leu Asp Gly Phe Leu
   2060 2065 2070
   Lys Gln Gln Leu Ser Arg Val Leu Lys Val Pro Ala Glu Gln Val
   2075 2080 2085
   Arg Asp Gly Leu Thr Phe Lys Glu Met Gly Phe Asp Ser Leu Met
   2090 2095 2100
   Ala Ile Glu Leu Arg Asn Arg Leu Glu Ala Gly Leu Thr Val Ser
   2105 2110 2115
   Leu Ser Ser Thr Leu Val Trp Arg Tyr Pro Thr Leu Glu Glu Met
   2120 2125 2130
   Ser Arg Phe Leu Leu Gln Leu Val Gly Gly Gly Ser Glu Thr Pro
   2135 2140 2145
   Ser Asp Ala Arg Pro Ala Ser Ala Ala Pro Ala Arg Arg Gly Arg
   2150 2155 2160
   Trp Leu Val Arg Glu Lys Pro Ser Pro Ser Ala Lys Leu Arg Leu
   2165 2170 2175
   Leu Cys Phe Pro His Gly Gly Ala Gly Ala Ser Ala Phe Gln Gly
   2180 2185 2190
   Trp Trp Gln Gln Phe Pro Asp Thr Val Glu Val Leu Thr Phe Gln
   2195 2200 2205
   Pro Pro Gly Arg Glu Glu Arg Leu Gln Glu Glu Pro Glu Arg Asp
   2210 2215 2220
   Met Gly Ala Tyr Ala Ser Ala Ile Val Glu Glu Leu Gln Pro Leu
   2225 2230 2235
   Leu Asp Leu Pro Phe Ala Val Phe Gly Tyr Ser Leu Gly Gly Leu
   2240 2245 2250
   Thr Gly Phe Ala Thr Val Ala Glu Leu Arg Arg Arg Asn Arg Val
   2255 2260 2265
   Thr Pro Arg His Leu Phe Val Ser Ser Ala Phe Ala Pro His Ile
   2270 2275 2280
   Pro Phe Ser Glu Thr Thr Leu Ala Arg Val Ser Thr Gln Thr Ser
   2285 2290 2295
   Ala Leu Lys Ala Met Ala Phe Tyr Gln Ser Thr Pro Asp Ser Val
   2300 2305 2310
   Phe Lys Asp Pro Glu Met Gln Ala Leu Leu Ala Arg Ser Met Asp
   2315 2320 2325
   Ala Asp Asn Ser Val Val Glu Ser Tyr Arg Phe Gly Ala Glu Lys
   2330 2335 2340
   Pro Leu Asp Cys Pro Ile Thr Ala Phe Ala Gly Arg Gln Asp Asp
   2345 2350 2355
   Leu Ala Pro Leu His Glu Gln Leu Arg Trp Lys Glu Leu Thr Thr
   2360 2365 2370
   Gln Ala Phe Glu Leu Arg Pro Phe Glu Gly Asp His Phe Phe Phe
   2375 2380 2385
   Leu Arg Asp Lys Arg Pro Val Met Asp Glu Leu Arg Arg His Leu
   2390 2395 2400
   Leu Ser Leu Ile Ala Asp Ser Arg Asp Gly Ser Ser Arg
   2405 2410 2415
<210> 41
   <211> 406
   <212> PRT
   <213> Pyxidicoccus fallax
<400> 41
   Met Thr Tyr Thr Gln Leu Leu His Arg Arg Val Gly Gly Gln Leu Arg
   1 5 10 15
   Arg Phe Leu Leu Leu Arg Pro Phe Gly Pro Gly Val Glu Val Asp Asp
   20 25 30
   Pro Leu His Pro Pro Glu Leu Pro Pro Tyr Asp Ala Glu Arg Asp Leu
   35 40 45
   Ala Pro Arg Thr Pro Gln Phe Leu Gln His Ala Leu Gln Pro Glu Glu
   50 55 60
   Arg Gly Arg Pro Gly Ala Pro Gly Leu Pro Val Thr Thr Ser Glu Leu
   65 70 75 80
   Ser Val Gly Cys Asn Leu Ser Gly Ala Cys Cys Ser Gln Tyr Pro Val
   85 90 95
   Ile Pro Thr Thr Val Glu Glu Ser Arg Arg Ala Val Glu Thr Leu Arg
   100 105 110
   Arg Leu Ala Pro Gly Ala Ala Pro Gly Arg Ala Glu Asp Ala Phe Ala
   115 120 125
   Pro Val Asn Pro Gly Arg Arg Asp Val Leu Ser Pro Ile Leu Gln Gly
   130 135 140
   Asp Gly Cys Pro Phe Leu Ala Gln Gly Ala Cys Gln Val His Lys Ala
   145 150 155 160
   Gly Gly Ala Glu Ala Lys Pro Trp Thr Cys Arg Gln Phe Pro Leu Gln
   165 170 175
   Val Ile His Cys Gly Asp His Phe Glu Val Ser Ile Leu Pro Gln Cys
   180 185 190
   Ala Cys Ala Ala Arg Thr Val Thr Arg Gly Ser Gly Gly Asp Trp Pro
   195 200 205
   Glu Asp Pro Leu Arg Gly Leu Thr Ser Val Pro Arg Val Pro Asp Ser
   210 215 220
   Val Val Val Glu Gly Arg Arg Thr Leu Ala Arg Gly Met Tyr Leu Gln
   225 230 235 240
   Trp Val Arg Ala Leu Ala Asp Arg Leu Ser Ala Pro Gly Leu Gly Glu
   245 250 255
   Pro Ala Leu Val Leu Asp Ser Ala Ser Gln Ser Leu Arg Val Ala Pro
   260 265 270
   Gly Ala Leu Thr Pro Gly Trp Leu Gly Glu Leu Arg Ala Arg Met Leu
   275 280 285
   Ser Glu Ala Glu Arg Leu Glu Gly Tyr Leu Asp Pro Ala Gly Pro Gln
   290 295 300
   Pro Lys Gly Thr Arg Trp Val Ala Ser Leu Ala Glu Ala Leu Leu Ala
   305 310 315 320
   Asp Ala Asp Ala Glu Pro Glu Pro Ala Pro Ser Asp Gly Leu Val Leu
   325 330 335
   Ala Leu Ala Leu Arg Ala His Leu Leu Leu Glu Leu Pro Ala Leu Gly
   340 345 350
   Ala Ala Leu Ala Asp Leu Thr Arg Leu Ala Arg Leu Ala Ser Leu Ser
   355 360 365
   Arg Thr Val Ala Pro Ala Glu Ala Ala Asp Ala Arg Phe Glu Pro Leu
   370 375 380
   Thr Leu Leu Leu Tyr Leu Trp Ala Thr Thr Arg Trp Pro Val Ser Ser
   385 390 395 400
   Glu Gly Val Thr Arg Pro
   405
<210> 42
   <211> 398
   <212> PRT
   <213> Pyxidicoccus fallax
<400> 42
   Met Thr Gly Thr Ser Gly Gln Gly Ala Gly Gly Gly Arg Ile Asp Thr
   1 5 10 15
   Pro Pro Glu Gly Ala Leu Arg Ala Val Arg Ser Leu Val Glu Leu Gln
   20 25 30
   Glu Thr Glu Gln Ala Ala Gln Leu Tyr Glu Glu Leu Gly Asp Ala Gln
   35 40 45
   Arg Glu Arg Leu Arg Arg Glu Ala Ala Arg Arg Pro Ala Arg Glu Arg
   50 55 60
   Arg Gly Leu Val Glu Val Leu Arg Arg Ala Arg Asp Phe Leu Gly Ala
   65 70 75 80
   Ala Arg Leu Met Glu Gly Asp Gly Glu Asp Ala Thr Val Ala Asp Leu
   85 90 95
   Tyr Phe Gln Ser Gly Gln Tyr Leu Glu Ala Ala Glu Ala Tyr Leu Arg
   100 105 110
   Ala Gly Glu Ala Glu Arg Ala Ala Ala Ala Phe Glu Arg Ala Gly Ala
   115 120 125
   Leu Glu Arg Ala Leu Asp Val Tyr Arg Arg Leu Gly Ala Arg Glu Ala
   130 135 140
   Met Ala Gln Cys Leu Val Arg Leu Gly Arg Pro Phe Glu Ala Ala Asp
   145 150 155 160
   Leu Tyr Arg Glu Leu Gly Gln Ala His Ala Glu Val Glu Ala Leu Gly
   165 170 175
   Gly Val Lys Pro Glu Asp Ser Arg Tyr Val Glu Ala Val Leu Arg Val
   180 185 190
   Cys Thr Leu Leu Asp Val Glu Gly Phe Thr His Arg Ala Leu Ala Val
   195 200 205
   Leu Ala Asp Ala Val Ser Ser Ser Glu Glu Ala Arg Ala Asp Glu Ala
   210 215 220
   Leu Val Lys Glu Lys Ala Arg Leu Leu Arg Arg Met Gly Met Glu Ala
   225 230 235 240
   Glu Ala Glu Ala Val Leu Leu Arg Arg Ala Thr Ala Ala Ala Thr Pro
   245 250 255
   Ala Ala Asn Gly Tyr Arg Phe Leu Lys Ala Ile Pro Ile Phe Gly Glu
   260 265 270
   Leu Ser Leu Glu Asp Met Lys Asp Leu Tyr Arg Leu Ala Arg Gln Val
   275 280 285
   Val Ile Ala Pro Gly Thr Val Leu Leu Glu Lys Gly Ala Arg Gly Thr
   290 295 300
   Gly Leu Phe Val Leu Leu Asp Gly Thr Val Glu Val Phe Ser Gly Pro
   305 310 315 320
   Glu Gln Asp Ala Arg His Leu Asn Thr Leu Gly Pro Gly Ala Tyr Leu
   325 330 335
   Gly Glu Ile Ser Leu Val Gln Asp Ala Pro Val Ser Ala His Val Arg
   340 345 350
   Ala Arg Thr Ser Val Arg Ala Leu Arg Ile Ser Arg Ala Asp Phe Glu
   355 360 365
   His Tyr Leu Asp Thr His Glu Ala Ala Ala Leu Arg Ile Tyr Arg Leu
   370 375 380
   Phe Thr Gln Asn Leu Ala Ala Arg Val Arg Ala Leu Ser Thr
   385 390 395
<210> 43
   <211> 210
   <212> PRT
   <213> Pyxidicoccus fallax
<400> 43
   Met Ala Ser Ala Arg Arg Pro Glu Lys Asp Leu Gln Ala Leu Ala Arg
   1 5 10 15
   Lys Ser Lys Lys Leu Ser Trp Leu Leu Arg His Gly Ala Arg Glu Met
   20 25 30
   Gly Leu Ala Met Asp Ser Ala Gly Phe Ala His Ile Gly Glu Val Leu
   35 40 45
   Arg Met Thr Gly Leu Ser Arg Asp Glu Leu Asp Glu Val Val Ala Glu
   50 55 60
   Asn Asn Lys Ser Arg Tyr Glu Val Arg Gly Lys Gln Val Arg Ala Val
   65 70 75 80
   Gln Gly His Ser Leu Asp Gly Thr Pro Val Thr Leu Asp Gly Leu Glu
   85 90 95
   Arg Ser Trp Glu Glu Val Leu Ala Asp Ala Pro Leu Tyr His Gly Thr
   100 105 110
   Ser Val Val Ala Ala Arg Ala Ile Leu Ser Gly Glu Gly Ile His Ser
   115 120 125
   Ala Ala Arg Thr His Val His Leu Ala Ala Ala Val Asp Ala Lys Val
   130 135 140
   Gly Lys Arg Ala Gly Val Glu Val Leu Leu Val Ile Ser Ala Ala Arg
   145 150 155 160
   Leu Arg Ala Ser Gly Leu Arg Val Phe Arg Ala Pro Asn Gly Val Leu
   165 170 175
   Leu Ala Arg Ala Ile Pro Ala Ala Ser Ile Val Asp Val Leu Ala Cys
   180 185 190
   Asn Gly Pro Gly Asn Ala Ala Leu Ala Glu Leu Gln Arg Phe Phe Arg
   195 200 205
   Glu Ala
   210
<210> 44
   <211> 284
   <212> PRT
   <213> Pyxidicoccus fallax
<400> 44
   Met Glu Thr Asp Gly Leu Arg Ala Leu Val Ala Phe Ala Glu Ala Gly
   1 5 10 15
   Cys Asn Leu Thr Leu Ala Gly Arg Ala Ile Gly Leu Ser Gln Pro Ala
   20 25 30
   Val His Ala Arg Leu Gln Gly Val Ala Arg Ala Leu Glu Thr Ser Leu
   35 40 45
   Tyr Glu Arg Arg Gly Arg Arg Leu Gln Leu Thr Pro Glu Gly Ala Arg
   50 55 60
   Val Leu Ala Trp Ala Arg Asp Val Leu Glu Arg Glu Arg Ala Leu Arg
   65 70 75 80
   Val Glu Leu Arg Gln Gly His Ala Glu Glu Arg Val Val Leu Ala Cys
   85 90 95
   Gly Glu Gly Ala Leu Val His Val Val Ala Glu Arg Val Ala Pro Leu
   100 105 110
   Leu Arg Glu Arg Thr Gly Val Leu Ser Phe Leu Val Leu Asp Gly Pro
   115 120 125
   Ala Ala Val Ala Ala Val Glu Arg Gly Ser Ala His Leu Ala Val Val
   130 135 140
   Ala Gly Pro Ala Ser Ser Pro Pro Gly Leu Arg Ser Gln Pro Leu Val
   145 150 155 160
   Thr Ala Ser Leu Leu Ala Val Ala Pro Arg Gln His Pro Leu Ala Arg
   165 170 175
   Ala Gly Arg Glu Val Glu Val Gly Ala Leu Leu Glu His Arg Leu Leu
   180 185 190
   Val Pro Pro Leu Gly Arg Ala Leu Arg Ala Thr Leu Glu Asp Ala Ala
   195 200 205
   Ala Ala His Gly Arg Thr Leu Glu Val Ala Ala Glu Val Thr Gly Trp
   210 215 220
   Glu Ala Val Cys Arg Leu Ala Ala Leu Gly Val Gly Val Gly Val Ile
   225 230 235 240
   Asn Asp Val Val Ala Thr Pro Gly Leu Ala Arg Leu Ser Val Arg Gly
   245 250 255
   Leu Pro Pro Val Thr Tyr Arg Leu Leu Met Arg Arg Gly Arg Gly Thr
   260 265 270
   Arg Leu Ala Glu Glu Val Val Arg Ala Leu Leu Gly
   275 280
<210> 45
   <211> 139
   <212> PRT
   <213> Pyxidicoccus fallax
<400> 45
   Met Ala Arg Pro Pro Arg Lys Gly Pro Pro Glu Pro Lys Pro Ala Gly
   1               5                   10                  15
   Thr Pro Pro Gly Lys Ser Ser Ser Gly Gly Phe Asn Ser Pro Phe Ala
               20                  25                  30
   Ala Leu Ala Gly Leu Arg Glu Ser Leu Pro Gly Arg Thr Pro Gln Leu
           35                  40                  45
   Arg Ala Glu Asp Phe Pro Ala Glu Pro Pro Pro Asp Gly Pro Glu Arg
       50                  55                  60
   Ala Val Val Arg Leu Thr Arg Arg Glu Arg Asp Gly Ala Glu Val Thr
   65                  70                  75                  80
   Arg Val Glu Glu Leu Gly Leu Ala Pro Asp Ala Leu Thr Ala Trp Ser
                   85                  90                  95
   Gln Ala Leu Gln Arg Gly Leu Ala Cys Arg Val Thr Val Glu Gly Glu
               100                 105                 110
   Ala Leu Val Leu Glu Gly Asp Gln Arg Arg Ser Val Pro Asp Leu Leu
           115                 120                 125
   Leu Arg Arg Gly Val Arg Arg Val Thr Gln Gly
       130                 135
<210> 46
   <211> 237
   <212> PRT
   <213> Pyxidicoccus fallax
<400> 46
   Met Ala Arg Ala Arg Ser Gly Ala Lys Ala Ser Ala Thr Pro Pro Glu
   1               5                   10                  15
   Pro Asp Ala Arg Ala Arg Leu Ile Ala Ala Gly Leu Arg Val Leu Ala
               20                  25                  30
   Glu Arg Gly Tyr Asp Ala Thr Thr Val Lys Glu Val Ala Arg Glu Ala
           35                  40                  45
   Gly Val Asn Gln Gly Leu Val His Tyr Tyr Phe Gly Ser Lys Asp Ala
       50                  55                  60
   Leu Leu Leu Ala Val Ala Gln Glu Ala Arg Gln Gln Tyr Met Ala Glu
   65                  70                  75                  80
   Leu Arg Lys Leu Arg Glu Glu Thr Pro Arg Glu Arg Leu Ala Ala Ala
                   85                  90                  95
   Ser Phe Ala Trp Gly Glu Gln Leu Leu Arg Glu Thr Pro Glu Gln Phe
               100                 105                 110
   Arg Val Arg Tyr Glu Leu Phe Ala Ile Gly Leu His Asn Gln Glu Leu
           115                 120                 125
   Lys Pro Ala Val Ala Glu Ser Leu Arg Cys Val Asp Glu Glu Val Ala
       130                 135                 140
   Leu Thr Val Ala Arg Ile Arg His Gly Asp Asp Ala Pro Pro Glu Pro
   145                 150                 155                 160
   Leu Asp Gln His Tyr Ala Ala Ile Met Lys Ala Cys Ile Asp Gly Leu
                   165                 170                 175
   Ser Leu His His Leu Leu Asp Asp Ser Phe Asp Pro Ala Pro Val Tyr
               180                 185                 190
   Gln Leu Leu Ser Arg Ile Val Leu Ala Ser Leu Glu Pro Pro Glu Gly
           195                 200                 205
   Lys Lys Pro Pro Gly Arg Lys Gly Lys Ala Gln Ala Arg Arg Gly Arg
       210                 215                 220
   Val Pro Val Pro Arg Gly Gly Arg Gly Ala Arg Arg Arg
   225                 230                 235